# EUROPEAN PATENT SPECIFICATION

(11) **EP 3 583 126 B1**
(45) Date of publication and mention of the grant of the patent: **27.08.2025**
(21) Application number: 18711427.7
(22) Date of filing: 16.02.2018
(51) Int. Cl.: C07K 16/18, C07K 16/28, A61K 39/395, A61P 21/00

(54) **MULTISPECIFIC BINDING MOLECULES HAVING SPECIFICITY TO DYSTROGLYCAN AND LAMININ-2**
MULTISPEZIFISCHE BINDUNGSMOLEKÜLE MIT SPEZIFITÄT FÜR DYSTROGLYCAN UND LAMININ-2
MOLÉCULES DE LIAISON MULTISPÉCIFIQUES AYANT UNE SPÉCIFICITÉ VIS-À-VIS DU DYSTROGLYCANE ET DE LA LAMININE-2

(30) Priority: 17.02.2017 US 201762460663 P
(43) Date of publication of application: 25.12.2019
(60) Divisional application: 25191539.3
(73) Proprietor: SANOFI, 75017 Paris (FR)
(72) Inventor: SEVIGNY, Leila, Bridgewater, NJ 08807 (US); BEIL, Christian, 65926 Frankfurt am Main (DE); BRONDYK, William, H., Bridgewater, NJ 08807 (US); CHEN, Yangde, Wellesley, MA 02482 (US); CHENG, Seng, H., Bridgewater, NJ 08807 (US); CONNORS, Timothy, D., Bridgewater, NJ 08807 (US); DEVAUD, Catherine, 91380 Chilly-Mazarin (FR); HOFFMANN, Dietmar, 75008 Paris (FR); LANGE, Christian, 65926 Frankfurt am Main (DE); MAGNAY, Maureen, Bridgewater, NJ 08807 (US); MAGNAY, Tristan, Bridgewater, NJ 08807 (US); PRADES, Catherine, 91380 Chilly-Mazarin (FR); RAO, Ercole, 65926 Frankfurt am Main (DE); WEI, Ronnie, Needham, MA 02494 (US); ZHAO, Hongmei, Bridgewater, NJ 08807 (US); ZHU, Yunxiang, Bridgewater, NJ 08807 (US)
(74) Representative: Lavoix
(86) International application number: PCT/US2018/000056
(87) International publication number: WO 2018/151841

(56) References cited:
- US-A1- 2003 224 981
- C. QIAO ET AL: "Amelioration of laminin- 2-deficient congenital muscular dystrophy by somatic gene transfer of miniagrin", PROCEEDINGS OF THE NATIONAL ACADEMY OF SCIENCES, vol. 102, no. 34, 23 August 2005 (2005-08-23), US, pages 11999 - 12004, XP055471666, ISSN: 0027-8424, DOI: 10.1073/pnas.0502137102
- CHARLES H. VANNOY ET AL: "Adeno-Associated Virus-Mediated Mini-Agrin Delivery Is Unable to Rescue Disease Phenotype in a Mouse Model of Limb Girdle Muscular Dystrophy Type 2I", AMERICAN JOURNAL OF PATHOLOGY., vol. 187, no. 2, 1 February 2017 (2017-02-01), US, pages 431 - 440, XP055471661, ISSN: 0002-9440, DOI: 10.1016/j.ajpath.2016.09.024
- SENG H. CHENG ET AL: "AB008. Bifunctional antibody as a surrogate molecular linker for the treatment of alpha-dystroglycan related muscular dystrophies", ANNALS OF TRANSLATIONAL MEDICINE, vol. 5, no. S2, 1 September 2017 (2017-09-01), pages AB008 - AB008, XP055471506, ISSN: 2305-5839, DOI: 10.21037/atm.2017.s008
- EMMA L. HUMPHREY ET AL: "A new monoclonal antibody DAG-6F4 against human alpha-dystroglycan reveals reduced core protein in some, but not all, dystroglycanopathy patients", NEUROMUSCULAR DISORDERS., vol. 25, no. 1, 1 January 2015 (2015-01-01), GB, pages 32 - 42, XP055471543, ISSN: 0960-8966, DOI: 10.1016/j.nmd.2014.09.005
- MARISA J. FORTUNATO ET AL: "Development of Rabbit Monoclonal Antibodies for Detection of Alpha-Dystroglycan in Normal and Dystrophic Tissue", PLOS ONE, vol. 9, no. 5, 13 May 2014 (2014-05-13), pages e97567, XP055471577, DOI: 10.1371/journal.pone.0097567

## Description

### CROSS-REFERENCE TO RELATED APPLICATIONS

This application claims the priority benefit of U.S. Provisional Application Serial No. 62/460,663, filed February 17, 2017, and EP Application No. EP18305168.9, filed February 16, 2018.

### SUBMISSION OF SEQUENCE LISTING ON ASCII TEXT FILE

The content of the following submission on ASCII text file is a computer readable form (CRF) of the Sequence Listing (file name: 183952028140SEQLIST.txt, date recorded: February 16, 2018, size: 315 KB).

### FIELD

The disclosure relates to multispecific (e.g., multispecific and trivalent, or bispecific and bivalent or tetravalent) binding molecules comprising a first binding domain that binds an extracellular portion of dystroglycan and a second binding domain that binds laminin-2. The disclosure also relates to methods for making such binding molecules and uses of such binding molecules for treating and/or preventing alpha-dystroglycanopathies.

### BACKGROUND

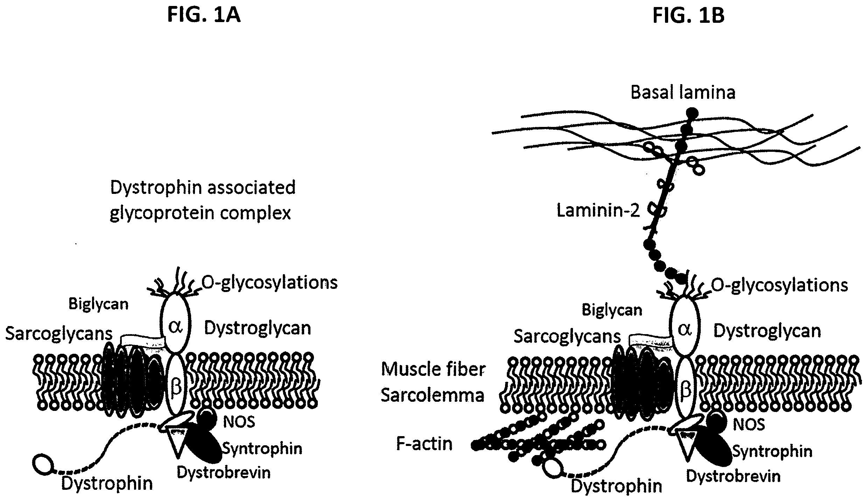

Alpha-dystroglycanopathy is a subgroup of congenital muscular dystrophy (CMD) characterized by reduced or absence of O-glycosylation in the mucin-like domain in alpha-dystroglycan (alpha-DG) (Muntoni, F. (2004) Acta. Myol. 23(2), 79-84; Toda, T. (2005) Rinsho Shinkeigaku 45(11), 932-934; Muntoni, F., et al. (2007) Acta. Myol. 26(3), 129-135; Hewitt, J. E. (2009). Biochim. Biophys. Acta. 1792(9), 853-861; Godfrey, C., et al. (2011) Curr. Opin. Genet. Dev. 21(3), 278-285). The lack or hypoglycosylation on alpha-dystroglycan leads to the loss or decreased binding of its ligands, which include laminin-2, agrin and perlecan in skeletal muscle, neurexin in the brain, and pikachurin in the eye. Alpha-dystroglycan is a peripheral membrane component of the dystrophin-glycoprotein complex (DGC) (**FIG. 1A**) common to all muscles and the heart (Matsumura, K., et al. (1993) Neuromuscul. Disord. 3(5-6), 533-535). In these tissues, the DGC complex functions to link the filamentous actin (F-actin) - associated cytoskeleton of the muscle fiber via dystrophin to the extracellular matrix (ECM, also called basal lamina) via laminin-2 (**FIG. 1B**).

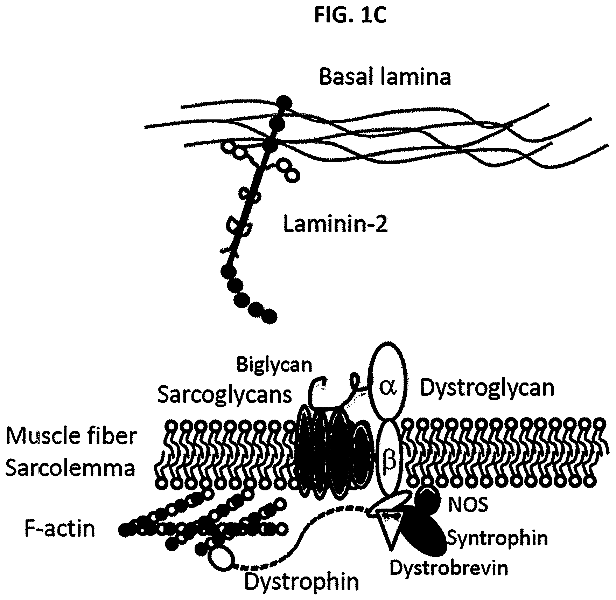

In alpha-dystroglycanopathies, mutations in at least 18 genes identified to date are linked to aberrant processing of O-glycosylation on alpha-DG and lack of binding to its ligands, leading to diseases. *See, e.g.,* Hara, Y., et al. (2011) N. Engl. J. Med. 364(10), 939-946; Kim, D. S., et al. (2004) Neurology 62(6), 1009-1011; van Reeuwijk, J., et al. (2005) J. Med. Genet. 42(12), 907-912; Murakami, T., et al. (2009) Brain Dev. 31(6), 465-468; Yanagisawa, A., et al. (2009) Eur. J. Med. Genet. 52(4), 201-206; Clement, E. M., et al. (2008) Arch. Neurol. 65(1), 137-141; Endo, T., et al. (2010) Methods Enzymol. 479, 343-352; Saredi, S., et al. (2012) J. Neurol. Sci. 318(1-2), 45-50; Longman, C., et al. (2003) Hum. Mol. Genet. 12(21), 2853-2861; Lefeber, D. J., et al. (2009) Am. J. Hum. Genet. 85(1), 76-86; Barone, R., et al. (2012) Ann. Neurol. 72(4), 550-558; Toda, T., et al. (2003) Congenit. Anom. (Kyoto) 43(2), 97-104; Toda, T. (2007). Rinsho Shinkeigaku 47(11), 743-748; Puckett, R. L., et al. (2009) Neuromuscul. Disord. 19(5), 352-356; Toda, T. (2009). Rinsho Shinkeigaku 49(11), 859-862; Yamamoto, T., et al. (2010) Cent. Nerv. Syst. Agents. Med. Chem. 10(2), 169-179; Kanagawa, M., et al. (2016) Cell. Rep. 14(9), 2209-2223; Yoshida-Moriguchi, T., et al. (2013) Science 341(6148), 896-899). These genes include, for instance, many glycosyltransferases, such as LARGE, which encodes a xylosyl- and glucuronyl- dual transferase responsible for adding xylose-glucuronic acid repeats to glycans to facilitate ligand binding (Inamori, K., et al. (2012) Science 335(6064), 93-96; Longman, C., et al. (2003) Hum. Mol. Genet. 12(21), 2853-2861). The main biological function of glycosyltransferases in this pathway (e.g. LARGE) are to properly assemble the O-glycosylation in the mucin-like domain in alpha-DG, which is necessary for tight binding to laminin-2 in the basal lamina of muscles, agrin and perlecan in neuromuscular junction, neurexin in the CNS, and pikachurin in the eye (Michele, D. E., et al. (2002) Nature 418(6896), 417-422; Muntoni, F., et al. (2002) Lancet 360(9343), 1419-1421). In the absence of proper O-glycosylation due to a defect in any of the aforementioned genes, binding of alpha-DG to laminin-2 in the extracellular matrix (ECM) is compromised or lost (**FIG. 1C**), causing a breakage of the mechanical link that is necessary for sarcolemma integrity. This renders the muscles prone to contraction-induced injury, resulting in damage to the sarcolemma of the muscle fiber and consequent muscular dystrophy (Barresi, R. and Campbell. K. P. (2006) J. Cell. Sci. 119, 199-207).

Due to the genetic heterogeneity, alpha-dystroglycanopathies include many subtypes of diseases which exhibit diverse yet overlapping clinical manifestations from very severe muscular dystrophy with central nervous system (CNS) and eye abnormalities to relatively mild muscular dystrophic phenotype without CNS manifestation or eye problem. There is no strict genetic and phenotypic correlation between different subtypes of alpha-dystroglycanopathies. Mutations in one gene can cause different subtypes of diseases with overlapping clinical manifestations, and mutations in different genes may lead to the same or similar disease (Godfrey, C., et al. (2007) Brain 130, 2725-2735). Because of this heterogeneity, strategies to treat individual alpha-dystroglycanopathies caused by mutations in individual genes have not been attractive for drug development due to the low cost effectiveness.

Alpha-dystroglycan and beta-dystroglycan are encoded by the same gene DAG1 and translated from a single mRNA as an intact type-1 transmembrane protein, dystroglycan. En route to the cell surface, dystroglycan is proteolytically cleaved to generate the transmembrane stud beta-dystroglycan and the noncovalently associated alpha-dystroglycan (Holt, K. H., et al. (2000) FEBS Lett. 468(1), 79-83). Theoretically, recombinant alpha-dystroglycan with proper O-glycosylation has been proposed as a protein replacement therapy for alpha-dystroglycanopathies. However, systemic delivery of recombinant alpha-dystroglycan indicated that this protein failed to reach the muscle interstitial space to be incorporated onto to the sarcolemma (Han, R., et al. (2009) PNAS 106(31), 12573-12579). Utilizing recombinant alpha-dystroglycan as protein replacement therapy for alpha-dystroglycanopathies is therefore thought to be technically impractical. US 2003/224981 discloses that muscular dystrophy diseases are coaused by mutations affecting the binding for laminins, including laminin-2 to alpha-dystrogylcan. The inventors recognized that the muscular dystrophy diseases can be treated by overexpressing in mice an artificial protein substituting for the natural binding of laminin to dystroglycan. Qiao C et al, PNAS, 2005, 102(34), 11999-12004 relates to a somatic gene therapy using AAV vectors as gene delivery system fo the mini-agrin, which showed therapeutic effect in laminin-2 knock-out mice. Vannoy CH et al.m American J Pathol, 2017, 187(2), 431-440 uses the same AAV mini-agrin vectors in a different type of dystroglycanopathy characterized by insufficient O-linked mannosyl glycosylation of alpha-dystroglycan.

Therefore, a need exists for therapeutic molecules for preventing and/or treating alpha-dystroglycanopathies and their associated pathologies.

### BRIEF SUMMARY

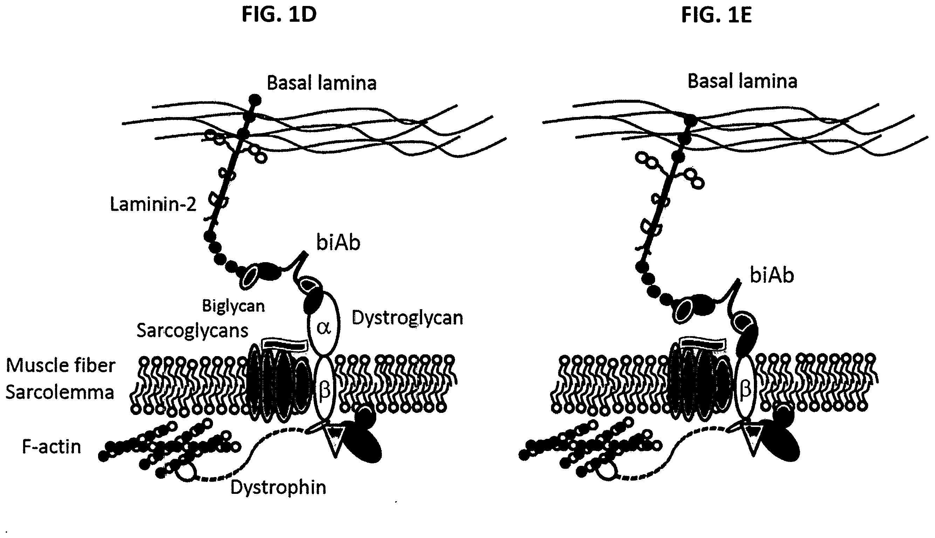

To meet this and other needs, provided herein, *inter alia,* are multispecific and bispecific binding molecules (*e.g.,* bispecific antibodies) and bifunctional biologics that can bind to laminin-2 and dystroglycan(s) simultaneously. When such a multispecific/bispecific antibody or bifunctional biologic is administered into patients with alpha-dystroglycanopathies, its concurrent binding to laminin-2 in the basal lamina and dystroglycan (alpha- or beta-) on the sarcolemma can restore the missing linkage (**FIGS. 1D** and **1E**). The present disclosure demonstrates that such an approach can ameliorate characteristic symptoms of alpha-dystroglycanopathies in an *in vivo* animal model system. In particular, antibodies are known to have prolonged circulation half-life (long pharmacokinetics) *in vivo* owing to their binding to neonatal Fc receptor, which mediates antibody recycling. Therefore, this multispecific/bispecific antibody strategy (or alternatively, bifunctional biologics strategy) represents a novel therapeutic approach for treating alpha-dystroglycanopathies.

The multispecific binding molecule comprises at least a first binding domain that binds an extracellular portion of dystroglycan and at least a second binding domain that binds laminin-2. The multispecific binding molecule is a multispecific binding protein comprising one or more polypeptide chains.

The multispecific binding molecule is a multispecific, trivalent binding protein comprising three antigen binding sites. The disclosure provides a multispecific binding molecule comprising at least a first binding domain that binds an extracellular portion of dystroglycan and at least a second binding domain that binds laminin-2, wherein the multispecific binding molecule is a multispecific binding protein comprising one or more polypeptide chains, wherein the binding protein comprises four polypeptide chains, wherein a first polypeptide chain comprises a structure represented by the formula:

V_{L2}-L₁-V_{L1}-L₂-C_{L} [I]

and a second polypeptide chain comprises a structure represented by the formula:

V_{H1}-L₃-V_{H2}-L₄-C_{H1}-hinge-C_{H2}-C_{H3} [II]

and a third polypeptide chain comprises a structure represented by the formula:

V_{H3}-C_{H1}-hinge-C_{H2}-C_{H3} [III]

and a fourth polypeptide chain comprises a structure represented by the formula:

V_{L3}-C_{L} [IV]

wherein:
V_{L1} is a first immunoglobulin light chain variable domain;
V_{L2} is a second immunoglobulin light chain variable domain;
V_{L3} is a third immunoglobulin light chain variable domain;
V_{H1} is a first immunoglobulin heavy chain variable domain;
V_{H2} is a second immunoglobulin heavy chain variable domain;
V_{H3} is a third immunoglobulin heavy chain variable domain;
C_{L} is an immunoglobulin light chain constant domain;
C_{H1} is an immunoglobulin C_{H1} heavy chain constant domain;
C_{H2} is an immunoglobulin C_{H2} heavy chain constant domain;
C_{H3} is an immunoglobulin C_{H3} heavy chain constant domain;
hinge is an immunoglobulin hinge region connecting the C_{H1} and C_{H2} domains; and
L₁, L₂, L₃ and L₄ are amino acid linkers;
wherein the polypeptide of formula I and the polypeptide of formula II form a cross-over light chain-heavy chain pair; and wherein V_{H1} and V_{L1} form an antigen binding site, wherein V_{H2} and V_{L2} form an antigen binding site, and wherein V_{H3} and V_{L3} form an antigen binding site for a total of three antigen binding sites, and wherein the three antigen binding sites comprise at least one antigen binding site that binds the extracellular portion of dystroglycan and at least one antigen binding site that binds laminin-2.

In some embodiments, the multispecific binding molecule comprises one antigen binding site that binds the extracellular portion of dystroglycan and two antigen binding sites that bind laminin-2. In some embodiments, the two antigen binding sites that bind laminin-2 bind different epitopes of laminin-2. In some embodiments, the two antigen binding sites that bind laminin-2 bind the same epitope of laminin-2. In some embodiments, V_{H1} and V_{L1} form a first antigen binding site that binds laminin-2, V_{H2} and V_{L2} form a second antigen binding site that binds laminin-2, and V_{H3} and V_{L3} form a third antigen binding site that binds the extracellular portion of dystroglycan.

In some embodiments, the multispecific binding molecule comprises two antigen binding sites that bind the extracellular portion of dystroglycan and one antigen binding site that binds laminin-2. In some embodiments, the two antigen binding sites that bind the extracellular portion of dystroglycan bind different epitopes of the extracellular portion of dystroglycan. In some embodiments, the two antigen binding sites that bind the extracellular portion of dystroglycan bind the same epitope of the extracellular portion of dystroglycan. In some embodiments, V_{H1} and V_{L1} form a first antigen binding site that binds the extracellular portion of dystroglycan, V_{H2} and V_{L2} form a second antigen binding site that binds the extracellular portion of dystroglycan, and V_{H3} and V_{L3} form a third antigen binding site that binds laminin-2.

In some embodiments, the at least one antigen binding site that binds the extracellular portion of dystroglycan binds the extracellular portion of dystroglycan with an equilibrium dissociation constant (K_{D}) lower than about 1µM when assayed as part of a multispecific binding protein. In some embodiments, the at least one antigen binding site that binds the extracellular portion of dystroglycan binds the extracellular portions of human and mouse dystroglycan. In some embodiments, the at least one antigen binding site that binds the extracellular portion of dystroglycan binds beta-dystroglycan. In some embodiments, the at least one antigen binding site that binds the extracellular portion of dystroglycan binds a polypeptide comprising the sequence SIVVEWTNN TLPLEPCPKE QIIGLSRRIA DENGKPRPAF SNALEPDFKA LSIAVTGSGS CRHLQFIPVA PPSPGSSAAP ATEVPDRDPE KSSEDD (SEQ ID NO:290). In some embodiments, the at least one antigen binding site that binds the extracellular portion of dystroglycan binds a polypeptide comprising the sequence SIVVEWT NNTLPLEPCP KEQIAGLSRR IAEDDGKPRP AFSNALEPDF KATSITVTGS GSCRHLQFIP VVPPRRVPSE APPTEVPDRD PEKSSEDDV (SEQ ID NO:291). In some embodiments, the at least one antigen binding site that binds the extracellular portion of dystroglycan binds alpha-dystroglycan. In some embodiments, the at least one antigen binding site that binds the extracellular portion of dystroglycan binds a polypeptide comprising the sequence

SIVVEWT NNTLPLEPCP KEQIAGLSRR IAEDDGKPRP AFSNALEPDF
KATSITVTGS GSCRHLQFIP VVPPRRVPSE APPTEVPDRD PEKSSEDDV (SEQ ID
NO:291).

In some embodiments, the at least one antigen binding site that binds laminin-2 binds human laminin-2. In some embodiments, the at least one antigen binding site that binds laminin-2 binds human laminin-2 with an equilibrium dissociation constant (K_{D}) lower than about 1µM when assayed as part of a multispecific binding protein. In some embodiments, the at least one antigen binding site that binds laminin-2 binds mouse and human laminin-2. In some embodiments, the at least one antigen binding site that binds laminin-2 binds a polypeptide comprising a laminin G-like (LG) domain 4 of laminin-2, a laminin G-like (LG) domain 5 of laminin-2, or both. In some embodiments, the at least one antigen binding site that binds laminin-2 binds a polypeptide comprising the laminin G-like (LG) domain 4 and laminin G-like (LG) domain 5 of laminin-2. In some embodiments, the at least one antigen binding site that binds laminin-2 binds a polypeptide comprising the sequence VQPQPV PTPAFPFPAP TMVHGPCVAE SEPALLTGSK QFGLSRNSHI AIAFDDTKVK NRLTIELEVR TEAESGLLFY MARINHADFA TVQLRNGFPY FSYDLGSGDT STMIPTKIND GQWHKIKIVR VKQEGILYVD DASSQTISPK KADILDVVGI LYVGGLPINY TTRRIGPVTY SLDGCVRNLH MEQAPVDLDQ PTSSFHVGTC FANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:300). In some embodiments, the at least one antigen binding site that binds laminin-2 binds a polypeptide comprising the sequence Q PEPVPTPAFP TPTPVLTHGP CAAESEPALL IGSKQFGLSR NSHIAIAFDD TKVKNRLTIE LEVRTEAESG LLFYMARINH ADFATVQLRN GLPYFSYDLG SGDTHTMIPT KINDGQWHKI KIMRSKQEGI LYVDGASNRT ISPKKADILD VVGMLYVGGL PINYTTRRIG PVTYSIDGCV RNLHMAEAPA DLEQPTSSFH VGTCFANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:301). In some embodiments, the at least one antigen binding site that binds laminin-2 binds a polypeptide comprising the laminin G-like (LG) domain 5 of laminin-2. In some embodiments, the at least one antigen binding site that binds laminin-2 binds a polypeptide comprising the sequence ANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:292). In some embodiments, the at least one antigen binding site that binds laminin-2 binds a polypeptide comprising the sequence ANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:293).

In some embodiments, the at least one antigen binding site that binds the extracellular portion of dystroglycan comprises: (a) a heavy chain variable domain (VH) comprising a CDR-H1 comprising an amino acid sequence selected from the group consisting of SEQ ID NOs:1-8, a CDR-H2 comprising an amino acid sequence selected from the group consisting of SEQ ID NOs:9-17, and a CDR-H3 comprising an amino acid sequence selected from the group consisting of SEQ ID NOs:18-27; and (b) a light chain variable domain (VL) comprising a CDR-L1 comprising an amino acid sequence selected from the group consisting of SEQ ID NOs:28-37, a CDR-L2 comprising an amino acid sequence selected from the group consisting of SEQ ID NOs:38-42, and a CDR-L3 comprising an amino acid sequence selected from the group consisting of SEQ ID NOs:43-50. In some embodiments, the VH domain of the at least one antigen binding site that binds the extracellular portion of dystroglycan comprises a sequence selected from the group consisting of SEQ ID NOs:170, 172, 174, 176, 178, 180, 182, 184, 186, and 188; and the VL domain of the at least one antigen binding site that binds the extracellular portion of dystroglycan comprises a sequence selected from the group consisting of SEQ ID NOs:171, 173, 175, 177, 179, 181, 183, 185, 187, and 189. In some embodiments, the at least one antigen binding site that binds the extracellular portion of dystroglycan comprises: (a) a heavy chain variable domain (VH) comprising a CDR-H1 comprising the sequence of SEQ ID NO:316, a CDR-H2 comprising the sequence of SEQ ID NO:318, and a CDR-H3 comprising the sequence of SEQ ID NO:320; and (b) a light chain variable domain (VL) comprising a CDR-L1 comprising the sequence of SEQ ID NO:332, a CDR-L2 comprising the sequence of SEQ ID NO:334, and a CDR-L3 comprising the sequence of SEQ ID NO:336. In some embodiments, the at least one antigen binding site that binds the extracellular portion of dystroglycan comprises a humanized VH domain and a humanized VL domain. In some embodiments, the VH domain of the at least one antigen binding site that binds the extracellular portion of dystroglycan comprises the sequence of SEQ ID NO:314; and the VL domain of the at least one antigen binding site that binds the extracellular portion of dystroglycan comprises the sequence of SEQ ID NO:330. In some embodiments, the VH domain of the at least one antigen binding site that binds the extracellular portion of dystroglycan comprises the sequence of SEQ ID NO:346; and the VL domain of the at least one antigen binding site that binds the extracellular portion of dystroglycan comprises the sequence of SEQ ID NO:362. In some embodiments, the at least one antigen binding site that binds the extracellular portion of dystroglycan comprises: (a) a heavy chain variable domain (VH) comprising a CDR-H1, a CDR-H2, and a CDR-H3 of AS30SS_Hu6 or AS30SS_Hu9 shown in Table A2, D2, or I4; and (b) a light chain variable domain (VL) comprising a CDR-L1, a CDR-L2, and a CDR-L3 of AS30SS_Hu6 or AS30SS_Hu9 shown in Table A2, D2, or I4. In some embodiments, the VH domain of the at least one antigen binding site that binds the extracellular portion of dystroglycan comprises the sequence of an AS30SS_Hu6 or AS30SS_Hu9 VH domain shown in Table D2 or I4; and the VL domain of the at least one antigen binding site that binds the extracellular portion of dystroglycan comprises the sequence of an AS30SS_Hu6 or AS30SS_Hu9 VL domain shown in Table D2 or I4.

In some embodiments, the at least one antigen binding site that binds laminin-2 comprises: (a) a heavy chain variable domain (VH) comprising a CDR-H1 comprising an amino acid sequence selected from the group consisting of SEQ ID NOs:51-55 and 81-95, a CDR-H2 comprising an amino acid sequence selected from the group consisting of SEQ ID NOs:56-60 and 96-110, and a CDR-H3 comprising an amino acid sequence selected from the group consisting of SEQ ID NOs:61-65 and 111-125; and (b) a light chain variable domain (VL) comprising a CDR-L1 comprising an amino acid sequence selected from the group consisting of SEQ ID NOs:66-70 and 126-140, a CDR-L2 comprising an amino acid sequence selected from the group consisting of SEQ ID NOs:38, 71-75, and 141-154, and a CDR-L3 comprising an amino acid sequence selected from the group consisting of SEQ ID NOs:76-80 and 155-169. In some embodiments, the VH domain of the at least one antigen binding site that binds laminin-2 comprises a sequence selected from the group consisting of SEQ ID NOs:190, 192, 194, 196, 198, 200, 202, 204, 206, 208, 210, 212, 214, 216, 218, 220, 222, 224, 226, and 228; and the VL domain of the at least one antigen binding site that binds laminin-2 comprises a sequence selected from the group consisting of SEQ ID NOs: 191, 193, 195, 197, 199, 201, 203, 205, 207, 209, 211, 213, 215, 217, 219, 221, 223, 225, 227, and 229. In some embodiments, the at least one antigen binding site that binds laminin-2 comprises: (a) a heavy chain variable domain (VH) comprising a CDR-H1 comprising the sequence of SEQ ID NO:380, a CDR-H2 comprising the sequence of SEQ ID NO:382, and a CDR-H3 comprising the sequence of SEQ ID NO:384; and (b) a light chain variable domain (VL) comprising a CDR-L1 comprising the sequence of SEQ ID NO:396, a CDR-L2 comprising the sequence of SEQ ID NO:398, and a CDR-L3 comprising the sequence of SEQ ID NO:400. In some embodiments, the at least one antigen binding site that binds laminin-2 comprises a humanized VH domain and a humanized VL domain. In some embodiments, the VH domain of the at least one antigen binding site that binds laminin-2 comprises the sequence of SEQ ID NO:378; and the VL domain of the at least one antigen binding site that binds laminin-2 comprises the sequence of SEQ ID NO:394. In some embodiments, the at least one antigen binding site that binds laminin-2 comprises: (a) a heavy chain variable domain (VH) comprising a CDR-H1 comprising the sequence of SEQ ID NO:380, a CDR-H2 comprising the sequence of SEQ ID NO:382, and a CDR-H3 comprising the sequence of SEQ ID NO:384; and (b) a light chain variable domain (VL) comprising a CDR-L1 comprising the sequence of SEQ ID NO:428, a CDR-L2 comprising the sequence of SEQ ID NO:398, and a CDR-L3 comprising the sequence of SEQ ID NO:400. In some embodiments, the at least one antigen binding site that binds laminin-2 comprises a humanized VH domain and a humanized VL domain. In some embodiments, the VH domain of the at least one antigen binding site that binds laminin-2 comprises the sequence of SEQ ID NO:410; and the VL domain of the at least one antigen binding site that binds laminin-2 comprises the sequence of SEQ ID NO:426. In some embodiments, the at least one antigen binding site that binds laminin-2 comprises: (a) a heavy chain variable domain (VH) comprising a CDR-H1 comprising the sequence of SEQ ID NO:444, a CDR-H2 comprising the sequence of SEQ ID NO:446, and a CDR-H3 comprising the sequence of SEQ ID NO:448; and (b) a light chain variable domain (VL) comprising a CDR-L1 comprising the sequence of SEQ ID NO:460, a CDR-L2 comprising the sequence of SEQ ID NO:462, and a CDR-L3 comprising the sequence of SEQ ID NO:464. In some embodiments, the at least one antigen binding site that binds laminin-2 comprises a humanized VH domain and a humanized VL domain. In some embodiments, the VH domain of the at least one antigen binding site that binds laminin-2 comprises the sequence of SEQ ID NO:442; and the VL domain of the at least one antigen binding site that binds laminin-2 comprises the sequence of SEQ ID NO:458. In some embodiments, the at least one antigen binding site that binds laminin-2 comprises: (a) a heavy chain variable domain (VH) comprising a CDR-H1 comprising the sequence of SEQ ID NO:444, a CDR-H2 comprising the sequence of SEQ ID NO:478, and a CDR-H3 comprising the sequence of SEQ ID NO:448; and (b) a light chain variable domain (VL) comprising a CDR-L1 comprising the sequence of SEQ ID NO:460, a CDR-L2 comprising the sequence of SEQ ID NO:462, and a CDR-L3 comprising the sequence of SEQ ID NO:464. In some embodiments, the at least one antigen binding site that binds laminin-2 comprises a humanized VH domain and a humanized VL domain. In some embodiments, the VH domain of the at least one antigen binding site that binds laminin-2 comprises the sequence of SEQ ID NO:474; and the VL domain of the at least one antigen binding site that binds laminin-2 comprises the sequence of SEQ ID NO:490. In some embodiments, the at least one antigen binding site that binds laminin-2 comprises: (a) a heavy chain variable domain (VH) comprising a CDR-H1, a CDR-H2, and a CDR-H3 of C3_Hu10, C3_Hu11, C21_Hu11, or C21_Hu21 shown in Table A2, D2, or I4; and (b) a light chain variable domain (VL) comprising a CDR-L1, a CDR-L2, and a CDR-L3 of C3_Hu10, C3_Hu11, C21_Hu11, or C21_Hu21 shown in Table A2, D2, or I4. In some embodiments, the VH domain of the at least one antigen binding site that binds laminin-2 comprises the sequence of a C3_Hu10, C3_Hu11, C21_Hu11, or C21_Hu21 VH domain shown in Table D2 or I4; and the VL domain of the at least one antigen binding site that binds laminin-2 comprises the sequence of a C3_Hu10, C3_Hu11, C21_Hu11, or C21_Hu21 VL domain shown in Table D2 or I4.

In some embodiments, V_{H1} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L1} comprises a CDR-L1 comprising the sequence of SEQ ID NO:396, a CDR-L2 comprising the sequence of SEQ ID NO:398, and a CDR-L3 comprising the sequence of SEQ ID NO:400; V_{H2} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L1} comprises a CDR-L1 comprising the sequence of SEQ ID NO:396, a CDR-L2 comprising the sequence of SEQ ID NO:398, and a CDR-L3 comprising the sequence of SEQ ID NO:400; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:378, and V_{L1} comprises the sequence of SEQ ID NO:394; V_{H2} comprises the sequence of SEQ ID NO:378, and V_{L2} comprises the sequence of SEQ ID NO:394; and V_{H3} comprises the sequence of SEQ ID NO:314, and V_{L3} comprises the sequence of SEQ ID NO:330. In some embodiments, V_{H1} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L1} comprises a CDR-L1 comprising the sequence of SEQ ID NO:396, a CDR-L2 comprising the sequence of SEQ ID NO:398, and a CDR-L3 comprising the sequence of SEQ ID NO:400; V_{H2} comprises a CDR-H1 comprising the sequence of SEQ ID NO:444, a CDR-H2 comprising the sequence of SEQ ID NO:446, and a CDR-H3 comprising the sequence of SEQ ID NO:448, and V_{L2} comprises a CDR-L1 comprising the sequence of SEQ ID NO:460, a CDR-L2 comprising the sequence of SEQ ID NO:462, and a CDR-L3 comprising the sequence of SEQ ID NO:464; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:378, and V_{L1} comprises the sequence of SEQ ID NO:394; V_{H2} comprises the sequence of SEQ ID NO:442, and V_{L2} comprises the sequence of SEQ ID NO:458; and V_{H3} comprises the sequence of SEQ ID NO:314, and V_{L3} comprises the sequence of SEQ ID NO:330. In some embodiments, V_{H1} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L1} comprises a CDR-L1 comprising the sequence of SEQ ID NO:428, a CDR-L2 comprising the sequence of SEQ ID NO:398, and a CDR-L3 comprising the sequence of SEQ ID NO:400; V_{H2} comprises a CDR-H1 comprising the sequence of SEQ ID NO:444, a CDR-H2 comprising the sequence of SEQ ID NO:478, and a CDR-H3 comprising the sequence of SEQ ID NO:448, and V_{L2} comprises a CDR-L1 comprising the sequence of SEQ ID NO:460, a CDR-L2 comprising the sequence of SEQ ID NO:462, and a CDR-L3 comprising the sequence of SEQ ID NO:464; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:410, and V_{L1} comprises the sequence of SEQ ID NO:426; V_{H2} comprises the sequence of SEQ ID NO:474, and V_{L2} comprises the sequence of SEQ ID NO:490; and V_{H3} comprises the sequence of SEQ ID NO:314, and V_{L3} comprises the sequence of SEQ ID NO:330. In some embodiments, V_{H1} comprises a CDR-H1 comprising the sequence of SEQ ID NO:444, a CDR-H2 comprising the sequence of SEQ ID NO:446, and a CDR-H3 comprising the sequence of SEQ ID NO:448, and V_{L1} comprises a CDR-L1 comprising the sequence of SEQ ID NO:460, a CDR-L2 comprising the sequence of SEQ ID NO:462, and a CDR-L3 comprising the sequence of SEQ ID NO:464; V_{H2} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L2} comprises a CDR-L1 comprising the sequence of SEQ ID NO:428, a CDR-L2 comprising the sequence of SEQ ID NO:398, and a CDR-L3 comprising the sequence of SEQ ID NO:400; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:442, and V_{L1} comprises the sequence of SEQ ID NO:458; V_{H2} comprises the sequence of SEQ ID NO:410, and V_{L2} comprises the sequence of SEQ ID NO:426; and V_{H3} comprises the sequence of SEQ ID NO:314, and V_{L3} comprises the sequence of SEQ ID NO:330. In some embodiments, V_{H1} comprises a CDR-H1 comprising the sequence of SEQ ID NO:444, a CDR-H2 comprising the sequence of SEQ ID NO:478, and a CDR-H3 comprising the sequence of SEQ ID NO:448, and V_{L1} comprises a CDR-L1 comprising the sequence of SEQ ID NO:460, a CDR-L2 comprising the sequence of SEQ ID NO:462, and a CDR-L3 comprising the sequence of SEQ ID NO:464; V_{H2} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L2} comprises a CDR-L1 comprising the sequence of SEQ ID NO:396, a CDR-L2 comprising the sequence of SEQ ID NO:398, and a CDR-L3 comprising the sequence of SEQ ID NO:400; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:474, and V_{L1} comprises the sequence of SEQ ID NO:490; V_{H2} comprises the sequence of SEQ ID NO:378, and V_{L2} comprises the sequence of SEQ ID NO:394; and V_{H3} comprises the sequence of SEQ ID NO:314, and V_{L3} comprises the sequence of SEQ ID NO:330.

In some embodiments of any of the above embodiments, L₁ and L₂ comprise the sequence DKTHT (SEQ ID NO: 534). In some embodiments, L₃ and L₄ comprise the sequence DKTHT (SEQ ID NO: 534). In some embodiments, L₁, L₂, L₃, and L₄ comprise the sequence DKTHT (SEQ ID NO: 534).

In some embodiments of any of the above embodiments, the C_{H3} domain of the second polypeptide chain comprises amino acid substitutions at positions corresponding to positions 354 and 366 of human IgG1 or IgG4 according to EU Index, wherein the amino acid substitutions are S354C and T366W; and wherein the C_{H3} domain of the third polypeptide chain comprises amino acid substitutions at positions corresponding to positions 349, 366, 368, and 407 of human IgG1 or IgG4 according to EU Index, wherein the amino acid substitutions are Y349C, T366S, L368A, and Y407V. In some embodiments, the C_{H3} domain of the second polypeptide chain comprises amino acid substitutions at positions corresponding to positions 349, 366, 368, and 407 of human IgG1 or IgG4 according to EU Index, wherein the amino acid substitutions are Y349C, T366S, L368A, and Y407V; and wherein the C_{H3} domain of the third polypeptide chain comprises amino acid substitutions at positions corresponding to positions 354 and 366 of human IgG1 or IgG4 according to EU Index, wherein the amino acid substitutions are S354C and T366W. In some embodiments, the C_{H3} domains of the second and the third polypeptide chains are human IgG1 or IgG4 C_{H3} domains, and wherein only one of the C_{H3} domains comprises amino acid substitutions at positions corresponding to positions 435 and 436 of human IgG1 or IgG4 according to EU Index, wherein the amino acid substitutions are H435R and Y436F. In some embodiments, the C_{H2} domains of the second and the third polypeptide chains are human IgG1 or IgG4 C_{H2} domains comprising an asparagine residue at position 297, an asparagine residue at position 298, an alanine residue at position 299, and a serine or threonine residue at position 300, numbering according to EU Index. In some embodiments, the C_{H2} domains of the second and the third polypeptide chains are human IgG1 or IgG4 C_{H2} domains comprising a tyrosine residue at position 252, a threonine residue at position 254, and a glutamic acid residue at position 256, numbering according to EU Index.

In some embodiments, the first polypeptide chain comprises the sequence of SEQ ID NO:500, the second polypeptide chain comprises the sequence of SEQ ID NO:498, the third polypeptide chain comprises the sequence of SEQ ID NO:499, and the fourth polypeptide chain comprises the sequence of SEQ ID NO:501. In some embodiments, the first polypeptide chain comprises the sequence of SEQ ID NO:504, the second polypeptide chain comprises the sequence of SEQ ID NO:502, the third polypeptide chain comprises the sequence of SEQ ID NO:503, and the fourth polypeptide chain comprises the sequence of SEQ ID NO:505. In some embodiments, the first polypeptide chain comprises the sequence of SEQ ID NO:508, the second polypeptide chain comprises the sequence of SEQ ID NO:506, the third polypeptide chain comprises the sequence of SEQ ID NO:507, and the fourth polypeptide chain comprises the sequence of SEQ ID NO:509. In some embodiments, the first polypeptide chain comprises the sequence of SEQ ID NO:512, the second polypeptide chain comprises the sequence of SEQ ID NO:510, the third polypeptide chain comprises the sequence of SEQ ID NO:511, and the fourth polypeptide chain comprises the sequence of SEQ ID NO:513. In some embodiments, the first polypeptide chain comprises the sequence of SEQ ID NO:516, the second polypeptide chain comprises the sequence of SEQ ID NO:514, the third polypeptide chain comprises the sequence of SEQ ID NO:515, and the fourth polypeptide chain comprises the sequence of SEQ ID NO:517. In some embodiments, the binding protein comprises one, two, three, or four polypeptides of triAb 3407, 3423, 3429, 3437, or 3439, as shown in Table I2 or I4.

In some embodiments, the binding protein comprises (a) a first antibody heavy chain comprising a first heavy chain variable (VH) domain and a first Fc region of an immunoglobulin comprising a first C_{H3} region, and a first antibody light chain comprising a first light chain variable (VL) domain, wherein the first VH and VL domains form a first antigen binding domain that binds an extracellular portion of dystroglycan, and (b) a second antibody heavy chain comprising a second heavy chain variable (VH) domain and a second Fc region of an immunoglobulin comprising a second C_{H3} region, and a second antibody light chain comprising a second light chain variable (VL) domain, wherein the second VH and VL domains form a second antigen binding domain that binds laminin-2; wherein the sequences of said first and second C_{H3} regions are different and are such that the heterodimeric interaction between said first and second C_{H3} regions is stronger than each of the homodimeric interactions of said first and second C_{H3} regions, and wherein said first homodimeric protein has an amino acid other than Lys, Leu or Met at position 409 and said second homodimeric protein has an amino- acid substitution at a position selected from the group consisting of: 366, 368, 370, 399, 405 and 407 and/or wherein the sequences of said first and second C_{H3} regions are such that the dissociation constants of homodimeric interactions of each of the C_{H3} regions are between 0.01 and 10 micromolar. In some embodiments, the first antibody heavy chain comprises the sequence of SEQ ID NO:518, wherein the second antibody heavy chain comprises the sequence of SEQ ID NO:519, wherein the first antibody light chain comprises the sequence of SEQ ID NO:520, and wherein the second antibody light chain comprises the sequence of SEQ ID NO:521. In some embodiments, the binding protein comprises one, two, three, or four polypeptides of AS30_Hu6 x C3_Hu10 Duobody, AS30_Hu6 x C21_Hu11 Duetmab, AS30_Hu6 x C3_Hu10 TBTI, AS30_Hu6 x C21_Hu11 TBTI, AS30_Hu9 x C3_Hu11 CODV, or AS30_Hu9 x C21_Hu21 CODV, as shown in Table I3 or I4.

Further provided herein are isolated nucleic acid molecules comprising a nucleotide sequence encoding the multispecific binding molecule of any one of the above embodiments. Also provided are isolated nucleic acid molecules comprising a nucleotide sequence of Table G2 or I4. Also provided are expression vectors comprising the nucleic acid molecules of any one of the above embodiments. Also provided are host cells (*e.g.,* isolated host cells) comprising the nucleic acid molecules or expression vectors of any one of the above embodiments. Also provided is a vector system comprising one or more vectors encoding a first, second, third, and fourth polypeptide chain of a multispecific binding molecule of any one of the above embodiments. Also provided is a host cell (*e.g.,* an isolated host cell) comprising the vector system of any one of the above embodiments. Also provided is a method of producing a multispecific binding molecule, the method comprising: culturing a host cell of any one of the above embodiments under conditions such that the host cell expresses the multispecific binding molecule; and isolating the multispecific binding molecule from the host cell.

Also provided herein is a use of the multispecific binding molecule of any one of the above embodiments for treating or preventing an alpha-dystroglycanopathy in an individual.

In some embodiments of any of the above embodiments, the individual has reduced expression of alpha-dystroglycan. In some embodiments, alpha-dystroglycan expressed in the individual has impaired or aberrant O-glycosylation. In some embodiments, the individual has a mutation in a gene selected from the group consisting of: dystroglycan (DAG1), protein O-mannosyltransferase-1 (POMT1), protein O-mannosyltransferase-2 (POMT2), protein O-linked mannose beta1,2-N-acetylglucosylaminyltransferase subunit 1 (POMGNT1), protein O-linked mannose beta1,4-N-acetylglucosylaminyltransferase subunit 2 (POMGNT2), xylosyl- and glucuronyltransferase 1 (LARGE1), xylosyl- and glucuronyltransferase 2 (LARGE2), dolichyl-phosphate mannosyltransferase subunit 1 (DPM1), dolichyl-phosphate mannosyltransferase subunit 2 (DPM2), dolichyl-phosphate mannosyltransferase subunit 3 (DPM3), fukutin, fukutin related protein (FKRP), isprenoid synthase domain containing (ISPD), protein O-mannose kinase (POMK), beta-1,3-N-acetylgalactosaminyltransferase 2 (B3GALNT2), beta-1,4-glucuronyltransferase 1 (B4GAT1), dolichol kinase (DOLK), transmembrane protein 5 (TMEM5), and GDP-mannose pyrophosphorylase B (GMPPB). In some embodiments, the multispecific binding molecule is administered via intravenous infusion. In some embodiments, the multispecific binding molecule is administered via intramuscular, intraperitoneal, or subcutaneous injection. In some embodiments, the individual is a human.

Further provided herein is a pharmaceutical composition comprising the multispecific binding molecule of any one of the above embodiments and a pharmaceutically acceptable carrier. In some embodiments, the individual has reduced expression of alpha-dystroglycan. In some embodiments, alpha-dystroglycan expressed in the individual has impaired or aberrant O-glycosylation. In some embodiments, the individual has a mutation in a gene selected from the group consisting of: dystroglycan (DAG1), protein O-mannosyltransferase-1 (POMT1), protein O-mannosyltransferase-2 (POMT2), protein O-linked mannose beta1,2-N-acetylglucosylaminyltransferase subunit 1 (POMGNT1), protein O-linked mannose beta1,4-N-acetylglucosylaminyltransferase subunit 2 (POMGNT2), xylosyl- and glucuronyltransferase 1 (LARGE1), xylosyl- and glucuronyltransferase 2 (LARGE2), dolichyl-phosphate mannosyltransferase subunit 1 (DPM1), dolichyl-phosphate mannosyltransferase subunit 2 (DPM2), dolichyl-phosphate mannosyltransferase subunit 3 (DPM3), fukutin, fukutin related protein (FKRP), isprenoid synthase domain containing (ISPD), protein O-mannose kinase (POMK), beta-1,3-N-acetylgalactosaminyltransferase 2 (B3GALNT2), beta-1,4-glucuronyltransferase 1 (B4GAT1), dolichol kinase (DOLK), transmembrane protein 5 (TMEM5), and GDP-mannose pyrophosphorylase B (GMPPB). In some embodiments, the individual is a human.

Further provided herein is an antibody that binds an extracellular portion of dystroglycan, wherein the antibody comprises: (a) an antibody heavy chain comprising a heavy chain variable domain (VH) comprising a CDR-H1 comprising an amino acid sequence selected from the group consisting of SEQ ID NOs:1-8, a CDR-H2 comprising an amino acid sequence selected from the group consisting of SEQ ID NOs:9-17, and a CDR-H3 comprising an amino acid sequence selected from the group consisting of SEQ ID NOs:18-27; and (b) an antibody light chain comprising a light chain variable domain (VL) comprising a CDR-L1 comprising an amino acid sequence selected from the group consisting of SEQ ID NOs:28-37, a CDR-L2 comprising an amino acid sequence selected from the group consisting of SEQ ID NOs:38-42, and a CDR-L3 comprising an amino acid sequence selected from the group consisting of SEQ ID NOs:43-50. In some embodiments, the VH domain comprises an amino acid sequence selected from the group consisting of SEQ ID NOs:170, 172, 174, 176, 178, 180, 182, 184, 186, and 188; and the VL domain comprises an amino acid sequence selected from the group consisting of SEQ ID NOs:171, 173, 175, 177, 179, 181, 183, 185, 187, and 189. In some embodiments, the antibody comprises 1, 2, 3, 4, 5, or 6 CDR sequences of a binding domain shown in Table A2, D2, or I4, or a VH and/or VL domain sequence of a binding domain shown in Table D2 or I4 or encoded by a polynucleotide sequence shown in Table G2.

Further provided herein is an antibody that binds an extracellular portion of dystroglycan, wherein the antibody comprises: (a) an antibody heavy chain comprising a heavy chain variable domain (VH) comprising a CDR-H1 comprising the sequence of SEQ ID NO:316, a CDR-H2 comprising the sequence of SEQ ID NO:318, and a CDR-H3 comprising the sequence of SEQ ID NO:320; and (b) an antibody light chain comprising a light chain variable domain (VL) comprising a CDR-L1 comprising the sequence of SEQ ID NO:332, a CDR-L2 comprising the sequence of SEQ ID NO:334, and a CDR-L3 comprising the sequence of SEQ ID NO:336. In some embodiments, the VH domain comprises the amino acid sequence of SEQ ID NO:314, and the VL domain comprises the amino acid sequence of SEQ ID NO:330; or the VH domain comprises the amino acid sequence of SEQ ID NO:346, and the VL domain comprises the amino acid sequence of SEQ ID NO:362. In some embodiments, the antibody comprises 1, 2, 3, 4, 5, or 6 CDR sequences of a binding domain shown in Table A2, D2, or I4, or a VH and/or VL domain sequence of a binding domain shown in Table D2 or I4 or encoded by a polynucleotide sequence shown in Table G2.

Further provided herein is an antibody that binds laminin-2, wherein the antibody comprises: (a) an antibody heavy chain comprising a CDR-H1 comprising an amino acid sequence selected from the group consisting of SEQ ID NOs:51-55 and 81-95, a CDR-H2 comprising an amino acid sequence selected from the group consisting of SEQ ID NOs:56-60 and 96-110, and a CDR-H3 comprising an amino acid sequence selected from the group consisting of SEQ ID NOs:61-65 and 111-125; and (b) an antibody light chain comprising a light chain variable domain (VL) comprising a CDR-L1 comprising an amino acid sequence selected from the group consisting of SEQ ID NOs:66-70 and 126-140, a CDR-L2 comprising an amino acid sequence selected from the group consisting of SEQ ID NOs:38, 71-75, and 141-154, and a CDR-L3 comprising an amino acid sequence selected from the group consisting of SEQ ID NOs:76-80 and 155-169. In some embodiments, the VH domain comprises an amino acid sequence selected from the group consisting of SEQ ID NOs:190, 192, 194, 196, 198, 200, 202, 204, 206, 208, 210, 212, 214, 216, 218, 220, 222, 224, 226, and 228; and the VL domain comprises an amino acid sequence selected from the group consisting of SEQ ID NOs: 191, 193, 195, 197, 199, 201, 203, 205, 207, 209, 211, 213, 215, 217, 219, 221, 223, 225, 227, and 229. In some embodiments, the antibody comprises 1, 2, 3, 4, 5, or 6 CDR sequences of a binding domain shown in Table A2, D2, or I4, or a VH and/or VL domain sequence of a binding domain shown in Table D2 or I4 or encoded by a polynucleotide sequence shown in Table G2.

Further provided herein is an antibody that binds laminin-2, wherein the antibody comprises: (a) an antibody heavy chain comprising a heavy chain variable domain (VH) comprising a CDR-H1 comprising the sequence of SEQ ID NO:380, a CDR-H2 comprising the sequence of SEQ ID NO:382, and a CDR-H3 comprising the sequence of SEQ ID NO:384, and an antibody light chain comprising a light chain variable domain (VL) comprising a CDR-L1 comprising the sequence of SEQ ID NO:428, a CDR-L2 comprising the sequence of SEQ ID NO:398, and a CDR-L3 comprising the sequence of SEQ ID NO:400; (b) an antibody heavy chain comprising a heavy chain variable domain (VH) comprising a CDR-H1 comprising the sequence of SEQ ID NO:380, a CDR-H2 comprising the sequence of SEQ ID NO:382, and a CDR-H3 comprising the sequence of SEQ ID NO:384, and an antibody light chain comprising a light chain variable domain (VL) comprising a CDR-L1 comprising the sequence of SEQ ID NO:428, a CDR-L2 comprising the sequence of SEQ ID NO:398, and a CDR-L3 comprising the sequence of SEQ ID NO:400; (c) an antibody heavy chain comprising a heavy chain variable domain (VH) comprising a CDR-H1 comprising the sequence of SEQ ID NO:444, a CDR-H2 comprising the sequence of SEQ ID NO:446, and a CDR-H3 comprising the sequence of SEQ ID NO:448, and an antibody light chain comprising a light chain variable domain (VL) comprising a CDR-L1 comprising the sequence of SEQ ID NO:460, a CDR-L2 comprising the sequence of SEQ ID NO:462, and a CDR-L3 comprising the sequence of SEQ ID NO:464; or (d) an antibody heavy chain comprising a heavy chain variable domain (VH) comprising a CDR-H1 comprising the sequence of SEQ ID NO:444, a CDR-H2 comprising the sequence of SEQ ID NO:478, and a CDR-H3 comprising the sequence of SEQ ID NO:448, and an antibody light chain comprising a light chain variable domain (VL) comprising a CDR-L1 comprising the sequence of SEQ ID NO:460, a CDR-L2 comprising the sequence of SEQ ID NO:462, and a CDR-L3 comprising the sequence of SEQ ID NO:464. In some embodiments, (a) the VH domain comprises the amino acid sequence of SEQ ID NO:378, and the VL domain comprises the amino acid sequence of SEQ ID NO:394; (b) the VH domain comprises the amino acid sequence of SEQ ID NO:410, and the VL domain comprises the amino acid sequence of SEQ ID NO:426; (c) the VH domain comprises the amino acid sequence of SEQ ID NO:442, and the VL domain comprises the amino acid sequence of SEQ ID NO:458; or (d) the VH domain comprises the amino acid sequence of SEQ ID NO:474, and the VL domain comprises the amino acid sequence of SEQ ID NO:490. In some embodiments, the antibody comprises 1, 2, 3, 4, 5, or 6 CDR sequences of a binding domain shown in Table A2, D2, or I4, or a VH and/or VL domain sequence of a binding domain shown in Table D2 or I4 or encoded by a polynucleotide sequence shown in Table G2.

Further provided herein are isolated nucleic acid molecules comprising a nucleotide sequence encoding the antibody of any one of the above embodiments. Also provided are expression vectors comprising the nucleic acid molecules of any one of the above embodiments. Also provided are host cells (*e.g.,* isolated host cells) comprising the nucleic acid molecules or expression vectors of any one of the above embodiments. Also provided is a method of producing an antibody, the method comprising: culturing a host cell of any one of the above embodiments under conditions such that the host cell expresses the antibody; and isolating the antibody from the host cell.

The bispecific binding molecule comprises a first binding domain that binds an extracellular portion of dystroglycan and a second binding domain that binds laminin-2. The bispecific binding molecule is a bispecific binding protein comprising one or more polypeptide chains.

The bispecific binding molecule is a bispecific, bivalent or tetravalent binding protein comprising two or four antigen binding sites. In some embodiments, the bispecific binding protein comprises a first binding domain that binds to an extracellular portion of dystroglycan, wherein the first binding domain comprises a first immunoglobulin heavy chain variable domain (V_{H1}) and a first immunoglobulin light chain variable domain (V_{L1}), and a second binding domain that binds to laminin-2, wherein the second binding domain comprises a second immunoglobulin heavy chain variable domain (V _{H2}) and a second immunoglobulin light chain variable domain (V_{L2}). In some embodiments, the V_{H1} domain comprises at least 1, at least 2, at least 3, at least 4, at least 5, or 6 CDR sequences of an antibody shown in Table A and/or the V_{L1} domain comprises at least 1, at least 2, at least 3, at least 4, at least 5, or 6 CDR sequences of an antibody shown in Table A. In some embodiments, the V_{H2} domain comprises at least 1, at least 2, at least 3, at least 4, at least 5, or 6 CDR sequences of an antibody shown in Table B or Table C and/or the V_{L2} domain comprises at least 1, at least 2, at least 3, at least 4, at least 5, or 6 CDR sequences of an antibody shown in Table B or Table C.

The disclosure thus provides a bispecific binding molecule comprising a first binding domain that binds an extracellular portion of dystroglycan and a second binding domain that binds laminin-2, wherein wherein the bispecific binding molecule is a bispecific binding protein comprising one or more polypeptide chains, wherein the bispecific binding molecule comprises four polypeptide chains that form four antigen binding sites, wherein two polypeptide chains comprise a structure represented by the formula:

V_{L1}-L₁-V_{L2}-L₂-C_{L} [I]

and two polypeptide chains comprise a structure represented by the formula:

V_{H2}-L₃-V_{H1}-L₄-C_{H1}-hinge-C_{H2}-C_{H3} [II]

wherein:
V_{L1} is a first immunoglobulin light chain variable domain;
V_{L2} is a second immunoglobulin light chain variable domain;
V_{H1} is a first immunoglobulin heavy chain variable domain;
V_{H2} is a second immunoglobulin heavy chain variable domain;
C_{L} is an immunoglobulin light chain constant domain;
C_{H1} is an immunoglobulin C_{H1} heavy chain constant domain;
C_{H2} is an immunoglobulin C_{H2} heavy chain constant domain;
C_{H3} is an immunoglobulin C_{H3} heavy chain constant domain;
hinge is an immunoglobulin hinge region connecting the C_{H1} and C_{H2} domains; and
L₁, L₂, L₃, and L₄ are amino acid linkers; wherein the V_{H1} and V_{L1} domains form a V_{H1}/V_{L1} binding pair, and wherein the V_{H2} and V_{L2} domains form a V_{H2}/V_{L2} binding pair.

In some embodiments, the V_{H1} and V_{L1} domains cross-over to form the V_{H1}/V_{L1} binding pair. In some embodiments, the V_{H2} and V_{L2} domains cross-over to form the V_{H2}/V_{L2} binding pair. In some embodiments, L₁, L₂, L₃, and L₄ are each 0 to 50 amino acid residues in length. In some embodiments, L₁, L₂, L₃, and L₄ are each 0 to 25 amino acid residues in length. In some embodiments, L₁, L₂, L₃, and L₄ are each 0 to 14 amino acid residues in length. In some embodiments, L₁ is 5 amino acid residues in length; L₂ is 5 amino acid residues in length; L₃ is 5 amino acid residues in length; and L₄ is 5 amino acid residues in length. In some embodiments, L₁ is 14 amino acid residues in length; L₂ is 2 amino acid residues in length; L₃ is 14 amino acid residue in length; and L₄ is 2 amino acid residues in length. In some embodiments, L₁ and L₃ each comprise the sequence EPKSDKTHTSPPSP (SEQ ID NO:296), and wherein L₂ and L₄ each comprise the sequence GG. In some embodiments, L₁ is 7 amino acid residues in length; L₂ is 5 amino acid residues in length; L₃ is 1 amino acid residue in length; and L₄ is 2 amino acid residues in length. In some embodiments, L₁ comprises the sequence GQPKAAP (SEQ ID NO:297); L₂ comprises the sequence TKGPS (SEQ ID NO:298); L₃ comprises a serine residue; and L₄ comprises the sequence RT. In some embodiments, L₁ is 10 amino acid residues in length; L₂ is 10 amino acid residues in length; L₃ is 0 amino acid residues in length; and L₄ is 0 amino acid residues in length. In some embodiments, L₁ and L₂ each comprise the sequence GGSGSSGSGG (SEQ ID NO:299). In some embodiments, one or both of the variable domains of the polypeptides of formula I and/or formula II are human, humanized, or mouse variable domains.

The disclosure also provides a bispecific binding molecule comprising a first binding domain that binds an extracellular portion of dystroglycan and a second binding domain that binds laminin-2, wherein the bispecific binding molecule is a bispecific binding protein comprising one or more polypeptide chains, wherein the bispecific binding molecule comprises two light chains comprising a structure represented by the formula:

V_{L1}-L₅-V_{L2}-L₆-C_{L} [III]

and two heavy chains comprising a structure represented by the formula:

V_{H1}-L₇-V_{H2}-L₈-C_{H1}-hinge-C_{H2}-C_{H3} [IV]

wherein:
V_{L1} is a first immunoglobulin light chain variable domain;
V_{L2} is a second immunoglobulin light chain variable domain;
V_{H1} is a first immunoglobulin heavy chain variable domain;
V_{H2} is a second immunoglobulin heavy chain variable domain;
C_{L} is an immunoglobulin light chain constant domain;
C_{H1} is an immunoglobulin C_{H1} heavy chain constant domain;
C_{H2} is an immunoglobulin C_{H2} heavy chain constant domain;
C_{H3} is an immunoglobulin C_{H3} heavy chain constant domain;
hinge is an immunoglobulin hinge region connecting the C_{H1} and C_{H2} domains; and
L₅, L₆, L₇, and L₈ are amino acid linkers; wherein the V_{H1} and V_{L1} domains form a V_{H1}/V_{L1} binding pair, and wherein the V_{H2} and V_{L2} domains form a V_{H2}/V_{L2} binding pair.

In some embodiments, L₅, L₆, L₇, and L₈ are each 0 to 50 amino acid residues in length. In some embodiments, L₅, L₆, L₇, and L₈ are each 0 to 25 amino acid residues in length. In some embodiments, L₅, L₆, L₇, and L₈ are each 0 to 14 amino acid residues in length. In some embodiments, the L₅ and L₇ linkers comprise the amino acid sequence of GGGGSGGGGS (SEQ ID NO:294), and wherein the L₆ and L₈ linkers are each 0 amino acid residues in length. In some embodiments, one or both of the variable domains of the polypeptides of formula III and/or formula IV are human, humanized, or mouse variable domains.

In some embodiments of any of the above embodiments, the V_{H1}/V_{L1} binding pair binds the extracellular portion of dystroglycan, and wherein the V_{H2}/V_{L2} binding pair binds laminin-2. In some embodiments, the V_{H1}/ V_{L1} binding pair binds the extracellular portion of human dystroglycan. In some embodiments, the V_{H1}/ V_{L1} binding pair binds the extracellular portion of human dystroglycan with an equilibrium dissociation constant (K_{D}) lower than about 1µM. In some embodiments, the V_{H1}/V_{L1} binding pair binds the extracellular portions of human and mouse dystroglycan. In some embodiments, the V_{H1}/V_{L1} binding pair binds beta-dystroglycan. In some embodiments, the V_{H1}/V_{L1} binding pair binds a polypeptide comprising the sequence SIVVEWTNN TLPLEPCPKE QIIGLSRRIA DENGKPRPAF SNALEPDFKA LSIAVTGSGS CRHLQFIPVA PPSPGSSAAP ATEVPDRDPE KSSEDD (SEQ ID NO:290). In some embodiments, the V_{H1}/V_{L1} binding pair binds a polypeptide comprising the sequence SIVVEWT NNTLPLEPCP KEQIAGLSRR IAEDDGKPRP AFSNALEPDF KATSITVTGS GSCRHLQFIP VVPPRRVPSE APPTEVPDRD PEKSSEDDV (SEQ ID NO:291). In some embodiments, the V_{H1}/V_{L1} binding pair binds alpha-dystroglycan. In some embodiments, the V_{H2}/V_{L2} binding pair binds human laminin-2. In some embodiments, the V_{H2}/V_{L2} binding pair binds human laminin-2 with an equilibrium dissociation constant (K_{D}) lower than about 1µM. In some embodiments, the V_{H2}/V_{L2} binding pair binds mouse and human laminin-2. In some embodiments, the V_{H2}/V_{L2} binding pair binds a polypeptide comprising a laminin G-like (LG) domain 4 of laminin-2, a laminin G-like (LG) domain 5 of laminin-2, or both. In some embodiments, the V_{H2}/V_{L2} binding pair binds a polypeptide comprising the laminin G-like (LG) domain 4 and laminin G-like (LG) domain 5 of laminin-2. In some embodiments, the V_{H2}/V_{L2} binding pair binds a polypeptide comprising the sequence VQPQPV PTPAFPFPAP TMVHGPCVAE SEPALLTGSK QFGLSRNSHI AIAFDDTKVK NRLTIELEVR TEAESGLLFY MARINHADFA TVQLRNGFPY FSYDLGSGDT STMIPTKIND GQWHKIKIVR VKQEGILYVD DASSQTISPK KADILDVVGI LYVGGLPINY TTRRIGPVTY SLDGCVRNLH MEQAPVDLDQ PTSSFHVGTC FANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:300). In some embodiments, the V_{H2}/V_{L2} binding pair binds a polypeptide comprising the sequence Q PEPVPTPAFP TPTPVLTHGP CAAESEPALL IGSKQFGLSR NSHIAIAFDD TKVKNRLTIE LEVRTEAESG LLFYMARINH ADFATVQLRN GLPYFSYDLG SGDTHTMIPT KINDGQWHKI KIMRSKQEGI LYVDGASNRT ISPKKADILD VVGMLYVGGL PINYTTRRIG PVTYSIDGCV RNLHMAEAPA DLEQPTSSFH VGTCFANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:301). In some embodiments, the V_{H2}/V_{L2} binding pair binds a polypeptide comprising the laminin G-like (LG) domain 5 of laminin-2. In some embodiments, the V_{H2}/V_{L2} binding pair binds a polypeptide comprising the sequence ANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:292). In some embodiments, the V_{H2}/V_{L2} binding pair binds a polypeptide comprising the sequence ANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:293).

In some embodiments, the V_{H1} domain comprises a CDR-H1 comprising an amino acid sequence selected from the group consisting of SEQ ID NOs:1-8, a CDR-H2 comprising an amino acid sequence selected from the group consisting of SEQ ID NOs:9-17, and a CDR-H3 comprising an amino acid sequence selected from the group consisting of SEQ ID NOs:18-27; and/or wherein the V_{L1} domain comprises a CDR-L1 comprising an amino acid sequence selected from the group consisting of SEQ ID NOs:28-37, a CDR-L2 comprising an amino acid sequence selected from the group consisting of SEQ ID NOs:38-42, and a CDR-L3 comprising an amino acid sequence selected from the group consisting of SEQ ID NOs:43-50. In some embodiments, the V_{H1} domain comprises an amino acid sequence selected from the group consisting of SEQ ID NOs:170, 172, 174, 176, 178, 180, 182, 184, 186, and 188. In some embodiments, the V_{L1} domain comprises an amino acid sequence selected from the group consisting of SEQ ID NOs:171, 173, 175, 177, 179, 181, 183, 185, 187, and 189. In some embodiments, the V_{H1} domain is encoded by a nucleic acid sequence selected from the group consisting of SEQ ID NOs:230, 232, 234, 236, 238, 240, 242, 244, 246, and 248. In some embodiments, the V_{L1} domain is encoded by a nucleic acid sequence selected from the group consisting of SEQ ID NOs:231, 233, 235, 237, 239, 241, 243, 245, 247, and 249. In some embodiments, the V_{H2} domain comprises a CDR-H1 comprising an amino acid sequence selected from the group consisting of SEQ ID NOs:51-55 and 81-95, a CDR-H2 comprising an amino acid sequence selected from the group consisting of SEQ ID NOs:56-60 and 96-110, and a CDR-H3 comprising an amino acid sequence selected from the group consisting of SEQ ID NOs:61-65 and 111-125; and/or wherein the V_{L2} domain comprises a CDR-L1 comprising an amino acid sequence selected from the group consisting of SEQ ID NOs:66-70 and 126-140, a CDR-L2 comprising an amino acid sequence selected from the group consisting of SEQ ID NOs:38, 71-75, and 141-154, and a CDR-L3 comprising an amino acid sequence selected from the group consisting of SEQ ID NOs:76-80 and 155-169. In some embodiments, the V_{H2} domain comprises an amino acid sequence selected from the group consisting of SEQ ID NOs:190, 192, 194, 196, 198, 200, 202, 204, 206, 208, 210, 212, 214, 216, 218, 220, 222, 224, 226, and 228. In some embodiments, the V_{L2} domain comprises an amino acid sequence selected from the group consisting of SEQ ID NOs: 191, 193, 195, 197, 199, 201, 203, 205, 207, 209, 211, 213, 215, 217, 219, 221, 223, 225, 227, and 229. In some embodiments, the V_{H2} domain is encoded by a nucleic acid sequence selected from the group consisting of SEQ ID NOs:250, 252, 254, 256, 258, 260, 262, 264, 266, 268, 270, 272, 274, 276, 278, 280, 282, 284, 286, and 288. In some embodiments, the V_{L2} domain is encoded by a nucleic acid sequence selected from the group consisting of SEQ ID NOs:251, 253, 255, 257, 259, 261, 263, 265, 267, 269, 271, 273, 275, 277, 279, 281, 283, 285, 287, and 289.

In some embodiments of any of the above embodiments, the V_{H1}/V_{L1} binding pair binds laminin-2, and wherein the V_{H2}/V_{L2} binding pair binds the extracellular portion of dystroglycan. In some embodiments, the V_{H2}/ V_{L2} binding pair binds the extracellular portion of human dystroglycan. In some embodiments, the V_{H2}/ V_{L2} binding pair binds the extracellular portion of human dystroglycan with an equilibrium dissociation constant (K_{D}) lower than about 1µM. In some embodiments, the V_{H2}/V_{L2} binding pair binds the extracellular portions of human and mouse dystroglycan. In some embodiments, the V_{H2}/V_{L2} binding pair binds beta-dystroglycan. In some embodiments, the V_{H2}/V_{L2} binding pair binds a polypeptide comprising the sequence SIVVEWTNN TLPLEPCPKE QIIGLSRRIA DENGKPRPAF SNALEPDFKA LSIAVTGSGS CRHLQFIPVA PPSPGSSAAP ATEVPDRDPE KSSEDD (SEQ ID NO:290). In some embodiments, the V_{H2}/V_{L2} binding pair binds a polypeptide comprising the sequence SIVVEWT NNTLPLEPCP KEQIAGLSRR IAEDDGKPRP AFSNALEPDF KATSITVTGS GSCRHLQFIP VVPPRRVPSE APPTEVPDRD PEKSSEDDV (SEQ ID NO:291). In some embodiments, the V_{H2}/V_{L2} binding pair binds alpha-dystroglycan. In some embodiments, the V_{H1}/V_{L1} binding pair binds human laminin-2. In some embodiments, the V_{H1}/V_{L1} binding pair binds human laminin-2 with an equilibrium dissociation constant (K_{D}) lower than about 1µM. In some embodiments, the V_{H1}/V_{L1} binding pair binds mouse and human laminin-2. In some embodiments, the V_{H1}/V_{L1} binding pair binds a polypeptide comprising a laminin G-like (LG) domain 4 of laminin-2, a laminin G-like (LG) domain 5 of laminin-2, or both. In some embodiments, the V_{H1}/V_{L1} binding pair binds a polypeptide comprising the laminin G-like (LG) domain 4 and laminin G-like (LG) domain 5 of laminin-2. In some embodiments, the V_{H1}/V_{L1} binding pair binds a polypeptide comprising the sequence VQPQPV PTPAFPFPAP TMVHGPCVAE SEPALLTGSK QFGLSRNSHI AIAFDDTKVK NRLTIELEVR TEAESGLLFY MARINHADFA TVQLRNGFPY FSYDLGSGDT STMIPTKIND GQWHKIKIVR VKQEGILYVD DASSQTISPK KADILDVVGI LYVGGLPINY TTRRIGPVTY SLDGCVRNLH MEQAPVDLDQ PTSSFHVGTC FANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:300). In some embodiments, the V_{H1}/V_{L1} binding pair binds a polypeptide comprising the sequence Q PEPVPTPAFP TPTPVLTHGP CAAESEPALL IGSKQFGLSR NSHIAIAFDD TKVKNRLTIE LEVRTEAESG LLFYMARINH ADFATVQLRN GLPYFSYDLG SGDTHTMIPT KINDGQWHKI KIMRSKQEGI LYVDGASNRT ISPKKADILD VVGMLYVGGL PINYTTRRIG PVTYSIDGCV RNLHMAEAPA DLEQPTSSFH VGTCFANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:301). In some embodiments, the V_{H1}/V_{L1} binding pair binds a polypeptide comprising the laminin G-like (LG) domain 5 of laminin-2. In some embodiments, the V_{H1}/V_{L1} binding pair binds a polypeptide comprising the sequence ANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:292). In some embodiments, the V_{H1}/V_{L1} binding pair binds a polypeptide comprising the sequence ANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:293).

In some embodiments, the V_{H2} domain comprises a CDR-H1 comprising an amino acid sequence selected from the group consisting of SEQ ID NOs:1-8, a CDR-H2 comprising an amino acid sequence selected from the group consisting of SEQ ID NOs:9-17, and a CDR-H3 comprising an amino acid sequence selected from the group consisting of SEQ ID NOs:18-27; and/or wherein the V_{L2} domain comprises a CDR-L1 comprising an amino acid sequence selected from the group consisting of SEQ ID NOs:28-37, a CDR-L2 comprising an amino acid sequence selected from the group consisting of SEQ ID NOs:38-42, and a CDR-L3 comprising an amino acid sequence selected from the group consisting of SEQ ID NOs:43-50. In some embodiments, the V_{H2} domain comprises an amino acid sequence selected from the group consisting of SEQ ID NOs:170, 172, 174, 176, 178, 180, 182, 184, 186, and 188. In some embodiments, the V_{L2} domain comprises an amino acid sequence selected from the group consisting of SEQ ID NOs:171, 173, 175, 177, 179, 181, 183, 185, 187, and 189. In some embodiments, the V_{H2} domain is encoded by a nucleic acid sequence selected from the group consisting of SEQ ID NOs:230, 232, 234, 236, 238, 240, 242, 244, 246, and 248. In some embodiments, the V_{L2} domain is encoded by a nucleic acid sequence selected from the group consisting of SEQ ID NOs:231, 233, 235, 237, 239, 241, 243, 245, 247, and 249. In some embodiments, the V_{H1} domain comprises a CDR-H1 comprising an amino acid sequence selected from the group consisting of SEQ ID NOs:51-55 and 81-95, a CDR-H2 comprising an amino acid sequence selected from the group consisting of SEQ ID NOs:56-60 and 96-110, and a CDR-H3 comprising an amino acid sequence selected from the group consisting of SEQ ID NOs:61-65 and 111-125; and/or wherein the V_{L1} domain comprises a CDR-L1 comprising an amino acid sequence selected from the group consisting of SEQ ID NOs:66-70 and 126-140, a CDR-L2 comprising an amino acid sequence selected from the group consisting of SEQ ID NOs:38, 71-75, and 141-154, and a CDR-L3 comprising an amino acid sequence selected from the group consisting of SEQ ID NOs:76-80 and 155-169. In some embodiments, the V_{H2} domain comprises an amino acid sequence selected from the group consisting of SEQ ID NOs:190, 192, 194, 196, 198, 200, 202, 204, 206, 208, 210, 212, 214, 216, 218, 220, 222, 224, 226, and 228. In some embodiments, the V_{L2} domain comprises an amino acid sequence selected from the group consisting of SEQ ID NOs: 191, 193, 195, 197, 199, 201, 203, 205, 207, 209, 211, 213, 215, 217, 219, 221, 223, 225, 227, and 229. In some embodiments, the V_{H2} domain is encoded by a nucleic acid sequence selected from the group consisting of SEQ ID NOs:250, 252, 254, 256, 258, 260, 262, 264, 266, 268, 270, 272, 274, 276, 278, 280, 282, 284, 286, and 288. In some embodiments, the V_{L2} domain is encoded by a nucleic acid sequence selected from the group consisting of SEQ ID NOs:251, 253, 255, 257, 259, 261, 263, 265, 267, 269, 271, 273, 275, 277, 279, 281, 283, 285, 287, and 289.

In one embodiment, the disclosure provides an isolated nucleic acid molecule comprising a nucleotide sequence encoding the bispecific binding molecule according to any one of the above embodiments. In one embodiment, the disclosure provides an expression vector comprising a nucleotide sequence encoding the bispecific binding molecule according to any one of the above embodiments. In one embodiment, the disclosure provides an isolated host cell comprising a nucleotide sequence encoding the bispecific binding molecule according to any one of the above embodiments or comprising an expression vector comprising a nucleotide sequence encoding the bispecific binding molecule according to any one of the above embodiments. In one embodiment, the disclosure provides a vector system comprising one or more vectors encoding a first, second, third, and fourth polypeptide chain of a bispecific binding molecule according to any one of the above embodiments. In one embodiment, the disclosure provides a vector system comprising one or more vectors encoding two light chains and two heavy chains of a bispecific binding molecule according to any one of the above embodiments. In one embodiment, the disclosure provides an isolated host cell comprising the vector system according to any one of the above embodiments.

In one embodiment, the disclosure provides a method of producing a bispecific binding molecule, the method comprising: a) culturing a host cell according to any one of the above embodiments under conditions such that the host cell expresses the bispecific binding molecule; and b) isolating the bispecific binding molecule from the host cell. In one embodiment, the disclosure provides a method of producing a bispecific binding protein comprising a first binding domain that binds an extracellular portion of dystroglycan and a second binding domain that binds laminin-2, the method comprising: a) culturing a first host cell that comprises a nucleic acid molecule encoding a first polypeptide chain comprising the first binding domain under conditions such that the host cell expresses the first polypeptide chain as part of a first monospecific binding protein with a first CH3 domain; b) culturing a second host cell that comprises a nucleic acid molecule encoding a second polypeptide chain comprising the second binding domain conditions such that the host cell expresses the second polypeptide chain as part of a second monospecific binding protein with a second CH3 domain; c) isolating the first monospecific binding protein from the first host cell; d) isolating the second monospecific binding protein from the second host cell; e) incubating the isolated first and second monospecific binding proteins under reducing conditions sufficient to allow cysteines in the hinge region to undergo disulfide bond isomerization; and f) obtaining the bispecific binding protein, wherein the first and second CH3 domains are different and are such that the heterodimeric interaction between said first and second CH3 domains is stronger than each of the homodimeric interactions of said first and second CH3 domains.

In one embodiment, the disclosure provides the bispecific binding molecule according to any one of the above embodiments for use in a method for treating or preventing an alpha-dystroglycanopathy in an individual. In some embodiments, the individual has reduced expression of alpha-dystroglycan. In some embodiments, alpha-dystroglycan expressed in the individual has impaired or aberrant O-glycosylation. In some embodiments, the individual has a mutation in a gene selected from the group consisting of: dystroglycan (DAG1), protein O-mannosyltransferase-1 (POMT1), protein O-mannosyltransferase-2 (POMT2), protein O-linked mannose beta1,2-N-acetylglucosylaminyltransferase subunit 1 (POMGNT1), protein O-linked mannose beta1,4-N-acetylglucosylaminyltransferase subunit 2 (POMGNT2), xylosyl- and glucuronyltransferase 1 (LARGE1), xylosyl- and glucuronyltransferase 2 (LARGE2), dolichyl-phosphate mannosyltransferase subunit 1 (DPM1), dolichyl-phosphate mannosyltransferase subunit 2 (DPM2), dolichyl-phosphate mannosyltransferase subunit 3 (DPM3), fukutin, fukutin related protein (FKRP), isprenoid synthase domain containing (ISPD), protein O-mannose kinase (POMK), beta-1,3-N-acetylgalactosaminyltransferase 2 (B3GALNT2), beta-1,4-glucuronyltransferase 1 (B4GAT1), dolichol kinase (DOLK), transmembrane protein 5 (TMEM5), and GDP-mannose pyrophosphorylase B (GMPPB). In some embodiments, the bispecific binding molecule is administered via intravenous infusion. In some embodiments, the bispecific binding molecule is administered via intramuscular, intraperitoneal, or subcutaneous injection. In some embodiments, the individual is a human.

In one embodiment, the disclosure provides a pharmaceutical composition comprising the bispecific binding molecule according to any one of the above embodiments and a pharmaceutically acceptable carrier. In some embodiments, the individual has reduced expression of alpha-dystroglycan. In some embodiments, alpha-dystroglycan expressed in the individual has impaired or aberrant O-glycosylation. In some embodiments, the individual has a mutation in a gene selected from the group consisting of: dystroglycan (DAG1), protein O-mannosyltransferase-1 (POMT1), protein O-mannosyltransferase-2 (POMT2), protein O-linked mannose beta1,2-N-acetylglucosylaminyltransferase subunit 1 (POMGNT1), protein O-linked mannose beta1,4-N-acetylglucosylaminyltransferase subunit 2 (POMGNT2), xylosyl- and glucuronyltransferase 1 (LARGE1), xylosyl- and glucuronyltransferase 2 (LARGE2), dolichyl-phosphate mannosyltransferase subunit 1 (DPM1), dolichyl-phosphate mannosyltransferase subunit 2 (DPM2), dolichyl-phosphate mannosyltransferase subunit 3 (DPM3), fukutin, fukutin related protein (FKRP), isprenoid synthase domain containing (ISPD), protein O-mannose kinase (POMK), beta-1,3-N-acetylgalactosaminyltransferase 2 (B3GALNT2), beta-1,4-glucuronyltransferase 1 (B4GAT1), dolichol kinase (DOLK), transmembrane protein 5 (TMEM5), and GDP-mannose pyrophosphorylase B (GMPPB). In some embodiments, the individual is a human.

Specific embodiments of the invention will become evident from the following more detailed description of certain embodiments and the claims.

It is to be understood that one, some, or all of the properties of the various embodiments described herein may be combined to form other embodiments of the present invention. These and other aspects of the invention will become apparent to one of skill in the art.

### BRIEF DESCRIPTION OF THE DRAWINGS

**FIGS. 1A** and **1B** show the normal protein interactions and functions of the dystrophin associated glycoprotein complex. The O-linked glycans in the mucin-like domain of alpha-dystroglycan serve as receptors for several ligands, including laminin-2 in muscles. **FIG. 1A** shows the dystrophin associated glycoprotein complex, where dystroglycan is O-glycosylated normally. **FIG. 1B** shows laminin-2 in the basal lamina interacting with O-glycoslyated alpha-dystroglycan. Beta-dystroglycan interacts with dystrophin, which in turn is associated with filamentous actin inside the cellular membrane.
**FIG. 1C** shows the etiology of alpha-dystroglycanopathy. In the absence of O-linked glycosylation on alpha-dystroglycan, the binding of laminin-2 and alpha-dystroglycan is lost, resulting in the detachment of the basal lamina from the muscle sarcolemma and leading to membrane damage during exercise and muscular dystrophy.
**FIGS. 1D** and **1E** show the strategy of employing multispecific of bispecific antibodies to treat alpha-dystroglycanopathy. Bispecific and multispecific antibodies specifically recognize and bind to laminin-2 with one or more arms and alpha-dystroglycan (**FIG. 1D**) or beta-dystroglycan (**FIG. 1E**) with one or more other arms, thus restoring the linkage between laminin-2 and dystroglycan for treating alpha-dystroglycanopathies.

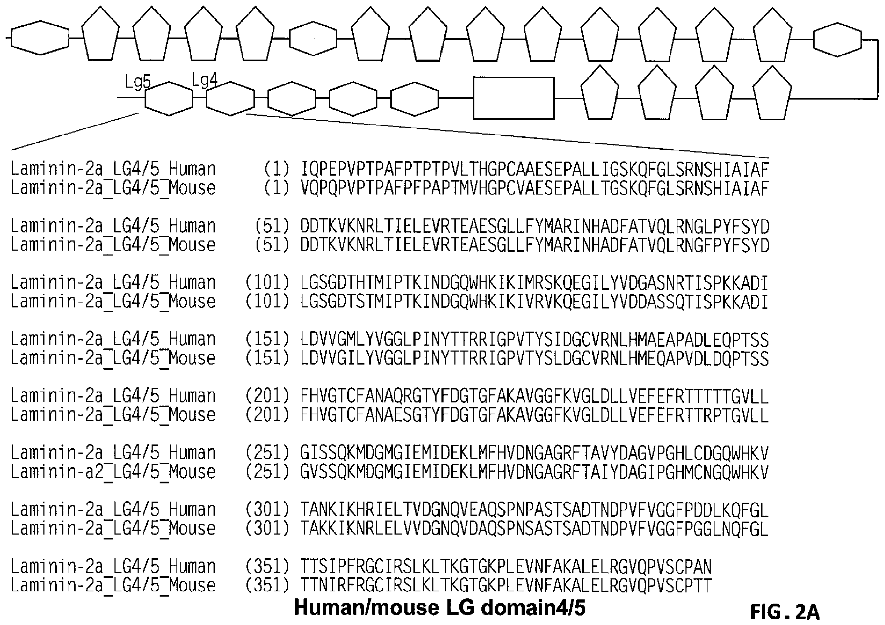

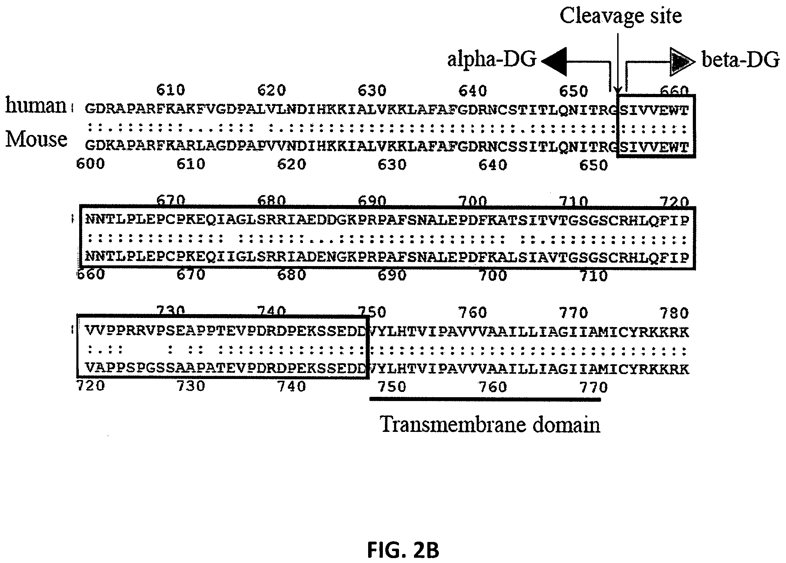

**FIG. 2A** shows a sequence alignment of human and mouse laminin globular (LG)-4/5 domains. The protein sequences of human and mouse LG-5 have significant homology, with 88% identity. Boxed sequences in the alpha-2 subunits of human and murine laminin-2 were used for protein expression and antibody generation. Shown are SEQ ID NOs:305 (upper) and 300 (lower). **FIG. 2B** shows a sequence alignment of human and mouse beta-dystroglycan (DG) extracellular domains. The protein sequences of human and mouse beta-DG extracellular domains have a homolog with 88.4% identity. Boxed sequences in human and murine beta-DGs were used for protein expression and antibody generation. Shown are SEQ ID NOs:303 (upper) and 304 (lower).

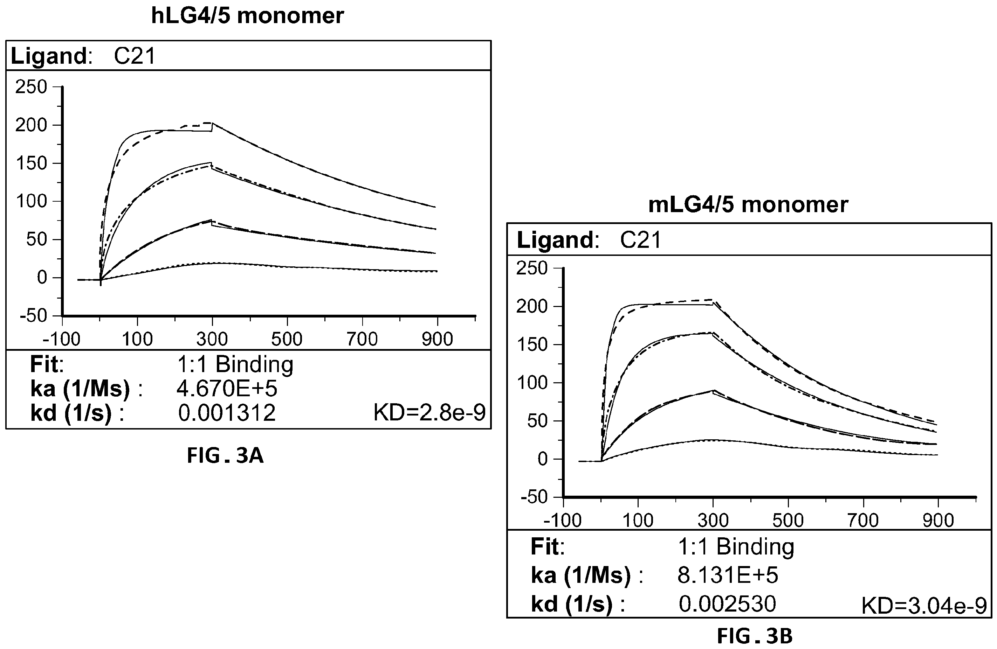

**FIGS. 3A** and **3B** show surface plasmon resonance (Biacore; GE Healthcare) kinetics assay data of the anti-laminin-2 antibody derived from hybridoma clone C21 binding to human (**FIG. 3A**) and mouse (**FIG. 3B**) LG-4/5.

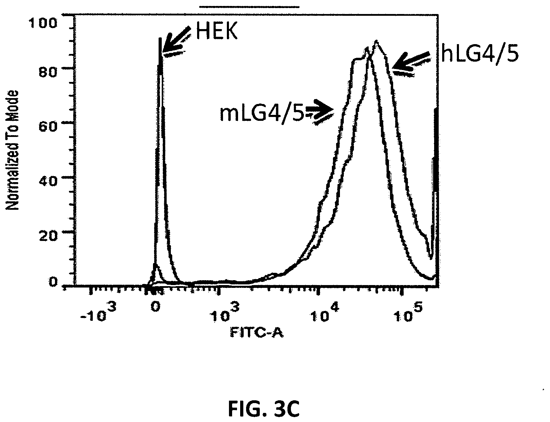

**FIG. 3C** shows fluorescence activated cell sorting (FACS) analysis of the anti-laminin-2 antibody derived from hybridoma clone C21 binding to human and mouse LG-4/5 expressed on HEK293 cells.

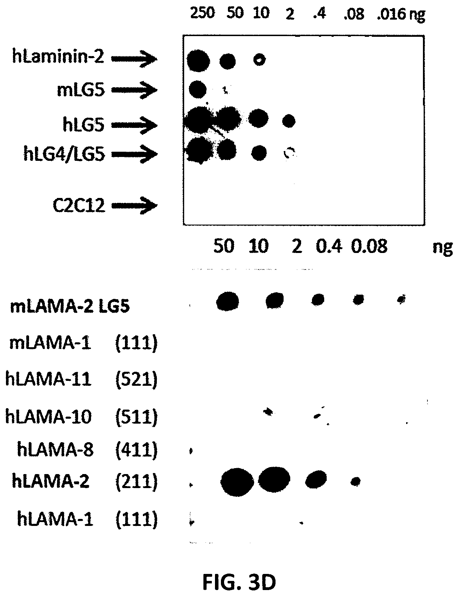

**FIG. 3D** shows a dot blot with various amounts of recombinant human laminin-2, murine LG-5 (mLG5), human LG-5 (hLG5), and human LG-4/5 (hLG4/LG5) dotted onto nitrocellulose then probed with anti-laminin-2 antibody derived from hybridoma clone C21. The amount of laminin-2 in the C2C12 cell lysate was below the detection limit.

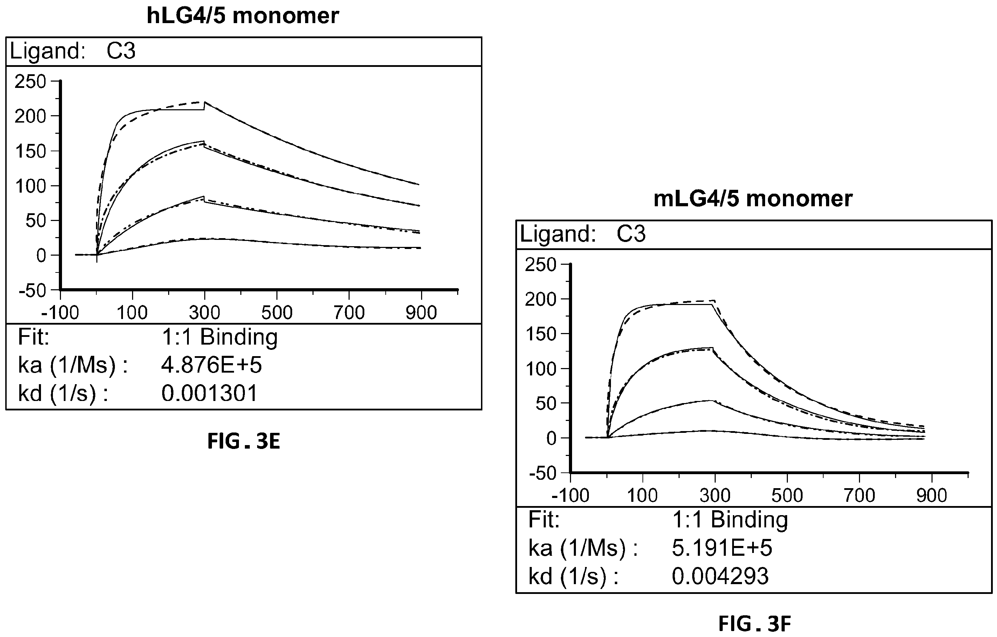

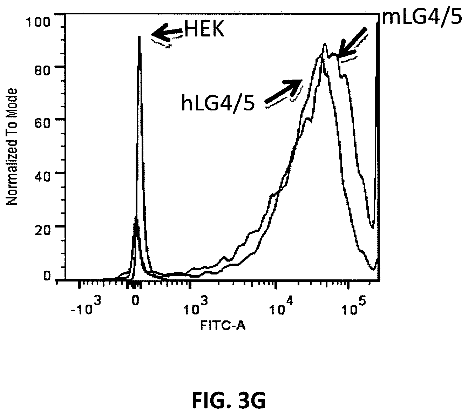

**FIGS. 3E** and **3F** show surface plasmon resonance (Biacore; GE Healthcare) kinetics assay data of the anti-laminin-2 antibody derived from hybridoma clone C3 binding to human (**FIG. 3F**) and mouse (**FIG. 3G**) LG-4/5.
**FIG. 3G** shows fluorescence activated cell sorting (FACS) analysis of the anti-laminin-2 antibody derived from hybridoma clone C3 binding to human and mouse LG-4/5 expressed on HEK293 cells.

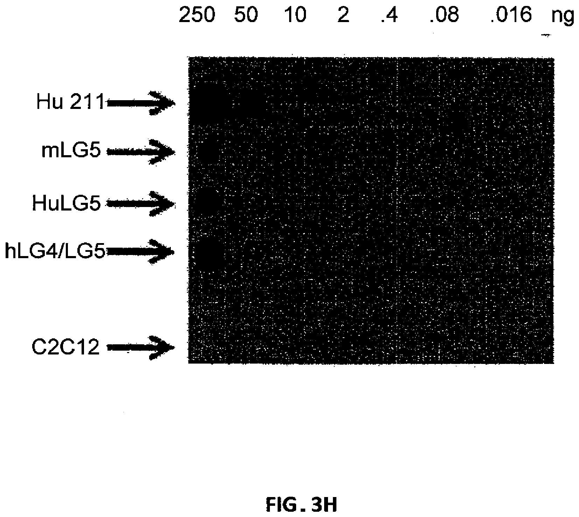

**FIG. 3H** shows a dot blot with various amounts of recombinant human laminin-2 (Hu 211), murine LG-5 (mLG5), human LG-5 (hLG5), and human LG-4/5 (hLG4/LG5) dotted onto nitrocellulose then probed with anti-laminin-2 antibody derived from hybridoma clone C3. The amount of laminin-2 in the C2C12 cell lysate was below the detection limit.

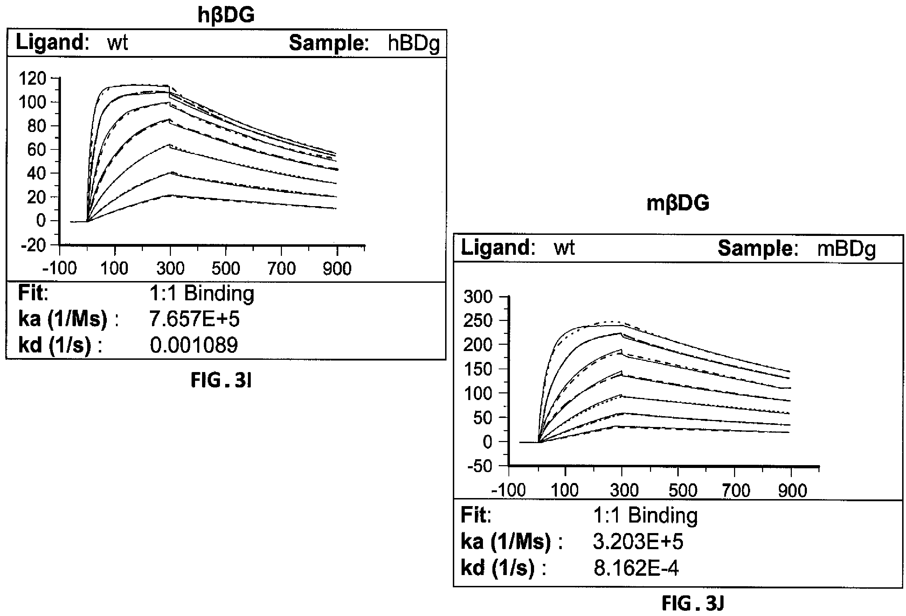

**FIGS. 3I** and **3J** show surface plasmon resonance (Biacore; GE Healthcare) kinetics assay data of the anti-beta-DG antibody derived from hybridoma clone AS30 binding to human (**FIG. 3I**) and mouse (**FIG. 3J**) beta-DG.

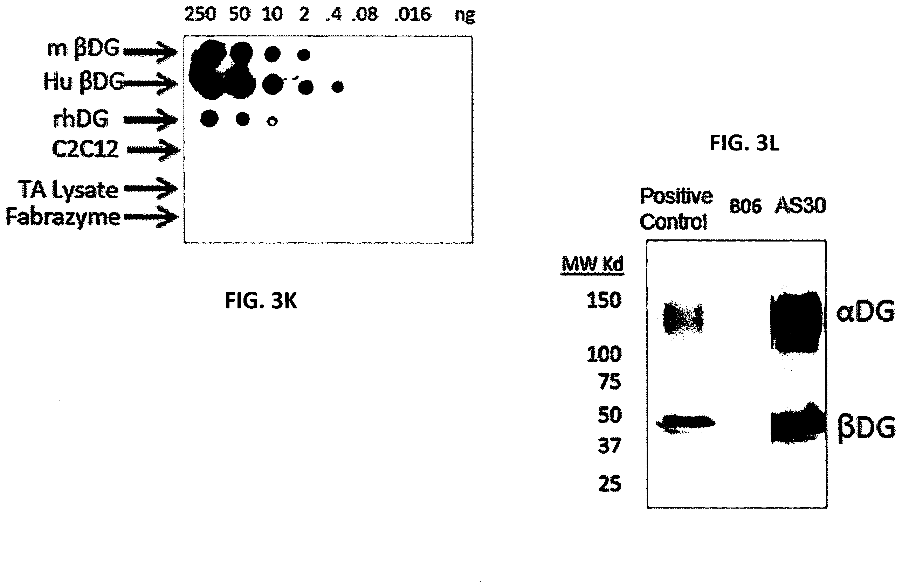

**FIG. 3K** shows a dot blot with various amounts of recombinant murine beta-DG ECD (m βDG), human beta-DG ECD (Hu βDG), or recombinant dystroglycan (rhDG) dotted onto nitrocellulose then probed with anti-beta-DG antibody derived from hybridoma clone AS30. The amount of βDG in C2C12 cell lysate and tibialis anterior muscle cell lysate (TA lysate) were below the detection limit. Fabrazyme (agalsidase beta, Genzyme) was used as a negative control.
**FIG. 3L** shows a Western blot of samples generated via immunoprecipitation of beta-DG from C2C12 cell lysates using anti-beta-DG antibody derived from hybridoma clone AS30. The first lane shows the positive control, the second lane shows the immunoprecipitation sample probed with the anti-beta-DG antibody derived from a phage display clone B06 (which has low affinity to βDG and thus minimal pulldown of βDG and alpha-DG), and the third lane shows the immunoprecipitation sample probed with the high affinity anti-beta-DG antibody derived from hybridoma clone AS30 (where abundant βDG and alpha-DG were immunoprecipitated).

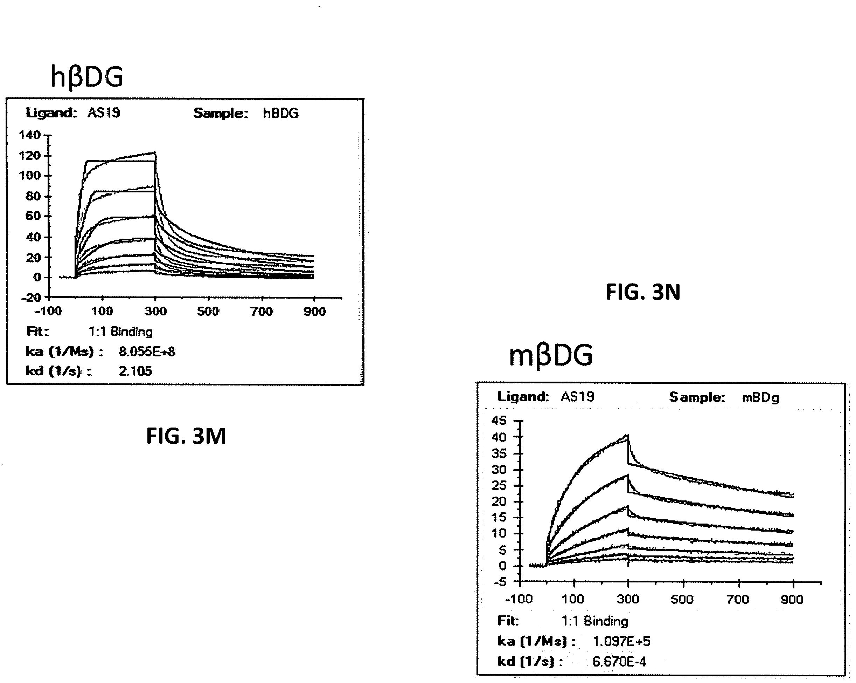

**FIGS. 3M** and **3N** show surface plasmon resonance (Biacore; GE Healthcare) kinetics assay data of the anti-beta-DG antibody derived from hybridoma clone AS19 binding to human (**FIG. 3M**) and mouse (**FIG. 3N**) beta-DG.

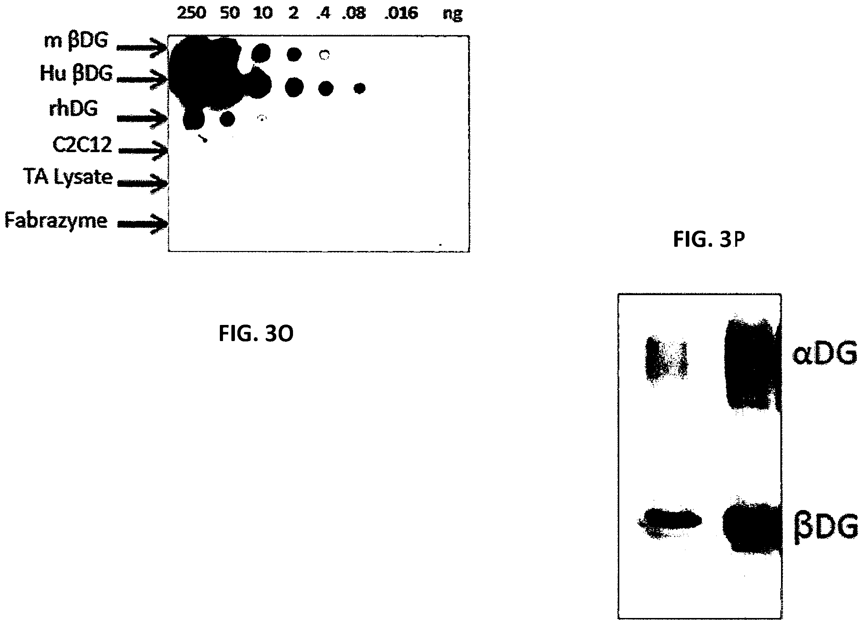

**FIG. 3O** shows a dot blot with various amounts of recombinant murine beta-DG ECD, human beta-DG ECD, or recombinant dystroglycan dotted onto nitrocellulose then probed with anti-beta-DG antibody derived from hybridoma clone AS19. C2C12 cell lysate, tibialislis anterior muscle cell lysate (TA lysate). Fabrazyme (agalsidase beta, Genzyme) was used as the negative control.
**FIG. 3P** shows a Western blot of samples generated via immunoprecipitation of beta-DG from C2C12 cell lysates using anti-beta-DG antibody derived from hybridoma clone AS19. The first lane shows the positive control, the second lane shows the immunoprecipitation sample probed with the anti-beta-DG antibody derived from phage display clone B06 (which has low affinity to βDG and thus minimal pulldown of βDG and alpha-DG), and the third lane shows the immunoprecipitation sample probed with the anti-beta-DG antibody derived from hybridoma clone AS19.

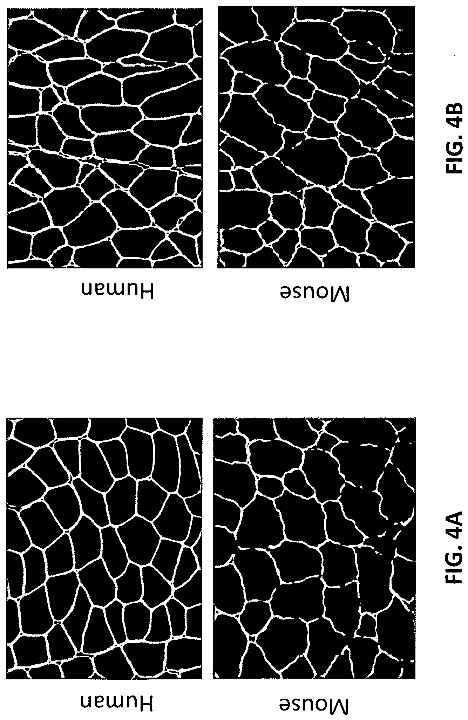

**FIGS. 4A** and **4B** show unfixed frozen human and mouse muscle tissue sections stained with anti-laminin-2 antibody derived from hybridoma clones C21 (**FIG. 4A**) and C3 (**FIG. 4B**), then detected with fluorescently labeled anti-mouse IgG secondary antibody. Secondary antibody only did not reveal any staining (not shown).

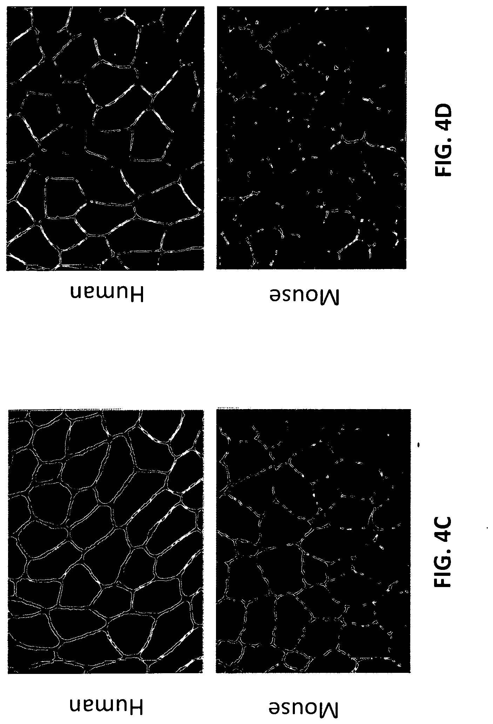

**FIGS. 4C** and **4D** show unfixed frozen human and mouse muscle tissue sections stained with anti-beta-DG antibody derived from hybridoma clones AS30 (**FIG. 4C**) and AS19 (**FIG. 4D**), then detected with fluorescently labeled anti-mouse IgG secondary antibody. Secondary antibody only did not reveal any staining (not shown).

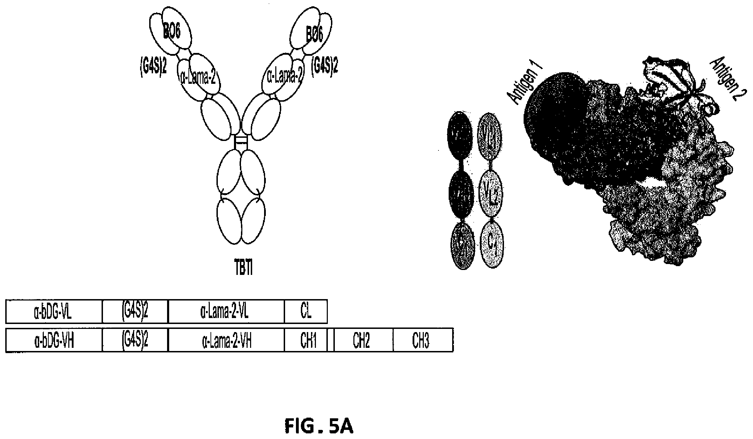

**FIG. 5A** shows a schematic design for tetravalent bispecific tandem IgG format antibodies (TBTI antibodies) that are specific to beta-DG and laminin-2, in accordance with some embodiments.

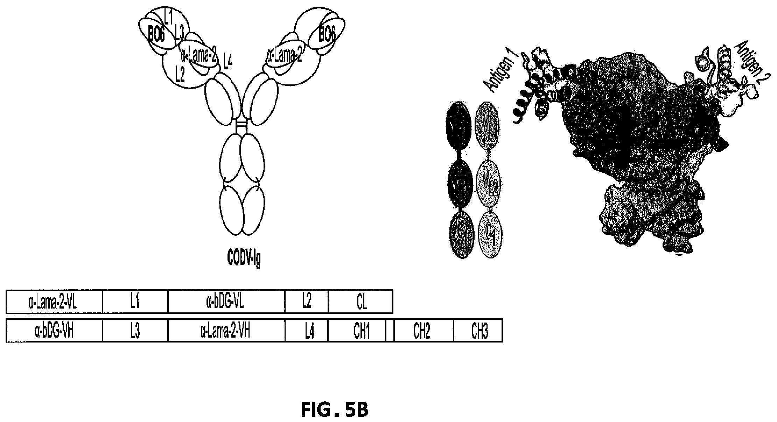

**FIG. 5B** shows a schematic design for crossover dual variable domain IgG format (CODV) bispecific antibodies that are specific to beta-DG and laminin-2, in accordance with some embodiments.

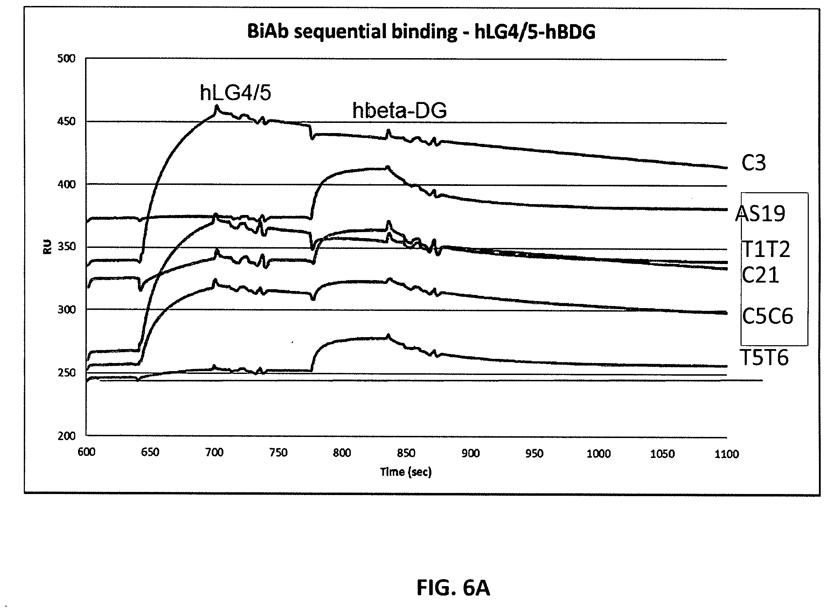

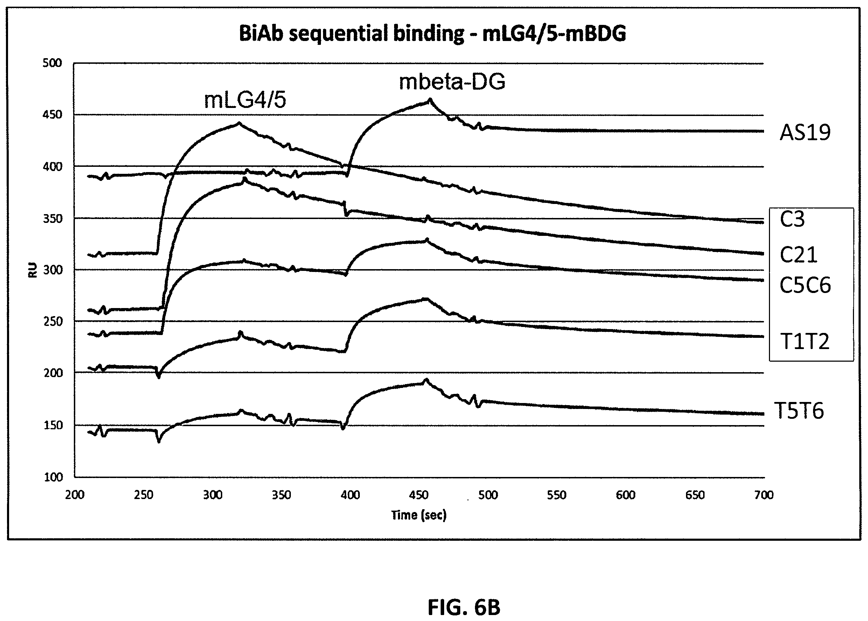

**FIGS. 6A** and **6B** show sequential surface plasmon resonance (Biacore; GE Healthcare) binding analysis data for parent monoclonal antibodies (AS19, C3, and C21) and bispecific antibodies (T1T2, C5C6, and T5T6) for human LG-4/5 and human beta-DG (**FIG. 6A**) or for murine LG-4/5 and murine beta-DG (**FIG. 6B**).

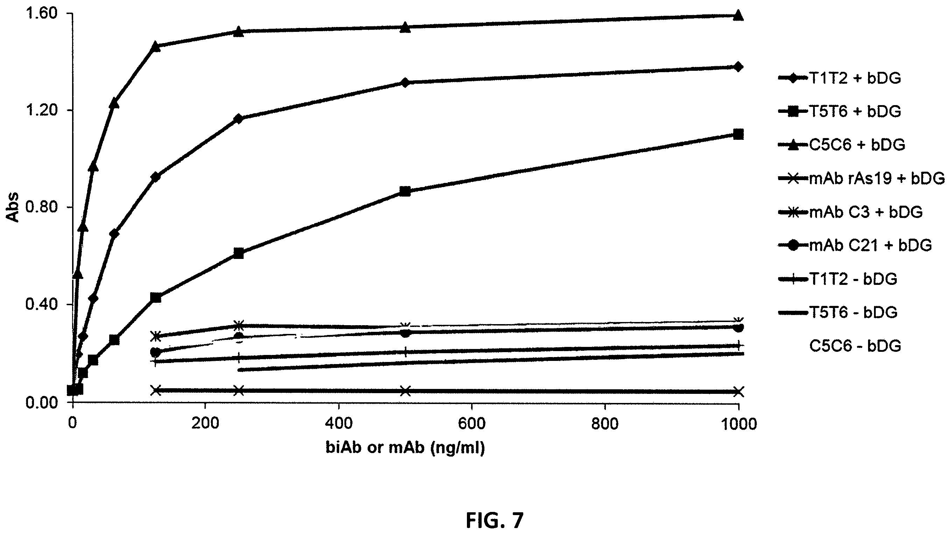

**FIG. 7** shows double deck Sandwich ELISA results for the simultaneous binding of LG-4/5 and beta-DG to bispecific antibodies. Parental monoclonal antibodies (AS19, C3, and C21) with beta-DG added and bispecific antibodies (T1T2, C5C6, or T5T6) with or without beta-DG added were assayed for binding. Only bispecific antibodies T1T2, T5T6, and C5C6 showed significant binding to LG-4/5 and beta-DG simultaneously.

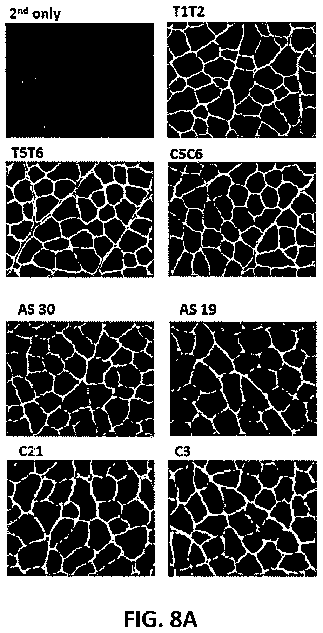

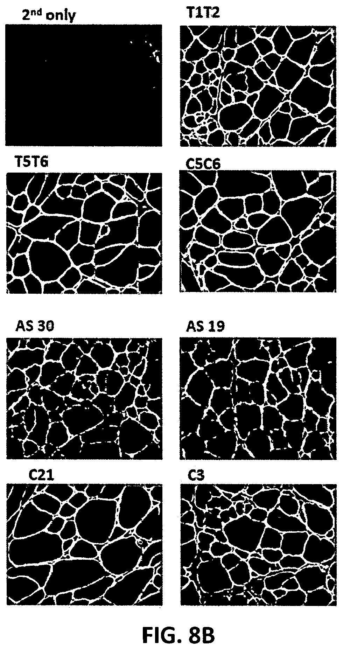

**FIGS. 8A** and **8B** show unfixed frozen sections of wild-type murine muscle tissue (**FIG. 8A**) or LARGE^{myd-3J/GrsrJ} murine muscle tissue (**FIG. 8B**) stained with parental monoclonal antibodies (AS19, C3, and C21) or bispecific antibodies (T1T2, C5C6, or T5T6) and detected with fluorescently labeled anti-mouse IgG secondary antibody.

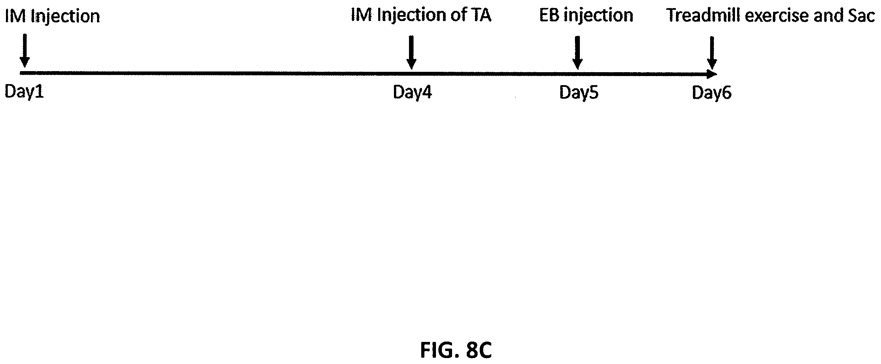

**FIG. 8C** outlines the bispecific intramuscular injection study plan for testing the effect of bispecific antibodies on exercise-induced muscle damage. Bispecific antibodies were injected intramuscularly into tibialis anterior (TA) muscles of LARGE^{myd-3J/GrsrJ} mice at day 1 and day 4 of the experiment. Evans blue dye (EB) was injected at day 5 to track muscle fiber damage. Mice underwent forced treadmill exercise and were sacrificed on day 6.

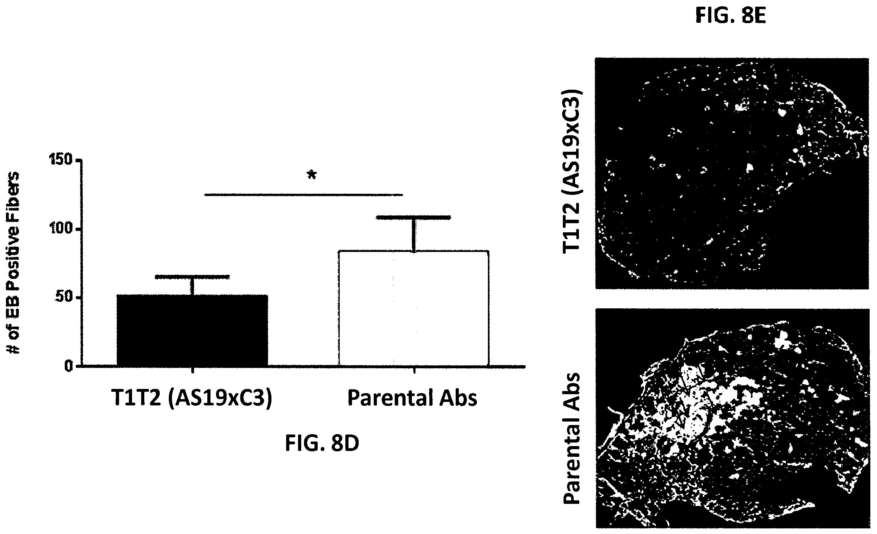

**FIG. 8D** shows the average number of Evans blue positive (i.e. damaged) muscle fibers for treatment with bispecific antibody T1T2 versus a mixture of parental monoclonal antibodies (AS19 and C3). Less damage was seen with bispecific antibody treatment than with the control parental antibody treatment.
**FIG. 8E** shows stained muscle tissue of LARGE^{myd-3J/GrsrJ} mice that were treated with bispecific antibody T1T2 or a mixture of parental monoclonal antibodies (AS19 and C3). Staining with Evans blue dye (arrows) is much more prominent in tissue treated with parental monoclonal antibodies than with bispecific antibody T1T2. Staining of bispecific antibody T1T2 or a mixture of parental monoclonal antibodies (AS19 and C3) using a fluorescent secondary antibody is shown.

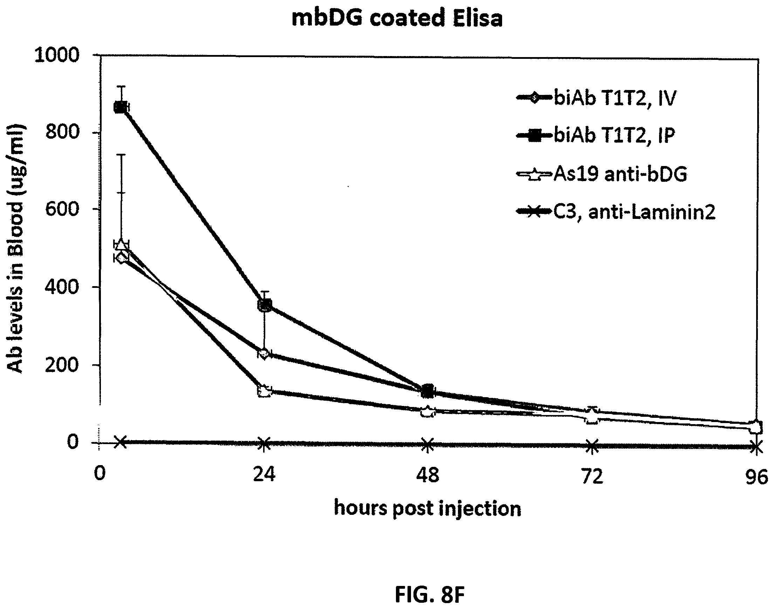

**FIG. 8F** shows the pharmacokinetics and biodistribution of bispecific antibody T1T2 (administered either by tail vein injection or intraperitoneally) and of parental antibodies derived from hybridoma clones AS19 or C3 after systemic delivery. Bispecific antibodies are still detectable in blood 4 days after dosing.

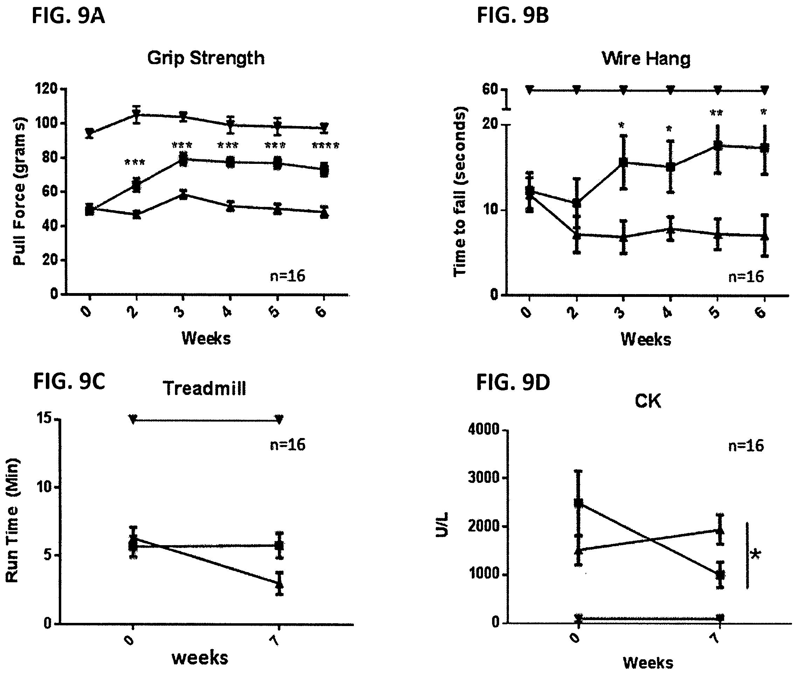

**FIGS. 9A** to **9C** show behavioral testing of wildtype mice (triangles pointing down), LARGE^{myd-3J/GrsrJ} mice treated with control monoclonal parental antibodies (triangles pointing up), and bispecific antibody-treated LARGE^{myd-3J/GrsrJ} mice (squares).
**FIG. 9A** shows that bispecific antibody-treated LARGE^{myd-3J/GrsrJ} mice showed improvement in the grip strength test compared to untreated LARGE^{myd-3J/GrsrJ} mice. **FIG. 9B** shows that bispecific antibody-treated LARGE^{myd-3J/GrsrJ} mice showed improvement in the wire hang test compared to untreated LARGE^{myd-3J/GrsrJ} mice. **FIG. 9C** shows that bispecific antibody-treated LARGE^{myd-3J/GrsrJ} mice showed improvement in the run time test over untreated LARGE^{myd-3J/GrsrJ} mice.
**FIG. 9D** shows that bispecific antibody-treated LARGE^{myd-3J/GrsrJ} mice have decreased creatine kinase levels compared to untreated LARGE^{myd-3J/GrsrJ} mice, suggesting less muscle damage.

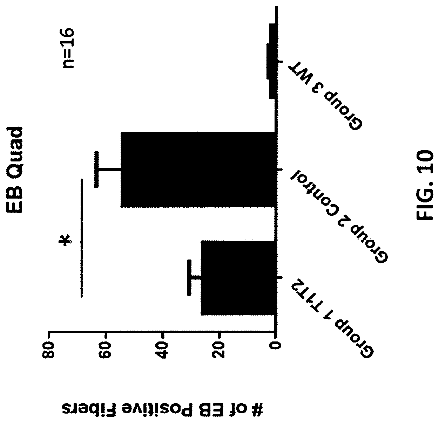

**FIG. 10** shows the average number of Evans blue positive (i.e. damaged) muscle fibers in tissue from LARGE^{myd-3J/GrsrJ} mice treated with bispecific antibody T1T2 (group 1 T1T2) versus a mixture of parental monoclonal antibodies AS19 and C3 (group 2 control). Wildtype untreated mice (group 3 WT) were used as a control. Less damage was seen with bispecific antibody T1T2 treatment than with the control parental antibody treatment in LARGE^{myd-3J/GrsrJ} mice.

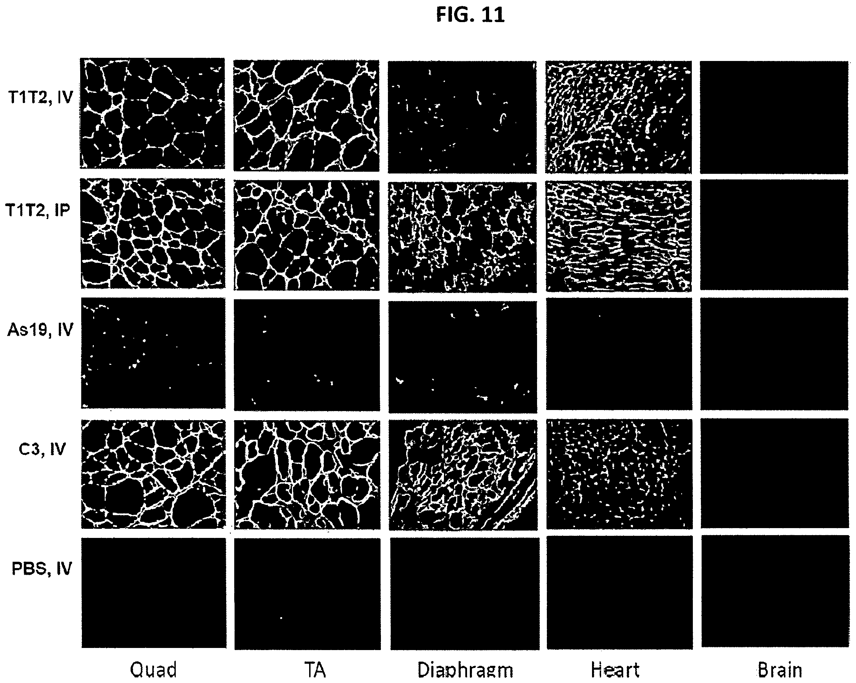

**FIG. 11** shows immunofluorescence staining of LARGE^{myd-3J/GrsrJ} mouse tissues 4 days after injection with bispecific antibody T1T2, parental monoclonal antibody AS19 or C3, or PBS as a negative control. Slides were washed and mounted using Vectashield mounting media with DAPI (Vector Labs). IV: intravenous injection; IP: intraperitoneal injection.

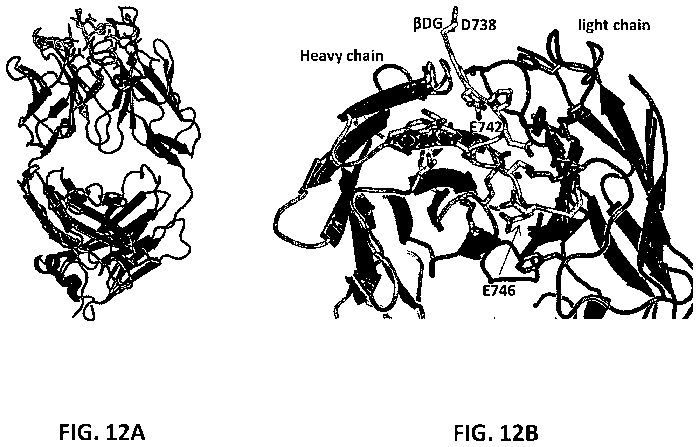

**FIG. 12A** shows the overall structure of the AS30 Fab bound to antigen human beta-DG, with the antigen shown between the heavy chain and light chain. **FIG. 12B** shows a close up view of the interaction between the CDRs of AS30 Fab and antigen beta-DG. Residues involved in the interaction are shown as sticks; arrows in CDRs indicate orientation from N-terminus to C-terminus.

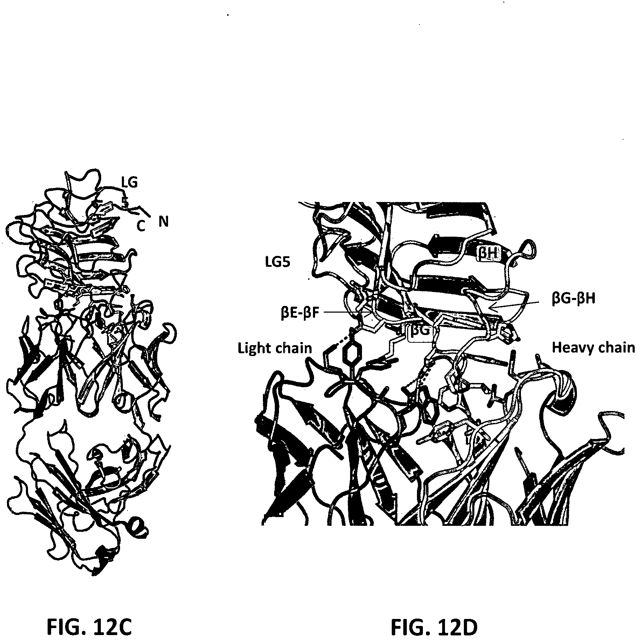

**FIG. 12C** shows the overall structure of the C21 Fab bound to antigen human laminin-2 LG-5 domain, with the antigen shown between the heavy chain and light chain.
**FIG. 12D** shows a close up view of the interaction between the CDRs of C21 Fab and antigen laminin-2 LG-5 domain. Residues involved in the interaction are shown as sticks; arrows in CDRs indicate orientation from N-terminus to C-terminus.

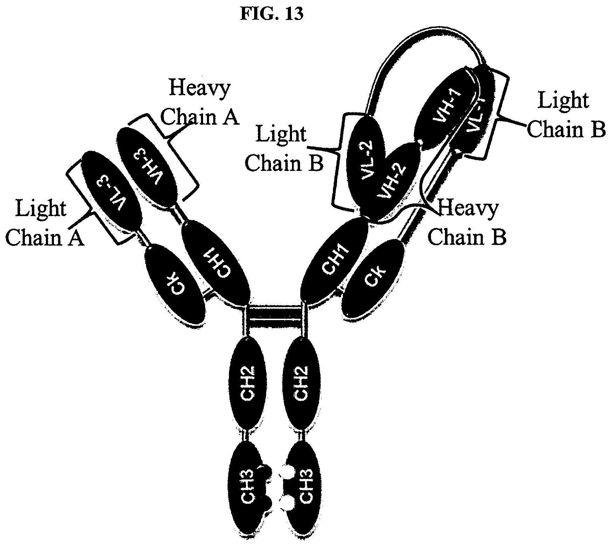

**FIG. 13** shows a schematic representation of a trivalent binding protein (triAb) comprising four polypeptide chains that form three antigen binding sites for binding laminin-2 or beta-DG, in accordance with some embodiments.

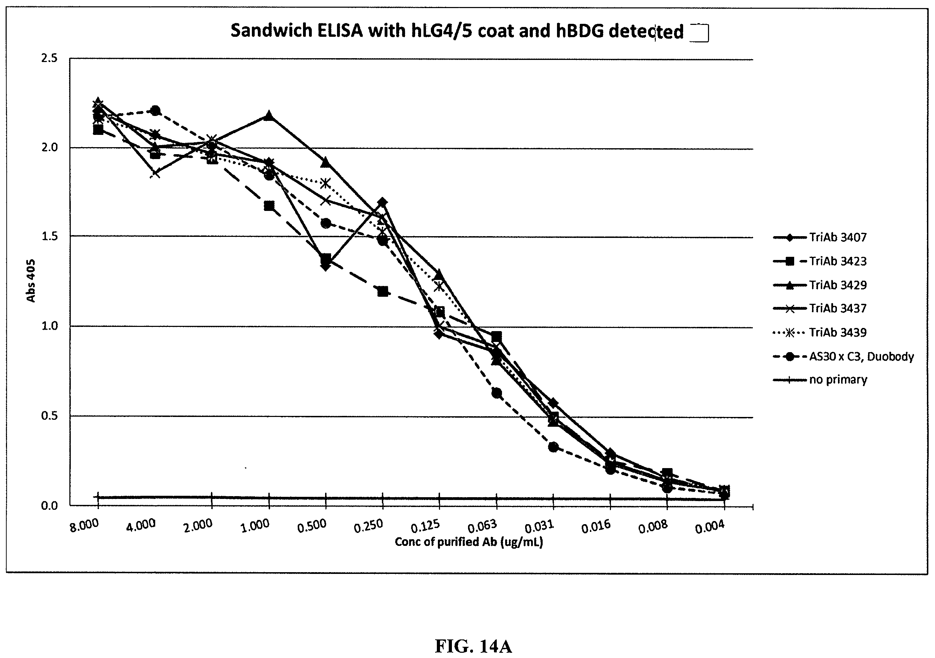

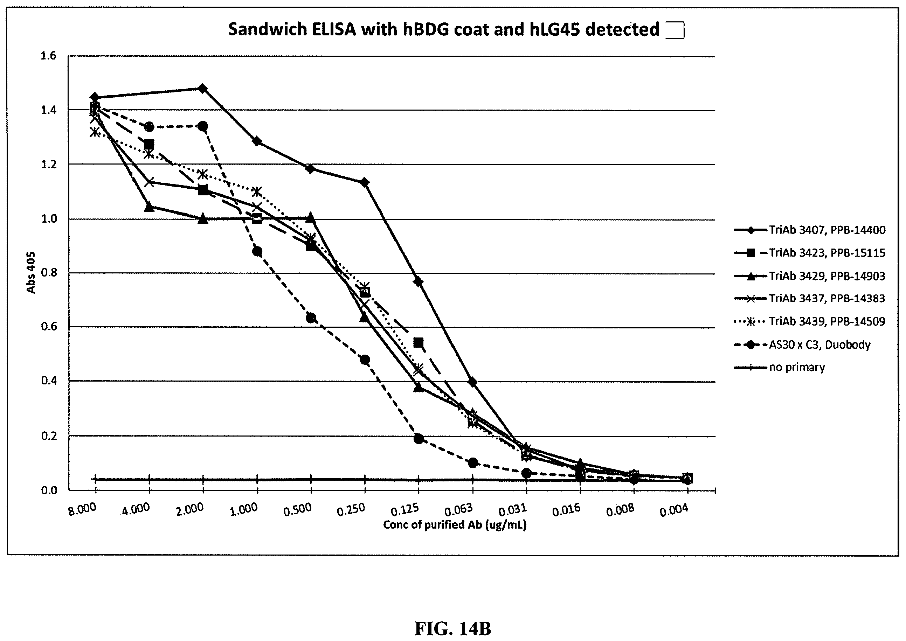

**FIGS. 14A** **&** **14B** show the results of dual binding sandwich ELISA assays examining triAb binding to human LG4/5 or beta-DG. In **FIG. 14A****,** plates were coated with biotinylated N'Avi-HPC4-human LG4/5, and binding to human beta-DG was detected. In **FIG. 14B****,** plates were coated with human-beta DG-HPC4-Avi-C', and binding to human human LG4/5 was detected.

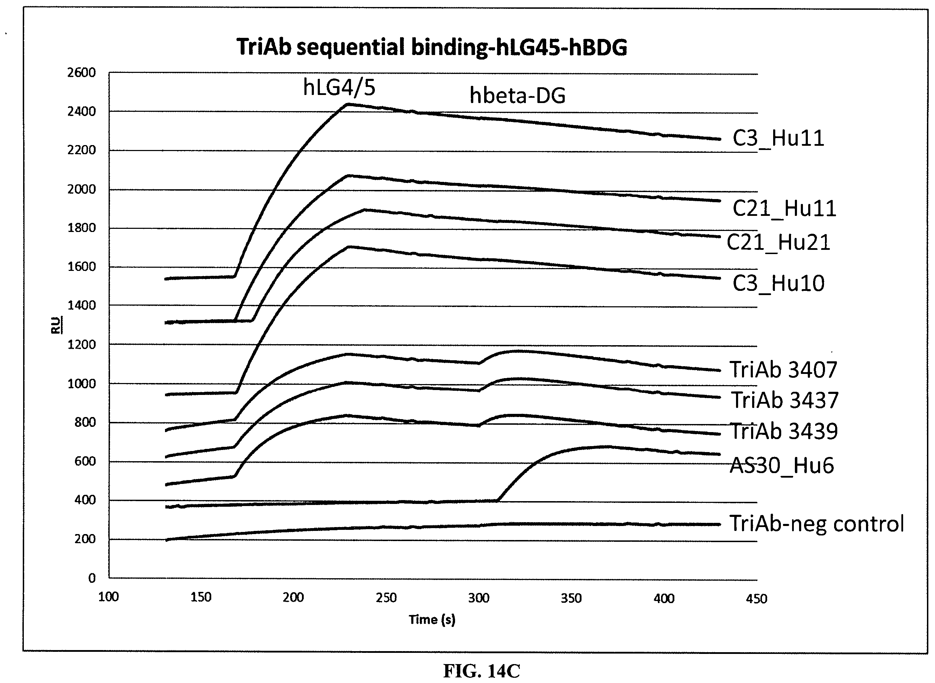

**FIG. 14C** shows sequential binding of triAb 3407, 3437, or 3439 to human LG4/5, then human beta-DG. In contrast, monovalent anti-LG4/5 antibodies C3_Hu11, C21_Hu11, C21_Hu21, and C3_Hu10 only bound LG4/5, while monovalent anti-beta-DG antibody AS30_Hu6 only bound beta-DG, and negative control triAb showed no binding.

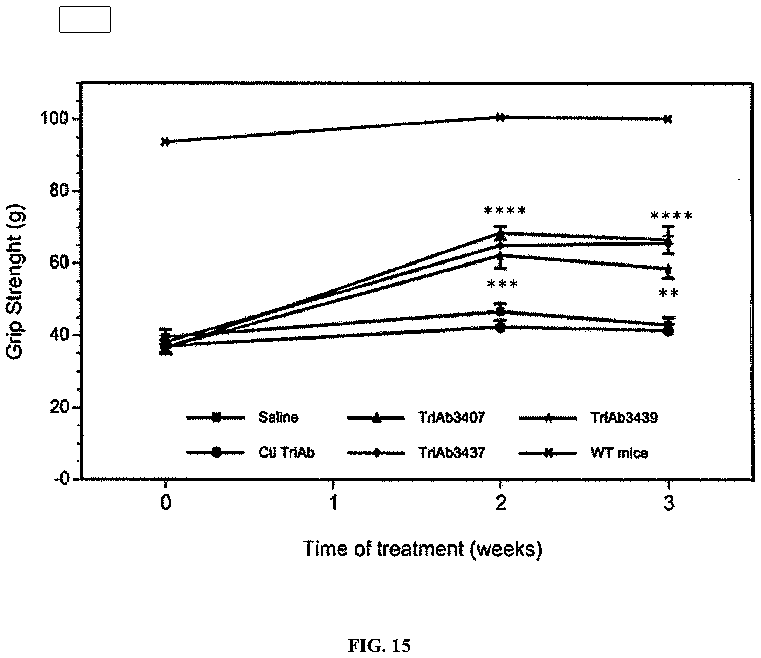

**FIG. 15** shows the effects of treatment with triAb 3407, 3437, or 3439 on muscle function in LARGE^{myd-3J/GrsrJ} mice using a grip strength assay. Administration with indicated triAbs was compared with administration of saline or negative control triAb. Performance of wild-type mice in the assay was also measured.

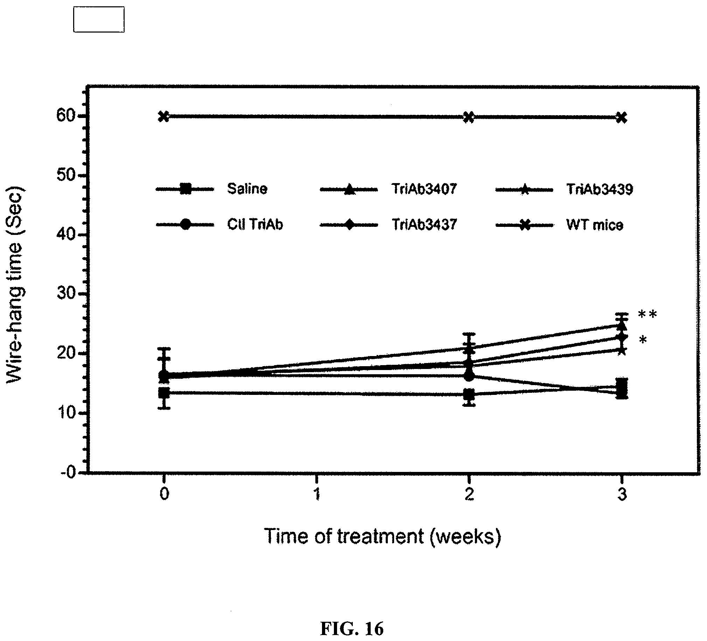

**FIG. 16** shows the effects of treatment with triAb 3407, 3437, or 3439 on muscle function in LARGE^{myd-3J/GrsrJ} mice using a wire-hang assay. Administration with indicated triAbs was compared with administration of saline or negative control triAb. Performance of wild-type mice in the assay was also measured.

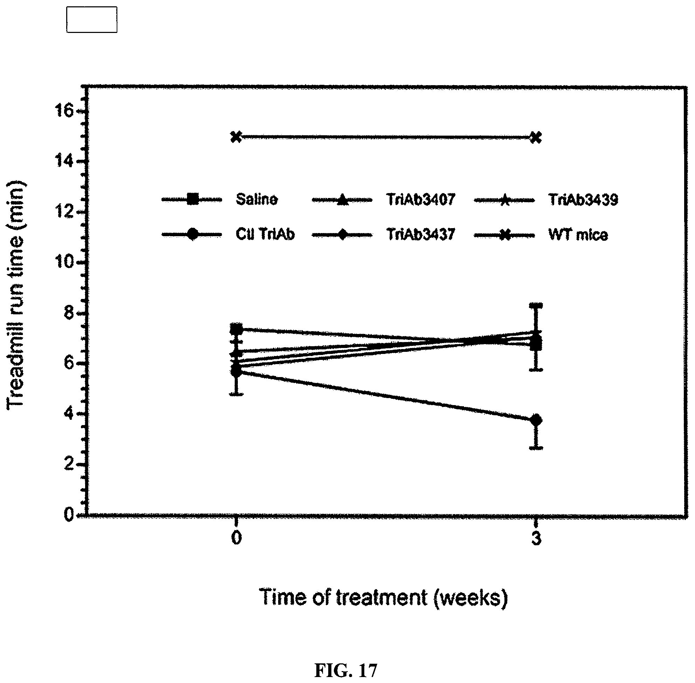

**FIG. 17** shows the effects of treatment with triAb 3407, 3437, or 3439 on muscle function in LARGE^{myd-3J/GrsrJ} mice using a treadmill assay. Administration with indicated triAbs was compared with administration of saline or negative control triAb. Performance of wild-type mice in the assay was also measured.

### DETAILED DESCRIPTION

The present disclosure provides multispecific and bispecific binding molecules comprising a first binding domain that binds an extracellular portion of dystroglycan and a second binding domain that binds laminin-2. In some embodiments, the binding molecules are bispecific and comprise a first binding domain that binds an extracellular portion of dystroglycan and a second binding domain that binds laminin-2. In some embodiments, the binding molecules are multispecific and comprise a first binding domain that binds an extracellular portion of dystroglycan, a second binding domain that binds laminin-2, and one or more additional binding domains that bind one or more additional targets. The present disclosure provides multiple configurations of multispecific/bispecific binding molecules that are able to bind dystroglycan and laminin-2 simultaneously and ameliorate the symptoms of alpha-dystroglycanopathy in an *in vivo* model system.

The following description sets forth exemplary methods, parameters, and the like.

### Definitions

As utilized in accordance with the present disclosure, the following terms, unless otherwise indicated, shall be understood to have the following meanings. Unless otherwise required by context, singular terms shall include pluralities and plural terms shall include the singular.

The term "antigen" or "target antigen" or "antigen target" as used herein refers to a molecule or a portion of a molecule that is capable of being bound by a binding protein, and additionally is capable of being used in an animal to produce antibodies capable of binding to an epitope of that antigen. A target antigen may have one or more epitopes. With respect to each target antigen recognized by a binding protein, the binding protein is capable of competing with an intact antibody that recognizes the target antigen. Exemplary target antigens described herein include dystroglycan (*e.g.,* an extracellular portion thereof) and laminin-2.

The term "epitope" includes any determinant, for example a polypeptide determinant, capable of specifically binding to an immunoglobulin or T-cell receptor. In certain embodiments, epitope determinants include chemically active surface groupings of molecules such as amino acids, sugar side chains, phosphoryl groups, or sulfonyl groups, and, in certain embodiments, may have specific three-dimensional structural characteristics and/or specific charge characteristics. An epitope is a region of an antigen that is bound by an antibody or binding protein. In certain embodiments, a binding protein is said to specifically bind an antigen when it preferentially recognizes its target antigen in a complex mixture of proteins and/or macromolecules. In some embodiments, a binding protein is said to specifically bind an antigen when the equilibrium dissociation constant is ≤ 10⁻⁶ M, for example when the equilibrium dissociation constant is ≤ 10⁻⁹ M, and for example when the dissociation constant is ≤ 10⁻¹⁰ M.

The dissociation constant (K_{D}) of a binding protein can be determined, for example, by surface plasmon resonance. Generally, surface plasmon resonance analysis measures real-time binding interactions between ligand (a target antigen on a biosensor matrix) and analyte (a binding protein in solution) by surface plasmon resonance (SPR) using the BIAcore system (Pharmacia Biosensor; Piscataway, NJ). Surface plasmon analysis can also be performed by immobilizing the analyte (binding protein on a biosensor matrix) and presenting the ligand (target antigen). The term "K_{D}," as used herein refers to the dissociation constant of the interaction between a particular binding protein and a target antigen.

The term "specifically binds" as used herein refers to the ability of a binding protein or an antigen-binding fragment thereof to bind to an antigen containing an epitope with an K_{D} of at least about 1 x 10⁻⁶ M, 1 x 10⁻⁷ M, 1 x 10⁻¹ M, 1 x 10⁻⁹ M, 1 x 10⁻¹⁰ M, 1 x 10⁻¹¹ M, 1 x 10⁻¹² M, or more, and/or to bind to an epitope with an affinity that is at least two-fold greater than its affinity for a nonspecific antigen.

The term "binding protein" as used herein refers to a non-naturally occurring (or recombinant or engineered) molecule that specifically binds to at least one target antigen.

The term "monospecific binding protein" refers to a binding protein that specifically binds to one antigen target.

The term "monovalent binding protein" refers to a binding protein that has one antigen binding site.

The term "bispecific binding protein" refers to a binding protein that specifically binds to two different antigen targets. A bispecific or bifunctional antibody typically is an artificial hybrid antibody having two different heavy chain/light chain pairs and two different binding sites or epitopes. Bispecific antibodies may be produced by a variety of methods including, but not limited to, fusion of hybridomas or linking of F(ab') fragments.

The term "bivalent binding protein" refers to a binding protein that has two binding sites or domains.

The term "polynucleotide" as used herein refers to single-stranded or double-stranded nucleic acid polymers of at least 10 nucleotides in length. In certain embodiments, the nucleotides comprising the polynucleotide can be ribonucleotides or deoxyribonucleotides or a modified form of either type of nucleotide. Such modifications include base modifications such as bromuridine, ribose modifications such as arabinoside and 2',3'-dideoxyribose, and internucleotide linkage modifications such as phosphorothioate, phosphorodithioate, phosphoroselenoate, phosphorodiselenoate, phosphoroanilothioate, phoshoraniladate and phosphoroamidate. The term "polynucleotide" specifically includes single-stranded and double-stranded forms of DNA.

An "isolated polynucleotide" is a polynucleotide of genomic, cDNA, or synthetic origin or some combination thereof, which: (1) is not associated with all or a portion of a polynucleotide in which the isolated polynucleotide is found in nature, (2) is linked to a polynucleotide to which it is not linked in nature, or (3) does not occur in nature as part of a larger sequence.

An "isolated polypeptide" is one that: (1) is free of at least some other polypeptides with which it would normally be found, (2) is essentially free of other polypeptides from the same source, *e.g.,* from the same species, (3) is expressed by a cell from a different species, (4) has been separated from at least about 50 percent of polynucleotides, lipids, carbohydrates, or other materials with which it is associated in nature, (5) is not associated (by covalent or noncovalent interaction) with portions of a polypeptide with which the "isolated polypeptide" is associated in nature, (6) is operably associated (by covalent or noncovalent interaction) with a polypeptide with which it is not associated in nature, or (7) does not occur in nature. Such an isolated polypeptide can be encoded by genomic DNA, cDNA, mRNA or other RNA, of synthetic origin, or any combination thereof. In some embodiments, the isolated polypeptide is substantially free from polypeptides or other contaminants that are found in its natural environment that would interfere with its use (therapeutic, diagnostic, prophylactic, research or otherwise).

Naturally occurring antibodies typically comprise a tetramer. Each such tetramer is typically composed of two identical pairs of polypeptide chains, each pair having one full-length "light" chain (typically having a molecular weight of about 25 kDa) and one full-length "heavy" chain (typically having a molecular weight of about 50-70 kDa). The terms "heavy chain" and "light chain" as used herein refer to any immunoglobulin polypeptide having sufficient variable domain sequence to confer specificity for a target antigen. The amino-terminal portion of each light and heavy chain typically includes a variable domain of about 100 to 110 or more amino acids that typically is responsible for antigen recognition. The carboxy-terminal portion of each chain typically defines a constant domain responsible for effector function. Thus, in a naturally occurring antibody, a full-length heavy chain immunoglobulin polypeptide includes a variable domain (V_{H}) and three constant domains (C_{H1}, C_{H2}, and C_{H3}), wherein the V_{H} domain is at the amino-terminus of the polypeptide and the C_{H3} domain is at the carboxyl-terminus, and a full-length light chain immunoglobulin polypeptide includes a variable domain (V_{L}) and a constant domain (C_{L}), wherein the V_{L} domain is at the amino-terminus of the polypeptide and the C_{L} domain is at the carboxyl-terminus.

Human light chains are typically classified as kappa and lambda light chains, and human heavy chains are typically classified as mu, delta, gamma, alpha, or epsilon, and define the antibody's isotype as IgM, IgD, IgG, IgA, and IgE, respectively. IgG has several subclasses, including, but not limited to, IgG1, IgG2, IgG3, and IgG4. IgM has subclasses including, but not limited to, IgM1 and IgM2. IgA is similarly subdivided into subclasses including, but not limited to, IgA1 and IgA2. Within full-length light and heavy chains, the variable and constant domains typically are joined by a "J" region of about 12 or more amino acids, with the heavy chain also including a "D" region of about 10 more amino acids. *See, e.g.,* FUNDAMENTAL IMMUNOLOGY (Paul, W., ed., Raven Press, 2nd ed., 1989). The variable regions of each light/heavy chain pair typically form an antigen binding site. The variable domains of naturally occurring antibodies typically exhibit the same general structure of relatively conserved framework regions (FR) joined by three hypervariable regions, also called complementarity determining regions or CDRs. The CDRs from the two chains of each pair typically are aligned by the framework regions, which may enable binding to a specific epitope. From the amino-terminus to the carboxyl-terminus, both light and heavy chain variable domains typically comprise the domains FR1, CDR1, FR2, CDR2, FR3, CDR3, and FR4.

The term "CDR set" refers to a group of three CDRs that occur in a single variable region capable of binding the antigen. The exact boundaries of these CDRs have been defined differently according to different systems. The system described by Kabat (Kabat et al., SEQUENCES OF PROTEINS OF IMMUNOLOGICAL INTEREST (National Institutes of Health, Bethesda, Md. (1987) and (1991)) not only provides an unambiguous residue numbering system applicable to any variable region of an antibody, but also provides precise residue boundaries defining the three CDRs. These CDRs may be referred to as Kabat CDRs. Chothia and coworkers (Chothia and Lesk, 1987, J. Mol. Biol. 196: 901-17; Chothia et al., 1989, Nature 342: 877-83) found that certain sub-portions within Kabat CDRs adopt nearly identical peptide backbone conformations, despite having great diversity at the level of amino acid sequence. These sub-portions were designated as L1, L2, and L3 or H1, H2, and H3 where the "L" and the "H" designates the light chain and the heavy chain regions, respectively. These regions may be referred to as Chothia CDRs, which have boundaries that overlap with Kabat CDRs. Other boundaries defining CDRs overlapping with the Kabat CDRs have been described by Padlan, 1995, FASEB J. 9: 133-39; MacCallum, 1996, J. Mol. Biol. 262(5): 732-45; and Lefranc, 2003, Dev. Comp. Immunol. 27: 55-77. Still other CDR boundary definitions may not strictly follow one of the herein systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although certain embodiments use Kabat or Chothia defined CDRs. Identification of predicted CDRs using the amino acid sequence is well known in the field, such as in Martin, A.C. "Protein sequence and structure analysis of antibody variable domains," In Antibody Engineering, Vol. 2. Kontermann R., Dübel S., eds. Springer-Verlag, Berlin, p. 33-51 (2010). The amino acid sequence of the heavy and/or light chain variable domain may be also inspected to identify the sequences of the CDRs by other conventional methods, *e.g.,* by comparison to known amino acid sequences of other heavy and light chain variable regions to determine the regions of sequence hypervariability. The numbered sequences may be aligned by eye, or by employing an alignment program such as one of the CLUSTAL suite of programs, as described in Thompson, 1994, Nucleic Acids Res. 22: 4673-80. Molecular models are conventionally used to correctly delineate framework and CDR regions and thus correct the sequence-based assignments.

The term "Fc" as used herein refers to a molecule comprising the sequence of a non-antigen-binding fragment resulting from digestion of an antibody or produced by other means, whether in monomeric or multimeric form, and can contain the hinge region. In some embodiments, the original immunoglobulin source of the native Fc is of human origin and can be any of the immunoglobulins, for example IgG1 and IgG2. Fc molecules are made up of monomeric polypeptides that can be linked into dimeric or multimeric forms by covalent (*i.e.,* disulfide bonds) and non-covalent association. The number of intermolecular disulfide bonds between monomeric subunits of native Fc molecules ranges from 1 to 4 depending on class (*e.g.,* IgG, IgA, and IgE) or subclass (*e.g.,* IgG1, IgG2, IgG3, IgA1, and IgGA2). One example of a Fc is a disulfide-bonded dimer resulting from papain digestion of an IgG. The term " Fc" as used herein is generic to the monomeric, dimeric, and multimeric forms.

A F(ab) fragment typically includes one light chain and the V_{H} and C_{H1} domains of one heavy chain, wherein the V_{H}-C_{H1} heavy chain portion of the F(ab) fragment cannot form a disulfide bond with another heavy chain polypeptide. As used herein, a F(ab) fragment can also include one light chain containing two variable domains separated by an amino acid linker and one heavy chain containing two variable domains separated by an amino acid linker and a C_{H1} domain.

A F(ab') fragment typically includes one light chain and a portion of one heavy chain that contains more of the constant region (between the C_{H1} and C_{H2} domains), such that an interchain disulfide bond can be formed between two heavy chains to form a F(ab')₂ molecule.

A "recombinant" molecule is one that has been prepared, expressed, created, or isolated by recombinant means.

One embodiment of the disclosure provides binding proteins having biological and immunological specificity to between one and three target antigens. Another embodiment of the disclosure provides nucleic acid molecules comprising nucleotide sequences encoding polypeptide chains that form such binding proteins. Another embodiment of the disclosure provides expression vectors comprising nucleic acid molecules comprising nucleotide sequences encoding polypeptide chains that form such binding proteins. Yet another embodiment of the disclosure provides host cells that express such binding proteins (*i.e.,* comprising nucleic acid molecules or vectors encoding polypeptide chains that form such binding proteins).

The term "swapability" as used herein refers to the interchangeability of variable domains within the binding protein format and with retention of folding and ultimate binding affinity. "Full swapability" refers to the ability to swap the order of both V_{H1} and V_{H2} domains, and therefore the order of V_{L1} and V_{L2} domains, in the polypeptide chain of formula I or the polypeptide chain of formula II (*i.e.,* to reverse the order) while maintaining full functionality of the binding protein as evidenced by the retention of binding affinity. Furthermore, it should be noted that the designations V_{H} and V_{L} refer only to the domain's location on a particular protein chain in the final format. For example, V_{H1} and V_{H2} could be derived from V_{L1} and V_{L2} domains in parent antibodies and placed into the V_{H1} and V_{H2} positions in the binding protein. Likewise, V_{L1} and V_{L2} could be derived from V_{H1} and V_{H2} domains in parent antibodies and placed in the V_{H1} and V_{H2} positions in the binding protein. Thus, the V_{H} and V_{L} designations refer to the present location and not the original location in a parent antibody. V_{H} and V_{L} domains are therefore "swappable."

An "isolated" binding molecule or protein is one that has been identified and separated and/or recovered from a component of its natural environment. Contaminant components of its natural environment are materials that would interfere with diagnostic or therapeutic uses for the binding protein, and may include enzymes, hormones, and other proteinaceous or non-proteinaceous solutes. In some embodiments, the binding molecule or protein will be purified: (1) to greater than 95% by weight of antibody as determined by the Lowry method, and in some embodiments more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or silver stain. Isolated binding molecules or proteins include the molecules/proteins *in situ* within recombinant cells since at least one component of the natural environment will not be present.

The terms "substantially pure" or "substantially purified" as used herein refer to a compound or species that is the predominant species present (*i.e.,* on a molar basis it is more abundant than any other individual species in the composition). In some embodiments, a substantially purified fraction is a composition wherein the species comprises at least about 50% (on a molar basis) of all macromolecular species present. In other embodiments, a substantially pure composition will comprise more than about 80%, 85%, 90%, 95%, or 99% of all macromolar species present in the composition. In still other embodiments, the species is purified to essential homogeneity (contaminant species cannot be detected in the composition by conventional detection methods) wherein the composition consists essentially of a single macromolecular species.

The term "vector" as used herein refers to any molecule (*e.g.,* nucleic acid, plasmid, or virus) that is used to transfer coding information to a host cell. The term "vector" includes a nucleic acid molecule that is capable of transporting another nucleic acid to which it has been linked. One type of vector is a "plasmid," which refers to a circular double-stranded DNA molecule into which additional DNA segments may be inserted. Another type of vector is a viral vector, wherein additional DNA segments may be inserted into the viral genome. Certain vectors are capable of autonomous replication in a host cell into which they are introduced (*e.g.,* bacterial vectors having a bacterial origin of replication and episomal mammalian vectors). Other vectors (*e.g.,* non-episomal mammalian vectors) can be integrated into the genome of a host cell upon introduction into the host cell and thereby are replicated along with the host genome. In addition, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. The terms "plasmid" and "vector" may be used interchangeably herein, as a plasmid is the most commonly used form of vector. However, the disclosure is intended to include other forms of expression vectors, such as viral vectors (*e.g.,* replication defective retroviruses, adenoviruses, and adeno-associated viruses), which serve equivalent functions.

The phrase "recombinant host cell" (or "host cell") as used herein refers to a cell into which a recombinant expression vector has been introduced. A recombinant host cell or host cell is intended to refer not only to the particular subject cell, but also to the progeny of such a cell. Because certain modifications may occur in succeeding generations due to either mutation or environmental influences, such progeny may not, in fact, be identical to the parent cell, but such cells are still included within the scope of the term "host cell" as used herein. A wide variety of host cell expression systems can be used to express the binding proteins, including bacterial, yeast, baculoviral, and mammalian expression systems (as well as phage display expression systems). An example of a suitable bacterial expression vector is pUC19. To express a binding protein recombinantly, a host cell is transformed or transfected with one or more recombinant expression vectors carrying DNA fragments encoding the polypeptide chains of the binding protein such that the polypeptide chains are expressed in the host cell and secreted into the medium in which the host cells are cultured, from which medium the binding protein can be recovered.

The term "transformation" as used herein refers to a change in a cell's genetic characteristics, and a cell has been transformed when it has been modified to contain a new

DNA. For example, a cell is transformed where it is genetically modified from its native state. Following transformation, the transforming DNA may recombine with that of the cell by physically integrating into a chromosome of the cell, or may be maintained transiently as an episomal element without being replicated, or may replicate independently as a plasmid. A cell is considered to have been stably transformed when the DNA is replicated with the division of the cell. The term "transfection" as used herein refers to the uptake of foreign or exogenous DNA by a cell, and a cell has been "transfected" when the exogenous DNA has been introduced inside the cell membrane. A number of transfection techniques are well known in the art. Such techniques can be used to introduce one or more exogenous DNA molecules into suitable host cells.

The term "naturally occurring" as used herein and applied to an object refers to the fact that the object can be found in nature and has not been manipulated by man. For example, a polynucleotide or polypeptide that is present in an organism (including viruses) that can be isolated from a source in nature and that has not been intentionally modified by man is naturally-occurring. Similarly, "non-naturally occurring" as used herein refers to an object that is not found in nature or that has been structurally modified or synthesized by man.

As used herein, the twenty conventional amino acids and their abbreviations follow conventional usage. Stereoisomers (*e.g.,* D-amino acids) of the twenty conventional amino acids; unnatural amino acids and analogs such as α-, α-disubstituted amino acids, N-alkyl amino acids, lactic acid, and other unconventional amino acids may also be suitable components for the polypeptide chains of the binding proteins. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (*e.g.,* 4-hydroxyproline). In the polypeptide notation used herein, the left-hand direction is the amino terminal direction and the right-hand direction is the carboxyl-terminal direction, in accordance with standard usage and convention.

Naturally occurring residues may be divided into classes based on common side chain properties:
(1) hydrophobic: Met, Ala, Val, Leu, Ile, Phe, Trp, Tyr, Pro;
(2) polar hydrophilic: Arg, Asn, Asp, Gln, Glu, His, Lys, Ser, Thr ;
(3) aliphatic: Ala, Gly, Ile, Leu, Val, Pro;
(4) aliphatic hydrophobic: Ala, Ile, Leu, Val, Pro;
(5) neutral hydrophilic: Cys, Ser, Thr, Asn, Gln;
(6) acidic: Asp, Glu;
(7) basic: His, Lys, Arg;
(8) residues that influence chain orientation: Gly, Pro;
(9) aromatic: His, Trp, Tyr, Phe; and
(10) aromatic hydrophobic: Phe, Trp, Tyr.

Conservative amino acid substitutions may involve exchange of a member of one of these classes with another member of the same class. Non-conservative substitutions may involve the exchange of a member of one of these classes for a member from another class.

A skilled artisan will be able to determine suitable variants of the polypeptide chains of the binding proteins using well-known techniques. For example, one skilled in the art may identify suitable areas of a polypeptide chain that may be changed without destroying activity by targeting regions not believed to be important for activity. Alternatively, one skilled in the art can identify residues and portions of the molecules that are conserved among similar polypeptides. In addition, even areas that may be important for biological activity or for structure may be subject to conservative amino acid substitutions without destroying the biological activity or without adversely affecting the polypeptide structure.

The term "patient" (the terms "individual" and "subject" can be used interchangeably herein) as used herein includes human and animal (*e.g.,* mammals, including but not limited to dogs, cats, and other domestic pets; horses, cows, goats, rabbits, buffalos, and other livestock; and research animals such as non-human primates, mice, etc.) subjects.

In some embodiments, the terms "treatment" or "treat" as used herein refer to therapeutic treatment, *e.g.*, reducing or mitigating the severity or presence of one or more symptoms.

In other embodiments, the term "prevent" as used herein can refer to prophylactic or preventative measures, *e.g.*, preventing or delaying onset of one or more symptoms, for instance in an individual at risk for developing a pathological condition described herein.

The terms "pharmaceutical composition" or "therapeutic composition" as used herein refer to a compound or composition capable of inducing a desired therapeutic effect when properly administered to a patient.

The term "pharmaceutically acceptable carrier" or "physiologically acceptable carrier" as used herein refers to one or more formulation materials suitable for accomplishing or enhancing the delivery of a binding protein.

The terms "effective amount" and "therapeutically effective amount" when used in reference to a pharmaceutical composition comprising one or more binding proteins refer to an amount or dosage sufficient to produce a desired therapeutic result. More specifically, a therapeutically effective amount is an amount of a binding protein sufficient to inhibit, for some period of time, one or more of the clinically defined pathological processes associated with the condition being treated. The effective amount may vary depending on the specific binding protein that is being used, and also depends on a variety of factors and conditions related to the patient being treated and the severity of the disorder. For example, if the binding protein is to be administered *in vivo,* factors such as the age, weight, and health of the patient as well as dose response curves and toxicity data obtained in preclinical animal work would be among those factors considered. The determination of an effective amount or therapeutically effective amount of a given pharmaceutical composition is well within the ability of those skilled in the art.

One embodiment of the disclosure provides a pharmaceutical composition comprising a pharmaceutically acceptable carrier and a therapeutically effective amount of a binding molecule.

### Binding Molecules

Certain aspects of the present disclosure relate to multispecific binding molecules comprising one or more binding domain(s) that bind an extracellular portion of dystroglycan and one or more binding domain(s) that bind laminin-2. The multispecific binding molecule is a multispecific binding protein comprising one or more polypeptide chains. The multispecific binding molecule is a bivalent or tetravalent bispecific binding molecule comprising two or four antigen binding sites. The multispecific binding molecule is a trivalent multispecific binding molecule comprising three antigen binding sites. The terms "binding domain" and "binding site" are used interchangeably herein.

Various formats and configurations for multispecific binding proteins are known in the art and suitable for use as described herein. Descriptions of exemplary formats are provided below. Any of the formats and optional features thereof described in International Publication No. WO2017/180913 may be used in the binding proteins (*e.g.,* multispecific binding proteins) described herein.

### Multispecific, trivalent binding proteins

The present disclosure provides a multispecific binding protein. The multispecific binding protein is a trivalent binding protein comprising three antigen binding sites and collectively targeting two or more target antigens. The multispecific binding protein comprises at least a first binding domain that binds an extracellular portion of dystroglycan and at least a second binding domain that binds laminin-2, wherein the multispecific binding molecule is a multispecific binding protein comprising one or more polypeptide chains, wherein the binding protein comprises four polypeptide chains, wherein a first polypeptide chain comprises a structure represented by the formula:

V_{L2}-L₁-V_{L1}-L₂-C_{L} [I]

and a second polypeptide chain comprises a structure represented by the formula:

V_{H1}-L₃-V_{H2}-L₄-C_{H1}-hinge-C_{H2}-C_{H3} [II]

and a third polypeptide chain comprises a structure represented by the formula:

V_{H3}-C_{H1}-hinge-C_{H2}-C_{H3} [III]

and a fourth polypeptide chain comprises a structure represented by the formula:

V_{L3}-C_{L} [IV]

where:
V_{L1} is a first immunoglobulin light chain variable domain;
V_{L2} is a second immunoglobulin light chain variable domain;
V_{L3} is a third immunoglobulin light chain variable domain;
V_{H1} is a first immunoglobulin heavy chain variable domain;
V_{H2} is a second immunoglobulin heavy chain variable domain;
V_{H3} is a third immunoglobulin heavy chain variable domain;
C_{L} is an immunoglobulin light chain constant domain;
C_{H1} is an immunoglobulin C_{H1} heavy chain constant domain;
C_{H2} is an immunoglobulin C_{H2} heavy chain constant domain;
C_{H3} is an immunoglobulin C_{H3} heavy chain constant domain;
hinge is an immunoglobulin hinge region connecting the C_{H1} and C_{H2} domains; and
L₁, L₂, L₃ and L₄ are amino acid linkers,

wherein the polypeptide of formula I and the polypeptide of formula II form a cross-over light chain-heavy chain pair; and
wherein V_{H1} and V_{L1} form an antigen binding site, wherein V_{H2} and V_{L2} form an antigen binding site, and wherein V_{H3} and V_{L3} form an antigen binding site for a total of three antigen binding sites, and wherein the three antigen binding sites comprise two antigen binding sites that bind the extracellular portion of dystroglycan and one antigen binding site that binds laminin-2 or one antigen binding site that binds the extracellular portion of dystroglycan and two antigen binding sites that bind laminin-2.

The polypeptide of formula I and the polypeptide of formula II form a cross-over light chain-heavy chain pair. V_{H1} and V_{L1} form an antigen binding site, V_{H2} and V_{L2} form an antigen binding site, and V_{H3} and V_{L3} form an antigen binding site for a total of three antigen binding sites. The three antigen binding sites comprise two antigen binding sites that bind the extracellular portion of dystroglycan and one antigen binding site that binds laminin-2, or one antigen binding site that binds the extracellular portion of dystroglycan and two antigen binding sites that bind laminin-2 and two antigen binding sites that bind the extracellular portion of dystroglycan and one antigen binding site that binds laminin-2.

In some embodiments, the two antigen binding sites that bind laminin-2 bind different epitopes of laminin-2. In some embodiments, the two antigen binding sites that bind laminin-2 bind the same or overlapping epitopes of laminin-2. In some embodiments, V_{H1} and V_{L1} form a first antigen binding site that binds laminin-2, V_{H2} and V_{L2} form a second antigen binding site that binds laminin-2, and V_{H3} and V_{L3} form a third antigen binding site that binds the extracellular portion of dystroglycan.

In some embodiments, the two antigen binding sites that bind the extracellular portion of dystroglycan bind different epitopes of the extracellular portion of dystroglycan. In some embodiments, the two antigen binding sites that bind the extracellular portion of dystroglycan bind the same or overlapping epitopes of the extracellular portion of dystroglycan. In some embodiments, V_{H1} and V_{L1} form a first antigen binding site that binds the extracellular portion of dystroglycan, V_{H2} and V_{L2} form a second antigen binding site that binds the extracellular portion of dystroglycan, and V_{H3} and V_{L3} form a third antigen binding site that binds laminin-2.

Any of the antigen binding sites described herein may find use in the binding proteins (*e.g.,* multispecific binding proteins) described herein, *e.g.,* including but not limited to, binding proteins comprising polypeptide(s) according to formulas I, II, III, and/or IV described *supra.*

In some embodiments, a binding protein (*e.g.,* multispecific binding protein) of the present disclosure comprises: a heavy chain variable domain (VH) comprising a CDR-H1 comprising the sequence of GFTFTDSV (SEQ ID NO:316), a CDR-H2 comprising the sequence of IYPGSGNF (SEQ ID NO:318), and a CDR-H3 comprising the sequence of AMRRSS (SEQ ID NO:320); and a light chain variable domain (VL) comprising a CDR-L1 comprising the sequence of QTIVHSNSKTY (SEQ ID NO:332), a CDR-L2 comprising the sequence of KVS (SEQ ID NO:334), and a CDR-L3 comprising the sequence of FQGSHVPLT (SEQ ID NO:336). In some embodiments, the VH and VL domains form an antigen binding site (*e.g.,* V_{H1} and V_{L1}, V_{H2} and V_{L2}, or V_{H3} and V_{L3}) that binds the extracellular portion of dystroglycan. In some embodiments, a binding protein (*e.g.,* multispecific binding protein) of the present disclosure comprises 1, 2, 3, 4, 5, or 6 CDR sequences of antibody AS30SS_Hu6 or AS30SS_Hu9 as shown in Table A2.

In some embodiments, the VH and/or VL domain are humanized. In some embodiments, a binding protein (*e.g.,* multispecific binding protein) of the present disclosure comprises a VH domain sequence having at least 90%, 91%, 92%, 93%, 94%, 95%, 96%, 97%, 98%, 99%, or 100% sequence identity to the amino acid sequence of SEQ ID NO: 314 and/ or a VL domain sequence having at least 90%, 91%, 92%, 93%, 94%, 95%, 96%, 97%, 98%, 99%, or 100% sequence identity to the amino acid sequence of SEQ ID NO:330. In some embodiments, a binding protein (*e.g.,* multispecific binding protein) of the present disclosure comprises a VH domain sequence having at least 90%, 91%, 92%, 93%, 94%, 95%, 96%, 97%, 98%, 99%, or 100% sequence identity to the amino acid sequence of SEQ ID NO: 346 and/ or a VL domain sequence having at least 90%, 91%, 92%, 93%, 94%, 95%, 96%, 97%, 98%, 99%, or 100% sequence identity to the amino acid sequence of SEQ ID NO:362. In some embodiments, a binding protein (*e.g.,* multispecific binding protein) of the present disclosure comprises a VH and/or VL domain sequence of antibody AS30SS_Hu6 or AS30SS_Hu9 as shown in Table D2. In some embodiments, a binding protein (*e.g.,* multispecific binding protein) of the present disclosure comprises a VH domain encoded by the polynucleotide sequence of SEQ ID NO:306 and/or a VL domain encoded by the polynucleotide sequence of SEQ ID NO:322. In some embodiments, a binding protein (*e.g.,* multispecific binding protein) of the present disclosure comprises a VH domain encoded by the polynucleotide sequence of SEQ ID NO:338 and/or a VL domain encoded by the polynucleotide sequence of SEQ ID NO:354.

In some embodiments, a binding protein (*e.g.,* multispecific binding protein) of the present disclosure comprises: a heavy chain variable domain (VH) comprising a CDR-H1 comprising the sequence of GFTFSSYT (SEQ ID NO:380), a CDR-H2 comprising the sequence of ISSSGSNT (SEQ ID NO:382), and a CDR-H3 comprising the sequence of ARFDYGSSLDS (SEQ ID NO:384); and a light chain variable domain (VL) comprising a CDR-L1 comprising the sequence of QSISNN (SEQ ID NO:396), a CDR-L2 comprising the sequence of YAS (SEQ ID NO:398), and a CDR-L3 comprising the sequence of QQSKSWPRT (SEQ ID NO:400). In some embodiments, the VH and VL domains form an antigen binding site (*e.g.,* V_{H1} and V_{L1}, V_{H2} and V_{L2}, or V_{H3} and V_{L3}) that binds laminin-2. In some embodiments, a binding protein (*e.g.,* multispecific binding protein) of the present disclosure comprises 1, 2, 3, 4, 5, or 6 CDR sequences of antibody C3_Hu10 as shown in Table A2.

In some embodiments, the VH and/or VL domain are humanized. In some embodiments, a binding protein (*e.g.,* multispecific binding protein) of the present disclosure comprises a VH domain sequence having at least 90%, 91%, 92%, 93%, 94%, 95%, 96%, 97%, 98%, 99%, or 100% sequence identity to the amino acid sequence of SEQ ID NO: 378 and/ or a VL domain sequence having at least 90%, 91%, 92%, 93%, 94%, 95%, 96%, 97%, 98%, 99%, or 100% sequence identity to the amino acid sequence of SEQ ID NO:394. In some embodiments, a binding protein (*e.g.,* multispecific binding protein) of the present disclosure comprises a VH and/or VL domain sequence of antibody C3_Hu10 as shown in Table D2. In some embodiments, a binding protein (*e.g.,* multispecific binding protein) of the present disclosure comprises a VH domain encoded by the polynucleotide sequence of SEQ ID NO:370 and/or a VL domain encoded by the polynucleotide sequence of SEQ ID NO:386.

In some embodiments, a binding protein (*e.g.,* multispecific binding protein) of the present disclosure comprises: a heavy chain variable domain (VH) comprising a CDR-H1 comprising the sequence of GFTFSSYT (SEQ ID NO:380), a CDR-H2 comprising the sequence of ISSSGSNT (SEQ ID NO:382), and a CDR-H3 comprising the sequence of ARFDYGSSLDS (SEQ ID NO:384); and a light chain variable domain (VL) comprising a CDR-L1 comprising the sequence of QSIGNN (SEQ ID NO:428), a CDR-L2 comprising the sequence of YAS (SEQ ID NO:398), and a CDR-L3 comprising the sequence of QQSKSWPRT (SEQ ID NO:400). In some embodiments, the VH and VL domains form an antigen binding site (*e.g.,* V_{H1} and V_{L1}, V_{H2} and V_{L2}, or V_{H3} and V_{L3}) that binds laminin-2. In some embodiments, a binding protein (*e.g.,* multispecific binding protein) of the present disclosure comprises 1, 2, 3, 4, 5, or 6 CDR sequences of antibody C3_Hu11 as shown in Table A2.

In some embodiments, the VH and/or VL domain are humanized. In some embodiments, a binding protein (*e.g.,* multispecific binding protein) of the present disclosure comprises a VH domain sequence having at least 90%, 91%, 92%, 93%, 94%, 95%, 96%, 97%, 98%, 99%, or 100% sequence identity to the amino acid sequence of SEQ ID NO: 410 and/ or a VL domain sequence having at least 90%, 91%, 92%, 93%, 94%, 95%, 96%, 97%, 98%, 99%, or 100% sequence identity to the amino acid sequence of SEQ ID NO:426. In some embodiments, a binding protein (*e.g*., multispecific binding protein) of the present disclosure comprises a VH and/or VL domain sequence of antibody C3_Hu11 as shown in Table D2. In some embodiments, a binding protein (*e.g*., multispecific binding protein) of the present disclosure comprises a VH domain encoded by the polynucleotide sequence of SEQ ID NO:402 and/or a VL domain encoded by the polynucleotide sequence of SEQ ID NO:418.

In some embodiments, a binding protein (*e.g*., multispecific binding protein) of the present disclosure comprises: a heavy chain variable domain (VH) comprising a CDR-H1 comprising the sequence of GFTFSSYT (SEQ ID NO:444), a CDR-H2 comprising the sequence of ISSSGSNT (SEQ ID NO:446), and a CDR-H3 comprising the sequence of ARFDYGSSLDS (SEQ ID NO:448); and a light chain variable domain (VL) comprising a CDR-L1 comprising the sequence of QSISNY (SEQ ID NO:460), a CDR-L2 comprising the sequence of YAS (SEQ ID NO:462), and a CDR-L3 comprising the sequence of QQSKSWPRT (SEQ ID NO:464). In some embodiments, the VH and VL domains form an antigen binding site (*e.g.*, V_{H1} and V_{L1}, V_{H2} and V_{L2}, or V_{H3} and V_{L3}) that binds laminin-2. In some embodiments, a binding protein (*e.g*., multispecific binding protein) of the present disclosure comprises 1, 2, 3, 4, 5, or 6 CDR sequences of antibody C21_Hu11 as shown in Table A2.

In some embodiments, the VH and/or VL domain are humanized. In some embodiments, a binding protein (*e.g*., multispecific binding protein) of the present disclosure comprises a VH domain sequence having at least 90%, 91%, 92%, 93%, 94%, 95%, 96%, 97%, 98%, 99%, or 100% sequence identity to the amino acid sequence of SEQ ID NO: 442 and/ or a VL domain sequence having at least 90%, 91%, 92%, 93%, 94%, 95%, 96%, 97%, 98%, 99%, or 100% sequence identity to the amino acid sequence of SEQ ID NO:458. In some embodiments, a binding protein (*e.g*., multispecific binding protein) of the present disclosure comprises a VH and/or VL domain sequence of antibody C21_Hu11 as shown in Table D2. In some embodiments, a binding protein (*e.g*., multispecific binding protein) of the present disclosure comprises a VH domain encoded by the polynucleotide sequence of SEQ ID NO:434 and/or a VL domain encoded by the polynucleotide sequence of SEQ ID NO:450.

In some embodiments, a binding protein (*e.g*., multispecific binding protein) of the present disclosure comprises: a heavy chain variable domain (VH) comprising a CDR-H1 comprising the sequence of GFTFSSYT (SEQ ID NO:444), a CDR-H2 comprising the sequence of ISSSGDNT (SEQ ID NO:478), and a CDR-H3 comprising the sequence of ARFDYGSSLDS (SEQ ID NO:448); and a light chain variable domain (VL) comprising a CDR-L1 comprising the sequence of QSISNY (SEQ ID NO:460), a CDR-L2 comprising the sequence of YAS (SEQ ID NO:462), and a CDR-L3 comprising the sequence of QQSKSWPRT (SEQ ID NO:464). In some embodiments, the VH and VL domains form an antigen binding site (*e.g.*, V_{H1} and V_{L1}, V_{H2} and V_{L2}, or V_{H3} and V_{L3}) that binds laminin-2. In some embodiments, a binding protein (*e.g*., multispecific binding protein) of the present disclosure comprises 1, 2, 3, 4, 5, or 6 CDR sequences of antibody C21_Hu21 as shown in Table A2.

In some embodiments, the VH and/or VL domain are humanized. In some embodiments, a binding protein (*e.g*., multispecific binding protein) of the present disclosure comprises a VH domain sequence having at least 90%, 91%, 92%, 93%, 94%, 95%, 96%, 97%, 98%, 99%, or 100% sequence identity to the amino acid sequence of SEQ ID NO: 474 and/ or a VL domain sequence having at least 90%, 91%, 92%, 93%, 94%, 95%, 96%, 97%, 98%, 99%, or 100% sequence identity to the amino acid sequence of SEQ ID NO:490. In some embodiments, a binding protein (*e.g*., multispecific binding protein) of the present disclosure comprises a VH and/or VL domain sequence of antibody C21_Hu21 as shown in Table D2. In some embodiments, a binding protein (*e.g*., multispecific binding protein) of the present disclosure comprises a VH domain encoded by the polynucleotide sequence of SEQ ID NO:466 and/or a VL domain encoded by the polynucleotide sequence of SEQ ID NO:482.

In some embodiments, V_{H1} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L1} comprises a CDR-L1 comprising the sequence of SEQ ID NO:396, a CDR-L2 comprising the sequence of SEQ ID NO:398, and a CDR-L3 comprising the sequence of SEQ ID NO:400; V_{H2} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L1} comprises a CDR-L1 comprising the sequence of SEQ ID NO:396, a CDR-L2 comprising the sequence of SEQ ID NO:398, and a CDR-L3 comprising the sequence of SEQ ID NO:400; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:378, and V_{L1} comprises the sequence of SEQ ID NO:394; V_{H2} comprises the sequence of SEQ ID NO:378, and V_{L2} comprises the sequence of SEQ ID NO:394; and V_{H3} comprises the sequence of SEQ ID NO:314, and V_{L3} comprises the sequence of SEQ ID NO:330. In some embodiments, a binding protein (*e.g*., multispecific binding protein) of the present disclosure comprises a first polypeptide chain that comprises the sequence of SEQ ID NO:500, a second polypeptide chain that comprises the sequence of SEQ ID NO:498, a third polypeptide chain that comprises the sequence of SEQ ID NO:499, and a fourth polypeptide chain that comprises the sequence of SEQ ID NO:501. In some embodiments, the binding protein comprises 1, 2, 3, or 4 polypeptide chains of triAb 3407, *e.g.,* as shown in Table I2 or I4.

In some embodiments, V_{H1} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L1} comprises a CDR-L1 comprising the sequence of SEQ ID NO:396, a CDR-L2 comprising the sequence of SEQ ID NO:398, and a CDR-L3 comprising the sequence of SEQ ID NO:400; V_{H2} comprises a CDR-H1 comprising the sequence of SEQ ID NO:444, a CDR-H2 comprising the sequence of SEQ ID NO:446, and a CDR-H3 comprising the sequence of SEQ ID NO:448, and V_{L2} comprises a CDR-L1 comprising the sequence of SEQ ID NO:460, a CDR-L2 comprising the sequence of SEQ ID NO:462, and a CDR-L3 comprising the sequence of SEQ ID NO:464; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:378, and V_{L1} comprises the sequence of SEQ ID NO:394; V_{H2} comprises the sequence of SEQ ID NO:442, and V_{L2} comprises the sequence of SEQ ID NO:458; and V_{H3} comprises the sequence of SEQ ID NO:314, and V_{L3} comprises the sequence of SEQ ID NO:330. In some embodiments, a binding protein (*e.g*., multispecific binding protein) of the present disclosure comprises a first polypeptide chain that comprises the sequence of SEQ ID NO:504, a second polypeptide chain that comprises the sequence of SEQ ID NO:502, a third polypeptide chain that comprises the sequence of SEQ ID NO:503, and a fourth polypeptide chain that comprises the sequence of SEQ ID NO:505. In some embodiments, the binding protein comprises 1, 2, 3, or 4 polypeptide chains of triAb 3423, *e.g.,* as shown in Table I2 or I4.

In some embodiments, V_{H1} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L1} comprises a CDR-L1 comprising the sequence of SEQ ID NO:428, a CDR-L2 comprising the sequence of SEQ ID NO:398, and a CDR-L3 comprising the sequence of SEQ ID NO:400; V_{H2} comprises a CDR-H1 comprising the sequence of SEQ ID NO:444, a CDR-H2 comprising the sequence of SEQ ID NO:478, and a CDR-H3 comprising the sequence of SEQ ID NO:448, and V_{L2} comprises a CDR-L1 comprising the sequence of SEQ ID NO:460, a CDR-L2 comprising the sequence of SEQ ID NO:462, and a CDR-L3 comprising the sequence of SEQ ID NO:464; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:410, and V_{L1} comprises the sequence of SEQ ID NO:426; V_{H2} comprises the sequence of SEQ ID NO:474, and V_{L2} comprises the sequence of SEQ ID NO:490; and V_{H3} comprises the sequence of SEQ ID NO:314, and V_{L3} comprises the sequence of SEQ ID NO:330. In some embodiments, a binding protein (*e.g*., multispecific binding protein) of the present disclosure comprises a first polypeptide chain that comprises the sequence of SEQ ID NO:508, a second polypeptide chain that comprises the sequence of SEQ ID NO:506, a third polypeptide chain that comprises the sequence of SEQ ID NO:507, and a fourth polypeptide chain that comprises the sequence of SEQ ID NO:509. In some embodiments, the binding protein comprises 1, 2, 3, or 4 polypeptide chains of triAb 3429, *e.g.,* as shown in Table I2 or I4.

In some embodiments, V_{H1} comprises a CDR-H1 comprising the sequence of SEQ ID NO:444, a CDR-H2 comprising the sequence of SEQ ID NO:446, and a CDR-H3 comprising the sequence of SEQ ID NO:448, and V_{L1} comprises a CDR-L1 comprising the sequence of SEQ ID NO:460, a CDR-L2 comprising the sequence of SEQ ID NO:462, and a CDR-L3 comprising the sequence of SEQ ID NO:464; V_{H2} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L2} comprises a CDR-L1 comprising the sequence of SEQ ID NO:428, a CDR-L2 comprising the sequence of SEQ ID NO:398, and a CDR-L3 comprising the sequence of SEQ ID NO:400; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:442, and V_{L1} comprises the sequence of SEQ ID NO:458; V_{H2} comprises the sequence of SEQ ID NO:410, and V_{L2} comprises the sequence of SEQ ID NO:426; and V_{H3} comprises the sequence of SEQ ID NO:314, and V_{L3} comprises the sequence of SEQ ID NO:330. In some embodiments, a binding protein (*e.g*., multispecific binding protein) of the present disclosure comprises a first polypeptide chain that comprises the sequence of SEQ ID NO:512, a second polypeptide chain that comprises the sequence of SEQ ID NO:510, a third polypeptide chain that comprises the sequence of SEQ ID NO:511, and a fourth polypeptide chain that comprises the sequence of SEQ ID NO:513. In some embodiments, the binding protein comprises 1, 2, 3, or 4 polypeptide chains of triAb 3437, *e.g.,* as shown in Table I2 or I4.

In some embodiments, V_{H1} comprises a CDR-H1 comprising the sequence of SEQ ID NO:444, a CDR-H2 comprising the sequence of SEQ ID NO:478, and a CDR-H3 comprising the sequence of SEQ ID NO:448, and V_{L1} comprises a CDR-L1 comprising the sequence of SEQ ID NO:460, a CDR-L2 comprising the sequence of SEQ ID NO:462, and a CDR-L3 comprising the sequence of SEQ ID NO:464; V_{H2} comprises a CDR-H1 comprising the sequence of SEQ ID NO:380, a CDR-H2 comprising the sequence of SEQ ID NO:382, and a CDR-H3 comprising the sequence of SEQ ID NO:384, and V_{L2} comprises a CDR-L1 comprising the sequence of SEQ ID NO:396, a CDR-L2 comprising the sequence of SEQ ID NO:398, and a CDR-L3 comprising the sequence of SEQ ID NO:400; and V_{H3} comprises a CDR-H1 comprising the sequence of SEQ ID NO:316, a CDR-H2 comprising the sequence of SEQ ID NO:318, and a CDR-H3 comprising the sequence of SEQ ID NO:320, and V_{L3} comprises a CDR-L1 comprising the sequence of SEQ ID NO:332, a CDR-L2 comprising the sequence of SEQ ID NO:334, and a CDR-L3 comprising the sequence of SEQ ID NO:336. In some embodiments, V_{H1} comprises the sequence of SEQ ID NO:474, and V_{L1} comprises the sequence of SEQ ID NO:490; V_{H2} comprises the sequence of SEQ ID NO:378, and V_{L2} comprises the sequence of SEQ ID NO:394; and V_{H3} comprises the sequence of SEQ ID NO:314, and V_{L3} comprises the sequence of SEQ ID NO:330. In some embodiments, a binding protein (*e.g*., multispecific binding protein) of the present disclosure comprises a first polypeptide chain that comprises the sequence of SEQ ID NO:516, a second polypeptide chain that comprises the sequence of SEQ ID NO:514, a third polypeptide chain that comprises the sequence of SEQ ID NO:515, and a fourth polypeptide chain that comprises the sequence of SEQ ID NO:517. In some embodiments, the binding protein comprises 1, 2, 3, or 4 polypeptide chains of triAb 3439, *e.g.,* as shown in Table I2 or I4.

### Bispecific binding proteins

The present disclosure provides a bispecific binding protein. The bispecific binding protein is a bivalent binding protein comprising two antigen binding sites and collectively targeting two target antigens or a tetravalent binding protein comprising four antigen binding sites and collectively targeting two target antigens.

In some embodiments, the bispecific binding molecule comprises a first binding domain that binds to an extracellular portion of dystroglycan, wherein the first binding domain comprises a first immunoglobulin heavy chain variable domain (V_{H1}) and a first immunoglobulin light chain variable domain (V_{L1}), and a second binding domain that binds to laminin-2, wherein the second binding domain comprises a second immunoglobulin heavy chain variable domain (V_{H2}) and a second immunoglobulin light chain variable domain (V_{L2}). In some embodiments, the bispecific binding molecule is a bispecific binding protein, such as a bispecific antibody.

The present disclosure provides a bispecific binding molecule comprising a first binding domain that binds an extracellular portion of dystroglycan and a second binding domain that binds laminin-2, wherein wherein the bispecific binding molecule is a bispecific binding protein comprising one or more polypeptide chains, wherein the bispecific binding molecule comprises four polypeptide chains that form four antigen binding sites, wherein two polypeptide chains comprise a structure represented by the formula:

V_{L1}-L₁-V_{L2}-L₂-C_{L} [I]

and two polypeptide chains comprise a structure represented by the formula:

V_{H2}-L₃-V_{H1}-L₄-C_{H1}-hinge-C_{H2}-C_{H3} [II]

wherein:
V_{L1} is a first immunoglobulin light chain variable domain;
V_{L2} is a second immunoglobulin light chain variable domain;
V_{H1} is a first immunoglobulin heavy chain variable domain;
V_{H2} is a second immunoglobulin heavy chain variable domain;
C_{L} is an immunoglobulin light chain constant domain;
C_{H1} is an immunoglobulin C_{H1} heavy chain constant domain;
C_{H2} is an immunoglobulin C_{H2} heavy chain constant domain;
C_{H3} is an immunoglobulin C_{H3} heavy chain constant domain;
hinge is an immunoglobulin hinge region connecting the C_{H1} and C_{H2} domains; and
L₁, L₂, L₃, and L₄ are amino acid linkers;
wherein the V_{H1} and V_{L1} domains form a V_{H1}/V_{L1} binding pair, and wherein the V_{H2} and V_{L2} domains form a V_{H2}/V_{L2} binding pair.

In some embodiments, formulae I and II describe the arrangement of domains within the respective polypeptide chains in order from N-terminus to C-terminus. In some embodiments, one or more of the polypeptide chains can comprise additional sequence(s), *e.g.,* at the N-terminal or C-terminal ends.

For exemplary descriptions of this format, *see, e.g.,* International Pub. No. WO2012/135345, US Pat. No. 9,221,917, and EP Pat. No. EP2691416B1.

In some embodiments, the bispecific binding molecule comprises two polypeptide chains according to formula II comprising the sequence of SEQ ID NO:530 and two polypeptide chains according to formula I comprising the sequence of SEQ ID NO:531. In some embodiments, the bispecific binding molecule comprises two polypeptide chains according to formula II comprising the sequence of SEQ ID NO:532 and two polypeptide chains according to formula I comprising the sequence of SEQ ID NO:533. In some embodiments, the binding protein comprises two polypeptide chains shown for AS30_Hu9xC3_Hu11 CODV or AS30_Hu9xC21_Hu21 CODV in Table I3 or 14. In some embodiments, the binding protein comprises a variable domain comprising 1, 2, 3, 4, 5, or 6 CDR sequences shown in Table A2. In some embodiments, the binding protein comprises 1, 2, 3, or 4 variable domains shown in Table D2, I3, or 14.

In some embodiments, the V_{H1}/V_{L1} binding pair binds the extracellular portion of dystroglycan, and the V_{H2}/V_{L2} binding pair binds laminin-2. In other embodiments, the V_{H2}/V_{L2} binding pair binds the extracellular portion of dystroglycan, and the V_{H1}/V_{L1} binding pair binds laminin-2.

In some embodiments of any of the multispecific binding molecules described *supra,* the polypeptides of formula I and the polypeptides of formula II form a cross-over light chain-heavy chain pair. In some embodiments, the V_{H1} and V_{L1} domains cross-over to form the V_{H1}/V_{L1} binding pair. In some embodiments, the V_{H2} and V_{L2} domains cross-over to form the V_{H2}/V_{L2} binding pair. In some embodiments, the term linker as used herein in reference to the format above refers to one or more amino acid residues inserted between immunoglobulin domains to provide sufficient mobility for the domains of the light and heavy chains to fold into cross over dual variable region immunoglobulins. A linker is inserted at the transition between variable domains or between variable and constant domains, respectively, at the sequence level. The transition between domains can be identified because the approximate size of the immunoglobulin domains are well understood. The precise location of a domain transition can be determined by locating peptide stretches that do not form secondary structural elements such as beta-sheets or alpha-helices as demonstrated by experimental data or as can be assumed by techniques of modeling or secondary structure prediction. The linkers L₁, L₂, L₃, and L₄ are independent, but they may in some cases have the same sequence and/or length.

In some embodiments, a linker of the present disclosure comprises the sequence DKTHT (SEQ ID NO: 534). In some embodiments, L₁ and L₂ comprise the sequence DKTHT (SEQ ID NO: 534). In some embodiments, L₃ and L₄ comprise the sequence DKTHT (SEQ ID NO: 534). In some embodiments, L₁, L₂, L₃, and L₄ comprise the sequence DKTHT (SEQ ID NO: 534). Any of the linkers and linker combinations described in International Publication No. WO2017/180913 may be used in the binding proteins (*e.g.*, multispecific binding proteins) described herein.

In some embodiments, L₁, L₂, L₃, and L₄ are each 0 to 50 amino acid residues in length, 0 to 40 amino acid residues in length, 0 to 30 amino acid residues in length, 0 to 25 amino acid residues in length, 0 to 20 amino acid residues in length, 0 to 18 amino acid residues in length, 0 to 16 amino acid residues in length, or 0 to 14 amino acid residues in length. In some embodiments, the linkers L₁, L₂, L₃, and L₄ range from no amino acids (length=0) to about 100 amino acids long, or less than 100, 50, 40, 30, 20, or 15 amino acids or less. The linkers can also be 10, 9, 8, 7, 6, 5, 4, 3, 2, or 1 amino acids long. L₁, L₂, L₃, and L₄ in one binding protein may all have the same amino acid sequence or may all have different amino acid sequences.

In certain embodiments, L₁ is 5 amino acid residues in length, L₂ is 5 amino acid residues in length, L₃ is 5 amino acid residues in length, L₄ is 5 amino acid residues in length. In certain embodiments, L₁ is 14 amino acid residues in length, L₂ is 2 amino acid residues in length, L₃ is 14 amino acid residues in length, L₄ is 2 amino acid residues in length. In some embodiments, L₁ and L₃ each comprise the sequence EPKSDKTHTSPPSP (SEQ ID NO:296), and/or L₂ and L₄ each comprise the sequence GG. In certain embodiments, L₁ is 7 amino acid residues in length, L₂ is 5 amino acid residues in length, L₃ is 1 amino acid residue in length, and L₄ is 2 amino acid residues in length. In some embodiments, L₁ comprises the sequence GQPKAAP (SEQ ID NO:297), L₂ comprises the sequence TKGPS (SEQ ID NO:298), L₃ comprises a serine residue (*e.g.*, the sequence S), and L₄ comprises the sequence RT. In certain embodiments, L₁ is 10 amino acid residues in length, L₂ is 10 amino acid residues in length, L₃ is 0 amino acid residues in length, and L₄ is 0 amino acid residues in length. In some embodiments, L₁ and L₂ each comprise the sequence GGSGSSGSGG (SEQ ID NO:299).

In some embodiments, one or both of the variable domains of the polypeptides of formula I and/or formula II are human, humanized, or mouse variable domains.

The disclosure also provides a bispecific binding molecule comprising a first binding domain that binds an extracellular portion of dystroglycan and a second binding domain that binds laminin-2, wherein the bispecific binding molecule is a bispecific binding protein comprising one or more polypeptide chains, wherein the bispecific binding molecule comprises two light chains comprising a structure represented by the formula:

V_{L1}-L₅-V_{L2}-L₆-C_{L} [III]

and two heavy chains comprising a structure represented by the formula:

V_{H1}-L₇-V_{H2}-L₈-C_{H1}-hinge-C_{H2}-C_{H3} [IV]

wherein:
V_{L1} is a first immunoglobulin light chain variable domain;
V_{L2} is a second immunoglobulin light chain variable domain;
V_{H1} is a first immunoglobulin heavy chain variable domain;
V_{H2} is a second immunoglobulin heavy chain variable domain;
C_{L} is an immunoglobulin light chain constant domain;
C_{H1} is an immunoglobulin C_{H1} heavy chain constant domain;
C_{H2} is an immunoglobulin C_{H2} heavy chain constant domain;
C_{H3} is an immunoglobulin C_{H3} heavy chain constant domain;
hinge is an immunoglobulin hinge region connecting the C_{H1} and C_{H2} domains; and L₅, L₆, L₇, and L₈ are amino acid linkers;
wherein the V_{H1} and V_{L1} domains form a V_{H1}/V_{L1} binding pair, and wherein the V_{H2} and V_{L2} domains form a V_{H2}/V_{L2} binding pair.

In some embodiments, formulae III and IV describe the arrangement of domains within the respective polypeptide chains in order from N-terminus to C-terminus. In some embodiments, one or more of the polypeptide chains can comprise additional sequence(s), *e.g.,* at the N-terminal or C-terminal ends.

For exemplary descriptions of this format, *see, e.g.,* US PG Pub. No. US20130209469.

In some embodiments, the bispecific binding molecule comprises two heavy chains comprising the sequence of SEQ ID NO:522 and two light chains comprising the sequence of SEQ ID NO:523. In some embodiments, the bispecific binding molecule comprises two heavy chains comprising the sequence of SEQ ID NO:528 and two light chains comprising the sequence of SEQ ID NO:529. In some embodiments, the binding protein comprises two polypeptide chains shown for AS30_Hu6xC3_Hu10 or AS30_Hu6xC21_Hu11 in Table I3 or I4. In some embodiments, the binding protein comprises a variable domain comprising 1, 2, 3, 4, 5, or 6 CDR sequences shown in Table A2. In some embodiments, the binding protein comprises 1, 2, 3, or 4 variable domains shown in Table D2, 13, or I4.

In some embodiments, the V_{H1}/V_{L1} binding pair binds the extracellular portion of dystroglycan, and the V_{H2}/V_{L2} binding pair binds laminin-2. In other embodiments, the V_{H2}/V_{L2} binding pair binds the extracellular portion of dystroglycan, and the V_{H1}/V_{L1} binding pair binds laminin-2.

In some embodiments, one or both of the variable domains of the polypeptides of formula III and/or formula IV are human, humanized, or mouse variable domains.

### Linkers

In some embodiments, L₅, L₆, L₇, and L₈ are each 0 to 50 amino acid residues in length, 0 to 40 amino acid residues in length, 0 to 30 amino acid residues in length, 0 to 25 amino acid residues in length, 0 to 20 amino acid residues in length, 0 to 18 amino acid residues in length, 0 to 16 amino acid residues in length, or 0 to 14 amino acid residues in length. In some embodiments, the linkers L₅, L₆, L₇, and L₈ range from no amino acids (length=0) to about 100 amino acids long, or less than 100, 50, 40, 30, 20, or 15 amino acids or less. The linkers can also be 10, 9, 8, 7, 6, 5, 4, 3, 2, or 1 amino acids long. L₅, L₆, L₇, and L₈ in one binding protein may all have the same amino acid sequence or may all have different amino acid sequences.

In some embodiments, a linker of the present disclosure comprises the sequence DKTHT (SEQ ID NO: 534). In some embodiments, L₁ and L₂ comprise the sequence DKTHT (SEQ ID NO: 534). In some embodiments, L₃ and L₄ comprise the sequence DKTHT (SEQ ID NO: 534). In some embodiments, L₁, L₂, L₃, and L₄ comprise the sequence DKTHT (SEQ ID NO: 534). Any of the linkers and linker combinations described in International Publication No. WO2017/180913 may be used in the binding proteins (*e.g.*, multispecific binding proteins) described herein.

In some embodiments, L₁, L₂, L₃, and L₄ are each 0 to 50 amino acid residues in length, 0 to 40 amino acid residues in length, 0 to 30 amino acid residues in length, 0 to 25 amino acid residues in length, 0 to 20 amino acid residues in length, 0 to 18 amino acid residues in length, 0 to 16 amino acid residues in length, or 0 to 14 amino acid residues in length. In some embodiments, the linkers L₁, L₂, L₃, and L₄ range from no amino acids (length=0) to about 100 amino acids long, or less than 100, 50, 40, 30, 20, or 15 amino acids or less. The linkers can also be 10, 9, 8, 7, 6, 5, 4, 3, 2, or 1 amino acids long. L₁, L₂, L₃, and L₄ in one binding protein may all have the same amino acid sequence or may all have different amino acid sequences.

In certain embodiments, L₁ is 5 amino acid residues in length, L₂ is 5 amino acid residues in length, L₃ is 5 amino acid residues in length, L₄ is 5 amino acid residues in length. In certain embodiments, L₁ is 14 amino acid residues in length, L₂ is 2 amino acid residues in length, L₃ is 14 amino acid residues in length, L₄ is 2 amino acid residues in length. In some embodiments, L₁ and L₃ each comprise the sequence EPKSDKTHTSPPSP (SEQ ID NO:296), and/or L₂ and L₄ each comprise the sequence GG. In certain embodiments, L₁ is 7 amino acid residues in length, L₂ is 5 amino acid residues in length, L₃ is 1 amino acid residue in length, and L₄ is 2 amino acid residues in length. In some embodiments, L₁ comprises the sequence GQPKAAP (SEQ ID NO:297), L₂ comprises the sequence TKGPS (SEQ ID NO:298), L₃ comprises a serine residue (*e.g.*, the sequence S), and L₄ comprises the sequence RT. In certain embodiments, L₁ is 10 amino acid residues in length, L₂ is 10 amino acid residues in length, L₃ is 0 amino acid residues in length, and L₄ is 0 amino acid residues in length. In some embodiments, L₁ and L₂ each comprise the sequence GGSGSSGSGG (SEQ ID NO:299).

In certain embodiments, the L₅ and L₇ linkers comprise the amino acid sequence of GGGGSGGGGS (SEQ ID NO:294), and/or the L₆ and L₈ linkers are each 0 amino acid residues in length.

The examples listed above (*e.g.*, for L₁, L₂, L₃, L₄, L₅, L₆, L₇, or L₈) are not intended to limit the scope of the disclosure in any way, and linkers comprising randomly selected amino acids selected from the group consisting of valine, leucine, isoleucine, serine, threonine, lysine, arginine, histidine, aspartate, glutamate, asparagine, glutamine, glycine, and proline are suitable in the binding proteins.

The identity and sequence of amino acid residues in the linker may vary depending on the type of secondary structural element necessary to achieve in the linker. For example, glycine, serine, and alanine are used for flexible linkers. Some combination of glycine, proline, threonine, and serine are useful if a more rigid and extended linker is necessary. Any amino acid residue may be considered as a linker in combination with other amino acid residues to construct larger peptide linkers as necessary depending on the desired properties.

### Constant/Fc regions

In some embodiments, a binding protein (*e.g*., multispecific binding protein) of the present disclosure comprises a "knob" mutation on the second polypeptide chain and a "hole" mutation on the third polypeptide chain. In some embodiments, a binding protein of the present disclosure comprises a "knob" mutation on the third polypeptide chain and a "hole" mutation on the second polypeptide chain. In some embodiments, the "knob" mutation comprises substitution(s) at positions corresponding to positions 354 and/or 366 of human IgG1 or IgG4 according to EU Index. In some embodiments, the amino acid substitutions are S354C, T366W, T366Y, S354C and T366W, or S354C and T366Y. In some embodiments, the "knob" mutation comprises substitutions at positions corresponding to positions 354 and 366 of human IgG1 or IgG4 according to EU Index. In some embodiments, the amino acid substitutions are S354C and T366W. In some embodiments, the "hole" mutation comprises substitution(s) at positions corresponding to positions 407 and, optionally, 349, 366, and/or 368 and of human IgG1 or IgG4 according to EU Index. In some embodiments, the amino acid substitutions are Y407V or Y407T and optionally Y349C, T366S, and/or L368A. In some embodiments, the "hole" mutation comprises substitutions at positions corresponding to positions 349, 366, 368, and 407 of human IgG1 or IgG4 according to EU Index. In some embodiments, the amino acid substitutions are Y349C, T366S, L368A, and Y407V.

In some embodiments, the C_{H3} domain of the second polypeptide chain comprises amino acid substitutions at positions corresponding to positions 354 and 366 of human IgG1 or IgG4 according to EU Index (*e.g.*, S354C and T366W); and the C_{H3} domain of the third polypeptide chain comprises amino acid substitutions at positions corresponding to positions 349, 366, 368, and 407 of human IgG1 or IgG4 according to EU Index (*e.g.*, Y349C, T366S, L368A, and Y407V). In some embodiments, the C_{H3} domain of the second polypeptide chain comprises amino acid substitutions at positions corresponding to positions 349, 366, 368, and 407 of human IgG1 or IgG4 according to EU Index (*e.g.*, Y349C, T366S, L368A, and Y407V); and the C_{H3} domain of the third polypeptide chain comprises amino acid substitutions at positions corresponding to positions 354 and 366 of human IgG1 or IgG4 according to EU Index (*e.g.*, S354C and T366W).

In some embodiments, a binding protein (*e.g*., multispecific binding protein) of the present disclosure comprises one or more mutations to improve purification, *e.g*., by modulating the affinity for a purification reagent. For example, it is known that heterodimeric binding proteins can be selectively purified away from their homodimeric forms if one of the two Fc regions of the heterodimeric form contains mutation(s) that reduce or eliminate binding to Protein A, because the heterodimeric form will have an intermediate affinity for Protein A-based purification than either homodimeric form and can be selectively eluted from Protein A, *e.g.,* by use of a different pH (*See e.g.*, Smith, E.J. et al. (2015) Sci. Rep. 5:17943). In some embodiments, the first and/or second Fc regions are human IgG1 Fc regions. In some embodiments, the first and/or second Fc regions are human IgG4 Fc regions. In some embodiments, the mutation comprises substitutions at positions corresponding to positions 435 and 436 of human IgG1 or IgG4 according to EU Index, wherein the amino acid substitutions are H435R and Y436F. In some embodiments, the C_{H3} domains of the second and the third polypeptide chains are human IgG1 or IgG4 C_{H3} domains, and only one of the C_{H3} domains comprises amino acid substitutions at positions corresponding to positions 435 and 436 of human IgG1 or IgG4 according to EU Index (*e.g.*, H435R and Y436F). In some embodiments, a binding protein of the present disclosure comprises knob and hole mutations and one or more mutations to improve purification.

In some embodiments, a binding protein (*e.g*., multispecific binding protein) of the present disclosure comprises one or more mutations to increase half-life, *e.g.*, *in vivo* half-life. In some embodiments, a binding protein comprises one or more of the mutations described in U.S. Pat. No. 7,083,784. For example, in some embodiments, the C_{H2} domains of the second and the third polypeptide chains are human IgG1 or IgG4 C_{H2} domains comprising a tyrosine residue at position 252, a threonine residue at position 254, and a glutamic acid residue at position 256, numbering according to EU Index.

In some embodiments, a binding protein (*e.g*., multispecific binding protein) of the present disclosure comprises one or more mutations resulting in an Fc region with altered glycosylation and/or reduced effector function. In some embodiments, a binding protein comprises one or more of the mutations described in U.S. Pat. No. 9,790,268. For example, in some embodiments, the C_{H2} domains of the second and the third polypeptide chains are human IgG1 or IgG4 C_{H2} domains comprising an asparagine residue at position 297, an

asparagine residue at position 298, an alanine residue at position 299, and a serine or threonine residue at position 300, numbering according to EU Index.

Another bispecific binding protein platform contemplated for use herein is described in US PG Pub. No. US2013/0039913 and Labrijn, A.F. et al. (2013) Proc. Natl. Acad. Sci. 110:5145-5150. In this approach, each binding domain is produced in a homodimeric form, then assembled *in vitro* to form a heterodimeric bispecific binding protein. This approach employs specific mutations (*e.g*., in the antibody CH3 domain) to promote Fab-arm exchange, leading to heterodimeric binding proteins that are more stable than either homodimeric form. In some embodiments, these mutations occur, *e.g.,* at positions 366, 368, 370, 399, 405, 407 and/or 409, according to the EU-index as described in Kabat et al. Specific mutations are described in greater detail in US PG Pub. No. US2013/0039913 and Labrijn, A.F. et al. (2013) Proc. Natl. Acad. Sci. 110:5145-5150*.*

Additional bispecific binding protein platforms contemplated for use herein are described briefly below. One strategy was proposed by Carter et al. (Ridgway et al., 1996, Protein Eng. 9(7): 617-21; Carter, 2011, J. Immunol. Methods 248(1-2): 7-15) to produce a Fc heterodimer using a set of "knob-into-hole" mutations in the C_{H3} domain of Fc. These mutations lead to the alteration of residue packing complementarity between the C_{H3} domain interface within the structurally conserved hydrophobic core so that formation of the heterodimer is favored as compared with homodimers, which achieves good heterodimer expression from mammalian cell culture. Although the strategy led to higher heterodimer yield, the homodimers were not completely suppressed (Merchant et al., 1998, Nat. Biotechnol. 16(7): 677-81).

To improve the yields of the binding proteins, in some embodiments, the C_{H3} domains can be altered by the "knob-into-holes" technology which is described in detail with several examples in, for example, International Publication No. WO 96/027011, Ridgway et al., 1996, Protein Eng. 9: 617-21; and Merchant et al., 1998, Nat. Biotechnol. 16: 677-81. Specifically, the interaction surfaces of the two C_{H3} domains are altered to increase the heterodimerisation of both heavy chains containing these two C_{H3} domains. Each of the two C_{H3} domains (of the two heavy chains) can be the "knob," while the other is the "hole." The introduction of a disulfide bridge further stabilizes the heterodimers (Merchant *et al.*, 1998; Atwell et al., 1997, J. Mol. Biol. 270: 26-35) and increases the yield. In particular embodiments, the knob is on the CH3 domain of one polypeptide chain. In other embodiments, the knob is on the first pair of polypeptides having the cross-over orientation. In yet other embodiments, the C_{H3} domains do not include a knob in hole.

In some embodiments, a binding protein of the present disclosure comprises a "knob" mutation on one polypeptide chain and a "hole" mutation on the other polypeptide chain. In some embodiments, the "knob" mutation comprises substitutions at positions corresponding to positions 354 and 366 of human IgG1 according to EU Index. In some embodiments, the amino acid substitutions are S354C and T366W. In some embodiments, the "hole" mutation comprises substitutions at positions corresponding to positions 349, 366, 368, and 407 of human IgG1 according to EU Index. In some embodiments, the amino acid substitutions are Y349C, T366S, L368A, and Y407V.

In some embodiments, a binding protein of the present disclosure comprises one or more mutations to improve serum half-life (*See e.g.,* Hinton, P.R. et al. (2006) J. Immunol. 176(1):346-56). In some embodiments, the mutation comprises substitutions at positions corresponding to positions 428 and 434 of human IgG1 according to EU Index, wherein the amino acid substitutions are M428L and N434S. In some embodiments, a binding protein of the present disclosure comprises knob and hole mutations and one or more mutations to improve serum half-life.

Another bispecific binding protein platform contemplated for use herein is the heterodimeric, bivalent antibody Fc-containing format described in WO2011131746. Any of the antigen binding sites described herein may be combined in this heterodimeric, bispecific format. In some embodiments, a multispecific (*e.g*., bispecific) binding protein of the present disclosure comprises a first antibody heavy chain comprising a first heavy chain variable (VH) domain and a first Fc region of an immunoglobulin comprising a first C_{H3} region, and a first antibody light chain comprising a first light chain variable (VL) domain, wherein the first VH and VL domains form a first antigen binding domain that binds an extracellular portion of dystroglycan, and a second antibody heavy chain comprising a second heavy chain variable (VH) domain and a second Fc region of an immunoglobulin comprising a second C_{H3} region, and a second antibody light chain comprising a second light chain variable (VL) domain, wherein the second VH and VL domains form a second antigen binding domain that binds laminin-2. In some embodiments, the sequences of said first and second C_{H3} regions are different and are such that the heterodimeric interaction between said first and second C_{H3} regions is stronger than each of the homodimeric interactions of said first and second C_{H3} regions. In some embodiments, the first homodimeric protein has an amino acid other than Lys, Leu or Met at position 409 and the second homodimeric protein has an amino- acid substitution at a position selected from: 366, 368, 370, 399, 405 and 407, and/or the sequences of said first and second C_{H3} regions are such that the dissociation constants of homodimeric interactions of each of the C_{H3} regions are between 0.01 and 10 micromolar. In some embodiments, the first antibody heavy chain comprises the sequence of SEQ ID NO:518, the second antibody heavy chain comprises the sequence of SEQ ID NO:519, the first antibody light chain comprises the sequence of SEQ ID NO:520, and the second antibody light chain comprises the sequence of SEQ ID NO:521. In some embodiments, the binding protein comprises two antibody light chains and two antibody heavy chains shown for AS30_Hu6xC3_Hu10 duobody in Table 13.

Another bispecific binding protein platform contemplated for use herein is the "DuetMab" design (Mazor, Y. et al. (2015) MAbs 7:377-389). Briefly, the "knob-into-hole" approach described above is combined with replacing a native disulfide bond in one of the CH1-CL interfaces with an engineered disulfide bond to increase the efficiency of cognate heavy and light chain pairing. In some embodiments, the heavy chain of one binding domain bears an F126C mutation, and the cognate light chain for that binding domain bears an S121C mutation, numbering according to Kabat. For example, in some embodiments, a multispecific (*e.g*., bispecific) binding protein of the present disclosure comprises a first antibody heavy chain comprising the sequence of SEQ ID NO:524, a second antibody heavy chain comprising the sequence of SEQ ID NO:525, a first antibody light chain comprising the sequence of SEQ ID NO:526, and a second antibody light chain comprising the sequence of SEQ ID NO:527. In some embodiments, the binding protein comprises two antibody light chains and two antibody heavy chains shown for AS30_Hu6xC21_Hu11 duetmab in Table I3.

Gunasekaran et al. explored the feasibility of retaining the hydrophobic core integrity while driving the formation of Fc heterodimer by changing the charge complementarity at the C_{H3} domain interface (Gunasekaran et al., 2010, J. Biol. Chem. 285(25): 19637-46). Taking advantage of the electrostatic steering mechanism, these constructs showed efficient promotion of Fc heterodimer formation with minimum contamination of homodimers through mutation of two pairs of peripherally located charged residues. In contrast to the knob-into-hole design, the homodimers were evenly suppressed due to the nature of the electrostatic repulsive mechanism, but not totally avoided.

Davis et al. describe an antibody engineering approach to convert Fc homodimers into heterodimers by interdigitating β-strand segments of human IgG and IgA C_{H3} domains, without the introduction of extra interchain disulfide bonds (Davis et al., 2010, Protein Eng. Des. Sel. 23(4): 195-202). Expression of SEEDbody (Sb) fusion proteins by mammalian cells yields Sb heterodimers in high yield that are readily purified to eliminate minor by-products.

U.S. Patent Application Publication No. US 2010/331527 A1 describes a bispecific antibody based on heterodimerization of the C_{H3} domain, introducing in one heavy chain the mutations H95R and Y96F within the C_{H3} domain. These amino acid substitutions originate from the C_{H3} domain of the IgG3 subtype and will heterodimerize with an IgG1 backbone. A common light chain prone to pair with every heavy chain is a prerequisite for all formats based on heterodimerization through the C_{H3} domain. A total of three types of antibodies are therefore produced: 50% having a pure IgG1 backbone, one-third having a pure H95R and Y96F mutated backbone, and one-third having two different heavy chains (bispecific). The desired heterodimer can be purified from this mixture because its binding properties to Protein A are different from those of the parental antibodies: IgG3-derived C_{H3} domains do not bind to Protein A, whereas the IgG1 does. Consequently, the heterodimer binds to Protein A, but elutes at a higher pH than the pure IgG1 homodimer, and this makes selective purification of the bispecific heterodimer possible.

U.S. Pat. No. 7,612,181 describes a Dual-Variable-Domain IgG (DVD-IgG) bispecific antibody that is based on the Dual-Fv format described in U.S. Pat. No. 5,989,830. A similar bispecific format was also described in U.S. Patent Application Publication No. US 2010/0226923 A1. The addition of constant domains to respective chains of the Dual-Fv (C_{H1}-Fc to the heavy chain and kappa or lambda constant domain to the light chain) led to functional bispecific antibodies without any need for additional modifications (i.e., obvious addition of constant domains to enhance stability). Some of the antibodies expressed in the DVD-Ig/TBTI format show a position effect on the second (or innermost) antigen binding position (Fv2). Depending on the sequence and the nature of the antigen recognized by the Fv2 position, this antibody domain displays a reduced affinity to its antigen (i.e., loss of on-rate in comparison to the parental antibody). One possible explanation for this observation is that the linker between V_{L1} and V_{L2} protrudes into the CDR region of Fv2, making the Fv2 somewhat inaccessible for larger antigens.

The second configuration of a bispecific antibody fragment described in U.S. Pat. No. 5,989,830 is the cross-over double head (CODH), having the following orientation of variable domains expressed on two chains:
V_{L1}-linker-V_{L2} for the light chain, and
V_{H2}-linker-V_{H1} for the heavy chain.

### CDR, VH, and VL domain sequences

Described *infra* are exemplary CDR, VH domain, and VL domain sequences that may be used in any of the multispecific or bispecific binding proteins of the present disclosure in any number, combination, or configuration.

In some embodiments of any of the formats described herein, a V_{H1}/V_{L1} binding pair of the present disclosure binds the extracellular portion of dystroglycan, and a V_{H2}/V_{L2} binding pair of the present disclosure binds laminin-2.

In some embodiments, the V_{H1} domain comprises a CDR-H1 comprising an amino acid sequence selected from the group consisting of SEQ ID NOs:1-8, a CDR-H2 comprising an amino acid sequence selected from the group consisting of SEQ ID NOs:9-17, and a CDR-H3 comprising an amino acid sequence selected from the group consisting of SEQ ID NOs:18-27; and/or wherein the V_{L1} domain comprises a CDR-L1 comprising an amino acid sequence selected from the group consisting of SEQ ID NOs:28-37, a CDR-L2 comprising an amino acid sequence selected from the group consisting of SEQ ID NOs:38-42, and a CDR-L3 comprising an amino acid sequence selected from the group consisting of SEQ ID NOs:43-50. In some embodiments of any of the formats described herein, the V_{H1} domain comprises an amino acid sequence selected from the group consisting of SEQ ID NOs:170, 172, 174, 176, 178, 180, 182, 184, 186, and 188. In some embodiments, the V_{L1} domain comprises an amino acid sequence selected from the group consisting of SEQ ID NOs:171, 173, 175, 177, 179, 181, 183, 185, 187, and 189. In some embodiments, the V_{H1} domain is encoded by a nucleic acid sequence selected from the group consisting of SEQ ID NOs:230, 232, 234, 236, 238, 240, 242, 244, 246, and 248. In some embodiments, the V_{L1} domain is encoded by a nucleic acid sequence selected from the group consisting of SEQ ID NOs:231, 233, 235, 237, 239, 241, 243, 245, 247, and 249.

In some embodiments of any of the formats described herein, a V_{H1}/V_{L1} binding pair of the present disclosure binds the extracellular portion of dystroglycan, and a V_{H2}/V_{L2} binding pair of the present disclosure binds laminin-2 (*e.g.*, a laminin G-like (LG) domain 5, or LG-5). In some embodiments of any of the formats described herein, the V_{H2} domain comprises a CDR-H1 comprising an amino acid sequence selected from the group consisting of SEQ ID NOs:51-55, a CDR-H2 comprising an amino acid sequence selected from the group consisting of SEQ ID NOs:56-60, and a CDR-H3 comprising an amino acid sequence selected from the group consisting of SEQ ID NOs:61-65; and/or wherein the V_{L2} domain comprises a CDR-L1 comprising an amino acid sequence selected from the group consisting of SEQ ID NOs:66-70, a CDR-L2 comprising an amino acid sequence selected from the group consisting of SEQ ID NOs: 71-75, and a CDR-L3 comprising an amino acid sequence selected from the group consisting of SEQ ID NOs:76-80. In some embodiments, the V_{H2} domain comprises an amino acid sequence selected from the group consisting of SEQ ID NOs:190, 192, 194, 196, and 198. In some embodiments, the V_{L2} domain comprises an amino acid sequence selected from the group consisting of SEQ ID NOs: 191, 193, 195, 197, and 199. In some embodiments, the V_{H2} domain is encoded by a nucleic acid sequence selected from the group consisting of SEQ ID NOs:250, 252, 254, 256, and 258. In some embodiments, the V_{L2} domain is encoded by a nucleic acid sequence selected from the group consisting of SEQ ID NOs:251, 253, 255, 257, and 259.

In some embodiments of any of the formats described herein, a V_{H1}/V_{L1} binding pair of the present disclosure binds the extracellular portion of dystroglycan, and a V_{H2}/V_{L2} binding pair of the present disclosure binds laminin-2 (*e.g*., laminin G-like (LG) 4 and/or 5 domains, or LG-4/5). In some embodiments of any of the formats described herein, the V_{H2} domain comprises a CDR-H1 comprising an amino acid sequence selected from the group consisting of SEQ ID NOs:81-95, a CDR-H2 comprising an amino acid sequence selected from the group consisting of SEQ ID NOs:96-110, and a CDR-H3 comprising an amino acid sequence selected from the group consisting of SEQ ID NOs:111-125; and/or wherein the V_{L2} domain comprises a CDR-L1 comprising an amino acid sequence selected from the group consisting of SEQ ID NOs:126-140, a CDR-L2 comprising an amino acid sequence selected from the group consisting of SEQ ID NOs:38 and 141-154, and a CDR-L3 comprising an amino acid sequence selected from the group consisting of SEQ ID NOs:155-169. In some embodiments, the V_{H2} domain comprises an amino acid sequence selected from the group consisting of SEQ ID NOs:200, 202, 204, 206, 208, 210, 212, 214, 216, 218, 220, 222, 224, 226, and 228. In some embodiments, the V_{L2} domain comprises an amino acid sequence selected from the group consisting of SEQ ID NOs:201, 203, 205, 207, 209, 211, 213, 215, 217, 219, 221, 223, 225, 227, and 229. In some embodiments, the V_{H2} domain is encoded by a nucleic acid sequence selected from the group consisting of SEQ ID NOs:260, 262, 264, 266, 268, 270, 272, 274, 276, 278, 280, 282, 284, 286, and 288. In some embodiments, the V_{L2} domain is encoded by a nucleic acid sequence selected from the group consisting of SEQ ID NOs:261, 263, 265, 267, 269, 271, 273, 275, 277, 279, 281, 283, 285, 287, and 289.

In some embodiments of any of the formats described herein, a V_{H2}/V_{L2} binding pair of the present disclosure binds the extracellular portion of dystroglycan, and a V_{H1}/V_{L1} binding pair of the present disclosure binds laminin-2. In some embodiments of any of the formats described herein, the V_{H2} domain comprises a CDR-H1 comprising an amino acid sequence selected from the group consisting of SEQ ID NOs:1-8, a CDR-H2 comprising an amino acid sequence selected from the group consisting of SEQ ID NOs:9-17, and a CDR-H3 comprising an amino acid sequence selected from the group consisting of SEQ ID NOs:18-27; and/or wherein the V_{L2} domain comprises a CDR-L1 comprising an amino acid sequence selected from the group consisting of SEQ ID NOs:28-37, a CDR-L2 comprising an amino acid sequence selected from the group consisting of SEQ ID NOs:38-42, and a CDR-L3 comprising an amino acid sequence selected from the group consisting of SEQ ID NOs:43-50. In some embodiments of any of the formats described herein, the V_{H2} domain comprises an amino acid sequence selected from the group consisting of SEQ ID NOs:170, 172, 174, 176, 178, 180, 182, 184, 186, and 188. In some embodiments, the V_{L2} domain comprises an amino acid sequence selected from the group consisting of SEQ ID NOs:171, 173, 175, 177, 179, 181, 183, 185, 187, and 189. In some embodiments, the V_{H2} domain is encoded by a nucleic acid sequence selected from the group consisting of SEQ ID NOs:230, 232, 234, 236, 238, 240, 242, 244, 246, and 248. In some embodiments, the V_{L2} domain is encoded by a nucleic acid sequence selected from the group consisting of SEQ ID NOs:231, 233, 235, 237, 239, 241, 243, 245, 247, and 249.

In some embodiments of any of the formats described herein, a V_{H2}/V_{L2} binding pair of the present disclosure binds the extracellular portion of dystroglycan, and a V_{H1}/V_{L1} binding pair of the present disclosure binds laminin-2 (*e.g.*, a laminin G-like (LG) domain 5, or LG-5). In some embodiments of any of the formats described herein, the V_{H2} domain comprises a CDR-H1 comprising an amino acid sequence selected from the group consisting of SEQ ID NOs:51-55, a CDR-H2 comprising an amino acid sequence selected from the group consisting of SEQ ID NOs:56-60, and a CDR-H3 comprising an amino acid sequence selected from the group consisting of SEQ ID NOs:61-65; and/or wherein the V_{L2} domain comprises a CDR-L1 comprising an amino acid sequence selected from the group consisting of SEQ ID NOs:66-70, a CDR-L2 comprising an amino acid sequence selected from the group consisting of SEQ ID NOs: 71-75, and a CDR-L3 comprising an amino acid sequence selected from the group consisting of SEQ ID NOs:76-80. In some embodiments, the V_{H2} domain comprises an amino acid sequence selected from the group consisting of SEQ ID NOs:190, 192, 194, 196, and 198. In some embodiments, the V_{L2} domain comprises an amino acid sequence selected from the group consisting of SEQ ID NOs: 191, 193, 195, 197, and 199. In some embodiments, the V_{H2} domain is encoded by a nucleic acid sequence selected from the group consisting of SEQ ID NOs:250, 252, 254, 256, and 258. In some embodiments, the V_{L2} domain is encoded by a nucleic acid sequence selected from the group consisting of SEQ ID NOs:251, 253, 255, 257, and 259.

In some embodiments of any of the formats described herein, a V_{H2}/V_{L2} binding pair of the present disclosure binds the extracellular portion of dystroglycan, and a V_{H1}/V_{L1} binding pair of the present disclosure binds laminin-2 (*e.g*., laminin G-like (LG) 4 and/or 5 domains, or LG-4/5). In some embodiments of any of the formats described herein, the V_{H2} domain comprises a CDR-H1 comprising an amino acid sequence selected from the group consisting of SEQ ID NOs:81-95, a CDR-H2 comprising an amino acid sequence selected from the group consisting of SEQ ID NOs:96-110, and a CDR-H3 comprising an amino acid sequence selected from the group consisting of SEQ ID NOs:111-125; and/or wherein the V_{L2} domain comprises a CDR-L1 comprising an amino acid sequence selected from the group consisting of SEQ ID NOs:126-140, a CDR-L2 comprising an amino acid sequence selected from the group consisting of SEQ ID NOs:38 and 141-154, and a CDR-L3 comprising an amino acid sequence selected from the group consisting of SEQ ID NOs:155-169. In some embodiments, the V_{H2} domain comprises an amino acid sequence selected from the group consisting of SEQ ID NOs:200, 202, 204, 206, 208, 210, 212, 214, 216, 218, 220, 222, 224, 226, and 228. In some embodiments, the V_{L2} domain comprises an amino acid sequence selected from the group consisting of SEQ ID NOs:201, 203, 205, 207, 209, 211, 213, 215, 217, 219, 221, 223, 225, 227, and 229. In some embodiments, the V_{H2} domain is encoded by a nucleic acid sequence selected from the group consisting of SEQ ID NOs:260, 262, 264, 266, 268, 270, 272, 274, 276, 278, 280, 282, 284, 286, and 288. In some embodiments, the V_{L2} domain is encoded by a nucleic acid sequence selected from the group consisting of SEQ ID NOs:261, 263, 265, 267, 269, 271, 273, 275, 277, 279, 281, 283, 285, 287, and 289.

Exemplary CDR sequences suitable for use in the binding proteins of the present disclosure are provided in Tables A-C below. Exemplary VH and VL sequences (polypeptide and nucleic acid) suitable for use in the binding proteins of the present disclosure are provided in Tables D-I below. In some embodiments, a bispecific binding protein of the present disclosure comprises a binding domain that binds an extracellular portion of dystroglycan, wherein the binding domain comprises a VH domain comprising at least 1, at least 2, at least 3, at least 4, at least 5, or 6 CDR sequences of an antibody shown in Table A below and/or a VL domain comprising at least 1, at least 2, at least 3, at least 4, at least 5, or 6 CDR sequences of an antibody shown in Table A below. In some embodiments, a bispecific binding protein of the present disclosure comprises a binding domain that binds laminin-2, wherein the binding domain comprises a VH domain comprising at least 1, at least 2, at least 3, at least 4, at least 5, or 6 CDR sequences of an antibody shown in Table B or Table C below and/or a VL domain comprising at least 1, at least 2, at least 3, at least 4, at least 5, or 6 CDR sequences of an antibody shown in Table B or Table C below. In some embodiments, a bispecific binding protein of the present disclosure comprises a binding domain that binds an extracellular portion of dystroglycan, wherein the binding domain comprises a VH domain comprising a sequence that is at least 90%, at least 91%, at least 92%, at least 93%, at least 94%, at least 95%, at least 96%, at least 97%, at least 98%, at least 99%, or 100% identical to a VH sequence shown in Table D below and/or a VL domain comprising a sequence that is at least 90%, at least 91%, at least 92%, at least 93%, at least 94%, at least 95%, at least 96%, at least 97%, at least 98%, at least 99%, or 100% identical to a VL sequence shown in Table D below. In some embodiments, a bispecific binding protein of the present disclosure comprises a binding domain that binds laminin-2, wherein the binding domain comprises a VH domain comprising a sequence that is at least 90%, at least 91%, at least 92%, at least 93%, at least 94%, at least 95%, at least 96%, at least 97%, at least 98%, at least 99%, or 100% identical to a VH sequence shown in Table E or Table F below and/or a VL domain comprising a sequence that is at least 90%, at least 91%, at least 92%, at least 93%, at least 94%, at least 95%, at least 96%, at least 97%, at least 98%, at least 99%, or 100% identical to a VL sequence shown in Table E or Table F below. In some embodiments, a bispecific binding protein of the present disclosure comprises a binding domain that binds an extracellular portion of dystroglycan, wherein the binding domain comprises a VH domain comprising a sequence that is at least 90%, at least 91%, at least 92%, at least 93%, at least 94%, at least 95%, at least 96%, at least 97%, at least 98%, at least 99%, or 100% identical to a VH sequence encoded by a polynucleotide sequence shown in Table G below and/or a VL domain comprising a sequence that is at least 90%, at least 91%, at least 92%, at least 93%, at least 94%, at least 95%, at least 96%, at least 97%, at least 98%, at least 99%, or 100% identical to a VL sequence encoded by a polynucleotide sequence shown in Table G below. In some embodiments, a bispecific binding protein of the present disclosure comprises a binding domain that binds laminin-2, wherein the binding domain comprises a VH domain comprising a sequence that is at least 90%, at least 91%, at least 92%, at least 93%, at least 94%, at least 95%, at least 96%, at least 97%, at least 98%, at least 99%, or 100% identical to a VH sequence encoded by a polynucleotide sequence shown in Table H or Table I below and/or a VL domain comprising a sequence that is at least 90%, at least 91%, at least 92%, at least 93%, at least 94%, at least 95%, at least 96%, at least 97%, at least 98%, at least 99%, or 100% identical to a VL sequence encoded by a polynucleotide sequence shown in Table H or Table I below.

In some embodiments, a bispecific binding protein of the present disclosure comprises (a) a VH domain comprising (i) CDR-H1 comprising the amino acid sequence of SEQ ID NO:1, (ii) CDR-H2 comprising the amino acid sequence of SEQ ID NO:9, and (iii) CDR-H3 comprising the amino acid sequence of SEQ ID NO:18; and/or (b) a VL domain comprising (i) CDR-L1 comprising the amino acid sequence of SEQ ID NO:28, (ii) CDR-L2 comprising the amino acid sequence of SEQ ID NO:38, and (iii) CDR-L3 comprising the amino acid sequence of SEQ ID NO:43. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:170 and/ or a VL domain sequence having at least 90%, 91%, 92%, 93%, 94%, 95%, 96%, 97%, 98%, 99%, or 100% sequence identity to the amino acid sequence of SEQ ID NO:171. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:230 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:231.

In some embodiments, a bispecific binding protein of the present disclosure comprises (a) a VH domain comprising (i) CDR-H1 comprising the amino acid sequence of SEQ ID NO:1, (ii) CDR-H2 comprising the amino acid sequence of SEQ ID NO:10, and (iii) CDR-H3 comprising the amino acid sequence of SEQ ID NO:19; and/or (b) a VL domain comprising (i) CDR-L1 comprising the amino acid sequence of SEQ ID NO:29, (ii) CDR-L2 comprising the amino acid sequence of SEQ ID NO:38, and (iii) CDR-L3 comprising the amino acid sequence of SEQ ID NO:43. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:172 and/ or a VL domain sequence having at least 90%, 91%, 92%, 93%, 94%, 95%, 96%, 97%, 98%, 99%, or 100% sequence identity to the amino acid sequence of SEQ ID NO:173. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:232 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:233.

In some embodiments, a bispecific binding protein of the present disclosure comprises (a) a VH domain comprising (i) CDR-H1 comprising the amino acid sequence of SEQ ID NO:2, (ii) CDR-H2 comprising the amino acid sequence of SEQ ID NO:11, and (iii) CDR-H3 comprising the amino acid sequence of SEQ ID NO:20; and/or (b) a VL domain comprising (i) CDR-L1 comprising the amino acid sequence of SEQ ID NO:30, (ii) CDR-L2 comprising the amino acid sequence of SEQ ID NO:39, and (iii) CDR-L3 comprising the amino acid sequence of SEQ ID NO:44. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:174 and/ or a VL domain sequence having at least 90%, 91%, 92%, 93%, 94%, 95%, 96%, 97%, 98%, 99%, or 100% sequence identity to the amino acid sequence of SEQ ID NO:175. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:234 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:235.

In some embodiments, a bispecific binding protein of the present disclosure comprises (a) a VH domain comprising (i) CDR-H1 comprising the amino acid sequence of SEQ ID NO:3, (ii) CDR-H2 comprising the amino acid sequence of SEQ ID NO:12, and (iii) CDR-H3 comprising the amino acid sequence of SEQ ID NO:21; and/or (b) a VL domain comprising (i) CDR-L1 comprising the amino acid sequence of SEQ ID NO:31, (ii) CDR-L2 comprising the amino acid sequence of SEQ ID NO:40, and (iii) CDR-L3 comprising the amino acid sequence of SEQ ID NO:45. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:176 and/ or a VL domain sequence having at least 90%, 91%, 92%, 93%, 94%, 95%, 96%, 97%, 98%, 99%, or 100% sequence identity to the amino acid sequence of SEQ ID NO:177. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:236 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:237.

In some embodiments, a bispecific binding protein of the present disclosure comprises (a) a VH domain comprising (i) CDR-H1 comprising the amino acid sequence of SEQ ID NO:4, (ii) CDR-H2 comprising the amino acid sequence of SEQ ID NO:13, and (iii) CDR-H3 comprising the amino acid sequence of SEQ ID NO:22; and/or (b) a VL domain comprising (i) CDR-L1 comprising the amino acid sequence of SEQ ID NO:32, (ii) CDR-L2 comprising the amino acid sequence of SEQ ID NO:41, and (iii) CDR-L3 comprising the amino acid sequence of SEQ ID NO:46. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:178 and/ or a VL domain sequence having at least 90%, 91%, 92%, 93%, 94%, 95%, 96%, 97%, 98%, 99%, or 100% sequence identity to the amino acid sequence of SEQ ID NO:179. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:238 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:239.

In some embodiments, a bispecific binding protein of the present disclosure comprises (a) a VH domain comprising (i) CDR-H1 comprising the amino acid sequence of SEQ ID NO:4, (ii) CDR-H2 comprising the amino acid sequence of SEQ ID NO:13, and (iii) CDR-H3 comprising the amino acid sequence of SEQ ID NO:23; and/or (b) a VL domain comprising (i) CDR-L1 comprising the amino acid sequence of SEQ ID NO:33, (ii) CDR-L2 comprising the amino acid sequence of SEQ ID NO:41, and (iii) CDR-L3 comprising the amino acid sequence of SEQ ID NO:46. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:180 and/ or a VL domain sequence having at least 90%, 91%, 92%, 93%, 94%, 95%, 96%, 97%, 98%, 99%, or 100% sequence identity to the amino acid sequence of SEQ ID NO:181. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:240 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:241.

In some embodiments, a bispecific binding protein of the present disclosure comprises (a) a VH domain comprising (i) CDR-H1 comprising the amino acid sequence of SEQ ID NO:5, (ii) CDR-H2 comprising the amino acid sequence of SEQ ID NO:14, and (iii) CDR-H3 comprising the amino acid sequence of SEQ ID NO:24; and/or (b) a VL domain comprising (i) CDR-L1 comprising the amino acid sequence of SEQ ID NO:34, (ii) CDR-L2 comprising the amino acid sequence of SEQ ID NO:42, and (iii) CDR-L3 comprising the amino acid sequence of SEQ ID NO:47. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:182 and/ or a VL domain sequence having at least 90%, 91%, 92%, 93%, 94%, 95%, 96%, 97%, 98%, 99%, or 100% sequence identity to the amino acid sequence of SEQ ID NO:183. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:242 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:243.

In some embodiments, a bispecific binding protein of the present disclosure comprises (a) a VH domain comprising (i) CDR-H1 comprising the amino acid sequence of SEQ ID NO:6, (ii) CDR-H2 comprising the amino acid sequence of SEQ ID NO:15, and (iii) CDR-H3 comprising the amino acid sequence of SEQ ID NO:25; and/or (b) a VL domain comprising (i) CDR-L1 comprising the amino acid sequence of SEQ ID NO:35, (ii) CDR-L2 comprising the amino acid sequence of SEQ ID NO:40, and (iii) CDR-L3 comprising the amino acid sequence of SEQ ID NO:48. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:184 and/ or a VL domain sequence having at least 90%, 91%, 92%, 93%, 94%, 95%, 96%, 97%, 98%, 99%, or 100% sequence identity to the amino acid sequence of SEQ ID NO:185. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:244 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:245.

In some embodiments, a bispecific binding protein of the present disclosure comprises (a) a VH domain comprising (i) CDR-H1 comprising the amino acid sequence of SEQ ID NO:7, (ii) CDR-H2 comprising the amino acid sequence of SEQ ID NO:16, and (iii) CDR-H3 comprising the amino acid sequence of SEQ ID NO:26; and/or (b) a VL domain comprising (i) CDR-L1 comprising the amino acid sequence of SEQ ID NO:36, (ii) CDR-L2 comprising the amino acid sequence of SEQ ID NO:40, and (iii) CDR-L3 comprising the amino acid sequence of SEQ ID NO:49. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:186 and/ or a VL domain sequence having at least 90%, 91%, 92%, 93%, 94%, 95%, 96%, 97%, 98%, 99%, or 100% sequence identity to the amino acid sequence of SEQ ID NO:187. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:246 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:247.

In some embodiments, a bispecific binding protein of the present disclosure comprises (a) a VH domain comprising (i) CDR-H1 comprising the amino acid sequence of SEQ ID NO:8, (ii) CDR-H2 comprising the amino acid sequence of SEQ ID NO:17, and (iii) CDR-H3 comprising the amino acid sequence of SEQ ID NO:27; and/or (b) a VL domain comprising (i) CDR-L1 comprising the amino acid sequence of SEQ ID NO:37, (ii) CDR-L2 comprising the amino acid sequence of SEQ ID NO:40, and (iii) CDR-L3 comprising the amino acid sequence of SEQ ID NO:50. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:188 and/ or a VL domain sequence having at least 90%, 91%, 92%, 93%, 94%, 95%, 96%, 97%, 98%, 99%, or 100% sequence identity to the amino acid sequence of SEQ ID NO:189. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:248 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:249.

In some embodiments, a bispecific binding protein of the present disclosure comprises (a) a VH domain comprising (i) CDR-H1 comprising the amino acid sequence of SEQ ID NO:51, (ii) CDR-H2 comprising the amino acid sequence of SEQ ID NO:56, and (iii) CDR-H3 comprising the amino acid sequence of SEQ ID NO:61; and/or (b) a VL domain comprising (i) CDR-L1 comprising the amino acid sequence of SEQ ID NO:66, (ii) CDR-L2 comprising the amino acid sequence of SEQ ID NO:71, and (iii) CDR-L3 comprising the amino acid sequence of SEQ ID NO:76. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:190 and/ or a VL domain sequence having at least 90%, 91%, 92%, 93%, 94%, 95%, 96%, 97%, 98%, 99%, or 100% sequence identity to the amino acid sequence of SEQ ID NO:191. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:250 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:251.

In some embodiments, a bispecific binding protein of the present disclosure comprises (a) a VH domain comprising (i) CDR-H1 comprising the amino acid sequence of SEQ ID NO:52, (ii) CDR-H2 comprising the amino acid sequence of SEQ ID NO:57, and (iii) CDR-H3 comprising the amino acid sequence of SEQ ID NO:62; and/or (b) a VL domain comprising (i) CDR-L1 comprising the amino acid sequence of SEQ ID NO:67, (ii) CDR-L2 comprising the amino acid sequence of SEQ ID NO:72, and (iii) CDR-L3 comprising the amino acid sequence of SEQ ID NO:77. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:192 and/ or a VL domain sequence having at least 90%, 91%, 92%, 93%, 94%, 95%, 96%, 97%, 98%, 99%, or 100% sequence identity to the amino acid sequence of SEQ ID NO:193. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:252 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:253.

In some embodiments, a bispecific binding protein of the present disclosure comprises (a) a VH domain comprising (i) CDR-H1 comprising the amino acid sequence of SEQ ID NO:53, (ii) CDR-H2 comprising the amino acid sequence of SEQ ID NO:58, and (iii) CDR-H3 comprising the amino acid sequence of SEQ ID NO:63; and/or (b) a VL domain comprising (i) CDR-L1 comprising the amino acid sequence of SEQ ID NO:68, (ii) CDR-L2 comprising the amino acid sequence of SEQ ID NO:73, and (iii) CDR-L3 comprising the amino acid sequence of SEQ ID NO:78. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:194 and/ or a VL domain sequence having at least 90%, 91%, 92%, 93%, 94%, 95%, 96%, 97%, 98%, 99%, or 100% sequence identity to the amino acid sequence of SEQ ID NO:195. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:254 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:255.

In some embodiments, a bispecific binding protein of the present disclosure comprises (a) a VH domain comprising (i) CDR-H1 comprising the amino acid sequence of SEQ ID NO:54, (ii) CDR-H2 comprising the amino acid sequence of SEQ ID NO:59, and (iii) CDR-H3 comprising the amino acid sequence of SEQ ID NO:64; and/or (b) a VL domain comprising (i) CDR-L1 comprising the amino acid sequence of SEQ ID NO:69, (ii) CDR-L2 comprising the amino acid sequence of SEQ ID NO:74, and (iii) CDR-L3 comprising the amino acid sequence of SEQ ID NO:79. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:196 and/ or a VL domain sequence having at least 90%, 91%, 92%, 93%, 94%, 95%, 96%, 97%, 98%, 99%, or 100% sequence identity to the amino acid sequence of SEQ ID NO:197. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:256 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:257.

In some embodiments, a bispecific binding protein of the present disclosure comprises (a) a VH domain comprising (i) CDR-H1 comprising the amino acid sequence of SEQ ID NO:55, (ii) CDR-H2 comprising the amino acid sequence of SEQ ID NO:60, and (iii) CDR-H3 comprising the amino acid sequence of SEQ ID NO:65; and/or (b) a VL domain comprising (i) CDR-L1 comprising the amino acid sequence of SEQ ID NO:70, (ii) CDR-L2 comprising the amino acid sequence of SEQ ID NO:75, and (iii) CDR-L3 comprising the amino acid sequence of SEQ ID NO:80. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:198 and/ or a VL domain sequence having at least 90%, 91%, 92%, 93%, 94%, 95%, 96%, 97%, 98%, 99%, or 100% sequence identity to the amino acid sequence of SEQ ID NO:199. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:258 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:259.

In some embodiments, a bispecific binding protein of the present disclosure comprises (a) a VH domain comprising (i) CDR-H1 comprising the amino acid sequence of SEQ ID NO:81, (ii) CDR-H2 comprising the amino acid sequence of SEQ ID NO:96, and (iii) CDR-H3 comprising the amino acid sequence of SEQ ID NO:111; and/or (b) a VL domain comprising (i) CDR-L1 comprising the amino acid sequence of SEQ ID NO:126, (ii) CDR-L2 comprising the amino acid sequence of SEQ ID NO:141, and (iii) CDR-L3 comprising the amino acid sequence of SEQ ID NO:155. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:200 and/ or a VL domain sequence having at least 90%, 91%, 92%, 93%, 94%, 95%, 96%, 97%, 98%, 99%, or 100% sequence identity to the amino acid sequence of SEQ ID NO:201. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:260 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:261.

In some embodiments, a bispecific binding protein of the present disclosure comprises (a) a VH domain comprising (i) CDR-H1 comprising the amino acid sequence of SEQ ID NO:82, (ii) CDR-H2 comprising the amino acid sequence of SEQ ID NO:97, and (iii) CDR-H3 comprising the amino acid sequence of SEQ ID NO:112; and/or (b) a VL domain comprising (i) CDR-L1 comprising the amino acid sequence of SEQ ID NO:127, (ii) CDR-L2 comprising the amino acid sequence of SEQ ID NO:142, and (iii) CDR-L3 comprising the amino acid sequence of SEQ ID NO:156. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:202 and/ or a VL domain sequence having at least 90%, 91%, 92%, 93%, 94%, 95%, 96%, 97%, 98%, 99%, or 100% sequence identity to the amino acid sequence of SEQ ID NO:203. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:262 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:263.

In some embodiments, a bispecific binding protein of the present disclosure comprises (a) a VH domain comprising (i) CDR-H1 comprising the amino acid sequence of SEQ ID NO:83, (ii) CDR-H2 comprising the amino acid sequence of SEQ ID NO:98, and (iii) CDR-H3 comprising the amino acid sequence of SEQ ID NO:113; and/or (b) a VL domain comprising (i) CDR-L1 comprising the amino acid sequence of SEQ ID NO:128, (ii) CDR-L2 comprising the amino acid sequence of SEQ ID NO:143, and (iii) CDR-L3 comprising the amino acid sequence of SEQ ID NO:157. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:204 and/ or a VL domain sequence having at least 90%, 91%, 92%, 93%, 94%, 95%, 96%, 97%, 98%, 99%, or 100% sequence identity to the amino acid sequence of SEQ ID NO:205. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:264 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:265.

In some embodiments, a bispecific binding protein of the present disclosure comprises (a) a VH domain comprising (i) CDR-H1 comprising the amino acid sequence of SEQ ID NO:84, (ii) CDR-H2 comprising the amino acid sequence of SEQ ID NO:99, and (iii) CDR-H3 comprising the amino acid sequence of SEQ ID NO:114; and/or (b) a VL domain comprising (i) CDR-L1 comprising the amino acid sequence of SEQ ID NO:129, (ii) CDR-L2 comprising the amino acid sequence of SEQ ID NO:144, and (iii) CDR-L3 comprising the amino acid sequence of SEQ ID NO:158. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:206 and/ or a VL domain sequence having at least 90%, 91%, 92%, 93%, 94%, 95%, 96%, 97%, 98%, 99%, or 100% sequence identity to the amino acid sequence of SEQ ID NO:207. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:266 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:267.

In some embodiments, a bispecific binding protein of the present disclosure comprises (a) a VH domain comprising (i) CDR-H1 comprising the amino acid sequence of SEQ ID NO:85, (ii) CDR-H2 comprising the amino acid sequence of SEQ ID NO:100, and (iii) CDR-H3 comprising the amino acid sequence of SEQ ID NO:115; and/or (b) a VL domain comprising (i) CDR-L1 comprising the amino acid sequence of SEQ ID NO:130, (ii) CDR-L2 comprising the amino acid sequence of SEQ ID NO:145, and (iii) CDR-L3 comprising the amino acid sequence of SEQ ID NO:159. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:208 and/ or a VL domain sequence having at least 90%, 91%, 92%, 93%, 94%, 95%, 96%, 97%, 98%, 99%, or 100% sequence identity to the amino acid sequence of SEQ ID NO:209. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:268 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:269.

In some embodiments, a bispecific binding protein of the present disclosure comprises (a) a VH domain comprising (i) CDR-H1 comprising the amino acid sequence of SEQ ID NO:86, (ii) CDR-H2 comprising the amino acid sequence of SEQ ID NO:101, and (iii) CDR-H3 comprising the amino acid sequence of SEQ ID NO:116; and/or (b) a VL domain comprising (i) CDR-L1 comprising the amino acid sequence of SEQ ID NO:131, (ii) CDR-L2 comprising the amino acid sequence of SEQ ID NO:146, and (iii) CDR-L3 comprising the amino acid sequence of SEQ ID NO:160. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:210 and/ or a VL domain sequence having at least 90%, 91%, 92%, 93%, 94%, 95%, 96%, 97%, 98%, 99%, or 100% sequence identity to the amino acid sequence of SEQ ID NO:211. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:270 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:271.

In some embodiments, a bispecific binding protein of the present disclosure comprises (a) a VH domain comprising (i) CDR-H1 comprising the amino acid sequence of SEQ ID NO:87, (ii) CDR-H2 comprising the amino acid sequence of SEQ ID NO:102, and (iii) CDR-H3 comprising the amino acid sequence of SEQ ID NO:117; and/or (b) a VL domain comprising (i) CDR-L1 comprising the amino acid sequence of SEQ ID NO:132, (ii) CDR-L2 comprising the amino acid sequence of SEQ ID NO:147, and (iii) CDR-L3 comprising the amino acid sequence of SEQ ID NO:161. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:212 and/ or a VL domain sequence having at least 90%, 91%, 92%, 93%, 94%, 95%, 96%, 97%, 98%, 99%, or 100% sequence identity to the amino acid sequence of SEQ ID NO:213. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:272 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:273.

In some embodiments, a bispecific binding protein of the present disclosure comprises (a) a VH domain comprising (i) CDR-H1 comprising the amino acid sequence of SEQ ID NO:88, (ii) CDR-H2 comprising the amino acid sequence of SEQ ID NO:103, and (iii) CDR-H3 comprising the amino acid sequence of SEQ ID NO:118; and/or (b) a VL domain comprising (i) CDR-L1 comprising the amino acid sequence of SEQ ID NO:133, (ii) CDR-L2 comprising the amino acid sequence of SEQ ID NO:148, and (iii) CDR-L3 comprising the amino acid sequence of SEQ ID NO:162. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:214 and/ or a VL domain sequence having at least 90%, 91%, 92%, 93%, 94%, 95%, 96%, 97%, 98%, 99%, or 100% sequence identity to the amino acid sequence of SEQ ID NO:215. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:274 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:275.

In some embodiments, a bispecific binding protein of the present disclosure comprises (a) a VH domain comprising (i) CDR-H1 comprising the amino acid sequence of SEQ ID NO:89, (ii) CDR-H2 comprising the amino acid sequence of SEQ ID NO:104, and (iii) CDR-H3 comprising the amino acid sequence of SEQ ID NO:119; and/or (b) a VL domain comprising (i) CDR-L1 comprising the amino acid sequence of SEQ ID NO:134, (ii) CDR-L2 comprising the amino acid sequence of SEQ ID NO:149, and (iii) CDR-L3 comprising the amino acid sequence of SEQ ID NO:163. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:216 and/ or a VL domain sequence having at least 90%, 91%, 92%, 93%, 94%, 95%, 96%, 97%, 98%, 99%, or 100% sequence identity to the amino acid sequence of SEQ ID NO:217. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:276 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:277.

In some embodiments, a bispecific binding protein of the present disclosure comprises (a) a VH domain comprising (i) CDR-H1 comprising the amino acid sequence of SEQ ID NO:90, (ii) CDR-H2 comprising the amino acid sequence of SEQ ID NO:105, and (iii) CDR-H3 comprising the amino acid sequence of SEQ ID NO:120; and/or (b) a VL domain comprising (i) CDR-L1 comprising the amino acid sequence of SEQ ID NO:135, (ii) CDR-L2 comprising the amino acid sequence of SEQ ID NO:150, and (iii) CDR-L3 comprising the amino acid sequence of SEQ ID NO:164. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:218 and/ or a VL domain sequence having at least 90%, 91%, 92%, 93%, 94%, 95%, 96%, 97%, 98%, 99%, or 100% sequence identity to the amino acid sequence of SEQ ID NO:219. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:278 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:279.

In some embodiments, a bispecific binding protein of the present disclosure comprises (a) a VH domain comprising (i) CDR-H1 comprising the amino acid sequence of SEQ ID NO:91, (ii) CDR-H2 comprising the amino acid sequence of SEQ ID NO:106, and (iii) CDR-H3 comprising the amino acid sequence of SEQ ID NO:121; and/or (b) a VL domain comprising (i) CDR-L1 comprising the amino acid sequence of SEQ ID NO:136, (ii) CDR-L2 comprising the amino acid sequence of SEQ ID NO:151, and (iii) CDR-L3 comprising the amino acid sequence of SEQ ID NO:165. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:220 and/ or a VL domain sequence having at least 90%, 91%, 92%, 93%, 94%, 95%, 96%, 97%, 98%, 99%, or 100% sequence identity to the amino acid sequence of SEQ ID NO:221. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:280 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:281.

In some embodiments, a bispecific binding protein of the present disclosure comprises (a) a VH domain comprising (i) CDR-H1 comprising the amino acid sequence of SEQ ID NO:92, (ii) CDR-H2 comprising the amino acid sequence of SEQ ID NO:107, and (iii) CDR-H3 comprising the amino acid sequence of SEQ ID NO:122; and/or (b) a VL domain comprising (i) CDR-L1 comprising the amino acid sequence of SEQ ID NO:137, (ii) CDR-L2 comprising the amino acid sequence of SEQ ID NO:152, and (iii) CDR-L3 comprising the amino acid sequence of SEQ ID NO:166. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:222 and/ or a VL domain sequence having at least 90%, 91%, 92%, 93%, 94%, 95%, 96%, 97%, 98%, 99%, or 100% sequence identity to the amino acid sequence of SEQ ID NO:223. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:282 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:283.

In some embodiments, a bispecific binding protein of the present disclosure comprises (a) a VH domain comprising (i) CDR-H1 comprising the amino acid sequence of SEQ ID NO:93, (ii) CDR-H2 comprising the amino acid sequence of SEQ ID NO:108, and (iii) CDR-H3 comprising the amino acid sequence of SEQ ID NO:123; and/or (b) a VL domain comprising (i) CDR-L1 comprising the amino acid sequence of SEQ ID NO:138, (ii) CDR-L2 comprising the amino acid sequence of SEQ ID NO:153, and (iii) CDR-L3 comprising the amino acid sequence of SEQ ID NO:167. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:224 and/ or a VL domain sequence having at least 90%, 91%, 92%, 93%, 94%, 95%, 96%, 97%, 98%, 99%, or 100% sequence identity to the amino acid sequence of SEQ ID NO:225. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:284 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:285.

In some embodiments, a bispecific binding protein of the present disclosure comprises (a) a VH domain comprising (i) CDR-H1 comprising the amino acid sequence of SEQ ID NO:94, (ii) CDR-H2 comprising the amino acid sequence of SEQ ID NO:109, and (iii) CDR-H3 comprising the amino acid sequence of SEQ ID NO:124; and/or (b) a VL domain comprising (i) CDR-L1 comprising the amino acid sequence of SEQ ID NO:139, (ii) CDR-L2 comprising the amino acid sequence of SEQ ID NO:38, and (iii) CDR-L3 comprising the amino acid sequence of SEQ ID NO:168. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:226 and/ or a VL domain sequence having at least 90%, 91%, 92%, 93%, 94%, 95%, 96%, 97%, 98%, 99%, or 100% sequence identity to the amino acid sequence of SEQ ID NO:227. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:286 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:287.

In some embodiments, a bispecific binding protein of the present disclosure comprises (a) a VH domain comprising (i) CDR-H1 comprising the amino acid sequence of SEQ ID NO:95, (ii) CDR-H2 comprising the amino acid sequence of SEQ ID NO:110, and (iii) CDR-H3 comprising the amino acid sequence of SEQ ID NO:125; and/or (b) a VL domain comprising (i) CDR-L1 comprising the amino acid sequence of SEQ ID NO:140, (ii) CDR-L2 comprising the amino acid sequence of SEQ ID NO:154, and (iii) CDR-L3 comprising the amino acid sequence of SEQ ID NO:169. In some embodiments, a bispecific binding protein of the present disclosure comprises a VH domain sequence having at least 90%, 91%, 92%, 93%, 94%, 95%, 96%, 97%, 98%, 99%, or 100% sequence identity to the amino acid sequence of SEQ ID NO:228 and/ or a VL domain sequence having at least 90%, 91%, 92%, 93%, 94%, 95%, 96%, 97%, 98%, 99%, or 100% sequence identity to the amino acid sequence of SEQ ID NO:229. In some embodiments, a bispecific binding protein of the present disclosure comprises a VH domain sequence having at least 90%, 91%, 92%, 93%, 94%, 95%, 96%, 97%, 98%, 99%, or 100% sequence identity to a VH domain sequence encoded by the polynucleotide sequence of SEQ ID NO:288 and/ or a VL domain sequence having at least 90%, 91%, 92%, 93%, 94%, 95%, 96%, 97%, 98%, 99%, or 100% sequence identity to a VL domain sequence encoded by the polynucleotide sequence of SEQ ID NO:289.

It will be appreciated by one of skill in the art that the CDRs and/or VH/VL domains of any of the anti-dystroglycan antibodies described herein may be combined in a bispecific binding protein with the CDRs and/or VH/VL domains of any of the anti-laminin-2 antibodies (*e.g*., antibodies that bind the LG-4/5 and/or LG-5 domains of laminin-2) described herein in any combination or configuration (*e.g.*, having a V_{H1}/V_{L1} binding pair specific for the extracellular domain of dystroglycan and a V_{H2}/V_{L2} binding pair specific for laminin-2, or having a V_{H2}/V_{L2} binding pair specific for the extracellular domain of dystroglycan and a V_{H1}/V_{L1} binding pair specific for laminin-2).

It will be appreciated by one of skill in the art that the CDRs and/or VH/VL domains of any of the anti-dystroglycan antibodies described herein may be combined in a multispecific binding protein with the CDRs and/or VH/VL domains of any of the anti-laminin-2 antibodies (*e.g*., antibodies that bind the LG-4/5 and/or LG-5 domains of laminin-2) described herein in any combination or configuration. In some embodiments, a binding protein (*e.g*., multispecific binding protein) of the present disclosure comprises 1, 2, 3, 4, 5, 6, or more CDRs shown in Table A2 or from a variable domain or polypeptide sequence shown in Tables D2, I2, I3, or I4. In some embodiments, a binding protein (*e.g*., multispecific binding protein) of the present disclosure comprises a 1, 2, 3, 4, 5, or 6 variable domain sequences shown in Tables D2, I2, I3, or I4, or a 1, 2, 3, 4, 5, or 6 variable domain sequences encoded by a polynucleotide shown in Table G2 (*e.g.*, 1, 2, or 3 VH/VL binding pairs, each comprising a VH and VL domain). In some embodiments, a binding protein (*e.g.*, multispecific binding protein) of the present disclosure comprises 1, 2, 3, or 4 variable domain framework sequences shown in Table I4.

**Table A. CDR sequences of anti-beta-DG VH and VL regions.**

| **bDG** | **Variable Heavy Chain (VH)** | | | | | | **Variable Light Chain (VL)** | | | | | |
|---|---|---|---|---|---|---|---|---|---|---|---|---|
| | **CDR 1** | **SEQ ID NO** | **CDR 2** | **SEQ ID NO** | **CDR 3** | **SEQ ID NO** | **CDR 1** | **SEQ ID NO** | **CDR 2** | **SEQ ID NO** | **CDR 3** | **SEQ ID NO** |
| AS19 | GFTFTDSV | 1 | IYPGSGSI | 9 | AMRRSY | 18 | QSIVHSNGNTY | 28 | KVS | 38 | FQGSHVPLT | 43 |
| AS30 S/S | GFTFTDSV | 1 | IYPGSGNF | 10 | AMRRSS | 19 | QTIVHSNSKTY | 29 | KVS | 38 | FQGSHVPLT | 43 |
| B04 | GFTFSSYA | 2 | ISGSGGST | 11 | ARLGYCSSTSCYLSAFDI | 20 | QSISSW | 30 | DAS | 39 | QQYNSYPLT | 44 |
| B06 | GYSFSNYW | 3 | IYPGDSDT | 12 | ARGVIINGTTSGFDY | 21 | QSVSSN | 31 | GAS | 40 | QHYNNLPLT | 45 |
| C107 | GFNIKDTY | 4 | IDPANGNT | 13 | GRSGGNYVGY | 22 | QSLLDSGNQKNY | 32 | WAS | 41 | QQYYTYPWT | 46 |
| D87/D3 9/D173 | GFNIKDTY | 4 | IDPANGNT | 13 | GRSRGNYFDY | 23 | QSLLYSSNQKNY | 33 | WAS | 41 | QQYYTYPWT | 46 |
| TDG-2 | GYTFTTYY | 5 | INPSAGNT | 14 | ARELDI | 24 | QDIRND | 34 | AAS | 42 | LQDFNFPFT | 47 |
| TDI-11 | GFTFSSYG | 6 | IWYDGSNK | 15 | AREGMVRGALFDY | 25 | QSVSSSY | 35 | GAS | 40 | QQDYNLPYT | 48 |
| TDI-23 | GYSFTSYW | 7 | IYPGDSDT | 16 | ARQLRDYYGMDV | 26 | QTISSNY | 36 | GAS | 40 | QQDYNLPRT | 49 |
| TDI-38 | GYSFTSYW | 8 | IYPGDSDT | 17 | ARQLRDYYSMDV | 27 | QSVSSSY | 37 | GAS | 40 | QQDYNLPRT | 50 |

**Table A2. CDR sequences of humanized antibodies.**

| | **Variable Heavy Chain (VH)** | | | | | | **Variable Ligth Chain (VL)** | | | | | |
|---|---|---|---|---|---|---|---|---|---|---|---|---|
| | **CDR 1** | **SEQ ID NO** | **CDR 2** | **SEQ ID NO** | **CDR 3** | **SEQ ID NO** | **CDR 1** | **SEQ ID NO** | **CDR 2** | **SEQ ID NO** | **CDR 3** | **SEQ ID NO** |
| **bDG** | | | | | | | | | | | | |
| AS30SS_Hu6 | GFTFTDSV | 316 | IYPGSGNF | 318 | AMRRSS | 320 | QTIVHSNSKTY | 332 | KVS | 334 | FQGSHVPLT | 336 |
| AS30SS_Hu9 | GFTFTDSV | 316 | IYPGSGNF | 318 | AM RRSS | 320 | QTIVHSNSKTY | 332 | KVS | 334 | FQGSHVPLT | 336 |

| **L-4/5** | | | | | | | | | | | | |
|---|---|---|---|---|---|---|---|---|---|---|---|---|
| C3_Hu10 | GFTFSSYT | 380 | ISSSGSNT | 382 | ARFDYGSSLDS | 384 | QSISNN | 396 | YAS | 398 | QQSKSWPRT | 400 |
| C3_Hu11 | GFTFSSYT | 412 | ISSSGSNT | 414 | ARFDYGSSLDS | 416 | QSIGNN | 428 | YAS | 430 | QQSKSWPRT | 432 |
| C21_Hu11 | GFTFSSYT | 444 | ISSSGSNT | 446 | ARFDYGSSLDS | 448 | QSISNY | 460 | YAS | 462 | QQSKSWPRT | 464 |
| C21_Hu21 | GFTFSSYT | 476 | ISSSGDNT | 478 | ARFDYGSSLDS | 480 | QSISNY | 492 | YAS | 494 | QQSKSWPRT | 496 |

**Table B. CDR sequences of anti-LG-5 VH and VL regions.**

| **L-5** | **Variable Heavy Chain (VH)** | | | | | | **Variable Light Chain (VL)** | | | | | |
|---|---|---|---|---|---|---|---|---|---|---|---|---|
| | **CDR 1** | **SEQ ID NO** | **CDR 2** | **SEQ ID NO** | **CDR 3** | **SEQ ID NO** | **CDR 1** | **SEQ ID NO** | **CDR 2** | **SEQ ID NO** | **CDR 3** | **SEQ ID NO** |
| AN01 | GYTFTSYN | 51 | INPYNDGT | 56 | AIYGNSY | 61 | KSLLHSNGNTY | 66 | YMS | 71 | MQGLEYPYT | 76 |
| C3 | GFTFSSYT | 52 | ISSGGGNT | 57 | ARFDYGSSLDS | 62 | QSISNN | 67 | YAS | 72 | QQSKNWPRT | 77 |
| C21 | GFTFSSYT | 53 | ISSGGDNT | 58 | ARFDYGSSLDC | 63 | QSISNY | 68 | YAS | 73 | QQSKSWPRT | 78 |
| TLF39 | GYSFTSYW | 54 | IYPGDSDT | 59 | ARRGYRSSWYFDY | 64 | QGIRND | 69 | AAS | 74 | LQDYNYPLT | 79 |
| TLF86 | GFTFDDYG | 55 | INWNGGST | 60 | AREGGELLMDY | 65 | QSVSTY | 70 | DAS | 75 | QQRSNWPPT | 80 |

**Table C. CDR sequences of anti-LG-4/5 VH and VL regions.**

| **L-4/5** | **Varirable Heavy Chain (VH)** | | | | | | **Variable Light Chain (VL)** | | | | | |
|---|---|---|---|---|---|---|---|---|---|---|---|---|
| | **CDR 1** | **SEQ ID NO** | **CDR 2** | **SEQ ID NO** | **CDR 3** | **SEQ ID NO** | **CDR 1** | **SEQ ID NO** | **CDR 2** | **SEQ ID NO** | **CDR 3** | **SEQ NO** |
| CL-40968 | GFTFSHYS | 81 | IYPSGGT | 96 | ARHWRGYSSSWYHPAYFDY | 111 | QSVSSY | 126 | DAS | 141 | QQRSNWPLT | 155 |
| CL-40992 | GFTFSWYP | 82 | IYPSGGTT | 97 | ARSYYYDSSGYYSHDAFDI | 112 | QSIDTY | 127 | AAS | 142 | QQSYSSPGIT | 156 |
| CL-41136 | GFTFSDYE | 83 | IWPSGGLT | 98 | ARDSYYYDSSGALGY | 113 | QSVSNW | 128 | KAS | 143 | LQYVSYPLT | 157 |
| CL-41400 | GFTFSYYD | 84 | IYSSGGHT | 99 | ARPGYSSGWYDGTYFDY | 114 | QSIDTW | 129 | SAS | 144 | QQYKTYPFT | 158 |
| CL-41500 | GFTFSHYQ | 85 | ISPSGGFT | 100 | TREPGRLWAFDI | 115 | QDIRNW | 130 | AAS | 145 | QQADSSPRT | 159 |
| TLG3/TL G4 | GYTFTGYY | 86 | INPNSGGT | 101 | AVFGSGSS | 116 | QGISNS | 131 | AAS | 146 | QQYKSYPYT | 160 |
| TLG26 | GNTFTGYY | 87 | IKPSTGDT | 102 | AVFGSGSS | 117 | QGISNY | 132 | AAS | 147 | QQYKTYPYT | 161 |
| TLI-3 | GFTFSSYG | 88 | IWYDGSN K | 103 | AREGGWYGGDYYYGMDV | 118 | QGISSA | 133 | DAS | 148 | HQFNNYPFT | 162 |
| TLI-7 | GFTFSSYA | 89 | ISGRGGSP | 104 | AKDGDGSGPPYYFDY | 119 | QGISSW | 134 | AAS | 149 | QQYNSYPYT | 163 |
| TTLK71-4-6 | GFTFSGYG | 90 | IWSDGSN R | 105 | ARDRGITMVRGLIIKYYYYYGLDV | 120 | QSVSSY | 135 | DAS | 150 | QQRSNWWT | 164 |
| TTLK123-3 | GFTFSSFG | 91 | IYYDGSNK | 106 | ARDDNWNDGDFDY | 121 | QGISSY | 136 | AAS | 151 | QQLNSYPRT | 165 |
| TTLK145-6-3 | GFTFNRFV | 92 | ISGSGGST | 107 | AKDFTYYYGSGNYYNWFDP | 122 | QSISSW | 137 | KAS | 152 | QQYNSYSRT | 166 |
| TTLK170-2 | GGSFSGYY | 93 | INHSGGT | 108 | ARTSDYDYYYYGMDV | 123 | SGINLGR YR | 138 | YYSDSSK | 153 | MIWHRSALFI | 167 |
| WJL10 | GYTFTSYE | 94 | IYPRDGDT | 109 | ARHTPGAF | 124 | QSLVHSN GDTY | 139 | KVS | 38 | SQSTHVPYT | 168 |
| WJL48 | GFTFSRYA | 95 | ISSGGDYI | 110 | TRVLFYYYGSSYVFFDY | 125 | QDISNF | 140 | YTS | 154 | QQGHTLPYT | 169 |

**Table D. Amino acid sequences of anti-beta-DG VH and VL regions.**

| **bDG** | **Chain** | | **SEQ ID NO** |
|---|---|---|---|
| AS19 | VH | QVQLQQSGPELVKPGASVKMSCKASGFTFTDSVISWVKQRTGQGLEWIGEIYPGSGSI YYNEKFKGKATLTADKSSNTAYMQLRSLTSEDSAVYFCAMRRSYWGQGTLVTVSA | 170 |
| | VL | DVLMTQTPLSLPVSLGDQASISCRSSQSIVHSNGNTYLEWYLQKPGQSPKLLIYKVSNR FSGVPDRFSGSGSGTDFTLKISRVEAEDLGVYYCFQGSHVPLTFGAGTKLELK | 171 |
| AS30 S/S | VH | QIQLQQSGPELVKPGASVKMSCEASGFTFTDSVITWVKQRPGQGLEWIGEIYPGSGN FYYNEKFKGKATLTADKSSNTAYMQLRSLTSEDSAVYFCAMRRSSWGQGTLVTVSA | 172 |
| | VL | DVLMTQTPLSLPVSLGDQASISCRSSQTIVHSNSKTYLEWYLQKPGQSPKLLIYKVSNRF SGVPDRFSGSGSGTDFTLKISRVEAEDLGVYYCFQGSHVPLTFGAGTKLELK | 173 |
| B04 | VH | QVQLQQSGGGLVQPGGSLRLSCAASGFTFSSYAMSWVRQAPGKGLEWVSAISGSGG STYYADSVKGRFTVSRDNSKNTLYLQMNSLRAEDTAVYYCARLGYCSSTSCYLSAFDIW GQGTMVTVSS | 174 |
| | VL | EIVLTQSPSTLSASVGDRVTITCRASQSISSWLAWYQQKPGKAPKLLIYDASSLESGVPS RFSGSGSGTEFTLTISSLQPDDFATYYCQQYNSYPLTFGGGTKVEIK | 175 |
| B06 | VH | QIQLVQSGAEVKKPGKSLKIACKGSGYSFSNYWIGWVRQMPGKGLEWMGIIYPGDS DTRYSPSFHGQVTISADKSISTAYLQWSSLKASDTAMYYCARGVIINGTTSGFDYWGQ GTLVIVSS | 176 |
| | VL | ETTLTQSPATLSVSPGERATLSCRASQSVSSNLAWYQQKPGQAPRLLMYGASTRATGI PARFSGSGSGTEFTLTISSLQSEDFAVYYCQHYNNLPLTFGGGTKVDLK | 177 |
| C107 | VH | EVQLQQSGAELVKPGASVKLSCTASGFNIKDTYIHWVKQRPEQGLEWIGRIDPANGN TKYDPKFQGKATITADTSSNIVYVQFSSLTSEDTAVYYCGRSGGNYVGYWGQGTTLTV SS | 178 |
| | VL | DIVMSQSPSSPTVSVGEKVTMTCKSSQSLLDSGNQKNYLAWYQQKPGQSPKLLIYWA STRKSGVPDRFTGSGSGTDFTLSISSVKAEDLAVYYCQQYYTYPWTFGGGTKLEIK | 179 |
| D87/D3 9/D173 | VH | EVQLQQSGAELVKPGASVKLSCTASGFNIKDTYMHWVKERPEQGLEWIGRIDPANG NTKYDPKFQGKATITADTSSNTAYLQLSSLTSEDTAVYYCGRSRGNYFDYWGQGTTLT VSS | 180 |
| | VL | DIVMSQPPSSLAVSVGEKVTMTCKSSQSLLYSSNQKNYLAWYQQKPGQSPKLLIYWA STRESGVPDRFTGSGSGTDFTLTISSVKAEDLAVYYCQQYYTYPWTFGGGTKLEIK | 181 |
| TDG-2 | VH | QVQLVQSGAEVKKPGTSVKVSCKASGYTFTTYYMHWVRQAPGQGLEWMGLINPSA GNTRNAQKFQGRVTMTRDTSTNTVYMELSSLRSEDTAVYYCARELDIWGQGTKVTV SS | 182 |
| | VL | AIQMTQSPSSLSASVGDRVTITCRASQDIRNDLGWYQQKPGKAPKLLIYAASSLQSGV PSRFSGNGSGTDFTLTINSLQPEDFATYYCLQDFNFPFTFGPGTTVDIN | 183 |
| TDI-11 | VH | QVQLVESGGGVVQSGRSLRLSCAASGFTFSSYGMHWVRQAPGKGLEWVAVIWYDG SNKYYADSVKDRFTISRDNSKKTVYLQMNSLRAEDTAVYYCAREGMVRGALFDYWG QGTLVTVSS | 184 |
| | VL | EIVMTQSPATLSLSPGERATLSCRASQSVSSSYLSWYQQKPGQAPRLLIYGASTRATGIP ARFSGSGSGPDLTLTISSLQPEDFAVYYCQQDYNLPYTFGQGTKLEIK | 185 |
| | | | |
| TDI-23 | VH | EVQLVQSGAEVKKPGESLKISCKGSGYSFTSYWIGWVRQMPGKGLEWMGVIYPGDS DTRYSPSFQGQVTMSADKSISTAYLQWSSLKASDSAMYYCARQLRDYYGMDVWGQ GTTVTVSS | 186 |
| | VL | EIVMTQSPATLSLSPGERATLSCRASQTISSNYFSWYQQKPGQAPRLLIYGASTRATGIP ARFSGSGSETDFTLTISSLQPEDFAVYYCQQDYNLPRTFGQGTKVEIK | 187 |
| TDI-38 | VH | EVQLVQSGAEVKKPGESLKISCKGSGYSFTSYWIGWVRQMPGKGLEWMGIIYPGDSD TRYSPSFQGQVTISADKSISTAYLHWSSLKASDTAMYYCARQLRDYYSMDVWGQGTT VTVSS | 188 |
| | VL | EIVMTQSPATLSLSPGERATLSCRASQSVSSSYLSWYQQKPGQAPRLLIYGASTRATGIA ARFSGSGSGTDFTLTISSLQPEDFAVYYCQQDYNLPRTFGQGTKVEIK | 189 |

**Table D2. Amino acid sequences of humanized VH and VL regions.**

| | **Chain** | | **SEQ ID NO** |
|---|---|---|---|
| AS30SS_Hu6 | VH | QVQLVQSGAEVVKPGSSVKVSCEASGFTFTDSVITWVRQRPGQGLEWIGEIYPG SGNFYYNEKFQGRVTITADKSTSTAYMELSSLRSEDTAVYFCAMRRSSWGQGTL VTVSS | 314 |
| | VL | DVVMTQTPLSLSVTPGQPASISCKSSQTIVHSNSKTYLEWYLQKPGQSPQLLIYKV SSRFSGVPDRFSGSGSGTDFTLKISRVEAEDVGVYYCFQGSHVPLTFGQGTKLEI K | 330 |
| AS30SS_Hu9 | VH | QVQLVQSGAEVVKPGSSVKVSCEASGFTFTDSVITWVRQRPGQGLEWIGEIYPG SGNFYYNEKFQGRVTITADKSTSTAYMELSSLRSEDTAVYFCAMRRSSWGQGTL VTVSS | 346 |
| | VL | DVVMTQTPLSLSVTPGQPASISCRSSQTIVHSNSKTYLEWYLQKPGQSPQLLIYK VSNRFSGVPDRFSGSGSGTDFTLKISRVEAEDVGVYYCFQGSHVPLTFGAGTKL EIK | 362 |
| C3_Hu10 | VH | EVQLVESGGGLVKPGGSLRLSCAASGFTFSSYTMSWVRQAPGKGLEWVASISS SGSNTYYPDSVKGRFTISRDNAKNNLYLQMNSLRAEDTALYYCARFDYGSSLDS WGQGTLLTVSS | 378 |
| | VL | EIVLTQSPDFLSVTPKEKVTLTCRASQSISNNLHWYQQKSDQSPKLLIKYASQSIS GIPSRFSGSGSGTDFTLTINSVEAEDAATYFCQQSKSWPRTFGGGTKLEIK | 394 |
| C3_Hu11 | VH | EVQLVESGGGLVKPGGSLRLSCAASGFTFSSYTMSWVRQAPGKGLEWVASISS SGSNTYYPDSVKGRFTISRDNAKNNLYLQMNSLRAEDTALYYCARFDYGSSLDS WGQGTLLTVSS | 410 |
| | VL | EIVLTQSPDFLSVTPKEKVTLTCRASQSIGNNLHWYQQKSDQSPKLLIKYASQSIS GIPSRFSGSGSGTDFTLTINSVEAEDAATYFCQQSKSWPRTFGGGTKLEIK | 426 |
| C21_Hu11 | VH | EVQLVESGGGLVKPGGSLRLSCAASGFTFSSYTMSWVRQAPGKGLEWVASISS SGSNTYYPDSVKGRFTISRDNAKNNLYLQMNSLRAEDTALYYCARFDYGSSLDS WGQGTLLTVSS | 442 |
| | VL | EIVLTQSPDFLSVTPKEKVTLSCRASQSISNYLHWYQQKSDQSPKLLIKYASQSIS GIPSRFSGSGSGTDFTLSINSVEAEDAATYFCQQSKSWPRTFGGGTKLEIK | 458 |
| C21_Hu21 | VH | EVQLVESGGGLVQPGGSLRLSCAASGFTFSSYTMSWVRQAPGKGLEWVATISS SGDNTYYPDSVKGRFTISRDNSKNTLYLQMSSLRAEDTALYYCARFDYGSSLDS WGQGTTLTVSS | 474 |
| | VL | EIVLTQSPDFLSVTPGEKVTLTCRASQSISNYLHWYQQKSDQSPKLLIKYASQSIS GVPSRFSGSGSGTDFTLTISSVEAEDFATYFCQQSKSWPRTFGGGTKLEIK | 490 |

**Table E. Amino acid sequences of anti-LG-5 VH and VL regions.**

| **LG-5** | **Chain** | **Amino Acid Sequence** | **SEQ ID NO** |
|---|---|---|---|
| AN01 | VH | EVQLQQSGPELVKPGASVKMSCKASGYTFTSYNIHWVKQKPGQGLEWIGYINPYND GTKYSEKFKGKATLTSDRSSSTAYMEVSSLTSEDSAVYYCAIYGNSYWGQGSTLTVSS | 190 |
| | VL | DIVMTQAAPSIPVTPGESVSISCRSSKSLLHSNGNTYLYWFLQRPGQSPQRLIYYMSNL DSGVPDRFSGRGSGTDFTLRISRVEAEDVGVYYCMQGLEYPYTFGGGTKLEIK | 191 |
| C3 | VH | DVMLVESGGDLVKPGGSLKLSCAASGFTFSSYTMSWVRQTPEKRLEWVASISSGGGN TYYPDSVKGRFTISRDNAKNNLYLQMSSLRSEDTALYYCARFDYGSSLDSWGQGTTLT VSS | 192 |
| | VL | DIVLTQSPATLSVTPGDSVSLSCRASQSISNNLHWYQQKSHESPRLLIKYASQSISGIPSR FSGSGSGTDFTLSINSVETEDFGMYFCQQSKNWPRTFGGGTKLEIK | 193 |
| C21 | VH | EVMLVESGGGLVKPGGSLKLSCAASGFTFSSYTMSWVRQTPEKRLEWVATISSGGDN TYYPDSVKGRFTISRDNAKNNLYLQMSSLRSEDTALYYCARFDYGSSLDCWGQGTTLT VSS | 194 |
| | VL | DIVLTQSPATLSVTPGDSVSLSCRASQSISNYLHWYQQKSHESPRLLIKYASQSISGIPSR FSGSGSGTDFTLSINSVETEDFGMYFCQQSKSWPRTFGGGTELEIK | 195 |
| TLF39 | VH | EVQLVQSGAEVKKPGESLKISCKGSGYSFTSYWIGWVRQMPGKGLEWMGIIYPGDSD TRYSPSFQGQVTISADKSISTAYLQWSSLKASDTAMYYCARRGYRSSWYFDYWGQGT LVTVSS | 196 |
| | VL | AIQMTQSPSSLSASVGDRVTITCRASQGIRNDLGWYQQKPGKAPKLLIYAASSLQSGV PSRFSGSGSGTDFTFTISSLQPEDFATYYCLQDYNYPLTFGGGTKVEIK | 197 |
| TLF86 | VH | EVQLVESGGGVVRPGGSLRLSCAASGFTFDDYGMSWVRQAPGKGLEWVSGINWNG GSTGYADSVKGRFTISRDNAKNSLYLQMNSLRAEDTALYHCAREGGELLMDYWGQG TLVTVSS | 198 |
| | VL | EIVLTQSPATLSLSPGERATLSCRASQSVSTYLAWYQQKPGQAPRLLIYDASNRATGIPP RFSGSGSGTDFTLTISSLEPEDFAVYYCQQRSNWPPTFGGGTTVEIK | 199 |

**Table F. Amino acid sequences of anti-LG-4/5 VH and VL regions.**

| **LG-4/5** | **Chain** | **Amino Acid Sequence** | **SEQ ID NO** |
|---|---|---|---|
| CL-40968 | VH | EVQLLESGGGLVQPGGSLRLSCAASGFTFSHYSMVWVRQAPGKGLEWVSYIYPSGGT SYADSVKGRFTISRDNSKNTLYLQMNSLRAEDTAVYYCARHWRGYSSSWYHPAYFDY WGQGTLVTVSS | 200 |
| | VL | DIQMTQSPATLSLSPGERATLSCRASQSVSSYLAWYQQKPGQAPRLLIYDASNRATGIP ARFSGSGSGTDFTLTISSLEPEDFAVYYCQQRSNWPLTFGGGTKVEIK | 201 |
| CL-40992 | VH | EVQLLESGGGLVQPGGSLRLSCAASGFTFSWYPMMWVRQAPGKGLEWVSSIYPSGG TTTYADSVKGRFTISRDNSKNTLYLQMNSLRAEDTAVYYCARSYYYDSSGYYSHDAFDI WGQGTMVTVSS | 202 |
| | VL | DIQMTQSPSSLSASVGDRVAITCRASQSIDTYLNWYQQKPGKAPKLLIYAASKLEDGVP SRFSGSGTGTDFTLTIRSLQPEDFASYFCQQSYSSPGITFGPGTKVEIK | 203 |
| CL-41136 | VH | EVQLLESGGGLVQPGGSLRLSCAASGFTFSDYEMHWVRQAPGKGLEWVSSIWPSGG LTKYADPVKGRFTISRDNSKNTLYLQMNSLRAEDTAVYYCARDSYYYDSSGALGYWGQ GTLVTVSS | 204 |
| | VL | DIQMTQSPSTLSASVGDRVTITCRASQSVSNWLAWYQQKPGKAPKLLIYKASSLESGV PSRFSGSGSGTEFTLTISSLQPDDFATYYCLQYVSYPLTFGGGTKVDIK | 205 |
| CL-41400 | VH | EVQLLESGGGLVQPGGSLRLSCAASGFTFSYYDMYWVRQAPGKGLEWVSRIYSSGGH TWYADSVKGRFTISRDNSKNTLYLQMNSLRAEDTAVYYCARPGYSSGWYDGTYFDY WGQGTLVTVSS | 206 |
| | VL | DIQMTQSPSTLSASVGDRVTITCRASQSIDTWLAWYRQKPGKAPNVVIHSASTLQSGV PARFSGSGFGTEWTLTITNLQPDDFATYYCQQYKTYPFTFGQGTKLEIK | 207 |
| CL-41500 | VH | EVQLLESGGGLVQPGGSLRLSCAASGFTFSHYQMEWVRQAPGKGLEWVSSISPSGGF TSYADSVKGRFTISRDNSKNTLYLQMNSLRAEDTAVYYCTREPGRLWAFDIWGQGTM VTVSS | 208 |
| | VL | DIQMTQSPSFVSASVGDRVTITCRASQDIRNWLAWYQQESGKAPRLLISAASSRHSGV SSRFSGSGSGTDFTLTITSLQPEDSATYFCQQADSSPRTFGQGTKVEIK | 209 |
| TLG3/TL G4 | VH | QVQLVQSGAEVKKPGASVKVSCKASGYTFTGYYMHWVRQAPGQGLEWMGWINPN SGGTNYAQKFQGRVTMTRDTSINTAYMELSRLRSDDTAVYYCAVFGSGSSWGQGTL VTVSS | 210 |
| | VL | DIQMTQSPSSLSASVGDRVTITCRASQGISNSLAWFQQKPGKAPKSLIYAASSLQSGVP SKFSGSGSGTDFTLTISSLQPEDFATYYCQQYKSYPYTFGQGTKLEIK | 211 |
| TLG26 | VH | QVQLVQSGAEVKKPGASVKVSCKASGNTFTGYYIHWVRQAPGQGLEWMGWIKPST GDTNYAQNFLDRVTMTRDTSISTAYMELSRLRSDDTAVYYCAVFGSGSSWGQGTLVT VSS | 212 |
| | VL | DIHMTQSPSSLSAFVGDRVTITCRASQGISNYLAWFQQKPGKAPKSLIYAASSLQSGVP SKFSGSGSGTDFTLTINNLQPEDFATYYCQQYKTYPYTFGQGTKLEIK | 213 |
| TLI-3 | VH | QVQLVESGGGVVQPGRSLRLSCAASGFTFSSYGMHWVRQAPGKGLEWVAVIWYDG SNKYYADSVKGRFTISRDNSKNTLYLQMNSLRAEDAAVYYCAREGGWYGGDYYYGM DVWGQGTTVTVSS | 214 |
| | VL | AIQLTQSPSSLSASVGDRVTITCRASQGISSALAWYQQKPGKAPKLLIYDASSLESGVPS RFSGSGSGTDFTLTISSLQPEDFATYYCHQFNNYPFTFGPGTKVDIK | 215 |
| TLI-7 | VH | EVQLLESGGGLVQPGGSLRLSCVASGFTFSSYAMSWVRQAPGKGLEWVSGISGRGGS PNYADSVKGRFTISRDNSKNTLYLQMNSLRAEDTAVYYCAKDGDGSGPPYYFDYWGQ GTLVTVSS | 216 |
| | VL | DIQMTQSPSSLSASVGDRVTITCRASQGISSWLAWYQQKPEKAPKSLIYAASSLQSGVP SRFSGSGSGTDFTLTISSLQPEDFATYYCQQYNSYPYTFGQGTKLEIK | 217 |
| TTLK71-4-6 | VH | QVQLMESGGGVVQPGRSLRLSCAASGFTFSGYGMHWVRQAPGKGLEWVAVIWSD GSNRYYTDSVKGRFTISRDNSKNTLSLQMNSLRAEDTAVYYCARDRGITMVRGLIIKYY YYYGLDVWGQGTSVTVSS | 218 |
| | VL | EIVLTQSPATLSLSPGERATLSCRASQSVSSYLAWYQQKPGQAPRLLIYDASNRATGIPA RFSGSGSGTDFTLTISSLEPEDFAVYYCQQRSNWWTFGQGTKVEIK | 219 |
| TTLK123 -3 | VH | QVQLVESGGGVVQPGRSLRLSCAASGFTFSSFGMHWVRQAPGKGLEWVAVIYYDGS NKYYADSVKGRFTISRDNSKNTLYLQMNSLRAEDTAVYYCARDDNWNDGDFDYWG QGTLVTVSS | 220 |
| | VL | DIQLTQSPSFLSASVGDRVTITCRASQGISSYLAWYQQKPGKAPKLLIYAASTLQSGVPS RFSGSGSGTEFTLTISSLQPEDFATYYCQQLNSYPRTFGQGTKVEIK | 221 |
| TTLK145 -6-3 | VH | EVQLLESGGGLVQPGESLRLSCAASGFTFNRFVMSWVRQAPGKGLEWVSTISGSGGS TYYADSVKGRFTISRDNSKNTLYLQMNSLRAEDTAVYYCAKDFTYYYGSGNYYNWFDP RGQGTLVTVSS | 222 |
| | VL | DIQMTQSPSTLSTSVGDRVTITCRASQSISSWLAWYQQKPGKAPKLLIYKASSLESGVP SRFSGSGSGTEFTLTISSLQPDDFATYYCQQYNSYSRTFGQGTKVEIK | 223 |
| TTLK170 -2 | VH | QVQLQQWGAGLLKPSETLSLTCAVYGGSFSGYYWSWIRQPPGKGLEWIGEINHSGGT NYNPSLKSRVTISVDTSKNHFSLKLSSVTAADTAVYYCARTSDYDYYYYGMDVWGQGT TVTVSS | 224 |
| | VL | QPVLTQPTSLSASPGASARLTCTLRSGINLGRYRIFWYQQKPESPPRYLLSYYSDSSKHQ GSGVPSRFSGSKDASSNAGILVISGLQSEDEADYYCMIWHRSALFIFGSGTKVTVL | 225 |
| WJL10 | VH | QVQLQQSGPELVKPGASVKLSCKASGYTFTSYEINWLKQRPGQGLEWIGLIYPRDGDT KYNEKFKGKATLTADTSSSTAYMELHSLTSEDSAVYFCARHTPGAFWGQGTLVTVSA | 226 |
| | VL | DVVMTQTPLSLPVSLGDQASISCRSSQSLVHSNGDTYLHWYLQKPGQSPKLLIYKVSN RFSGVPDRFSGSGSGTDFTLKISRVEAEDLEIYFCSQSTHVPYTFGGGTKLEIK | 227 |
| WJL48 | VH | DVKLVESGEGLVKPGGSLKLSCAASGFTFSRYAMSWVRQTPEKRLEWVAYISSGGDYI HYGETVKGRFTISRDNARNTLYLQMSSLKSEDTAMYYCTRVLFYYYGSSYVFFDYWGQ GTTLTVSS | 228 |
| | VL | DIQMTQTTSSLSVSLGDRVTISCRASQDISNFLNWYQQKPDGTVNLLIYYTSKLHSGVP SRFSGGGSGRDYSLTINNLEQEDIASYFCQQGHTLPYTFGGGTKLEIK | 229 |

**Table G. Nucleic acid sequences of anti-beta-DG VH and VL regions.**

| **bDG** | **Chain** | **Nucleotide Sequence** | **SEQ ID NO** |
|---|---|---|---|
| AS19 | VH | CAGGTGCAGCTGCAGCAGAGCGGTCCCGAGCTGGTGAAACCTGGCGCATCAGTC AAAATGAGCTGCAAGGCCTCCGGCTTCACTTTTACCGACTCAGTGATCAGCTGGGT CAAGCAGCGAACCGGTCAGGGACTGGAGTGGATCGGAGAAATCTACCCTGGATC TGGGAGTATCTACTATAACGAGAAGTTCAAAGGGAAGGCAACACTGACTGCCGAC AAAAGCTCCAATACAGCCTATATGCAGCTGCGATCCCTGACTTCTGAAGATAGCGC CGTGTACTTTTGCGCAATGCGGAGGTCCTATTGGGGTCAGGGCACCCTGGTGACA GTCTCTGCT | 230 |
| | VL | GACGTGCTGATGACCCAGACACCCCTGAGTCTGCCTGTCTCACTGGGAGATCAGG CTTCTATCAGTTGCCGAAGCTCCCAGAGCATCGTGCATTCCAACGGAAATACCTAC CTGGAGTGGTATCTGCAGAAGCCAGGGCAGTCCCCCAAGCTGCTGATCTACAAAG TGTCTAACCGGTTCAGTGGCGTCCCAGACAGGTTTTCAGGTAGCGGCTCCGGAACT GATTTCACCCTGAAAATTTCCCGGGTGGAGGCAGAAGACCTGGGTGTCTACTATTG CTTCCAGGGCAGCCATGTGCCCCTGACTTTTGGGGCCGGTACCAAGCTGGAGCTG AAA | 231 |
| AS30 S/S | VH | CAGATCCAGCTGCAGCAGTCCGGTCCCGAGCTGGTGAAACCTGGCGCATCTGTCA AGATGAGTTGCGAAGCCTCAGGCTTCACTTTTACCGACTCCGTGATTACCTGGGTC AAACAGCGCCCAGGCCAGGGACTGGAGTGGATCGGAGAAATCTACCCCGGATCT GGGAACTTCTACTATAATGAGAAGTTTAAAGGGAAGGCAACACTGACTGCCGACA AGAGCTCCAACACCGCCTACATGCAGCTGCGATCACTGACAAGCGAAGATAGCGC CGTGTACTTCTGCGCAATGCGGAGGTCTAGTTGGGGTCAGGGCACCCTGGTGACA GTCTCTGCT | 232 |
| | VL | GACGTGCTGATGACCCAGACACCCCTGTCTCTGCCTGTCAGTCTGGGAGATCAGGC TTCTATCAGTTGCCGAAGCTCCCAGACCATCGTGCATTCAAACAGCAAGACATACC TGGAGTGGTATCTGCAGAAACCAGGCCAGTCCCCCAAGCTGCTGATCTACAAAGT GTCAAATCGGTTCTCTGGAGTCCCAGACAGGTTTTCCGGTTCTGGCAGTGGAACTG ATTTCACCCTGAAGATTTCTCGGGTGGAGGCAGAAGACCTGGGTGTCTACTATTGC TTCCAGGGGAGCCATGTGCCCCTGACTTTTGGGGCCGGTACCAAGCTGGAGCTGA AA | 233 |
| B04 | VH | CAGGTGCAGCTGCAGCAGTCGGGGGGAGGCTTGGTACAGCCTGGGGGGTCCCTG AGACTCTCCTGTGCAGCCTCTGGATTCACCTTTAGCAGCTATGCCATGAGCTGGGT CCGCCAGGCTCCAGGGAAGGGGCTGGAGTGGGTCTCAGCTATTAGTGGTAGTGG TGGTAGCACATACTACGCAGACTCCGTGAAGGGCCGGTTCACCGTCTCCAGAGAC AATTCCAAGAACACGCTGTATCTGCAAATGAACAGCCTGAGAGCCGAGGACACGG CCGTATATTACTGTGCGAGGCTAGGATATTGTAGTAGTACCAGCTGCTATTTGTCT GCTTTTGATATCTGGGGCCAAGGGACAATGGTCACCGTCTCTTCA | 234 |
| | VL | GAAATTGTGTTGACACAGTCTCCTTCCACCCTGTCTGCATCTGTAGGAGACAGAGT CACCATCACTTGCCGGGCCAGTCAGAGTATTAGTAGCTGGTTGGCCTGGTATCAGC AGAAACCAGGGAAAGCCCCTAAGCTCCTGATCTATGATGCCTCCAGTTTGGAAAG TGGGGTCCCATCAAGGTTCAGCGGCAGTGGATCTGGGACAGAATTCACTCTCACC ATCAGCAGCCTGCAGCCTGATGATTTTGCAACTTATTACTGCCAACAGTATAATAG TTACCCGCTCACTTTCGGCGGAGGGACCAAGGTGGAGATCAAA | 235 |
| B06 | VH | CAGATCCAGCTGGTGCAGTCTGGCGCCGAAGTGAAGAAGCCTGGCAAGAGCCTG AAGATCGCCTGCAAGGGCTCCGGCTACAGCTTCAGCAACTACTGGATCGGCTGGG TGCGCCAGATGCCTGGAAAAGGACTGGAATGGATGGGCATTATCTACCCTGGCGA CAGCGACACCCGGTACAGCCCCAGCTTCCACGGCCAGGTGACAATCAGCGCCGAC AAGAGCATCTCCACCGCCTACCTGCAGTGGTCCTCCCTGAAGGCCAGCGACACCGC CATGTACTATTGTGCCAGAGGCGTGATCATCAACGGCACCACCAGCGGCTTCGACT ATTGGGGACAGGGCACCCTGGTGATCGTGTCCTCC | 236 |
| | VL | GAGACAACCCTGACCCAGAGCCCCGCCACCCTGTCCGTGTCTCCAGGCGAGAGAG CCACCCTGAGCTGCAGAGCCAGCCAGAGCGTGTCCAGCAACCTGGCCTGGTATCA GCAGAAGCCCGGCCAGGCCCCCAGACTGCTGATGTACGGCGCCAGCACCAGAGC CACCGGCATCCCTGCCAGATTCAGCGGCAGCGGCTCCGGCACCGAGTTCACCCTG ACCATCAGCAGCCTGCAGAGCGAGGACTTCGCCGTGTACTACTGCCAGCACTACA ACAACCTGCCCCTGACCTTCGGCGGAGGCACCAAGGTGGACCTGAAG | 237 |
| C107 | VH | GAAGTCCAACTCCAACAGTCTGGGGCAGAACTTGTCAAACCTGGGGCTTCAGTAA AATTGAGTTGCACAGCAAGTGGCTTTAACATCAAAGACACATATATTCATTGGGTG AAGCAACGACCAGAACAAGGCTTGGAGTGGATCGGTAGGATTGACCCTGCAAAC GGGAATACAAAATATGACCCTAAATTCCAGGGAAAGGCTACAATAACAGCAGACA CCAGCAGTAACATTGTCTATGTGCAATTTAGCTCTCTTACCTCTGAGGACACTGCTG TCTATTATTGCGGACGTAGTGGCGGGAATTATGTGGGTTATTGGGGCCAGGGGAC AACACTCACCGTATCCTCT | 238 |
| | VL | GATATAGTAATGTCCCAGTCTCCTTCATCACCTACTGTGTCAGTTGGAGAAAAAGT CACCATGACCTGTAAGTCCTCACAGTCCCTCTTGGACAGCGGGAATCAGAAAAATT ATCTCGCATGGTATCAGCAAAAGCCAGGGCAGTCCCCTAAGCTGTTGATCTATTGG GCAAGTACAAGGAAAAGTGGCGTGCCTGATAGATTCACAGGGAGCGGCAGCGGG ACAGACTTCACTTTGAGCATCTCTTCAGTAAAAGCCGAAGACCTGGCAGTGTACTA CTGTCAGCAATATTATACCTACCCTTGGACTTTTGGTGGCGGGACCAAACTGGAAA TAAAA | 239 |
| D87/D3 9/D173 | VH | GAGGTTCAACTTCAGCAATCAGGGGCTGAGCTTGTAAAACCTGGAGCCTCTGTAA AACTCTCTTGTACCGCCTCCGGGTTCAACATAAAAGATACATATATGCACTGGGTA AAGGAGCGGCCCGAACAGGGACTCGAATGGATCGGGAGGATTGACCCAGCTAAC GGAAATACCAAGTATGATCCAAAATTTCAGGGGAAAGCTACAATAACCGCCGATA CTTCTAGTAATACAGCATATCTTCAGCTCAGCAGCTTGACAAGCGAAGATACCGCA GTTTACTACTGCGGTCGATCCCGAGGGAATTATTTTGACTACTGGGGCCAGGGTAC TACTCTCACAGTAAGTAGC | 240 |
| | VL | GACATAGTAATGAGCCAGCCACCTAGTTCACTTGCCGTAAGTGTGGGTGAAAAGG TGACTATGACCTGTAAAAGTAGTCAGAGCCTCCTTTACTCATCAAATCAGAAGAAT TACTTGGCCTGGTATCAACAGAAACCTGGACAAAGCCCCAAACTCCTCATATACTG GGCCTCTACCCGAGAGTCCGGCGTACCAGATCGGTTTACCGGTTCTGGATCAGGT ACAGACTTTACACTTACCATCTCTTCAGTGAAGGCTGAGGACTTGGCCGTGTATTA TTGTCAACAATATTATACATATCCTTGGACTTTTGGCGGAGGGACAAAGCTCGAAA TAAAG | 241 |
| TDG-2 | VH | CAGGTGCAGCTGGTGCAGTCTGGCGCCGAAGTGAAGAAACCCGGCACCTCCGTG AAGGTGTCCTGCAAGGCTTCCGGCTACACCTTTACCACCTACTACATGCACTGGGT GCGACAGGCCCCTGGACAGGGCCTGGAATGGATGGGCCTGATCAACCCTTCCGCC GGCAACACCAGAAACGCCCAGAAATTCCAGGGCAGAGTGACCATGACCCGGGAC ACCTCCACCAACACCGTGTACATGGAACTGTCCTCCCTGCGGAGCGAGGACACCG CCGTGTACTACTGTGCCAGAGAGCTGGACATCTGGGGCCAGGGCACCAAAGTGAC CGTGTCCTCT | 242 |
| | VL | GCCATCCAGATGACCCAGTCCCCCAGCTCCCTGTCTGCCTCTGTGGGCGACAGAGT GACCATCACCTGTCGGGCCTCTCAGGACATCCGGAACGACCTGGGCTGGTATCAG CAGAAGCCTGGCAAGGCCCCCAAGCTGCTGATCTACGCCGCTTCCAGTCTCCAGTC CGGCGTGCCCTCCAGATTCTCCGGCAATGGCTCTGGCACCGACTTCACCCTGACCA TCAACTCCCTCCAGCCCGAGGACTTCGCCACCTACTACTGTCTCCAAGACTTCAACT TCCCCTTCACCTTCGGCCCTGGCACCACCGTGGACATCAAC | 243 |
| TDI-11 | VH | CAGGTGCAGCTGGTGGAGTCTGGGGGAGGCGTGGTCCAGTCTGGGAGGTCCCTG AGACTCTCCTGTGCAGCGTCTGGATTCACCTTCAGTAGTTATGGCATGCACTGGGT CCGCCAGGCTCCAGGCAAGGGGCTGGAGTGGGTGGCAGTTATATGGTATGATGG AAGTAATAAATACTATGCAGACTCCGTGAAGGACCGATTCACCATCTCCAGAGACA ATTCCAAGAAAACGGTGTATCTGCAAATGAACAGTCTGAGAGCCGAGGACACGGC TGTTTATTACTGTGCGAGAGAAGGGATGGTTCGGGGAGCCCTCTTTGACTACTGG GGTCAGGGAACCCTGGTCACCGTCTCCTCA | 244 |
| | VL | GAAATTGTAATGACACAGTCTCCAGCCACCCTGTCTTTGTCTCCAGGGGAAAGAGC CACCCTCTCCTGCAGGGCCAGTCAGAGTGTTAGCAGCAGCTACTTATCCTGGTACC AACAGAAACCTGGGCAGGCTCCCAGGCTCCTCATCTATGGTGCATCCACCAGGGC CACTGGCATCCCAGCCAGGTTCAGTGGCAGTGGGTCTGGGCCAGACCTCACTCTC ACCATCAGCAGCCTGCAGCCTGAAGATTTTGCAGTTTATTACTGTCAGCAGGATTA TAACTTACCGTACACTTTTGGCCAGGGGACCAAGCTGGAGATCAAA | 245 |
| TDI-23 | VH | GAGGTGCAGCTGGTGCAGTCTGGAGCAGAGGTGAAAAAGCCCGGGGAGTCTCTG AAGATCTCCTGTAAGGGTTCTGGATACAGTTTTACTAGTTACTGGATCGGCTGGGT GCGCCAGATGCCCGGGAAAGGCCTGGAGTGGATGGGAGTCATCTATCCTGGTGA CTCTGATACCAGATATAGCCCGTCCTTCCAAGGCCAGGTCACCATGTCAGCCGACA AGTCCATCAGTACCGCCTACCTGCAGTGGAGCAGCCTGAAGGCCTCGGACAGCGC CATGTATTACTGTGCGAGACAGCTACGAGACTACTACGGTATGGACGTCTGGGGC CAAGGGACCACGGTCACCGTCTCCTCA | 246 |
| | VL | GAAATTGTAATGACACAGTCTCCAGCCACCCTGTCTTTGTCTCCAGGGGAAAGAGC CACCCTCTCCTGCAGGGCCAGTCAGACTATCAGCAGCAACTACTTTTCCTGGTACC AGCAGAAACCTGGGCAGGCTCCCAGGCTCCTCATCTATGGTGCATCCACCAGGGC CACTGGCATTCCAGCCAGGTTCAGTGGCAGTGGGTCTGAGACAGACTTCACTCTCA CCATCAGCAGCCTGCAGCCTGAAGATTTTGCAGTTTATTACTGTCAGCAGGATTAT AACTTACCTCGGACGTTCGGCCAAGGGACCAAGGTGGAAATCAAA | 247 |
| TDI-38 | VH | GAGGTGCAGCTGGTGCAGTCTGGAGCAGAGGTGAAAAAGCCCGGGGAGTCTCTG AAGATCTCCTGTAAGGGTTCTGGATACAGCTTTACCAGCTACTGGATCGGCTGGGT GCGCCAGATGCCCGGGAAAGGCCTGGAGTGGATGGGGATCATCTATCCTGGTGA CTCTGATACCAGATACAGCCCGTCCTTCCAAGGCCAGGTCACCATCTCAGCCGACA AGTCCATCAGCACCGCCTACCTGCACTGGAGCAGCCTGAAGGCCTCGGACACCGC CATGTATTACTGTGCGAGACAGCTACGAGACTACTACAGTATGGACGTCTGGGGC CAAGGGACCACGGTCACCGTCTCCTCA | 248 |
| | VL | GAAATTGTAATGACACAGTCTCCAGCCACCCTGTCTTTGTCTCCAGGGGAAAGAGC CACCCTCTCCTGCAGGGCCAGTCAGAGTGTTAGCAGCAGCTACTTATCCTGGTACC AGCAGAAACCTGGGCAGGCTCCCAGGCTCCTCATCTATGGTGCATCCACCAGGGC CACTGGCATTGCAGCCAGGTTCAGTGGCAGTGGGTCTGGGACAGACTTCACTCTC ACCATCAGCAGCCTGCAGCCTGAAGATTTTGCAGTTTATTACTGTCAGCAGGATTA TAACTTACCTCGGACGTTCGGCCAAGGGACCAAGGTGGAAATCAAA | 249 |

**Table G2. Nucleic acid sequences of humanized VH and VL regions.**

| | **Doma in** | **Nucleotide Sequence** | **SEQ ID NO** |
|---|---|---|---|
| AS30SS_ Hu6 | VH | CAAGTACAACTGGTTCAATCAGGCGCAGAAGTCGTAAAACCTGGTTCCAGCGTAA AAGTCAGTTGTGAGGCTAGTGGATTCACCTTCACCGATAGCGTTATTACATGGGTT CGTCAGCGCCCAGGTCAAGGGCTCGAGTGGATTGGGGAAATTTACCCAGGAAGT GGAAATTTCTACTACAATGAAAAATTTCAAGGCCGGGTGACCATCACTGCTGATAA AAGCACTTCAACAGCCTATATGGAATTGTCCAGCTTGCGCTCCGAAGACACTGCCG TTTATTTCTGCGCCATGCGTAGGTCTTCCTGGGGACAGGGTACACTTGTAACTGTC AGCTCT | 306 |
| | VL | GACGTCGTAATGACTCAAACACCCCTCTCTCTTTCTGTTACCCCCGGACAGCCTGCT TCAATCAGTTGTAAATCATCCCAAACCATAGTTCATTCTAATAGTAAAACTTACCTC GAATGGTATCTCCAAAAACCTGGTCAGTCACCACAGCTCCTTATTTACAAGGTTAG TTCCAGATTCTCTGGCGTCCCTGACCGCTTCTCTGGCTCCGGTTCAGGCACCGACTT TACTCTGAAAATCTCACGGGTTGAAGCTGAAGATGTTGGAGTGTACTACTGCTTCC AGGGTTCTCACGTCCCATTGACCTTTGGACAGGGAACTAAGCTCGAAATAAAA | 322 |
| AS30SS_ Hu9 | VH | CAAGTACAACTGGTTCAATCAGGCGCAGAAGTCGTAAAACCTGGTTCCAGCGTAA AAGTCAGTTGTGAGGCTAGTGGATTCACCTTCACCGATAGCGTTATTACATGGGTT CGTCAGCGCCCAGGTCAAGGGCTCGAGTGGATTGGGGAAATTTACCCAGGAAGT GGAAATTTCTACTACAATGAAAAATTTCAAGGCCGGGTGACCATCACTGCTGATAA AAGCACTTCAACAGCCTATATGGAATTGTCCAGCTTGCGCTCCGAAGACACTGCCG TTTATTTCTGCGCCATGCGTAGGTCTTCCTGGGGACAGGGTACACTTGTAACTGTC AGCTCT | 338 |
| | VL | GATGTGGTGATGACTCAGACACCCCTGAGTCTCAGCGTAACACCTGGTCAACCCGC CTCTATTAGTTGTCGAAGCTCTCAAACAATCGTACATAGTAATAGTAAAACCTATCT CGAATGGTATCTTCAGAAACCAGGGCAGTCTCCTCAACTCCTTATATACAAAGTAT CCAACAGGTTTTCCGGTGTACCCGATAGGTTTTCCGGTTCCGGCTCCGGAACTGAC TTTACCCTCAAAATAAGTCGAGTGGAGGCTGAGGATGTTGGCGTTTATTATTGCTT TCAGGGGTCACACGTACCTCTTACCTTCGGCGCAGGCACAAAATTGGAGATTAAA | 354 |
| C3_Hu10 | VH | GAAGTTCAACTGGTCGAGTCTGGAGGAGGCCTCGTGAAGCCGGGCGGTAGTTTG CGCCTCTCTTGTGCCGCCTCAGGGTTTACGTTCTCTAGTTATACTATGAGTTGGGTG CGGCAGGCACCGGGAAAAGGGCTGGAATGGGTGGCCTCAATCTCTAGTAGCGGC AGCAATACTTATTATCCTGATAGTGTGAAGGGGAGGTTTACCATCTCACGGGATAA CGCTAAGAACAACCTGTATCTTCAAATGAATAGCCTCCGAGCAGAGGATACAGCA CTTTACTACTGCGCTCGCTTTGACTATGGCAGCAGTCTTGATAGTTGGGGGCAGGG CACCTTGCTTACGGTTTCATCC | 370 |
| | VL | GAGATCGTTCTTACCCAATCCCCGGATTTCCTTTCTGTGACCCCCAAAGAAAAAGTC ACACTCACCTGCCGAGCAAGCCAGTCTATTAGTAACAATTTGCACTGGTATCAGCA GAAGAGTGACCAATCTCCCAAACTCCTTATTAAGTACGCCTCTCAGTCAATATCCG GCATACCTAGCCGCTTTTCCGGTTCTGGTAGTGGCACCGACTTTACTCTCACTATCA ATTCAGTGGAGGCTGAGGATGCCGCCACGTATTTTTGTCAGCAATCAAAGAGTTG GCCCCGGACATTTGGAGGGGGAACTAAGCTGGAGATTAAG | 386 |
| C3_Hu11 | VH | GAAGTTCAACTGGTCGAGTCTGGAGGAGGCCTCGTGAAGCCGGGCGGTAGTTTG CGCCTCTCTTGTGCCGCCTCAGGGTTTACGTTCTCTAGTTATACTATGAGTTGGGTG CGGCAGGCACCGGGAAAAGGGCTGGAATGGGTGGCCTCAATCTCTAGTAGCGGC AGCAATACTTATTATCCTGATAGTGTGAAGGGGAGGTTTACCATCTCACGGGATAA CGCTAAGAACAACCTGTATCTTCAAATGAATAGCCTCCGAGCAGAGGATACAGCA CTTTACTACTGCGCTCGCTTTGACTATGGCAGCAGTCTTGATAGTTGGGGGCAGGG CACCTTGCTTACGGTTTCATCC | 402 |
| | VL | GAAATTGTGCTTACCCAGTCCCCAGACTTCCTGTCCGTGACCCCTAAAGAGAAGGT GACACTGACTTGCAGGGCCTCACAATCCATTGGCAATAACCTTCACTGGTATCAGC AGAAGTCCGACCAGTCTCCGAAACTCCTCATCAAGTATGCCAGCCAGTCAATTAGC GGAATACCGTCTCGGTTTAGCGGATCTGGGTCTGGTACTGACTTCACGCTGACGAT CAATAGCGTGGAAGCGGAGGACGCCGCCACCTATTTCTGCCAGCAATCTAAGTCC TGGCCGAGAACGTTCGGAGGCGGTACTAAACTTGAGATCAAG | 418 |
| C21_Hu1 1 | VH | GAGGTACAGCTCGTCGAAAGTGGCGGCGGTCTTGTCAAGCCGGGAGGAAGTTTG CGCCTGTCCTGTGCAGCATCCGGATTCACGTTTTCTTCTTATACGATGAGTTGGGTC CGGCAGGCACCGGGGAAAGGATTGGAATGGGTTGCGTCTATTAGTAGCTCTGGAT CTAACACATACTACCCAGACTCAGTTAAAGGTCGCTTCACGATAAGTCGGGACAAC GCTAAAAATAACCTGTATTTGCAAATGAACAGCTTGCGAGCTGAGGACACCGCCCT CTACTACTGTGCCCGATTTGATTATGGATCAAGTTTGGATTCATGGGGCCAAGGGA CCCTGCTCACAGTAAGCTCT | 434 |
| | VL | GAAATCGTTCTTACTCAGTCCCCGGATTTTTTGAGTGTAACGCCTAAAGAGAAGGT GACCCTGTCCTGCCGCGCTTCCCAATCTATATCAAACTATCTTCATTGGTACCAGCA AAAAAGCGACCAGTCCCCGAAACTGCTCATCAAATACGCTAGCCAATCAATAAGC GGCATCCCTAGCAGGTTTTCCGGTAGCGGTAGTGGCACAGACTTCACATTGAGCAT AAACAGCGTGGAAGCCGAGGATGCAGCAACATACTTTTGCCAACAGAGCAAGTCC TGGCCGAGGACGTTCGGTGGGGGCACCAAATTGGAAATAAAG | 450 |
| C21_Hu2 1 | VH | GAGGTCCAACTTGTTGAATCCGGTGGAGGGCTGGTGCAGCCTGGTGGATCCCTCC GCCTTTCCTGTGCAGCATCAGGTTTTACTTTTTCCTCATACACCATGTCTTGGGTTCG CCAGGCTCCAGGGAAAGGATTGGAATGGGTGGCAACTATCAGTAGTAGCGGGGA CAATACATACTATCCCGATTCCGTGAAAGGGAGATTTACGATTTCACGCGACAACA GCAAAAATACCCTTTACCTGCAAATGAGTTCCTTGCGGGCCGAGGACACTGCCCTC TACTACTGCGCTCGCTTCGATTACGGTTCCAGCCTGGACTCATGGGGTCAAGGGAC TACACTGACTGTTTCTTCC | 466 |
| | VL | GAGATCGTGCTCACCCAATCTCCTGACTTCCTTAGCGTTACACCAGGGGAGAAAGT AACTCTTACGTGCCGGGCCTCCCAGAGCATCTCCAATTATTTGCATTGGTACCAGC AAAAGAGTGACCAGAGCCCTAAGCTGCTCATCAAATACGCATCACAGAGTATTAG CGGCGTTCCCTCACGGTTCTCTGGCTCCGGTTCCGGTACAGACTTCACTTTGACGAT TTCAAGTGTAGAGGCCGAGGACTTCGCAACTTACTTTTGTCAGCAAAGCAAATCCT GGCCTCGAACTTTCGGCGGGGGTACAAAACTCGAAATCAAG | 482 |

**Table H. Nucleic acid sequences of anti-LG-5 VH and VL regions.**

| **LG-5** | **Chain** | **Nucleotide Sequence** | **SEQ ID NO** |
|---|---|---|---|
| AN01 | VH | GAGGTGCAGCTGCAGCAGAGCGGCCCTGAGCTGGTGAAACCTGGCGCCAGCGTG AAGATGAGCTGCAAGGCCAGCGGCTACACCTTCACCAGCTACAACATCCACTGGG TGAAACAGAAGCCCGGCCAGGGCCTGGAATGGATTGGCTACATCAACCCCTACAA CGACGGCACCAAGTACAGCGAGAAGTTCAAGGGCAAGGCCACCCTGACCAGCGA CAGAAGCAGCAGCACCGCCTACATGGAAGTGTCCAGCCTGACCTCCGAGGACAGC GCCGTGTACTACTGCGCCATCTACGGCAACAGCTACTGGGGCCAGGGCAGCACCC TGACCGTGTCCAGC | 250 |
| | VL | GACATCGTGATGACCCAGGCTGCCCCCAGCATCCCCGTGACACCTGGCGAGTCCG TGTCCATCAGCTGCAGAAGCAGCAAGAGCCTGCTGCACAGCAACGGCAATACCTA CCTGTACTGGTTCCTGCAGCGGCCTGGCCAGTCCCCCCAGCGGCTGATCTACTACA TGAGCAACCTGGACAGCGGCGTGCCCGACCGGTTTAGCGGCAGAGGCAGCGGCA CAGACTTTACCCTGCGGATCAGCCGGGTGGAAGCCGAGGACGTGGGCGTGTACTA TTGCATGCAGGGCCTGGAATACCCCTACACCTTTGGAGGCGGCACCAAGCTGGAA ATCAAG | 251 |
| C3 | VH | GACGTGATGCTGGTCGAGAGCGGCGGAGATCTGGTCAAACCCGGGGGTTCTCTG AAGCTGAGTTGTGCCGCTTCAGGCTTCACTTTTTCTAGTTACACCATGAGCTGGGT GCGACAGACCCCAGAGAAGCGGCTGGAATGGGTCGCTAGCATCTCAAGCGGCGG AGGGAACACCTACTATCCCGACTCTGTGAAAGGCAGATTCACAATTAGTCGCGATA ATGCAAAGAACAATCTGTACCTGCAGATGTCCTCTCTGAGGTCCGAAGATACTGCC CTGTACTATTGTGCTAGATTTGACTATGGAAGTTCACTGGATTCTTGGGGACAGGG GACCACACTGACAGTGAGCTCC | 252 |
| | VL | GACATCGTCCTGACCCAGAGTCCTGCCACCCTGTCTGTGACACCAGGCGATTCTGT CAGTCTGTCATGTAGAGCTAGCCAGTCCATCTCTAACAATCTGCACTGGTACCAGC AGAAGTCACATGAAAGCCCCAGACTGCTGATCAAGTATGCCAGTCAGTCAATCAG CGGTATTCCTTCCCGCTTCTCCGGCTCTGGAAGTGGGACAGACTTTACTCTGTCCAT CAACTCTGTGGAGACAGAAGATTTCGGCATGTATTTTTGTCAGCAGAGCAAGAATT GGCCCAGGACATTTGGCGGAGGGACTAAGCTGGAGATCAAG | 253 |
| C21 | VH | GAAGTGATGCTGGTCGAAAGTGGAGGAGGACTGGTGAAACCAGGTGGAAGCCTG AAGCTGTCCTGTGCCGCTTCTGGCTTCACTTTTTCAAGCTATACCATGAGCTGGGTG CGACAGACACCTGAGAAGCGGCTGGAATGGGTCGCTACAATCTCCTCTGGAGGG GACAACACTTACTATCCAGATAGCGTGAAAGGCAGATTCACTATTTCCCGCGACAA TGCAAAGAACAATCTGTACCTGCAGATGAGTTCACTGAGGAGCGAGGATACCGCC CTGTACTATTGCGCTAGATTTGACTATGGAAGCTCCCTGGATTGTTGGGGACAGGG GACCACACTGACCGTGTCTAGT | 254 |
| | VL | GACATCGTCCTGACTCAGAGCCCTGCCACCCTGTCCGTGACACCAGGCGATTCAGT CAGCCTGTCCTGTAGAGCTTCTCAGAGTATCTCAAACTACCTGCACTGGTATCAGC AGAAGAGTCATGAATCACCCAGACTGCTGATCAAGTACGCCAGCCAGTCCATCTCT GGGATTCCTAGCCGCTTCAGTGGCTCAGGAAGCGGGACAGACTTTACTCTGAGCA TCAATTCCGTGGAGACAGAAGATTTCGGCATGTATTTTTGTCAGCAGTCCAAGTCT TGGCCCAGGACATTTGGCGGAGGGACTGAGCTGGAGATCAAG | 255 |
| TLF39 | VH | GAGGTGCAACTGGTGCAGTCTGGAGCAGAGGTGAAAAAGCCCGGGGAGTCTCTG AAGATCTCCTGTAAGGGTTCTGGATACAGCTTTACCAGCTACTGGATCGGCTGGGT GCGCCAGATGCCCGGGAAAGGCCTGGAGTGGATGGGGATCATCTATCCTGGTGA CTCTGATACCAGATACAGCCCGTCCTTCCAAGGCCAGGTCACCATCTCAGCCGACA AGTCCATCAGCACCGCCTACCTGCAGTGGAGCAGCCTGAAGGCCTCGGACACCGC CATGTATTACTGTGCGAGACGCGGGTATCGCAGCAGCTGGTACTTTGACTACTGG GGCCAGGGAACCCTGGTCACCGTCTCCTCA | 256 |
| | VL | GCCATCCAGATGACCCAGTCTCCATCCTCCCTGTCTGCATCTGTAGGAGACAGAGT CACCATCACTTGCCGGGCAAGTCAGGGCATTAGAAATGATTTAGGCTGGTATCAG CAGAAACCAGGGAAAGCCCCTAAGCTCCTGATCTATGCTGCATCCAGTTTACAAAG TGGGGTCCCATCAAGGTTCAGCGGCAGTGGATCAGGCACAGATTTCACTTTCACCA TCAGCAGCCTGCAGCCTGAAGATTTTGCAACTTATTACTGTCTACAAGATTACAATT ACCCGCTCACTTTCGGCGGAGGGACCAAGGTGGAGATCAAA | 257 |
| TLF86 | VH | GAGGTGCAGCTGGTGGAGTCTGGGGGAGGTGTGGTACGGCCGGGGGGGTCCCT GAGACTCTCCTGTGCAGCCTCTGGATTCACCTTTGATGATTATGGCATGAGCTGGG TCCGCCAAGCTCCAGGGAAGGGGCTGGAGTGGGTCTCTGGTATTAATTGGAATGG TGGTAGCACAGGTTATGCAGACTCTGTGAAGGGCCGATTCACCATCTCCAGAGAC AACGCCAAGAACTCCCTGTATCTGCAAATGAACAGTCTGAGAGCCGAGGACACGG CCTTGTATCACTGTGCGAGAGAAGGGGGGGAGCTATTAATGGACTATTGGGGCCA GGGAACCCTGGTCACCGTCTCCTCA | 258 |
| | VL | GAAATTGTGTTGACACAGTCTCCAGCCACCCTGTCTTTGTCTCCAGGGGAAAGAGC CACCCTCTCCTGCAGGGCCAGTCAGAGTGTTAGCACCTACTTAGCCTGGTACCAAC AGAAACCTGGCCAGGCTCCCAGGCTCCTCATCTATGATGCATCCAACAGGGCCACT GGCATCCCACCCAGGTTCAGTGGCAGTGGGTCTGGGACAGACTTCACTCTCACCAT CAGCAGCCTAGAGCCTGAAGATTTTGCAGTTTATTACTGTCAGCAGCGTAGCAACT GGCCTCCTACTTTCGGCGGAGGGACCACGGTGGAGATCAAA | 259 |

**Table I. Nucleic acid sequences of anti-LG-4/5 VH and VL regions.**

| **LG-4/5** | **Chain** | **Nucleotide Sequence** | **SEQ ID NO** |
|---|---|---|---|
| CL-40968 | VH | GAAGTTCAATTGTTAGAGTCTGGTGGCGGTCTTGTTCAGCCTGGTGGTTCTTTACG TCTTTCTTGCGCTGCTTCCGGATTCACTTTCTCTCATTACTCTATGGTTTGGGTTCGC CAAGCTCCTGGTAAAGGTTTGGAGTGGGTTTCTTATATCTATCCTTCTGGTGGCAC TTCGTATGCTGACTCCGTTAAAGGTCGCTTCACTATCTCTAGAGACAACTCTAAGAA TACTCTCTACTTGCAGATGAACAGCTTAAGGGCTGAGGACACGGCCGTGTATTACT GTGCGAGACATTGGCGGGGGTATAGCAGCAGCTGGTACCACCCGGCGTACTTTGA CTACTGGGGCCAGGGCACCCTGGTCACCGTCTCAAGC | 260 |
| | VL | GACATCCAGATGACCCAGTCTCCAGCCACCCTGTCTTTGTCTCCAGGGGAAAGAGC CACCCTCTCCTGCAGGGCCAGTCAGAGTGTTAGCAGCTACTTAGCCTGGTACCAAC AGAAACCTGGCCAGGCTCCCAGGCTCCTCATCTATGATGCATCCAACAGGGCCACT GGCATCCCAGCCAGGTTCAGTGGCAGTGGGTCTGGGACAGACTTCACTCTCACCA TCAGCAGCCTAGAGCCTGAAGATTTTGCAGTTTATTACTGTCAGCAGCGTAGCAAC TGGCCTCTCACTTTCGGCGGAGGGACCAAGGTGGAGATCAAA | 261 |
| CL-40992 | VH | GAAGTTCAATTGTTAGAGTCTGGTGGCGGTCTTGTTCAGCCTGGTGGTTCTTTACG TCTTTCTTGCGCTGCTTCCGGATTCACTTTCTCTTGGTACCCTATGATGTGGGTTCGC CAAGCTCCTGGTAAAGGTTTGGAGTGGGTTTCTTCTATCTATCCTTCTGGTGGCACT ACTACTTATGCTGACTCCGTTAAAGGTCGCTTCACTATCTCTAGAGACAACTCTAAG AATACTCTCTACTTGCAGATGAACAGCTTAAGGGCTGAGGACACGGCCGTGTATTA CTGTGCGAGGTCGTATTACTATGATAGTAGTGGTTATTACTCACATGATGCTTTTGA TATCTGGGGCCAAGGGACAATGGTCACCGTCTCAAGC | 262 |
| | VL | GACATCCAGATGACCCAGTCTCCATCCTCCCTGTCTGCATCTGTGGGAGACAGAGT CGCCATCACTTGCCGCGCAAGTCAGAGCATCGACACCTATTTAAATTGGTATCAGC AGAAACCAGGGAAAGCCCCTAAACTCCTGATCTATGCTGCATCCAAGTTGGAAGA CGGGGTCCCATCAAGATTCAGTGGCAGTGGAACTGGGACAGATTTCACTCTCACC ATCAGAAGTCTGCAACCTGAAGATTTTGCAAGTTATTTCTGTCAACAGAGCTACTCT AGTCCAGGGATCACTTTCGGCCCTGGGACCAAGGTGGAGATCAAA | 263 |
| CL-41136 | VH | GAAGTTCAATTGTTAGAGTCTGGTGGCGGTCTTGTTCAGCCTGGTGGTTCTTTACG TCTTTCTTGCGCTGCTTCCGGATTCACTTTCTCTGATTACGAGATGCATTGGGTTCG CCAAGCTCCTGGTAAAGGTTTGGAGTGGGTTTCTTCTATCTGGCCTTCTGGTGGCC TTACTAAGTATGCTGACCCCGTTAAAGGTCGCTTCACTATCTCTAGAGACAACTCTA AGAATACTCTCTACTTGCAGATGAACAGCTTAAGGGCTGAGGACACGGCCGTGTA TTACTGTGCGAGAGATTCCTATTACTATGATAGTAGTGGTGCTCTTGGCTACTGGG GCCAGGGAACCCTGGTCACCGTCTCAAGC | 264 |
| | VL | GACATCCAGATGACCCAGTCTCCTTCCACCCTGTCTGCATCTGTAGGAGACAGAGT CACCATCACTTGCCGGGCCAGTCAGAGTGTTAGTAACTGGTTGGCCTGGTATCAGC AGAAACCAGGGAAAGCCCCTAAGCTCCTGATCTATAAGGCGTCTAGTTTAGAAAG TGGGGTCCCATCGAGGTTCAGCGGCAGTGGATCTGGGACAGAATTCACTCTCACC ATCAGCAGCCTGCAGCCTGATGATTTTGCAACTTATTACTGCCTACAGTATGTGAG TTATCCCCTCACTTTTGGCGGAGGGACCAAGGTGGACATCAAA | 265 |
| CL-41400 | VH | GAAGTTCAATTGTTAGAGTCTGGTGGCGGTCTTGTTCAGCCTGGTGGTTCTTTACG TCTTTCTTGCGCTGCTTCCGGATTCACTTTCTCTTATTACGATATGTATTGGGTTCGC CAAGCTCCTGGTAAAGGTTTGGAGTGGGTTTCTCGTATCTATTCTTCTGGTGGCCA TACTTGGTATGCTGACTCCGTTAAAGGTCGCTTCACTATCTCTAGAGACAACTCTAA GAATACTCTCTACTTGCAGATGAACAGCTTAAGGGCTGAGGACACGGCTGTGTATT ACTGTGCGAGGCCCGGGTATAGCAGTGGCTGGTACGATGGCACCTACTTTGACTA CTGGGGCCAGGGAACCCTGGTCACCGTCTCAAGC | 266 |
| | VL | GACATCCAGATGACCCAGTCTCCTTCCACCCTGTCTGCATCTGTTGGCGACAGAGT CACCATTACTTGTCGGGCCAGTCAGAGTATTGATACTTGGCTGGCCTGGTATCGGC AGAAACCAGGGAAAGCCCCTAATGTCGTAATTCATTCCGCGTCTACTTTACAAAGT GGCGTCCCCGCAAGGTTCAGCGGCAGTGGATTTGGGACAGAATGGACTCTCACTA TCACCAACCTGCAGCCTGATGATTTTGCCACCTATTATTGCCAACAATATAAGACTT ATCCGTTCACTTTTGGCCAGGGGACGAAGCTGGAGATCAAG | 267 |
| CL-41500 | VH | GAAGTTCAATTGTTAGAGTCTGGTGGCGGTCTTGTTCAGCCTGGTGGTTCTTTACG TCTTTCTTGCGCTGCTTCCGGATTCACTTTCTCTCATTACCAGATGGAGTGGGTTCG CCAAGCTCCTGGTAAAGGTTTGGAGTGGGTTTCTTCTATCTCTCCTTCTGGTGGCTT TACTTCTTATGCTGACTCCGTTAAAGGTCGCTTCACTATCTCTAGAGACAACTCTAA GAATACTCTCTACTTGCAGATGAACAGCTTAAGGGCTGAGGACACAGCCGTGTATT ACTGTACGAGAGAGCCGGGGAGGTTGTGGGCTTTTGATATCTGGGGCCAAGGGA CAATGGTCACCGTCTCAAGC | 268 |
| | VL | GACATCCAGATGACCCAGTCTCCATCTTTCGTGTCTGCATCTGTCGGAGACAGAGT CACCATCACTTGCCGGGCGAGTCAGGATATTCGTAATTGGCTAGCCTGGTATCAAC AGGAGTCCGGGAAAGCCCCTCGGCTCCTGATCTCTGCTGCATCCAGTAGGCACAG TGGCGTCTCATCTAGATTCAGCGGCAGTGGATCTGGGACAGACTTCACCCTCACCA TCACCAGTCTGCAGCCTGAAGATTCAGCAACTTATTTTTGTCAACAGGCTGACAGT TCCCCTCGGACGTTCGGCCAAGGGACCAAGGTGGAAATCAAA | 269 |
| TLG3/TL G4 | VH | CAGGTGCAACTGGTGCAGTCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGATGGATCAACCCTAACAG TGGTGGCACAAACTATGCACAGAAGTTTCAGGGCAGGGTCACCATGACCAGGGAC ACGTCCATCAACACAGCCTACATGGAACTGAGCAGGCTGAGATCTGACGACACGG CCGTGTATTACTGTGCGGTCTTTGGTTCGGGGAGTTCTTGGGGCCAGGGAACCCT GGTCACCGTCTCCTCA | 270 |
| | VL | GACATCCAGATGACCCAGTCTCCATCCTCACTGTCTGCATCTGTGGGAGACAGAGT CACCATCACTTGTCGGGCGAGTCAGGGTATTAGCAATTCTTTAGCCTGGTTTCAGC AGAAACCAGGGAAAGCCCCTAAGTCCCTGATCTATGCTGCATCCAGTTTGCAAAGT GGGGTCCCATCAAAGTTCAGCGGCAGTGGATCTGGGACAGATTTCACTCTCACCAT CAGCAGCCTGCAGCCTGAAGATTTTGCAACTTATTACTGCCAACAATATAAGAGTT ACCCGTACACATTTGGCCAGGGGACCAAGCTGGAGATCAAA | 271 |
| TLG26 | VH | CAGGTGCAGCTGGTGCAGTCTGGGGCTGAGGTGAAGAAGCCTGGGGCCTCAGTG AAGGTCTCCTGCAAGGCTTCTGGAAACACCTTCACCGGCTACTATATACACTGGGT TCGACAGGCCCCTGGACAAGGGCTTGAGTGGATGGGATGGATTAAACCTAGTACT GGTGACACAAACTATGCACAGAATTTTCTGGACAGGGTCACCATGACCAGGGACA CGTCCATCAGCACAGCCTACATGGAACTCAGCAGGCTGAGATCTGACGACACGGC CGTGTATTACTGTGCGGTCTTTGGTTCGGGGAGTTCTTGGGGCCAGGGAACCCTG GTCACCGTCTCCTCA | 272 |
| | VL | GACATCCACATGACCCAGTCTCCATCCTCACTGTCTGCATTTGTAGGAGACAGAGT CACCATCACTTGTCGGGCGAGTCAGGGCATTAGCAATTATTTAGCCTGGTTTCAGC AGAAACCAGGGAAAGCCCCTAAGTCCCTGATCTATGCTGCATCCAGTTTGCAAAGT GGGGTCCCATCAAAGTTCAGCGGCAGTGGATCTGGGACAGATTTCACTCTCACCAT CAACAACCTGCAGCCTGAAGATTTTGCAACTTATTACTGCCAACAGTATAAGACTT ACCCGTACACATTTGGCCAGGGGACCAAGCTGGAGATCAAA | 273 |
| TLI-3 | VH | CAGGTGCAGCTGGTGGAGTCTGGGGGAGGCGTGGTCCAGCCTGGGAGGTCCCTG AGACTCTCCTGTGCAGCGTCTGGATTCACCTTCAGTAGCTATGGCATGCACTGGGT CCGCCAGGCTCCAGGCAAGGGGCTGGAGTGGGTGGCAGTTATATGGTATGATGG AAGTAATAAATACTATGCAGACTCCGTGAAGGGCCGATTCACCATCTCCAGAGACA ATTCCAAGAACACGCTGTATCTGCAAATGAACAGTCTGAGAGCCGAGGACGCGGC TGTGTATTACTGTGCGAGAGAAGGTGGCTGGTACGGCGGGGACTACTACTACGGT ATGGACGTCTGGGGCCAAGGGACCACGGTCACCGTCTCCTCA | 274 |
| | VL | GCCATCCAGTTGACCCAGTCTCCATCCTCCCTGTCTGCATCTGTAGGAGACAGAGT CACCATCACTTGCCGGGCAAGTCAGGGCATTAGCAGTGCTTTAGCCTGGTATCAGC AGAAACCAGGGAAAGCTCCTAAGCTCCTGATCTATGATGCCTCCAGTTTGGAAAGT GGGGTCCCATCAAGGTTCAGCGGCAGTGGATCTGGGACAGATTTCACTCTCACCA TCAGCAGCCTGCAGCCTGAAGATTTTGCAACTTATTACTGTCATCAGTTTAATAATT ACCCATTCACTTTCGGCCCTGGGACCAAAGTGGATATCAAA | 275 |
| TLI-7 | VH | GAAGTGCAGCTGCTGGAATCTGGCGGCGGACTGGTGCAGCCTGGCGGATCTCTG AGACTGTCCTGTGTGGCCTCCGGCTTCACCTTCTCCAGCTACGCCATGTCCTGGGT GCGACAGGCTCCTGGCAAGGGCCTGGAATGGGTGTCCGGCATCTCTGGCAGGGG CGGCTCTCCTAACTACGCCGACTCTGTGAAGGGCCGGTTCACCATCTCCCGGGACA ACTCCAAGAACACCCTGTACCTCCAGATGAACTCCCTGCGGGCCGAGGACACCGC CGTGTACTACTGTGCTAAGGACGGCGACGGCTCCGGCCCTCCCTACTACTTTGATT ACTGGGGCCAGGGCACCCTCGTGACCGTGTCATCT | 276 |
| | VL | GACATCCAGATGACCCAGTCCCCCTCCAGCCTGTCTGCCTCTGTGGGCGACAGAGT GACCATCACCTGTCGGGCCTCCCAGGGCATCTCTTCTTGGCTGGCCTGGTATCAGC AGAAGCCCGAGAAGGCCCCCAAGTCCCTGATCTACGCCGCCAGCTCTCTCCAGTCT GGCGTGCCCTCCAGATTCTCCGGCTCTGGCTCTGGCACCGACTTTACCCTGACCATC AGCTCCCTCCAGCCCGAGGACTTCGCCACCTACTACTGCCAGCAGTACAACTCCTA CCCCTACACCTTCGGCCAGGGCACCAAGCTGGAAATCAAG | 277 |
| TTLK71-4-6 | VH | CAGGTGCAGCTGATGGAGTCTGGGGGAGGCGTGGTCCAGCCTGGGAGGTCCCTG AGACTCTCCTGTGCAGCGTCTGGATTCACCTTCAGTGGCTATGGCATGCACTGGGT CCGCCAGGCTCCAGGGAAGGGGCTGGAGTGGGTGGCAGTTATATGGTCTGATGG AAGTAATAGATACTATACAGACTCCGTGAAGGGCCGATTCACCATCTCCAGAGACA ATTCCAAGAACACGCTGTCTCTGCAAATGAACAGCCTGAGAGCCGAGGACACGGC TGTGTATTACTGTGCGAGAGATAGGGGGATTACTATGGTTCGGGGACTTATTATA AAATACTACTACTACTACGGTTTGGACGTCTGGGGCCAAGGGACCTCGGTCACCGT CTCCTCA | 278 |
| | VL | GAAATTGTGTTGACACAGTCTCCAGCCACCCTGTCTTTGTCTCCAGGGGAAAGAGC CACCCTCTCCTGCAGGGCCAGTCAGAGTGTTAGCAGCTACTTAGCCTGGTACCAAC AGAAACCTGGCCAGGCTCCCAGGCTCCTCATCTATGATGCATCCAACAGGGCCACT GGCATCCCAGCCAGGTTCAGTGGCAGTGGGTCTGGGACAGACTTCACTCTCACCA TCAGCAGCCTAGAGCCTGAAGATTTTGCAGTTTATTACTGTCAGCAGCGTAGCAAC TGGTGGACGTTCGGCCAAGGGACCAAGGTGGAAATCAAA | 279 |
| TTLK123 -3 | VH | CAGGTGCAGCTGGTGGAGTCTGGGGGAGGCGTGGTCCAGCCTGGGAGGTCCCTG AGACTCTCCTGTGCAGCGTCTGGATTCACCTTCAGTAGCTTTGGCATGCACTGGGT CCGCCAGGCTCCAGGCAAGGGGCTGGAGTGGGTGGCAGTTATATACTATGATGG AAGTAATAAATACTATGCAGACTCCGTGAAGGGCCGATTCACCATCTCCAGAGACA ATTCCAAGAACACGCTGTATCTGCAAATGAACAGCCTGAGAGCCGAGGACACGGC TGTGTATTACTGTGCGAGAGATGACAACTGGAACGACGGGGACTTTGACTACTGG GGCCAGGGAACCCTGGTCACCGTCTCCTCA | 280 |
| | VL | GACATCCAGTTGACCCAGTCTCCATCCTTCCTGTCTGCATCTGTAGGAGACAGAGT CACCATCACTTGCCGGGCCAGTCAGGGCATTAGTAGTTATTTAGCCTGGTATCAGC AAAAACCAGGGAAAGCCCCTAAGCTCCTGATCTATGCTGCATCCACTTTGCAAAGT GGGGTCCCATCAAGGTTCAGCGGCAGTGGATCTGGGACAGAATTCACTCTCACAA TCAGCAGCCTGCAGCCTGAAGATTTTGCAACTTATTACTGTCAACAGCTTAATAGTT ACCCTCGGACGTTCGGCCAAGGGACCAAGGTGGAAATCAAA | 281 |
| TTLK145 -6-3 | VH | GAGGTGCAGCTGTTGGAGTCTGGGGGAGGCTTGGTACAGCCTGGGGAGTCCCTG AGACTCTCCTGTGCAGCCTCTGGATTCACCTTTAACAGATTTGTCATGAGTTGGGTC CGCCAGGCTCCAGGGAAGGGGCTGGAGTGGGTCTCAACTATTAGTGGTAGTGGT GGTAGCACATACTACGCAGACTCCGTGAAGGGCCGGTTCACCATCTCCAGAGACA ATTCCAAGAACACGCTGTATCTGCAAATGAACAGCCTGAGAGCCGAGGACACGGC CGTGTATTACTGTGCGAAAGATTTTACGTATTACTATGGTTCGGGGAATTATTATA ACTGGTTCGACCCCAGGGGCCAGGGAACCCTGGTCACCGTCTCCTCA | 282 |
| | VL | GACATCCAGATGACCCAGTCTCCTTCCACCCTGTCTACATCTGTAGGAGACAGAGT CACCATCACTTGCCGGGCCAGTCAGAGTATTAGTAGCTGGTTGGCCTGGTATCAGC AGAAACCAGGGAAAGCCCCTAAGCTCCTGATCTATAAGGCGTCTAGTTTAGAAAG TGGGGTCCCATCAAGGTTCAGCGGCAGTGGATCTGGGACAGAATTCACTCTCACC ATCAGCAGCCTGCAGCCTGATGATTTTGCAACTTATTACTGCCAACAGTATAATAG TTATTCTCGGACGTTCGGCCAAGGGACCAAGGTGGAAATCAAA | 283 |
| TTLK170 -2 | VH | CAGGTGCAGCTACAGCAGTGGGGCGCAGGACTGTTGAAGCCTTCGGAGACCCTGT CCCTCACCTGCGCTGTCTATGGTGGGTCCTTTAGTGGTTACTACTGGAGCTGGATC CGCCAGCCCCCAGGGAAGGGGCTGGAGTGGATTGGGGAAATCAATCATAGTGGA GGCACCAACTACAACCCGTCCCTCAAGAGTCGAGTCACCATATCAGTAGACACGTC CAAGAACCACTTCTCCCTGAAGCTGAGCTCTGTGACCGCCGCGGACACGGCTGTGT ATTACTGTGCGAGAACTAGTGACTACGATTACTACTACTACGGTATGGACGTCTGG GGCCAAGGGACCACGGTCACCGTCTCCTCA | 284 |
| | VL | CAGCCTGTGCTGACTCAGCCAACTTCCCTCTCAGCATCTCCTGGAGCATCAGCCAG ACTCACCTGCACCTTGCGCAGTGGCATCAATCTTGGTCGCTACAGGATATTCTGGT ACCAGCAGAAGCCAGAGAGCCCTCCCCGGTATCTCCTGAGCTACTACTCAGACTCA AGTAAGCATCAGGGCTCTGGAGTCCCCAGCCGCTTCTCTGGATCCAAAGATGCTTC GAGCAATGCAGGGATTTTAGTCATCTCTGGGCTCCAGTCTGAGGATGAGGCTGAC TATTACTGTATGATTTGGCACAGGAGTGCTTTGTTTATTTTCGGCAGTGGAACCAA GGTCACTGTCCTA | 285 |
| WIJL10 | VH | CAGGTTCAGCTACAGCAGTCTGGACCTGAGCTGGTGAAGCCTGGGGCTTCAGTGA AGTTGTCCTGCAAGGCTTCTGGTTACACCTTCACAAGCTACGAGATAAACTGGTTG AAGCAGAGGCCTGGACAGGGACTTGAGTGGATTGGATTGATTTATCCTAGAGATG GAGATACTAAGTACAATGAGAAGTTCAAGGGCAAGGCCACATTGACTGCAGACAC ATCCTCCAGCACAGCGTACATGGAGCTCCACAGCCTGACATCTGAGGACTCTGCG GTCTATTTCTGTGCAAGACACACCCCAGGGGCTTTCTGGGGCCAAGGGACTCTGGT CACTGTCTCTGCA | 286 |
| | VL | GATGTTGTGATGACCCAAACTCCCCTCTCCCTGCCGGTCAGTCTTGGAGATCAAGC CTCCATCTCTTGCAGATCTAGTCAGAGCCTTGTTCACAGTAATGGAGACACCTATTT ACATTGGTACCTACAGAAGCCAGGCCAGTCTCCAAAGCTCCTGATCTACAAAGTTT CCAACCGATTTTCTGGGGTCCCAGACAGGTTCAGTGGCAGTGGATCAGGGACAGA TTTCACACTCAAGATCAGCAGAGTGGAGGCTGAGGATCTGGAAATTTATTTCTGCT CTCAAAGCACACATGTTCCGTACACGTTCGGAGGGGGGACCAAACTGGAAATAAA A | 287 |
| WJL48 | VH | GACGTGAAGCTGGTGGAGTCTGGGGAAGGCTTAGTGAAGCCCGGAGGGTCCCTG AAACTCTCTTGTGCAGCCTCTGGATTCACTTTCAGTAGGTATGCCATGTCTTGGGTT CGCCAGACTCCAGAAAAGAGGCTGGAATGGGTCGCATATATTAGTAGTGGAGGT GATTACATCCACTATGGAGAAACTGTGAAGGGCCGATTCACCATCTCCAGAGACA ATGCCAGGAACACCCTGTACCTGCAAATGAGCAGTCTGAAGTCTGAGGACACAGC CATGTATTACTGTACAAGAGTTCTCTTTTATTATTACGGCAGTAGCTACGTCTTTTTT GACTACTGGGGCCAAGGCACCACTCTCACAGTCTCCTCA | 288 |
| | VL | GATATCCAGATGACACAGACTACATCCTCCCTGTCAGTCTCTCTGGGAGACAGAGT CACCATCAGTTGCAGGGCAAGTCAGGACATCAGCAATTTTCTAAACTGGTATCAGC AGAAACCAGATGGAACTGTTAATCTCCTGATCTACTACACATCAAAATTACACTCA GGAGTCCCATCAAGGTTCAGTGGCGGTGGGTCTGGAAGAGATTATTCTCTCACCA TTAATAACCTGGAGCAAGAAGATATTGCCTCTTACTTTTGCCAACAGGGTCATACG CTTCCGTATACGTTCGGAGGGGGGACCAAGCTGGAAATAAAA | 289 |

**Table I2. Amino acid sequences of humanized, multispecific binding proteins.**

| **Name** | **Chain** | **Sequence** | **SEQ ID NO** |
|---|---|---|---|
| 3407 | I | EIVLTQSPDFLSVTPKEKVTLTCRASQSISNNLHWYQQKSDQSPKLLIKYASQSISGIPSR FSGSGSGTDFTLTINSVEAEDAATYFCQQSKSWPRTFGGGTKLEIKDKTHTEIVLTQSP DFLSVTPKEKVTLTCRASQSISNNLHWYQQKSDQSPKLLIKYASQSISGIPSRFSGSGSG TDFTLTINSVEAEDAATYFCQQSKSWPRTFGGGTKLEIKDKTHTRTVAAPSVFIFPPSDE QLKSGTASVVCLLNNFYPREAKVQWKVDNALQSGNSQESVTEQDSKDSTYSLSSTLTL SKADYEKHKVYACEVTHQGLSSPVTKSFNRGEC | 500 |
| | II | EVQLVESGGGLVKPGGSLRLSCAASGFTFSSYTMSWVRQAPGKGLEWVASISSSGSN TYYPDSVKGRFTISRDNAKNNLYLQMNSLRAEDTALYYCARFDYGSSLDSWGQGTLLT VSSDKTHTEVQLVESGGGLVKPGGSLRLSCAASGFTFSSYTMSWVRQAPGKGLEWVA SISSSGSNTYYPDSVKGRFTISRDNAKNNLYLQMNSLRAEDTALYYCARFDYGSSLDSW GQGTLLTVSSDKTHTASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSCD KTHTCPPCPAPELLGGPSVFLFPPKPKDTLYITREPEVTCVVVDVSHEDPEVKFNWYVD GVEVHNAKTKPREEQYNNASRVVSVLTVLHQDWLNGKEYKCKVSNKALPAPIEKTISK AKGQPREPQVYTLPPCRDELTKNQVSLWCLVKGFYPSDIAVEWESNGQPENNYKTTP PVLDSDGSFFLYSKLTVDKSRWQQGNVFSCSVMHEALHNHYTQKSLSLSPG | 498 |
| | III | QVQLVQSGAEVVKPGSSVKVSCEASGFTFTDSVITWVRQRPGQGLEWIGEIYPGSGN FYYNEKFQGRVTITADKSTSTAYMELSSLRSEDTAVYFCAMRRSSWGQGTLVTVSSAS TKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGP SVFLFPPKPKDTLYITREPEVTCVVVDVSHEDPEVKFNWYVDGVEVHNAKTKPREEQY NNASRVVSVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFFLVSKLTV DKSRWQQGNVFSCSVMHEALHNRFTQKSLSLSPG | 499 |
| | IV | DVVMTQTPLSLSVTPGQPASISCKSSQTIVHSNSKTYLEWYLQKPGQSPQLLIYKVSSRF SGVPDRFSGSGSGTDFTLKISRVEAEDVGVYYCFQGSHVPLTFGQGTKLEIKRTVAAPS VFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC | 501 |
| 3423 | I | EIVLTQSPDFLSVTPKEKVTLSCRASQSISNYLHWYQQKSDQSPKLLIKYASQSISGIPSRF SGSGSGTDFTLSINSVEAEDAATYFCQQSKSWPRTFGGGTKLEIKDKTHTEIVLTQSPD FLSVTPKEKVTLTCRASQSISNNLHWYQQKSDQSPKLLIKYASQSISGIPSRFSGSGSGT DFTLTINSVEAEDAATYFCQQSKSWPRTFGGGTKLEIKDKTHTRTVAAPSVFIFPPSDE QLKSGTASVVCLLNNFYPREAKVQWKVDNALQSGNSQESVTEQDSKDSTYSLSSTLTL SKADYEKHKVYACEVTHQGLSSPVTKSFNRGEC | 504 |
| | II | EVQLVESGGGLVKPGGSLRLSCAASGFTFSSYTMSWVRQAPGKGLEWVASISSSGSN TYYPDSVKGRFTISRDNAKNNLYLQMNSLRAEDTALYYCARFDYGSSLDSWGQGTLLT VSSDKTHTEVQLVESGGGLVKPGGSLRLSCAASGFTFSSYTMSWVRQAPGKGLEWVA SISSSGSNTYYPDSVKGRFTISRDNAKNNLYLQMNSLRAEDTALYYCARFDYGSSLDSW GQGTLLTVSSDKTHTASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSCD KTHTCPPCPAPELLGGPSVFLFPPKPKDTLYITREPEVTCVVVDVSHEDPEVKFNWYVD GVEVHNAKTKPREEQYNNASRVVSVLTVLHQDWLNGKEYKCKVSNKALPAPIEKTISK AKGQPREPQVYTLPPCRDELTKNQVSLWCLVKGFYPSDIAVEWESNGQPENNYKTTP PVLDSDGSFFLYSKLTVDKSRWQQGNVFSCSVMHEALHNHYTQKSLSLSPG | 502 |
| | III | QVQLVQSGAEVVKPGSSVKVSCEASGFTFTDSVITWVRQRPGQGLEWIGEIYPGSGN FYYNEKFQGRVTITADKSTSTAYMELSSLRSEDTAVYFCAMRRSSWGQGTLVTVSSAS TKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGP SVFLFPPKPKDTLYITREPEVTCVVVDVSHEDPEVKFNWYVDGVEVHNAKTKPREEQY NNASRVVSVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFFLVSKLTV DKSRWQQGNVFSCSVMHEALHNRFTQKSLSLSPG | 503 |
| | IV | DVVMTQTPLSLSVTPGQPASISCKSSQTIVHSNSKTYLEWYLQKPGQSPQLLIYKVSSRF SGVPDRFSGSGSGTDFTLKISRVEAEDVGVYYCFQGSHVPLTFGQGTKLEIKRTVAAPS VFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC | 505 |
| 3429 | I | EIVLTQSPDFLSVTPGEKVTLTCRASQSISNYLHWYQQKSDQSPKLLIKYASQSISGVPSR FSGSGSGTDFTLTISSVEAEDFATYFCQQSKSWPRTFGGGTKLEIKDKTHTEIVLTQSPD FLSVTPKEKVTLTCRASQSIGNNLHWYQQKSDQSPKLLIKYASQSISGIPSRFSGSGSGT DFTLTINSVEAEDAATYFCQQSKSWPRTFGGGTKLEIKDKTHTRTVAAPSVFIFPPSDE QLKSGTASVVCLLNNFYPREAKVQWKVDNALQSGNSQESVTEQDSKDSTYSLSSTLTL SKADYEKHKVYACEVTHQGLSSPVTKSFNRGEC | 508 |
| | II | EVQLVESGGGLVKPGGSLRLSCAASGFTFSSYTMSWVRQAPGKGLEWVASISSSGSN TYYPDSVKGRFTISRDNAKNNLYLQMNSLRAEDTALYYCARFDYGSSLDSWGQGTLLT VSSDKTHTEVQLVESGGGLVQPGGSLRLSCAASGFTFSSYTMSWVRQAPGKGLEWV ATISSSGDNTYYPDSVKGRFTISRDNSKNTLYLQMSSLRAEDTALYYCARFDYGSSLDS WGQGTTLTVSSDKTHTASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKKVEPKS CDKTHTCPPCPAPELLGGPSVFLFPPKPKDTLYITREPEVTCVVVDVSHEDPEVKFNWY VDGVEVHNAKTKPREEQYNNASRVVSVLTVLHQDWLNGKEYKCKVSNKALPAPIEKTI SKAKGQPREPQVYTLPPCRDELTKNQVSLWCLVKGFYPSDIAVEWESNGQPENNYKT TPPVLDSDGSFFLYSKLTVDKSRWQQGNVFSCSVMHEALHNHYTQKSLSLSPG | 506 |
| | III | QVQLVQSGAEVVKPGSSVKVSCEASGFTFTDSVITWVRQRPGQGLEWIGEIYPGSGN FYYNEKFQGRVTITADKSTSTAYMELSSLRSEDTAVYFCAMRRSSWGQGTLVTVSSAS TKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGP SVFLFPPKPKDTLYITREPEVTCVVVDVSHEDPEVKFNWYVDGVEVHNAKTKPREEQY NNASRVVSVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFFLVSKLTV DKSRWQQGNVFSCSVMHEALHNRFTQKSLSLSPG | 507 |
| | IV | DVVMTQTPLSLSVTPGQPASISCKSSQTIVHSNSKTYLEWYLQKPGQSPQLLIYKVSSRF SGVPDRFSGSGSGTDFTLKISRVEAEDVGVYYCFQGSHVPLTFGQGTKLEIKRTVAAPS VFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC | 509 |
| 3437 | I | EIVLTQSPDFLSVTPKEKVTLTCRASQSIGNNLHWYQQKSDQSPKLLIKYASQSISGIPSR FSGSGSGTDFTLTINSVEAEDAATYFCQQSKSWPRTFGGGTKLEIKDKTHTEIVLTQSP DFLSVTPKEKVTLSCRASQSISNYLHWYQQKSDQSPKLLIKYASQSISGIPSRFSGSGSGT DFTLSINSVEAEDAATYFCQQSKSWPRTFGGGTKLEIKDKTHTRTVAAPSVFIFPPSDE QLKSGTASVVCLLNNFYPREAKVQWKVDNALQSGNSQESVTEQDSKDSTYSLSSTLTL SKADYEKHKVYACEVTHQGLSSPVTKSFNRGEC | 512 |
| | II | EVQLVESGGGLVKPGGSLRLSCAASGFTFSSYTMSWVRQAPGKGLEWVASISSSGSN TYYPDSVKGRFTISRDNAKNNLYLQMNSLRAEDTALYYCARFDYGSSLDSWGQGTLLT VSSDKTHTEVQLVESGGGLVKPGGSLRLSCAASGFTFSSYTMSWVRQAPGKGLEWVA SISSSGSNTYYPDSVKGRFTISRDNAKNNLYLQMNSLRAEDTALYYCARFDYGSSLDSW GQGTLLTVSSDKTHTASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSCD KTHTCPPCPAPELLGGPSVFLFPPKPKDTLYITREPEVTCVVVDVSHEDPEVKFNWYVD GVEVHNAKTKPREEQYNNASRVVSVLTVLHQDWLNGKEYKCKVSNKALPAPIEKTISK AKGQPREPQVYTLPPCRDELTKNQVSLWCLVKGFYPSDIAVEWESNGQPENNYKTTP PVLDSDGSFFLYSKLTVDKSRWQQGNVFSCSVMHEALHNHYTQKSLSLSPG | 510 |
| | III | QVQLVQSGAEVVKPGSSVKVSCEASGFTFTDSVITWVRQRPGQGLEWIGEIYPGSGN FYYNEKFQGRVTITADKSTSTAYMELSSLRSEDTAVYFCAMRRSSWGQGTLVTVSSAS TKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGP SVFLFPPKPKDTLYITREPEVTCVVVDVSHEDPEVKFNWYVDGVEVHNAKTKPREEQY NNASRVVSVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFFLVSKLTV DKSRWQQGNVFSCSVMHEALHNRFTQKSLSLSPG | 511 |
| | IV | DVVMTQTPLSLSVTPGQPASISCKSSQTIVHSNSKTYLEWYLQKPGQSPQLLIYKVSSRF SGVPDRFSGSGSGTDFTLKISRVEAEDVGVYYCFQGSHVPLTFGQGTKLEIKRTVAAPS VFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC | 513 |
| 3439 | I | EIVLTQSPDFLSVTPKEKVTLTCRASQSISNNLHWYQQKSDQSPKLLIKYASQSISGIPSR FSGSGSGTDFTLTINSVEAEDAATYFCQQSKSWPRTFGGGTKLEIKDKTHTEIVLTQSP DFLSVTPGEKVTLTCRASQSISNYLHWYQQKSDQSPKLLIKYASQSISGVPSRFSGSGSG TDFTLTISSVEAEDFATYFCQQSKSWPRTFGGGTKLEIKDKTHTRTVAAPSVFIFPPSDE QLKSGTASVVCLLNNFYPREAKVQWKVDNALQSGNSQESVTEQDSKDSTYSLSSTLTL SKADYEKHKVYACEVTHQGLSSPVTKSFNRGEC | 516 |
| | II | EVQLVESGGGLVQPGGSLRLSCAASGFTFSSYTMSWVRQAPGKGLEWVATISSSGDN TYYPDSVKGRFTISRDNSKNTLYLQMSSLRAEDTALYYCARFDYGSSLDSWGQGTTLTV SSDKTHTEVQLVESGGGLVKPGGSLRLSCAASGFTFSSYTMSWVRQAPGKGLEWVAS ISSSGSNTYYPDSVKGRFTISRDNAKNNLYLQMNSLRAEDTALYYCARFDYGSSLDSW GQGTLLTVSSDKTHTASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSCD KTHTCPPCPAPELLGGPSVFLFPPKPKDTLYITREPEVTCVVVDVSHEDPEVKFNWYVD GVEVHNAKTKPREEQYNNASRVVSVLTVLHQDWLNGKEYKCKVSNKALPAPIEKTISK AKGQPREPQVYTLPPCRDELTKNQVSLWCLVKGFYPSDIAVEWESNGQPENNYKTTP PVLDSDGSFFLYSKLTVDKSRWQQGNVFSCSVMHEALHNHYTQKSLSLSPG | 514 |
| | III | QVQLVQSGAEVVKPGSSVKVSCEASGFTFTDSVITWVRQRPGQGLEWIGEIYPGSGN FYYNEKFQGRVTITADKSTSTAYMELSSLRSEDTAVYFCAMRRSSWGQGTLVTVSSAS TKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGP SVFLFPPKPKDTLYITREPEVTCVVVDVSHEDPEVKFNWYVDGVEVHNAKTKPREEQY NNASRVVSVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFFLVSKLTV DKSRWQQGNVFSCSVMHEALHNRFTQKSLSLSPG | 515 |
| | IV | DVVMTQTPLSLSVTPGQPASISCKSSQTIVHSNSKTYLEWYLQKPGQSPQLLIYKVSSRF SGVPDRFSGSGSGTDFTLKISRVEAEDVGVYYCFQGSHVPLTFGQGTKLEIKRTVAAPS VFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC | 517 |

**Table I3. Amino acid sequences of humanized, bispecific binding proteins.**

| **Name** | **Chain** | **Sequence** | **SEQ ID NO** |
|---|---|---|---|
| AS30_Hu6 x C3_Hu10 Duobody | HC1 | QVQLVQSGAEVVKPGSSVKVSCEASGFTFTDSVITWVRQRPGQGLEWIGEIYPGSG NFYYNEKFQGRVTITADKSTSTAYMELSSLRSEDTAVYFCAMRRSSWGQGTLVTVS SASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYVDGVEVHNA KTKPREEQYNNASRVVSVLTVLHQDWLNGKEYKCKVSNKALPAPIEKTISKAKGQPR EPQVYTLPPSRDELTKNQVSLTCLVKGFYPSDIAVEWESNGQPENNYKTTPPVLDSD GSFFLYSRLTVDKSRWQQGNVFSCSVMHEALHNHYTQKSLSLSPG | 518 |
| | HC2 | EVQLVESGGGLVKPGGSLRLSCAASGFTFSSYTMSWVRQAPGKGLEWVASISSSGS NTYYPDSVKGRFTISRDNAKNNLYLQMNSLRAEDTALYYCARFDYGSSLDSWGQGT LLTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEV HNAKTKPREEQYNNSARVVSVLTVLHQDWLNGKEYKCKVSNKALPAPIEKTISKAK GQPREPQVYTLPPSRDELTKNQVSLTCLVKGFYPSDIAVEWESNGQPENNYKTTPP VLDSDGSFLLYSKLTVDKSRWQQGNVFSCSVMHEALHNHYTQKSLSLSPG | 519 |
| | LC1 | DVVMTQTPLSLSVTPGQPASISCKSSQTIVHSNSKTYLEWYLQKPGQSPQLLIYKVSS RFSGVPDRFSGSGSGTDFTLKISRVEAEDVGVYYCFQGSHVPLTFGQGTKLEIKRTVA APSVFIFPPSDEQLKSGTASVVCLLNNFYPREAKVQWKVDNALQSGNSQESVTEQD SKDSTYSLSSTLTLSKADYEKHKVYACEVTHQGLSSPVTKSFNRGEC | 520 |
| | LC2 | EIVLTQSPDFLSVTPKEKVTLTCRASQSISNNLHWYQQKSDQSPKLLIKYASQSISGIPS RFSGSGSGTDFTLTINSVEAEDAATYFCQQSKSWPRTFGGGTKLEIKRTVAAPSVFIF PPSDEQLKSGTASVVCLLNNFYPREAKVQWKVDNALQSGNSQESVTEQDSKDSTYS LSSTLTLSKADYEKHKVYACEVTHQGLSSPVTKSFNRGEC | 521 |
| AS30_Hu6 x C21_Hu11 Duetmab | HC1 | QVQLVQSGAEVVKPGSSVKVSCEASGFTFTDSVITWVRQRPGQGLEWIGEIYPGSG NFYYNEKFQGRVTITADKSTSTAYMELSSLRSEDTAVYFCAMRRSSWGQGTLVTVS SASTKGPSVCPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSVDKTHTCPPCPA PELLGGPSVFLFPPKPKDTLMISRTPEVTCVVVDVSHEDPEVKFNWYVDGVEVHNA KTKPREEQYNNASRVVSVLTVLHQDWLNGKEYKCKVSNKALPAPIEKTISKAKGQPR EPQVYTLPPCRDELTKNQVSLWCLVKGFYPSDIAVEWESNGQPENNYKTTPPVLDS DGSFFLYSKLTVDKSRWQQGNVFSCSVMHEALHNHYTQKSLSLSPG | 524 |
| | HC2 | EVQLVESGGGLVKPGGSLRLSCAASGFTFSSYTMSWVRQAPGKGLEWVASISSSGS NTYYPDSVKGRFTISRDNAKNNLYLQMNSLRAEDTALYYCARFDYGSSLDSWGQGT LLTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEV HNAKTKPREEQYNNASRVVSVLTVLHQDWLNGKEYKCKVSNKALPAPIEKTISKAK GQPREPQVCTLPPSRDELTKNQVSLSCAVKGFYPSDIAVEWESNGQPENNYKTTPP VLDSDGSFFLVSKLTVDKSRWQQGNVFSCSVMHEALHNRFTQKSLSLSPG | 525 |
| | LC1 | DVVMTQTPLSLSVTPGQPASISCKSSQTIVHSNSKTYLEWYLQKPGQSPQLLIYKVSS RFSGVPDRFSGSGSGTDFTLKISRVEAEDVGVYYCFQGSHVPLTFGQGTKLEIKRTVA APSVFIFPPCDEQLKSGTASVVCLLNNFYPREAKVQWKVDNALQSGNSQESVTEQD SKDSTYSLSSTLTLSKADYEKHKVYACEVTHQGLSSPVTKSFNRGEV | 526 |
| | LC2 | EIVLTQSPDFLSVTPKEKVTLSCRASQSISNYLHWYQQKSDQSPKLLIKYASQSISGIPS RFSGSGSGTDFTLSINSVEAEDAATYFCQQSKSWPRTFGGGTKLEIKRTVAAPSVFIF PPSDEQLKSGTASVVCLLNNFYPREAKVQWKVDNALQSGNSQESVTEQDSKDSTYS LSSTLTLSKADYEKHKVYACEVTHQGLSSPVTKSFNRGEC | 527 |
| AS30_Hu6 x C3_Hu10 TBTI | HC | QVQLVQSGAEVVKPGSSVKVSCEASGFTFTDSVITWVRQRPGQGLEWIGEIYPGSG NFYYNEKFQGRVTITADKSTSTAYMELSSLRSEDTAVYFCAMRRSSWGQGTLVTVS SGGGGSGGGGSEVQLVESGGGLVKPGGSLRLSCAASGFTFSSYTMSWVRQAPGKG LEWVASISSSGSNTYYPDSVKGRFTISRDNAKNNLYLQMNSLRAEDTALYYCARFDY GSSLDSWGQGTLLTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHEDPE VKFNWYVDGVEVHNAKTKPREEQYNNASRVVSVLTVLHQDWLNGKEYKCKVSNK ALPAPIEKTISKAKGQPREPQVYTLPPSRDELTKNQVSLTCLVKGFYPSDIAVEWESN GQPENNYKTTPPVLDSDGSFFLYSKLTVDKSRWQQGNVFSCSVMHEALHNHYTQK SLSLSPG | 522 |
| | LC | DVVMTQTPLSLSVTPGQPASISCKSSQTIVHSNSKTYLEWYLQKPGQSPQLLIYKVSS RFSGVPDRFSGSGSGTDFTLKISRVEAEDVGVYYCFQGSHVPLTFGQGTKLEIKGGG GSGGGGSEIVLTQSPDFLSVTPKEKVTLTCRASQSISNNLHWYQQKSDQSPKLLIKYA SQSISGIPSRFSGSGSGTDFTLTINSVEAEDAATYFCQQSKSWPRTFGGGTKLEIKRTV AAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC | 523 |
| AS30_Hu6 x C21_Hu11 TBTI | HC | QVQLVQSGAEVVKPGSSVKVSCEASGFTFTDSVITWVRQRPGQGLEWIGEIYPGSG NFYYNEKFQGRVTITADKSTSTAYMELSSLRSEDTAVYFCAMRRSSWGQGTLVTVS SGGGGSGGGGSEVQLVESGGGLVKPGGSLRLSCAASGFTFSSYTMSWVRQAPGKG LEWVASISSSGSNTYYPDSVKGRFTISRDNAKNNLYLQMNSLRAEDTALYYCARFDY GSSLDSWGQGTLLTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKK | 528 |
| | | VEPKSCDKTHTCPPCPAPELLGGPSVFLFPPKPKDTLMISRTPEVTCVVVDVSHEDPE VKFNWYVDGVEVHNAKTKPREEQYNNASRVVSVLTVLHQDWLNGKEYKCKVSNK ALPAPIEKTISKAKGQPREPQVYTLPPSRDELTKNQVSLTCLVKGFYPSDIAVEWESN GQPENNYKTTPPVLDSDGSFFLYSKLTVDKSRWQQGNVFSCSVMHEALHNHYTQK SLSLSPG | |
| | LC | DVVMTQTPLSLSVTPGQPASISCKSSQTIVHSNSKTYLEWYLQKPGQSPQLLIYKVSS RFSGVPDRFSGSGSGTDFTLKISRVEAEDVGVYYCFQGSHVPLTFGQGTKLEIKGGG GSGGGGSEIVLTQSPDFLSVTPKEKVTLSCRASQSISNYLHWYQQKSDQSPKLLIKYA SQSISGIPSRFSGSGSGTDFTLSINSVEAEDAATYFCQQSKSWPRTFGGGTKLEIKRTV AAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC | 529 |
| AS30_Hu9 x C3_Hu11 CODV | HC | QVQLVQSGAEVVKPGSSVKVSCEASGFTFTDSVITWVRQRPGQGLEWIGEIYPGSG NFYYNEKFQGRVTITADKSTSTAYMELSSLRSEDTAVYFCAMRRSSWGQGTLVTVS SSEVQLVESGGGLVKPGGSLRLSCAASGFTFSSYTMSWVRQAPGKGLEWVASISSS GSNTYYPDSVKGRFTISRDNAKNNLYLQMNSLRAEDTALYYCARFDYGSSLDSWGQ GTLLTVSSRTASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFNWYVD GVEVHNAKTKPREEQYNNASRVVSVLTVLHQDWLNGKEYKCKVSNKALPAPIEKTI SKAKGQPREPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKSLSLSPG | 530 |
| | LC | EIVLTQSPDFLSVTPKEKVTLTCRASQSIGNNLHWYQQKSDQSPKLLIKYASQSISGIP SRFSGSGSGTDFTLTINSVEAEDAATYFCQQSKSWPRTFGGGTKLEIKGQPKAAPDV VMTQTPLSLSVTPGQPASISCRSSQTIVHSNSKTYLEWYLQKPGQSPQLLIYKVSNRF SGVPDRFSGSGSGTDFTLKISRVEAEDVGVYYCFQGSHVPLTFGAGTKLEIKTKGPSR TVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC | 531 |
| AS30_Hu9 x C21_Hu21 CODV | HC | QVQLVQSGAEVVKPGSSVKVSCEASGFTFTDSVITWVRQRPGQGLEWIGEIYPGSG NFYYNEKFQGRVTITADKSTSTAYMELSSLRSEDTAVYFCAMRRSSWGQGTLVTVS SSEVQLVESGGGLVQPGGSLRLSCAASGFTFSSYTMSWVRQAPGKGLEWVATISSS GDNTYYPDSVKGRFTISRDNSKNTLYLQMSSLRAEDTALYYCARFDYGSSLDSWGQ GTTLTVSSRTASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFNWYVD GVEVHNAKTKPREEQYNNASRVVSVLTVLHQDWLNGKEYKCKVSNKALPAPIEKTI SKAKGQPREPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKSLSLSPG | 532 |
| | LC | EIVLTQSPDFLSVTPGEKVTLTCRASQSISNYLHWYQQKSDQSPKLLIKYASQSISGVP SRFSGSGSGTDFTLTISSVEAEDFATYFCQQSKSWPRTFGGGTKLEIKGQPKAAPDV VMTQTPLSLSVTPGQPASISCRSSQTIVHSNSKTYLEWYLQKPGQSPQLLIYKVSNRF SGVPDRFSGSGSGTDFTLKISRVEAEDVGVYYCFQGSHVPLTFGAGTKLEIKTKGPSR TVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC | 533 |

**Table I4. Amino acid and DNA sequences of humanized, multispecific and bispecific binding proteins.**

| **SEQ ID NO** | **Binding Protein** | **Chain** | **Sequence** | **DNA/Protein** |
|---|---|---|---|---|
| 306 | AS30SS_Hu6 | VH | VH domain | DNA |
| 307 | AS30SS_Hu6 | VH | FW1 | DNA |
| 308 | AS30SS_Hu6 | VH | CDR-H1 | DNA |
| 309 | AS30SS_Hu6 | VH | FW2 | DNA |
| 310 | AS30SS_Hu6 | VH | CDR-H2 | DNA |
| 311 | AS30SS_Hu6 | VH | FW3 | DNA |
| 312 | AS30SS_Hu6 | VH | CDR-H3 | DNA |
| 313 | AS30SS_Hu6 | VH | FW4 | DNA |
| 314 | AS30SS_Hu6 | VH | VH domain | protein |
| 315 | AS30SS_Hu6 | VH | FW1 | protein |
| 316 | AS30SS_Hu6 | VH | CDR-H1 | protein |
| 317 | AS30SS_Hu6 | VH | FW2 | protein |
| 318 | AS30SS_Hu6 | VH | CDR-H2 | protein |
| 319 | AS30SS_Hu6 | VH | FW3 | protein |
| 320 | AS30SS_Hu6 | VH | CDR-H3 | protein |
| 321 | AS30SS_Hu6 | VH | FW4 | protein |
| 322 | AS30SS_Hu6 | VL | VL domain | DNA |
| 323 | AS30SS_Hu6 | VL | FW1 | DNA |
| 324 | AS30SS_Hu6 | VL | CDR-L1 | DNA |
| 325 | AS30SS_Hu6 | VL | FW2 | DNA |
| 326 | AS30SS_Hu6 | VL | CDR-L2 | DNA |
| 327 | AS30SS_Hu6 | VL | FW3 | DNA |
| 328 | AS30SS_Hu6 | VL | CDR-L3 | DNA |
| 329 | AS30SS_Hu6 | VL | FW4 | DNA |
| 330 | AS30SS_Hu6 | VL | VL domain | protein |
| 331 | AS30SS_Hu6 | VL | FW1 | protein |
| 332 | AS30SS_Hu6 | VL | CDR-L1 | protein |
| 333 | AS30SS_Hu6 | VL | FW2 | protein |
| 334 | AS30SS_Hu6 | VL | CDR-L2 | protein |
| 335 | AS30SS_Hu6 | VL | FW3 | protein |
| 336 | AS30SS_Hu6 | VL | CDR-L3 | protein |
| 337 | AS30SS_Hu6 | VL | FW4 | protein |
| 338 | AS30SS_Hu9 | VH | VH domain | DNA |
| 339 | AS30SS_Hu9 | VH | FW1 | DNA |
| 340 | AS30SS_Hu9 | VH | CDR-H1 | DNA |
| 341 | AS30SS_Hu9 | VH | FW2 | DNA |
| 342 | AS30SS_Hu9 | VH | CDR-H2 | DNA |
| 343 | AS30SS_Hu9 | VH | FW3 | DNA |
| 344 | AS30SS_Hu9 | VH | CDR-H3 | DNA |
| 345 | AS30SS_Hu9 | VH | FW4 | DNA |
| 346 | AS30SS_Hu9 | VH | VH domain | protein |
| 347 | AS30SS_Hu9 | VH | FW1 | protein |
| 348 | AS30SS_Hu9 | VH | CDR-H1 | protein |
| 349 | AS30SS_Hu9 | VH | FW2 | protein |
| 350 | AS30SS_Hu9 | VH | CDR-H2 | protein |
| 351 | AS30SS_Hu9 | VH | FW3 | protein |
| 352 | AS30SS_Hu9 | VH | CDR-H3 | protein |
| 353 | AS30SS_Hu9 | VH | FW4 | protein |
| 354 | AS30SS_Hu9 | VL | VL domain | DNA |
| 355 | AS30SS_Hu9 | VL | FW1 | DNA |
| 356 | AS30SS_Hu9 | VL | CDR-L1 | DNA |
| 357 | AS30SS_Hu9 | VL | FW2 | DNA |
| 358 | AS30SS_Hu9 | VL | CDR-L2 | DNA |
| 359 | AS30SS_Hu9 | VL | FW3 | DNA |
| 360 | AS30SS_Hu9 | VL | CDR-L3 | DNA |
| 361 | AS30SS_Hu9 | VL | FW4 | DNA |
| 362 | AS30SS_Hu9 | VL | VL domain | protein |
| 363 | AS30SS_Hu9 | VL | FW1 | protein |
| 364 | AS30SS_Hu9 | VL | CDR-L1 | protein |
| 365 | AS30SS_Hu9 | VL | FW2 | protein |
| 366 | AS30SS_Hu9 | VL | CDR-L2 | protein |
| 367 | AS30SS_Hu9 | VL | FW3 | protein |
| 368 | AS30SS_Hu9 | VL | CDR-L3 | protein |
| 369 | AS30SS_Hu9 | VL | FW4 | protein |
| 370 | C3_Hu10 | VH | VH domain | DNA |
| 371 | C3_Hu10 | VH | FW1 | DNA |
| 372 | C3_Hu10 | VH | CDR-H1 | DNA |
| 373 | C3_Hu10 | VH | FW2 | DNA |
| 374 | C3_Hu10 | VH | CDR-H2 | DNA |
| 375 | C3_Hu10 | VH | FW3 | DNA |
| 376 | C3_Hu10 | VH | CDR-H3 | DNA |
| 377 | C3_Hu10 | VH | FW4 | DNA |
| 378 | C3_Hu10 | VH | VH domain | protein |
| 379 | C3_Hu10 | VH | FW1 | protein |
| 380 | C3_Hu10 | VH | CDR-H1 | protein |
| 381 | C3_Hu10 | VH | FW2 | protein |
| 382 | C3_Hu10 | VH | CDR-H2 | protein |
| 383 | C3_Hu10 | VH | FW3 | protein |
| 384 | C3_Hu10 | VH | CDR-H3 | protein |
| 385 | C3_Hu10 | VH | FW4 | protein |
| 386 | C3_Hu10 | VL | VL domain | DNA |
| 387 | C3_Hu10 | VL | FW1 | DNA |
| 388 | C3_Hu10 | VL | CDR-L1 | DNA |
| 389 | C3_Hu10 | VL | FW2 | DNA |
| 390 | C3_Hu10 | VL | CDR-L2 | DNA |
| 391 | C3_Hu10 | VL | FW3 | DNA |
| 392 | C3_Hu10 | VL | CDR-L3 | DNA |
| 393 | C3_Hu10 | VL | FW4 | DNA |
| 394 | C3_Hu10 | VL | VL domain | protein |
| 395 | C3_Hu10 | VL | FW1 | protein |
| 396 | C3_Hu10 | VL | CDR-L1 | protein |
| 397 | C3_Hu10 | VL | FW2 | protein |
| 398 | C3_Hu10 | VL | CDR-L2 | protein |
| 399 | C3_Hu10 | VL | FW3 | protein |
| 400 | C3_Hu10 | VL | CDR-L3 | protein |
| 401 | C3_Hu10 | VL | FW4 | protein |
| 402 | C3_Hu11 | VH | VH domain | DNA |
| 403 | C3_Hu11 | VH | FW1 | DNA |
| 404 | C3_Hu11 | VH | CDR-H1 | DNA |
| 405 | C3_Hu11 | VH | FW2 | DNA |
| 406 | C3_Hu11 | VH | CDR-H2 | DNA |
| 407 | C3_Hu11 | VH | FW3 | DNA |
| 408 | C3_Hu11 | VH | CDR-H3 | DNA |
| 409 | C3_Hu11 | VH | FW4 | DNA |
| 410 | C3_Hu11 | VH | VH domain | protein |
| 411 | C3_Hu11 | VH | FW1 | protein |
| 412 | C3_Hu11 | VH | CDR-H1 | protein |
| 413 | C3_Hu11 | VH | FW2 | protein |
| 414 | C3_Hu11 | VH | CDR-H2 | protein |
| 415 | C3_Hu11 | VH | FW3 | protein |
| 416 | C3_Hu11 | VH | CDR-H3 | protein |
| 417 | C3_Hu11 | VH | FW4 | protein |
| 418 | C3_Hu11 | VL | VL domain | DNA |
| 419 | C3_Hu11 | VL | FW1 | DNA |
| 420 | C3_Hu11 | VL | CDR-L1 | DNA |
| 421 | C3_Hu11 | VL | FW2 | DNA |
| 422 | C3_Hu11 | VL | CDR-L2 | DNA |
| 423 | C3_Hu11 | VL | FW3 | DNA |
| 424 | C3_Hu11 | VL | CDR-L3 | DNA |
| 425 | C3_Hu11 | VL | FW4 | DNA |
| 426 | C3_Hu11 | VL | VL domain | protein |
| 427 | C3_Hu11 | VL | FW1 | protein |
| 428 | C3_Hu11 | VL | CDR-L1 | protein |
| 429 | C3_Hu11 | VL | FW2 | protein |
| 430 | C3_Hu11 | VL | CDR-L2 | protein |
| 431 | C3_Hu11 | VL | FW3 | protein |
| 432 | C3_Hu11 | VL | CDR-L3 | protein |
| 433 | C3_Hu11 | VL | FW4 | protein |
| 434 | C21_Hu11 | VH | VH domain | DNA |
| 435 | C21_Hu11 | VH | FW1 | DNA |
| 436 | C21_Hu11 | VH | CDR-H1 | DNA |
| 437 | C21_Hu11 | VH | FW2 | DNA |
| 438 | C21_Hu11 | VH | CDR-H2 | DNA |
| 439 | C21_Hu11 | VH | FW3 | DNA |
| 440 | C21_Hu11 | VH | CDR-H3 | DNA |
| 441 | C21_Hu11 | VH | FW4 | DNA |
| 442 | C21_Hu11 | VH | VH domain | protein |
| 443 | C21_Hu11 | VH | FW1 | protein |
| 444 | C21_Hu11 | VH | CDR-H1 | protein |
| 445 | C21_Hu11 | VH | FW2 | protein |
| 446 | C21_Hu11 | VH | CDR-H2 | protein |
| 447 | C21_Hu11 | VH | FW3 | protein |
| 448 | C21_Hu11 | VH | CDR-H3 | protein |
| 449 | C21_Hu11 | VH | FW4 | protein |
| 450 | C21_Hu11 | VL | VL domain | DNA |
| 451 | C21_Hu11 | VL | FW1 | DNA |
| 452 | C21_Hu11 | VL | CDR-L1 | DNA |
| 453 | C21_Hu11 | VL | FW2 | DNA |
| 454 | C21_Hu11 | VL | CDR-L2 | DNA |
| 455 | C21_Hu11 | VL | FW3 | DNA |
| 456 | C21_Hu11 | VL | CDR-L3 | DNA |
| 457 | C21_Hu11 | VL | FW4 | DNA |
| 458 | C21_Hu11 | VL | VL domain | protein |
| 459 | C21_Hu11 | VL | FW1 | protein |
| 460 | C21_Hu11 | VL | CDR-L1 | protein |
| 461 | C21_Hu11 | VL | FW2 | protein |
| 462 | C21_Hu11 | VL | CDR-L2 | protein |
| 463 | C21_Hu11 | VL | FW3 | protein |
| 464 | C21_Hu11 | VL | CDR-L3 | protein |
| 465 | C21_Hu11 | VL | FW4 | protein |
| 466 | C21_Hu21 | VH | VH domain | DNA |
| 467 | C21_Hu21 | VH | FW1 | DNA |
| 468 | C21_Hu21 | VH | CDR-H1 | DNA |
| 469 | C21_Hu21 | VH | FW2 | DNA |
| 470 | C21_Hu21 | VH | CDR-H2 | DNA |
| 471 | C21_Hu21 | VH | FW3 | DNA |
| 472 | C21_Hu21 | VH | CDR-H3 | DNA |
| 473 | C21_Hu21 | VH | FW4 | DNA |
| 474 | C21_Hu21 | VH | VH domain | protein |
| 475 | C21_Hu21 | VH | FW1 | protein |
| 476 | C21_Hu21 | VH | CDR-H1 | protein |
| 477 | C21_Hu21 | VH | FW2 | protein |
| 478 | C21_Hu21 | VH | CDR-H2 | protein |
| 479 | C21_Hu21 | VH | FW3 | protein |
| 480 | C21_Hu21 | VH | CDR-H3 | protein |
| 481 | C21_Hu21 | VH | FW4 | protein |
| 482 | C21_Hu21 | VL | VL domain | DNA |
| 483 | C21_Hu21 | VL | FW1 | DNA |
| 484 | C21_Hu21 | VL | CDR-L1 | DNA |
| 485 | C21_Hu21 | VL | FW2 | DNA |
| 486 | C21_Hu21 | VL | CDR-L2 | DNA |
| 487 | C21_Hu21 | VL | FW3 | DNA |
| 488 | C21_Hu21 | VL | CDR-L3 | DNA |
| 489 | C21_Hu21 | VL | FW4 | DNA |
| 490 | C21_Hu21 | VL | VL domain | protein |
| 491 | C21_Hu21 | VL | FW1 | protein |
| 492 | C21_Hu21 | VL | CDR-L1 | protein |
| 493 | C21_Hu21 | VL | FW2 | protein |
| 494 | C21_Hu21 | VL | CDR-L2 | protein |
| 495 | C21_Hu21 | VL | FW3 | protein |
| 496 | C21_Hu21 | VL | CDR-L3 | protein |
| 497 | C21_Hu21 | VL | FW4 | protein |
| 498 | Triab 3407 | HC1 | Full chain | protein |
| 499 | Triab 3407 | HC2 | Full chain | protein |
| 500 | Triab 3407 | LC1 | Full chain | protein |
| 501 | Triab 3407 | LC2 | Full chain | protein |
| 502 | Triab 3423 | HC1 | Full chain | protein |
| 503 | Triab 3423 | HC2 | Full chain | protein |
| 504 | Triab 3423 | LC1 | Full chain | protein |
| 505 | Triab 3423 | LC2 | Full chain | protein |
| 506 | Triab 3429 | HC1 | Full chain | protein |
| 507 | Triab 3429 | HC2 | Full chain | protein |
| 508 | Triab 3429 | LC1 | Full chain | protein |
| 509 | Triab 3429 | LC2 | Full chain | protein |
| 510 | Triab 3437 | HC1 | Full chain | protein |
| 511 | Triab 3437 | HC2 | Full chain | protein |
| 512 | Triab 3437 | LC1 | Full chain | protein |
| 513 | Triab 3437 | LC2 | Full chain | protein |
| 514 | Triab 3439 | HC1 | Full chain | protein |
| 515 | Triab 3439 | HC2 | Full chain | protein |
| 516 | Triab 3439 | LC1 | Full chain | protein |
| 517 | Triab 3439 | LC2 | Full chain | protein |
| 518 | AS30_Hu6 x C3_Hu10 duobody | HC1 | Full chain | protein |
| 519 | AS30_Hu6 x C3_Hu10 duobody | HC2 | Full chain | protein |
| 520 | AS30_Hu6 x C3_Hu10 duobody | LC1 | Full chain | protein |
| 521 | AS30_Hu6 x C3_Hu10 duobody | LC2 | Full chain | protein |
| 522 | AS30_Hu6 x C3_Hu10 TBTI | HC | Full chain | protein |
| 523 | AS30_Hu6 x C3_Hu10 TBTI | LC | Full chain | protein |
| 524 | AS30_Hu6 x C21_Hu11 duetmab | HC1 | Full chain | protein |
| 525 | AS30_Hu6 x C21_Hu11 | HC2 | Full chain | protein |
| | duetmab | | | |
| 526 | AS30_Hu6 x C21_Hu11 duetmab | LC1 | Full chain | protein |
| 527 | AS30_Hu6 x C21_Hu11 duetmab | LC2 | Full chain | protein |
| 528 | AS30_Hu6 x C21_Hu11 TBTI | HC | Full chain | protein |
| 529 | AS30_Hu6 x C21_Hu11 TBTI | LC | Full chain | protein |
| 530 | AS30_Hu9 x C3_Hu11 CODV | HC | Full chain | protein |
| 531 | AS30_Hu9 x C3_Hu11 CODV | LC | Full chain | protein |
| 532 | AS30_Hu9 x C21_Hu21 CODV | HC | Full chain | protein |
| 533 | AS30_Hu9 x C21_Hu21 CODV | LC | Full chain | protein |

### Target Proteins

Provided herein are multispecific binding molecules (e.g., binding proteins) that include a binding domain that binds an extracellular portion of dystroglycan and a binding domain that binds laminin-2. The terms "binds" and "specifically binds" are used interchangeably herein. In some embodiments, a binding domain that "binds" an antigen (*e.g.,* laminin-2 or an extracellular portion of dystroglycan) binds to the antigen with an K_{D} of less than or equal to about 1 x 10⁻⁶ M. In some embodiments, binding affinity (*e.g.,* K_{D}) of the antigen binding domain to the antigen (*e.g.*, an antigen epitope) is assayed using the antigen binding domain in a monovalent antibody or antigen-binding fragment thereof. In some embodiments, binding affinity (*e.g.,* K_{D}) of the antigen binding domain to the antigen (*e.g.,* an antigen epitope) is assayed using the antigen binding domain in a multispecific format of the present disclosure.

As used herein, dystroglycan (DG) refers to the dystrophin-associated protein that acts as a component of the dystrophin complex linking the extracellular matrix (ECM, also known as the basal lamina) to the F-actin-associated cytoskeleton of muscle fibers. Dystroglycan comprises two subunits, alpha dystroglycan and beta dystroglycan, that are post-translationally cleaved and associate non-covalently with each other. In some embodiments, the dystroglycan is human dystroglycan (*e.g.,* a protein encoded by the human *DAG1* gene as set forth in NCBI Ref. Seq. Gene ID No. 1605, or a protein corresponding to UniProt Entry Q14118). In some embodiments, the dystroglycan is mouse dystroglycan (*e.g*., a protein encoded by the mouse *Dag1* gene as set forth in NCBI Ref. Seq. Gene ID No. 13138, or a protein corresponding to UniProt Entry Q62165).

In some embodiments, a binding domain of the present disclosure binds alpha-dystroglycan. In some embodiments, a binding domain of the present disclosure binds beta-dystroglycan. In some embodiments, a binding domain of the present disclosure binds a polypeptide comprising the sequence SIVVEWTNN TLPLEPCPKE QIIGLSRRIA DENGKPRPAF SNALEPDFKA LSIAVTGSGS CRHLQFIPVA PPSPGSSAAP ATEVPDRDPE KSSEDD (SEQ ID NO:290). In some embodiments, a binding domain of the present disclosure binds an epitope or region within the sequence SIVVEWTNN TLPLEPCPKE QIIGLSRRIA DENGKPRPAF SNALEPDFKA LSIAVTGSGS CRHLQFIPVA PPSPGSSAAP ATEVPDRDPE KSSEDD (SEQ ID NO:290). In some embodiments, a binding domain of the present disclosure binds a polypeptide comprising the sequence SIVVEWT NNTLPLEPCP KEQIAGLSRR IAEDDGKPRP AFSNALEPDF KATSITVTGS GSCRHLQFIP VVPPRRVPSE APPTEVPDRD PEKSSEDDV (SEQ ID NO:291). In some embodiments, a binding domain of the present disclosure binds an epitope or region within the sequence SIVVEWT NNTLPLEPCP KEQIAGLSRR IAEDDGKPRP AFSNALEPDF KATSITVTGS GSCRHLQFIP VVPPRRVPSE APPTEVPDRD PEKSSEDDV (SEQ ID NO:291). In some embodiments, a binding domain of the present disclosure binds the extracellular portion of human dystroglycan. In some embodiments, a binding domain of the present disclosure binds the extracellular portion of mouse dystroglycan. In some embodiments, a binding domain of the present disclosure binds the extracellular portions of human and mouse dystroglycan.

In some embodiments, a binding domain of the present disclosure binds the extracellular portion of human dystroglycan with an equilibrium dissociation constant (K_{D}) lower than about 1µM, lower than about 500nM, lower than about 400nM, lower than about 300nM, lower than about 200nM, lower than about 100nM, lower than about 50nM, lower than about 25nM, lower than about 10nM, or lower than about 1nM. In some embodiments, the affinity of binding between a binding domain of the present disclosure and the extracellular portion of human dystroglycan is measured when the binding domain is in a bispecific format, rather than as a monospecific binding domain (such as a monospecific antibody). In some embodiments, an antigen binding site of the present disclosure that binds the extracellular portion of dystroglycan binds the extracellular portion of human dystroglycan with an equilibrium dissociation constant (K_{D}) lower than about 1µM, lower than about 500nM, lower than about 400nM, lower than about 300nM, lower than about 200nM, lower than about 100nM, lower than about 50nM, lower than about 25nM, lower than about 10nM, or lower than about 1nM when assayed as part of a multispecific binding protein.

As used herein, laminin-2 (also known as merosin) refers to the extracellular basement membrane protein that binds to dystroglycan. Laminin-2 is composed of three subunits: alpha, beta, and gamma. In some embodiments, the laminin-2 is human laminin subunit alpha 2 (*e.g.,* a protein encoded by the human *LAMA2* gene as set forth in NCBI Ref. Seq. Gene ID No. 3908, or a protein corresponding to UniProt Entry P24043). In some embodiments, the dystroglycan is mouse laminin subunit alpha 2 (*e.g.,* a protein encoded by the mouse *Lama2* gene as set forth in NCBI Ref. Seq. Gene ID No. 16773, or a protein corresponding to UniProt Entry Q60675).

In some embodiments, a binding domain of the present disclosure binds laminin-2. In some embodiments, a binding domain of the present disclosure binds a polypeptide comprising a laminin G-like (LG) domain 4 of laminin-2, a laminin G-like (LG) domain 5 of laminin-2, or both. In some embodiments, a binding domain of the present disclosure binds a polypeptide comprising the sequence VQPQPV PTPAFPFPAP TMVHGPCVAE SEPALLTGSK QFGLSRNSHI AIAFDDTKVK NRLTIELEVR TEAESGLLFY MARINHADFA TVQLRNGFPY FSYDLGSGDT STMIPTKIND GQWHKIKIVR VKQEGILYVD DASSQTISPK KADILDVVGI LYVGGLPINY TTRRIGPVTY SLDGCVRNLH MEQAPVDLDQ PTSSFHVGTC FANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:300). In some embodiments, a binding domain of the present disclosure binds an epitope or region within the sequence VQPQPV PTPAFPFPAP TMVHGPCVAE SEPALLTGSK QFGLSRNSHI AIAFDDTKVK NRLTIELEVR TEAESGLLFY MARINHADFA TVQLRNGFPY FSYDLGSGDT STMIPTKIND GQWHKIKIVR VKQEGILYVD DASSQTISPK KADILDVVGI LYVGGLPINY TTRRIGPVTY SLDGCVRNLH MEQAPVDLDQ PTSSFHVGTC FANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:300). In some embodiments, a binding domain of the present disclosure binds a polypeptide comprising the sequence ANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:292). In some embodiments, a binding domain of the present disclosure binds an epitope or region within the sequence ANAESGTYF DGTGFAKAVG GFKVGLDLLV EFEFRTTRPT GVLLGVSSQK MDGMGIEMID EKLMFHVDNG AGRFTAIYDA GIPGHMCNGQ WHKVTAKKIK NRLELVVDGN QVDAQSPNSA STSADTNDPV FVGGFPGGLN QFGLTTNIRF RGCIRSLKLT KGTGKPLEVN FAKALELRGV QPVSCPTT (SEQ ID NO:292). In some embodiments, a binding domain of the present disclosure binds a polypeptide comprising the sequence Q PEPVPTPAFP TPTPVLTHGP CAAESEPALL IGSKQFGLSR NSHIAIAFDD TKVKNRLTIE LEVRTEAESG LLFYMARINH ADFATVQLRN GLPYFSYDLG SGDTHTMIPT KINDGQWHKI KIMRSKQEGI LYVDGASNRT ISPKKADILD VVGMLYVGGL PINYTTRRIG PVTYSIDGCV RNLHMAEAPA DLEQPTSSFH VGTCFANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:301). In some embodiments, a binding domain of the present disclosure binds an epitope or region within the sequence Q PEPVPTPAFP TPTPVLTHGP CAAESEPALL IGSKQFGLSR NSHIAIAFDD TKVKNRLTIE LEVRTEAESG LLFYMARINH ADFATVQLRN GLPYFSYDLG SGDTHTMIPT KINDGQWHKI KIMRSKQEGI LYVDGASNRT ISPKKADILD VVGMLYVGGL PINYTTRRIG PVTYSIDGCV RNLHMAEAPA DLEQPTSSFH VGTCFANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:301). In some embodiments, a binding domain of the present disclosure binds a polypeptide comprising the sequence ANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:293). In some embodiments, a binding domain of the present disclosure binds an epitope or region within the sequence ANAQR GTYFDGTGFA KAVGGFKVGL DLLVEFEFRT TTTTGVLLGI SSQKMDGMGI EMIDEKLMFH VDNGAGRFTA VYDAGVPGHL CDGQWHKVTA NKIKHRIELT VDGNQVEAQS PNPASTSADT NDPVFVGGFP DDLKQFGLTT SIPFRGCIRS LKLTKGTGKP LEVNFAKALE LRGVQPVSCP AN (SEQ ID NO:293). In some embodiments, a binding domain of the present disclosure binds human laminin-2. In some embodiments, a binding domain of the present disclosure binds mouse laminin-2. In some embodiments, a binding domain of the present disclosure binds human and mouse laminin-2.

In some embodiments, a binding domain of the present disclosure binds human laminin-2 with an equilibrium dissociation constant (K_{D}) lower than about 1µM, lower than about 500nM, lower than about 400nM, lower than about 300nM, lower than about 200nM, lower than about 100nM, lower than about 50nM, lower than about 25nM, lower than about 10nM, or lower than about 1nM. In some embodiments, the affinity of binding between a binding domain of the present disclosure and human laminin-2 is measured when the binding domain is in a bispecific format, rather than as a monospecific binding domain (such as a monospecific antibody). In some embodiments, an antigen binding site of the present disclosure that binds laminin-2 binds human laminin-2 with an equilibrium dissociation constant (K_{D}) lower than about 1µM, lower than about 500nM, lower than about 400nM, lower than about 300nM, lower than about 200nM, lower than about 100nM, lower than about 50nM, lower than about 25nM, lower than about 10nM, or lower than about 1nM when assayed as part of a multispecific binding protein.

In some embodiments, a V_{H1}/V_{L1} binding pair of the present disclosure binds the extracellular portion of dystroglycan, and a V_{H2}/V_{L2} binding pair of the present disclosure binds laminin-2. In some embodiments, a V_{H2}/V_{L2} binding pair of the present disclosure binds the extracellular portion of dystroglycan, and a V_{H1}/V_{L1} binding pair of the present disclosure binds laminin-2.

### Antibodies

The present disclosure also provides antibodies (*e.g*., monovalent and/or monoclonal antibodies) comprising 1, 2, 3, 4, 5, or 6 CDR sequences of a binding domain shown in Table A2, D2, or I4, or a VH and/or VL domain sequence of a binding domain shown in Table D2 or I4 or encoded by a polynucleotide sequence shown in Table G2. In some embodiments, the antibody binds an extracellular portion of dystroglycan. In some embodiments, the antibody binds laminin-2. In some embodiments, the antibody comprises (a) an antibody heavy chain comprising a heavy chain variable domain (VH) comprising a CDR-H1 comprising an amino acid sequence selected from the group consisting of SEQ ID NOs:1-8, a CDR-H2 comprising an amino acid sequence selected from the group consisting of SEQ ID NOs:9-17, and a CDR-H3 comprising an amino acid sequence selected from the group consisting of SEQ ID NOs:18-27; and (b) an antibody light chain comprising a light chain variable domain (VL) comprising a CDR-L1 comprising an amino acid sequence selected from the group consisting of SEQ ID NOs:28-37, a CDR-L2 comprising an amino acid sequence selected from the group consisting of SEQ ID NOs:38-42, and a CDR-L3 comprising an amino acid sequence selected from the group consisting of SEQ ID NOs:43-50. In some embodiments, the VH domain comprises an amino acid sequence selected from the group consisting of SEQ ID NOs:170, 172, 174, 176, 178, 180, 182, 184, 186, and 188; and the VL domain comprises an amino acid sequence selected from the group consisting of SEQ ID NOs:171, 173, 175, 177, 179, 181, 183, 185, 187, and 189. In some embodiments, the antibody comprises (a) an antibody heavy chain comprising a heavy chain variable domain (VH) comprising a CDR-H1 comprising the sequence of SEQ ID NO:316, a CDR-H2 comprising the sequence of SEQ ID NO:318, and a CDR-H3 comprising the sequence of SEQ ID NO:320; and (b) an antibody light chain comprising a light chain variable domain (VL) comprising a CDR-L1 comprising the sequence of SEQ ID NO:332, a CDR-L2 comprising the sequence of SEQ ID NO:334, and a CDR-L3 comprising the sequence of SEQ ID NO:336. In some embodiments, the VH domain comprises the amino acid sequence of SEQ ID NO:314, and the VL domain comprises the amino acid sequence of SEQ ID NO:330. In some embodiments, the VH domain comprises the amino acid sequence of SEQ ID NO:346, and the VL domain comprises the amino acid sequence of SEQ ID NO:362. In some embodiments, the antibody comprises (a) an antibody heavy chain comprising a CDR-H1 comprising an amino acid sequence selected from the group consisting of SEQ ID NOs:51-55 and 81-95, a CDR-H2 comprising an amino acid sequence selected from the group consisting of SEQ ID NOs:56-60 and 96-110, and a CDR-H3 comprising an amino acid sequence selected from the group consisting of SEQ ID NOs:61-65 and 111-125; and (b) an antibody light chain comprising a light chain variable domain (VL) comprising a CDR-L1 comprising an amino acid sequence selected from the group consisting of SEQ ID NOs:66-70 and 126-140, a CDR-L2 comprising an amino acid sequence selected from the group consisting of SEQ ID NOs:38, 71-75, and 141-154, and a CDR-L3 comprising an amino acid sequence selected from the group consisting of SEQ ID NOs:76-80 and 155-169. In some embodiments, the VH domain comprises an amino acid sequence selected from the group consisting of SEQ ID NOs: 190, 192, 194, 196, 198, 200, 202, 204, 206, 208, 210, 212, 214, 216, 218, 220, 222, 224, 226, and 228; and the VL domain comprises an amino acid sequence selected from the group consisting of SEQ ID NOs: 191, 193, 195, 197, 199, 201, 203, 205, 207, 209, 211, 213, 215, 217, 219, 221, 223, 225, 227, and 229. In some embodiments, the antibody comprises an antibody heavy chain comprising a heavy chain variable domain (VH) comprising a CDR-H1 comprising the sequence of SEQ ID NO:380, a CDR-H2 comprising the sequence of SEQ ID NO:382, and a CDR-H3 comprising the sequence of SEQ ID NO:384, and an antibody light chain comprising a light chain variable domain (VL) comprising a CDR-L1 comprising the sequence of SEQ ID NO:428, a CDR-L2 comprising the sequence of SEQ ID NO:398, and a CDR-L3 comprising the sequence of SEQ ID NO:400. In some embodiments, the antibody comprises an antibody heavy chain comprising a heavy chain variable domain (VH) comprising a CDR-H1 comprising the sequence of SEQ ID NO:380, a CDR-H2 comprising the sequence of SEQ ID NO:382, and a CDR-H3 comprising the sequence of SEQ ID NO:384, and an antibody light chain comprising a light chain variable domain (VL) comprising a CDR-L1 comprising the sequence of SEQ ID NO:428, a CDR-L2 comprising the sequence of SEQ ID NO:398, and a CDR-L3 comprising the sequence of SEQ ID NO:400. In some embodiments, the antibody comprises an antibody heavy chain comprising a heavy chain variable domain (VH) comprising a CDR-H1 comprising the sequence of SEQ ID NO:444, a CDR-H2 comprising the sequence of SEQ ID NO:446, and a CDR-H3 comprising the sequence of SEQ ID NO:448, and an antibody light chain comprising a light chain variable domain (VL) comprising a CDR-L1 comprising the sequence of SEQ ID NO:460, a CDR-L2 comprising the sequence of SEQ ID NO:462, and a CDR-L3 comprising the sequence of SEQ ID NO:464. In some embodiments, the antibody comprises an antibody heavy chain comprising a heavy chain variable domain (VH) comprising a CDR-H1 comprising the sequence of SEQ ID NO:444, a CDR-H2 comprising the sequence of SEQ ID NO:478, and a CDR-H3 comprising the sequence of SEQ ID NO:448, and an antibody light chain comprising a light chain variable domain (VL) comprising a CDR-L1 comprising the sequence of SEQ ID NO:460, a CDR-L2 comprising the sequence of SEQ ID NO:462, and a CDR-L3 comprising the sequence of SEQ ID NO:464. In some embodiments, the VH domain comprises the amino acid sequence of SEQ ID NO:378, and the VL domain comprises the amino acid sequence of SEQ ID NO:394. In some embodiments, the VH domain comprises the amino acid sequence of SEQ ID NO:410, and the VL domain comprises the amino acid sequence of SEQ ID NO:426. In some embodiments, the VH domain comprises the amino acid sequence of SEQ ID NO:442, and the VL domain comprises the amino acid sequence of SEQ ID NO:458. In some embodiments, the VH domain comprises the amino acid sequence of SEQ ID NO:474, and the VL domain comprises the amino acid sequence of SEQ ID NO:490.

### Nucleic acids

Provided herein are isolated nucleic acid molecules comprising a nucleotide sequence encoding any of the multispecific (*e.g.,* bispecific) binding molecules (*e.g.,* bispecific binding proteins) of the present disclosure.

Standard recombinant DNA methodologies are used to construct the polynucleotides that encode the polypeptides which form the binding proteins, incorporate these polynucleotides into recombinant expression vectors, and introduce such vectors into host cells. *See e.g.,* Sambrook et al., 2001, MOLECULAR CLONING: A LABORATORY MANUAL (Cold Spring Harbor Laboratory Press, 3rd ed.). Enzymatic reactions and purification techniques may be performed according to manufacturer's specifications, as commonly accomplished in the art, or as described herein. Unless specific definitions are provided, the nomenclature utilized in connection with, and the laboratory procedures and techniques of, analytical chemistry, synthetic organic chemistry, and medicinal and pharmaceutical chemistry described herein are those well-known and commonly used in the art. Similarly, conventional techniques may be used for chemical syntheses, chemical analyses, pharmaceutical preparation, formulation, delivery, and treatment of patients.

Other aspects of the present disclosure relate to isolated nucleic acid molecules comprising a nucleotide sequence encoding any of the binding proteins or polypeptide chains thereof described herein. In some embodiments, the isolated nucleic acid is operably linked to a heterologous promoter to direct transcription of the binding protein-coding nucleic acid sequence. A promoter may refer to nucleic acid control sequences which direct transcription of a nucleic acid. A first nucleic acid sequence is operably linked to a second nucleic acid sequence when the first nucleic acid sequence is placed in a functional relationship with the second nucleic acid sequence. For instance, a promoter is operably linked to a coding sequence of a binding protein if the promoter affects the transcription or expression of the coding sequence. Examples of promoters may include, but are not limited to, promoters obtained from the genomes of viruses (such as polyoma virus, fowlpox virus, adenovirus (such as Adenovirus 2), bovine papilloma virus, avian sarcoma virus, cytomegalovirus, a retrovirus, hepatitis-B virus, Simian Virus 40 (SV40), and the like), from heterologous eukaryotic promoters (such as the actin promoter, an immunoglobulin promoter, from heat-shock promoters, and the like), the CAG-promoter (Niwa et al., Gene 108(2):193-9, 1991), the phosphoglycerate kinase (PGK)-promoter, a tetracycline-inducible promoter (Masui et al., Nucleic Acids Res. 33:e43, 2005), the lac system, the trp system, the tac system, the trc system, major operator and promoter regions of phage lambda, the promoter for 3-phosphoglycerate kinase, the promoters of yeast acid phosphatase, and the promoter of the yeast alpha-mating factors. Polynucleotides encoding binding proteins of the present disclosure may be under the control of a constitutive promoter, an inducible promoter, or any other suitable promoter described herein or other suitable promoter that will be readily recognized by one skilled in the art.

In some embodiments, the isolated nucleic acid is incorporated into a vector. In some embodiments, the vector is an expression vector. Expression vectors may include one or more regulatory sequences operatively linked to the polynucleotide to be expressed. The term "regulatory sequence" includes promoters, enhancers and other expression control elements (e.g., polyadenylation signals). Examples of suitable enhancers may include, but are not limited to, enhancer sequences from mammalian genes (such as globin, elastase, albumin, α-fetoprotein, insulin and the like), and enhancer sequences from a eukaryotic cell virus (such as SV40 enhancer on the late side of the replication origin (bp 100-270), the cytomegalovirus early promoter enhancer, the polyoma enhancer on the late side of the replication origin, adenovirus enhancers, and the like). Examples of suitable vectors may include, for example, plasmids, cosmids, episomes, transposons, and viral vectors (e.g., adenoviral, vaccinia viral, Sindbis-viral, measles, herpes viral, lentiviral, retroviral, adeno-associated viral vectors, etc.). Expression vectors can be used to transfect host cells, such as, for example, bacterial cells, yeast cells, insect cells, and mammalian cells. Biologically functional viral and plasmid DNA vectors capable of expression and replication in a host are known in the art, and can be used to transfect any cell of interest.

Further provided herein are vector systems comprising multiple vectors, wherein the multiple vectors collectively encode a bispecific binding protein of the present disclosure. For example, in some embodiments, a vector system comprises one or more vectors encoding a first, second, third, and fourth polypeptide chain of a bispecific binding molecule of the present disclosure. In some embodiments, a vector system comprises a first, second, third, and fourth polypeptide chain of a bispecific binding molecule of the present disclosure.

### Host cells and methods of producing binding proteins

Other aspects of the present disclosure relate to a host cell (*e.g.,* an isolated host cell) comprising one or more isolated polynucleotides, vectors, and/or vector systems described herein. In some embodiments, an isolated host cell of the present disclosure is cultured *in vitro.* In some embodiments, the host cell is a bacterial cell (*e.g.,* an *E. coli* cell). In some embodiments, the host cell is a yeast cell (*e.g.,* an *S*. *cerevisiae* cell). In some embodiments, the host cell is an insect cell. Examples of insect host cells may include, for example, *Drosophila* cells (*e.g.,* S2 cells), *Trichoplusia ni* cells (*e.g.,* High Five^{™} cells), and *Spodoptera frugiperda* cells (*e.g.,* Sf21 or Sf9 cells). In some embodiments, the host cell is a mammalian cell. Examples of mammalian host cells may include, for example, human embryonic kidney cells (*e.g.,* 293 or 293 cells subcloned for growth in suspension culture), Expi293^{™} cells, CHO cells, baby hamster kidney cells (*e.g.,* BHK, ATCC CCL 10), mouse sertoli cells (*e.g.,* TM4 cells), monkey kidney cells (*e.g.,* CV1 ATCC CCL 70), African green monkey kidney cells (*e.g.,* VERO-76, ATCC CRL-1587), human cervical carcinoma cells (*e.g.,* HELA, ATCC CCL 2), canine kidney cells (*e.g.,* MDCK, ATCC CCL 34), buffalo rat liver cells (*e.g.,* BRL 3A, ATCC CRL 1442), human lung cells (*e.g.,* W138, ATCC CCL 75), human liver cells (*e.g.,* Hep G2, HB 8065), mouse mammary tumor cells (*e.g.,* MMT 060562, ATCC CCL51), TRI cells, MRC 5 cells, FS4 cells, a human hepatoma line (*e.g.,* Hep G2), and myeloma cells (*e.g.,* NS0 and Sp2/0 cells).

Other aspects of the present disclosure relate to a method of producing any of the binding proteins described herein. In some embodiments, the method includes a) culturing a host cell (*e.g.,* any of the host cells described herein) comprising an isolated nucleic acid, vector, and/or vector system (*e.g*., any of the isolated nucleic acids, vectors, and/or vector systems described herein) under conditions such that the host cell expresses the binding molecule; and b) isolating the binding molecule from the host cell.

In some embodiments, multiple host cells can be used to produce components of a bispecific binding molecule (*e.g*., protein), which are then assembled into the bispecific binding molecule. In some embodiments, provided herein is a method of producing a bispecific binding protein comprising a first binding domain that binds an extracellular portion of dystroglycan and a second binding domain that binds laminin-2, the method comprising: a) culturing a first host cell that comprises a nucleic acid molecule encoding a first polypeptide chain comprising the first binding domain under conditions such that the host cell expresses the first polypeptide chain as part of a first monospecific binding protein with a first CH3 domain; b) culturing a second host cell that comprises a nucleic acid molecule encoding a second polypeptide chain comprising the second binding domain conditions such that the host cell expresses the second polypeptide chain as part of a second monospecific binding protein with a second CH3 domain; c) isolating the first monospecific binding protein from the first host cell; d) isolating the second monospecific binding protein from the second host cell; e) incubating the isolated first and second monospecific binding proteins under reducing conditions sufficient to allow cysteines in the hinge region to undergo disulfide bond isomerization; and f) obtaining the bispecific binding protein, wherein the first and second CH3 domains are different and are such that the heterodimeric interaction between said first and second CH3 domains is stronger than each of the homodimeric interactions of said first and second CH3 domains. For greater description, see, *e.g.,* US PG Pub. No. US2013/0039913 and Labrijn, A.F. et al. (2013) Proc. Natl. Acad. Sci. 110:5145-5150.

Methods of culturing host cells under conditions to express a protein are well known to one of ordinary skill in the art. Methods of isolating proteins from cultured host cells are well known to one of ordinary skill in the art, including, for example, by affinity chromatography (*e.g.,* two step affinity chromatography comprising protein A affinity chromatography followed by size exclusion chromatography).

### Use for Binding Proteins

Further provided herein is a bispecific binding molecule of the present disclosure for use in methods for treating or preventing an alpha-dystroglycanopathy in an individualIn some embodiments, the individual is a human.

Further provided herein is a multispecific binding molecule of the present disclosure for use in methods for treating or preventing an alpha-dystroglycanopathy in an individual, the method comprising administering to the individual a multispecific binding molecule of the present disclosure. In some embodiments, the individual is a human.

**In** some embodiments, the individual has reduced expression of alpha-dystroglycan (*e.g.,* as compared to expression in a control individual, or one lacking a genetic mutation described herein). In some embodiments, expression refers to expression in one or more tissues, *e.g*., muscle tissue.

**In** some embodiments, alpha-dystroglycan expressed in the individual has impaired or aberrant O-glycosylation (*e.g*., as compared to expression in a control individual, or one lacking a genetic mutation described herein).

**In** some embodiments, the individual has, has been diagnosed with, or has a propensity for developing an alpha-dystroglycanopathy. In some embodiments, the individual has a mutation in a gene selected from the group consisting of: dystroglycan (DAG1), protein O-mannosyltransferase-1 (POMT1), protein O-mannosyltransferase-2 (POMT2), protein O-linked mannose beta1,2-N-acetylglucosylaminyltransferase subunit 1 (POMGNT1), protein O-linked mannose betal,4-N-acetylglucosylaminyltransferase subunit 2 (POMGNT2), xylosyl- and glucuronyltransferase 1 (LARGE1), xylosyl- and glucuronyltransferase 2 (LARGE2), dolichyl-phosphate mannosyltransferase subunit 1 (DPM1), dolichyl-phosphate mannosyltransferase subunit 2 (DPM2), dolichyl-phosphate mannosyltransferase subunit 3 (DPM3), fukutin, fukutin related protein (FKRP), isprenoid synthase domain containing (ISPD), protein O-mannose kinase (POMK), beta-1,3-N-acetylgalactosaminyltransferase 2 (B3GALNT2), beta-1,4-glucuronyltransferase 1 (B4GAT1), dolichol kinase (DOLK), transmembrane protein 5 (TMEM5), and GDP-mannose pyrophosphorylase B (GMPPB).

In some embodiments, a bispecific binding molecule of the present disclosure is administered by intravenous infusion, intramuscular injection, intraperitoneal injection, or subcutaneous injection.

The binding proteins can be employed in any known assay method, such as competitive binding assays, direct and indirect sandwich assays, and immunoprecipitation assays for the detection and quantitation of one or more target antigens. The binding proteins will bind the one or more target antigens with an affinity that is appropriate for the assay method being employed.

Also provided herein are pharmaceutical compositions comprising a bispecific binding molecule of the present disclosure and an optional pharmaceutically acceptable carrier.

Also provided herein are pharmaceutical compositions comprising a multispecific binding molecule of the present disclosure and an optional pharmaceutically acceptable carrier.

The pharmaceutical composition can contain formulation materials for modifying, maintaining, or preserving, for example, the pH, osmolarity, viscosity, clarity, color, isotonicity, odor, sterility, stability, rate of dissolution or release, adsorption, or penetration of the composition. Suitable formulation materials include, but are not limited to, amino acids (such as glycine, glutamine, asparagine, arginine, or lysine), antimicrobials, antioxidants (such as ascorbic acid, sodium sulfite, or sodium hydrogen-sulfite), buffers (such as borate, bicarbonate, Tris-HCl, citrates, phosphates, or other organic acids), bulking agents (such as mannitol or glycine), chelating agents (such as ethylenediamine tetraacetic acid (EDTA)), complexing agents (such as caffeine, polyvinylpyrrolidone, beta-cyclodextrin, or hydroxypropyl-beta-cyclodextrin), fillers, monosaccharides, disaccharides, and other carbohydrates (such as glucose, mannose, or dextrins), proteins (such as serum albumin, gelatin, or immunoglobulins), coloring, flavoring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as polysorbate 20 or polysorbate 80; triton; tromethamine; lecithin; cholesterol or tyloxapal), stability enhancing agents (such as sucrose or sorbitol), tonicity enhancing agents (such as alkali metal halides - *e.g*., sodium or potassium chloride - or mannitol sorbitol), delivery vehicles, diluents, excipients and/or pharmaceutical adjuvants (*see, e.g.,* REMINGTON'S PHARMACEUTICAL SCIENCES (18th Ed., A.R. Gennaro, ed., Mack Publishing Company 1990), and subsequent editions of the sameAcceptable formulation materials are nontoxic to recipients at the dosages and concentrations employed.

The optimal pharmaceutical composition will be determined by a skilled artisan depending upon, for example, the intended route of administration, delivery format, and desired dosage. Such compositions can influence the physical state, stability, rate of *in vivo* release, and rate of *in vivo* clearance of the binding protein.

The primary vehicle or carrier in a pharmaceutical composition can be either aqueous or non-aqueous in nature. For example, a suitable vehicle or carrier for injection can be water, physiological saline solution, or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffer of about pH 7.0-8.5, or acetate buffer of about pH 4.0-5.5, which can further include sorbitol or a suitable substitute. In one embodiment of the disclosure, binding protein compositions can be prepared for storage by mixing the selected composition having the desired degree of purity with optional formulation agents in the form of a lyophilized cake or an aqueous solution. Further, the binding protein can be formulated as a lyophilizate using appropriate excipients such as sucrose.

The formulation components are present in concentrations that are acceptable to the site of administration. For example, buffers are used to maintain the composition at physiological pH or at a slightly lower pH, typically within a pH range of from about 5 to about 8.

When parenteral administration is contemplated, the therapeutic compositions for use can be in the form of a pyrogen-free, parenterally acceptable, aqueous solution comprising the desired binding protein in a pharmaceutically acceptable vehicle. A particularly suitable vehicle for parenteral injection is sterile distilled water in which a binding protein is formulated as a sterile, isotonic solution, properly preserved. Yet another preparation can involve the formulation of the desired molecule with an agent, such as injectable microspheres, bio-erodible particles, polymeric compounds (such as polylactic acid or polyglycolic acid), beads, or liposomes, that provides for the controlled or sustained release of the product which can then be delivered via a depot injection. Hyaluronic acid can also be used, and this can have the effect of promoting sustained duration in the circulation. Other suitable means for the introduction of the desired molecule include implantable drug delivery devices.

It is also contemplated that certain formulations can be administered orally. In one embodiment of the disclosure, binding proteins that are administered in this fashion can be formulated with or without those carriers customarily used in the compounding of solid dosage forms such as tablets and capsules. For example, a capsule can be designed to release the active portion of the formulation at the point in the gastrointestinal tract where bioavailability is maximized and pre-systemic degradation is minimized. Additional agents can be included to facilitate absorption of the binding protein. Diluents, flavorings, low melting point waxes, vegetable oils, lubricants, suspending agents, tablet disintegrating agents, and binders can also be employed.

Another pharmaceutical composition can involve an effective quantity of binding proteins in a mixture with non-toxic excipients that are suitable for the manufacture of tablets. By dissolving the tablets in sterile water, or another appropriate vehicle, solutions can be prepared in unit-dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional pharmaceutical compositions of the disclosure will be evident to those skilled in the art, including formulations involving binding proteins in sustained- or controlled-delivery formulations. Techniques for formulating a variety of other sustained- or controlled-delivery means, such as liposome carriers, bio-erodible microparticles or porous beads and depot injections, are also known to those skilled in the art. Additional examples of sustained-release preparations include semipermeable polymer matrices in the form of shaped articles, *e.g.* films, or microcapsules. Sustained release matrices can include polyesters, hydrogels, polylactides, copolymers of L-glutamic acid and gamma ethyl-L-glutamate, poly(2-hydroxyethylmethacrylate), ethylene vinyl acetate, or poly-D(-)-3-hydroxybutyric acid. Sustained-release compositions can also include liposomes, which can be prepared by any of several methods known in the art.

Pharmaceutical compositions to be used for *in vivo* administration typically must be sterile. This can be accomplished by filtration through sterile filtration membranes. Where the composition is lyophilized, sterilization using this method can be conducted either prior to, or following, lyophilization and reconstitution. The composition for parenteral administration can be stored in lyophilized form or in a solution. In addition, parenteral compositions generally are placed into a container having a sterile access port, for example, an intravenous solution bag or vial having a stopper pierceable by a hypodermic injection needle.

Once the pharmaceutical composition has been formulated, it can be stored in sterile vials as a solution, suspension, gel, emulsion, solid, or as a dehydrated or lyophilized powder. Such formulations can be stored either in a ready-to-use form or in a form (*e.g*., lyophilized) requiring reconstitution prior to administration.

The effective amount of a binding protein pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the binding protein is being used, the route of administration, and the size (body weight, body surface, or organ size) and condition (the age and general health) of the patient. Accordingly, the clinician can titer the dosage and modify the route of administration to obtain the optimal therapeutic effect.

Dosing frequency will depend upon the pharmacokinetic parameters of the binding protein in the formulation being used. Typically, a clinician will administer the composition until a dosage is reached that achieves the desired effect. The composition can therefore be administered as a single dose, as two or more doses (which may or may not contain the same amount of the desired molecule) over time, or as a continuous infusion via an implantation device or catheter. Further refinement of the appropriate dosage is routinely made by those of ordinary skill in the art and is within the ambit of tasks routinely performed by them. Appropriate dosages can be ascertained through use of appropriate dose-response data.

### EXAMPLES

The present disclosure will be more fully understood by reference to the following examples.

### Example 1: Identification of anti-beta-DG ECD, anti-LG-5, and anti-LG-4/5 antibodies.

### Methods

### Protein expression

To express murine beta-DG extracellular domain (mbeta-DG ECD), a construct was generated that contained an *E. coli* codon-optimized cassette encoding an N-terminal maltose binding protein, TEV cleavage site, mbeta-DG (UniProt Q62165, amino acids 652-746), and a C-terminal HPC4 tag, with pET22b as the parent vector backbone. The construct was transformed into chemically competent Origami B (DE3) pLysS cells (Novagen). Expression was performed at 37 °C, with ITPG induction at OD = 0.6. Cells were pelleted and resuspended in lysis buffer containing EDTA-free protease inhibitors (Roche) and lysed by sonication. mbeta-DG-HPC4 was purified from clarified cell lysate by processing cell lysate over an amylose resin column (New England Biolabs), cleaving off the maltose binding protein with Turbo TEV protease (Eton Biosciences), processing the digest over an amylose resin and His-Trap FastFlow column (GE Healthcare) to remove undigested fusion protein and cleaved maltose binding protein, and processing the flow through over NHS-activated Sepharose 4 FastFlow resin (GE Healthcare) coupled with mouse anti-HPC4 antibody. Further purification was carried out on a Superdex 75 size exclusion column (GE Healthcare), and eluate fractions with highly purified mbeta-DG-HPC4 (as determined by running fraction samples on an SDS-PAGE gel and coomassie staining) were collected and pooled.

To express murine or laminin G-like 5 domain (mLG-5 or hLG-5) constructs were generated that contained a mammalian codon-optimized cassette encoding N-terminal Avi and HPC4 tags, and either mLG-5 (*see* UniProt Q60675, amino acids 2932-3118; SEQ ID NO:292) or hLG-5 (UniProt P24043, amino acids 2936-3122; SEQ ID NO:293). The construct was used to transfect Expi293F cells using Expifectamine reagent (Thermo Fisher). After 7 days expression, soluble biotinylated protein was purified from the supernatant with NHS-activated Sepharose 4 FastFlow resin (GE Healthcare) coupled with mouse anti-HPC4 antibody.

To express murine or human laminin G-like 4 and 5 domains (mLG-4/5 or hLG-4/5), constructs were generated that contained a mammalian codon-optimized cassette encoding an N-terminal mIgG2a fusion partner, TEV cleavage site, an Avi tag, an HPC4 tag, and either mLG-4/5 (UniProt Q60675, amino acids 2725-3118; SEQ ID NO:292) or hLG-4/5 (UniProt P24043, amino acids 2729-3122; SEQ ID NO:293). The constructs were used to transfect Expi293F cells using Expifectamine reagent (Thermo Fisher). After 7 days expression, soluble protein was purified from the supernatant with a HiTrap MabSelect SuRe column (GE Healthcare). mIgG2a was cleaved off the mLG-4/5 protein using Turbo TEV protease (Nacalai USA), and the digest was processed over MabSelect SuRe resin (GE Healthcare) and Ni-NTA resin (Qiagen) to remove mIgG2a and TEV protease from purified mLG-4/5.

### Phage display

Purified mbeta-DG, mLG-5, or mLG-4/5 and hLG-4/5 (*e.g*., alternating between using mouse and human peptides) was coupled to magnetic tosyl-activated beads (Invitrogen) and used to enrich phage display libraries for mbeta-DG, mLG-5, or mLG-4/5 binders. Antibody phage display libraries were used in mbeta-DG selections, and the Dyax FAB 310 antibody phage display library was used for hLG-4/5 and mLG-4/5 selections. Libraries were first depleted of non-specific binders using uncoated beads and an HPC4-6xHis-Avi tagged unrelated protein. Three rounds of selection were then performed on the depleted libraries, using diminishing concentrations of antigen at each round (500 nM antigen at round 1, to 1 nM antigen at round 3). The enriched libraries were plated, individual library clones were picked and cultured in a 96-well format, and phage monoclonal antibodies were produced for each clone for phage ELISA binding assay.

### Phage ELISA binding assay

Purified antigen (mbeta-DG, mLG-5, hLG-5, mLG-4/5, or hLG-4/5) was coated on Nunc MaxiSorp 96-well ELISA plates (Thermo Scientific) at lug/ml. Phage monoclonal antibodies from the selected library clones were added to each well and positive or negative binding was detected using anti-M13 Europium labelled secondary (GE Healthcare, antibody custom labelled by Perkin Elmer).

### Variable region sequencing

Bacterial stocks of positive binding clones were PCR amplified and sequenced, and unique variable heavy chain (VH) and variable light chain (VL) sequences were identified.

### Results

Several phage library clones with specific binding affinity for beta-DG, LG-5, and LG-4/5 were identified: 10 clones specifically bound beta-DG, and 15 clones specifically bound LG-4/5. Sequencing of these clones revealed that variable heavy and variable light regions of each clone were distinct, as shown in Tables D through I *supra* (*see, e.g.,* clones B04, B06, CL-40968, CL-40992, CL-41136, CL-41400, and CL-41500). Complementarity-determining regions (CDRs) of these clones are identified in Tables A through C *supra.*

### Example 2: Generation of hybridomas, monoclonal antibodies, and chimeric antibodies targeted against beta-DG, LG-5, and LG-4/5.

### Methods

### Cell line production

Stable cell lines with either human or murine beta-DG surface expression were created by codon optimizing constructs containing an N-terminal myc tag and the extracellular and endogenous transmembrane domains of beta-DG (mouse UniProt Q62165, amino acids 652-893; human UniProt Q14118, amino acids 654-895). Adherent human embryonic kidney cells (HEK) and adherent Chinese hamster ovarian cells (CHO-K1) were transfected using lipofectamine (Thermo Fisher) and cells were selected with Geneticin (Gibco). Surviving cells were serial diluted for single cell clonality and surface expression of beta-DG was confirmed by anti-myc flow cytometry.

Stable cell lines with either human or murine LG-5 surface expression were created by codon optimizing constructs containing a N-terminal myc tag, a Gly/Ser linker, LG-5 (mouse UniProt Q60675, amino acids 2932-3118; human UniProt P24043, amino acids 2936-3122), and a Tfrl transmembrane domain for mammalian expression. Adherent Chinese hamster ovarian cells (CHO-K1) were transfected using lipofectamine (Thermo Fisher) and cells were selected with Geneticin (Gibco). Surviving cells were serial diluted for single cell clonality and surface expression of beta-DG was confirmed by anti-myc flow cytometry.

Stable cell lines with either human or murine LG-4/5 surface expression were created by codon optimizing constructs containing a N-terminal myc tag, a Gly/Ser linker, LG-4/5 (mouse UniProt Q60675, amino acids 2725-3118; human UniProt P24043, amino acids 2729-3122), and a Tfrl transmembrane domain for mammalian expression. Adherent human embryonic kidney cells (HEK) were transfected using lipofectamine (Thermo Fisher) and cells were selected with Geneticin (Gibco). Surviving cells were serial diluted for single cell clonality and surface expression of beta-DG was confirmed by anti-myc flow cytometry.

### Mouse immunization

Balb/c and Trianni mice were immunized with hbeta-DG, hLG-5, or hLG-4/5, then boosted with these proteins 3-4 times every two weeks. For mice immunized with hLG-4/5, mice were additionally boosted 3 times with human merosin every 2 weeks and once with a synthetic peptide that has identical sequence between human and mouse LG-5 (amino acid sequence =GFAKAVGGFKVGLDLLVEFE; SEQ ID NO:295).

### Hybridoma generation

Hybridoma cells were made by fusing mouse myeloma cells (from a Balb/c B-lymphoblast cell line, SP2/0, fused with Sendai virus) that are deficient in adenosine phosphoribosyltransferase (APRT) with spleen cells from the immunized mice. HAT selection (hypoxanthine, azaserine, and thymidine) and serial dilutions were performed to achieve single cell clonality.

### ELISA antibody binding assay

For ELISA assays, plates coated in either human beta-DG or LG-4/5 were blocked with 5% fetal bovine serum in PBS, and each well was incubated with a distinct culture supernatant. Plates were washed with PBS, incubated with HRP conjugated anti-mouse Fc secondary antibody, washed again with PBS, and developed for colorimetric measuring.

### Fluorescence activated cell sorting (FACS) antibody binding assay

For FACS assays, stable cells with either human or murine beta-DG or LG-4/5 surface expression (see above) were incubated with antibody-containing culture supernatant, washed with PBS, incubated with FITC-conjugated anti-mouse Fc secondary antibody (Thermo Fisher), washed again with PBS, and analyzed on a flow cytometer.

### Surface plasmon resonance (Biacore) kinetics assay

Hybridoma antibodies (contained in culture supernatant)t were further characterized by measuring antibody/antigen binding affinity and on/off-rate by Biacore kinetics assay, as per manufacturer's protocol (GE Healthcare). Antigens used for binding were human or murine beta-DG or LG-4/5.

### Monoclonal antibody generation

Hybridoma clones were expanded and terminal flasks with ultra-low IgG fetal bovine serum supplement were seeded. After 7 days, supernatant was harvested and monoclonal antibodies were purified using a HiTrap MabSelect SuRe column (GE Healthcare). Resulting antibodies were tested again by ELISA, FACS, and Biacore kinetics assay (GE Healthcare) to confirm antibody binding properties.

### Immunofluorescence

Immunofluorescence staining with unfixed frozen human and mouse muscle tissue sections was performed. Muscle tissue sections were stained with purified antibodies against beta-DG or LG-4/5, washed, stained with fluorescently labeled anti-mouse IgG secondary antibody, washed, mounted, and imaged using a fluorescence microscope.

### Variable region sequencing

Total RNA was isolated from hybridoma cells that produced high affinity antibodies using the RNeasy Mini Kit (Qiagen) and first-strand cDNA was synthesized using the SMARTer RACE cDNA Amplification Kit (Clontech). The VH and VL gene segments were amplified by 5'-Rapid Amplification of cDNA Ends (5'-RACE) PCR using isotype specific primers. Amplified PCR fragments were cloned and sequenced. *See, e.g.,* clones TDG-2, TDI-11, TDI-23, TDI-38, TLF39, TLF86, TLG3/TLG4, TLG26, TLI-3, TLI-7, TTLK71-4-6, TTLK123-3, TTLK145-6-3, TTLK170-2, WJL10, and WJL48.

### Chimeric antibody production

VH and VL sequences generated from 5'-RACE PCR were codon optimized for mammalian expression and synthesized. VH sequences were subcloned into a mammalian expression vector with human IgG1 and VL sequences were subcloned into a mammalian expression vector with the constant human kappa chain. Expi293F cells were co-transfected with these constructs using Expifectamine reagent (Thermo Fisher) to express chimeric antibodies. After 7 days expression, antibodies were purified from the supernatant with a HiTrap MabSelect SuRe column (GE). Purified antibodies were rescreened by ELISA, FACS, and Biacore (GE Healthcare) to confirm binding affinity to beta-DG, LG-5, or LG-4/5. To confirm that antibodies bound to their respective antigens in muscle tissue, immunofluorescence staining with unfixed frozen human and mouse muscle tissue sections was performed.

### Results

To screen for and select hybridomas that produced antibodies specific to beta-DG, LG-5, or LG-4/5, ELISA, FACS analysis, and Biacore kinetics assay were used to assess antibody binding. ELISA assays showed a range of binding affinities of antibodies to beta-DG, LG-5, or LG-4/5, with several samples giving strong colorimetric signal (exemplary data for three antibodies are provided in Table J below).

**Table J. Monoclonal antibody anti-LG-5 binding kinetics**

| **Clone name** | **Immobilized mAb with hLG-5 in flow** | | | **Immobilized mAb with mLG-5 in flow** | | |
|---|---|---|---|---|---|---|
| | **ka(1/Ms)** | **kd (1/s)** | **KD(M)** | **ka(1/Ms)** | **kd (1/s)** | **KD(M)** |
| AN01 | 9.90E+04 | 9.15E-04 | 9.24E-09 | nb | nb | nb |
| C3 | 4.88E+05 | 1.30E-03 | 2.66E-09 | 5.19E+05 | 4.29E-03 | 8.27E-09 |
| C21 | 4.67E+05 | 1.31E-03 | 2.80E-09 | 8.13E+05 | 2.53E-03 | 3.04E-09 |

| | | | | | | |
|---|---|---|---|---|---|---|
| nb: no binding. | | | | | | |

Samples giving a strong colorimetric signal were assayed using FACS for binding affinity to cells expressing beta-DG or LG-4/5 on their surface. FACS analysis revealed that antibodies derived from clones C21 and C3 had binding affinity for both murine and human LG-4/5 (**FIGS. 3C** **&** **3G****,** respectively). These antibodies did not bind control cells that lacked surface expression of beta-DG or LG-4/5, as shown by insignificant fluorescence detection.

Various amounts of recombinant human laminin-2 (from Biolamina), murine LG-5, human LG-5, human LG4/5 were dot blotted onto nitrocellulose membrane and probed with anti-laminin-2 antibody. Results indicated that the antibodies recognized Laminin-2 or its fragments containing LG-5 (**FIG. 3D****,** top for C21). To determine the antibody specificity, different laminin isoforms with different alpha chains were dotted onto the blot. Only mLG-5 and human laminin-2 containing alpha-2 were recognized, supporting the antibodies' binding specificity (**FIG. 3D****,** bottom for C21; **FIG. 3H** for C3).

Anti-dystroglycan antibody clones were also characterized. Kinetics revealed that all antibodies tested displayed high affinity to their respective antigens, with most KDs in the 10⁻⁹ M range (nanomolar sensitivity), as shown in Table K and **FIGS. 3I & 3J** (clone AS30) and **3M & 3N** (clone AS19). In addition, on and off-rates for tested antibodies were fairly typical for high affinity antibodies, with the exception of anti-beta-DG clone AS19, which had very high on and off rates (Table K).

**Table K. Monoclonal antibody anti-beta-DG binding kinetics.**

| **Clone name** | **Immobilized mAb with hBeta-DG in flow** | | | **Immobilized mAb with mBeta-DG in flow** | | |
|---|---|---|---|---|---|---|
| | **ka(1/Ms)** | **kd (1/s)** | **KD(M)** | **ka(1/Ms)** | **kd (1/s)** | **KD(M)** |
| B06 | 9.68E+05 | 2.16E-03 | 2.36E-09 | 1.43E+04 | 2.32E-03 | 1.62E-07 |
| B04 | 6.20E+04 | 1.60E+03 | 2.57E-09 | 6.20E+04 | 1.60E-03 | 2.57E-08 |
| AS30 | 8.90E+05 | 9.63E-04 | 1.08E-09 | 7.73E+05 | 1.13E-03 | 1.48E-09 |
| AS19 | 4.43E+09 | 1.04E+01 | 2.06E-09 | 2.02E+09 | 9.33E+00 | 3.38E-09 |
| AS55 | 1.47E+05 | 7.27E-04 | 5.00E-09 | 3.04E+05 | 1.36E-03 | 4.47E-09 |

To characterize clones AS30 and AS19, various amount of recombinant mouse or human beta-DG ECD, recombinant dystroglycan (from R&D Systems), C2C12 cell lysate, TA lysate and Fabrazyme as negative control were dot blotted onto nitrocellulose membrane and probed with anti-beta-DG antibody (**FIG. 3K** for AS30; **FIG. 3O** for AS19). Results indicate that all proteins containing beta-DG were detected. No signal was detected with C2C12 or TA lysate, probably due to very low amount of beta-DG in these samples. As expected, antibodies also did not detect negative control Fabrazyme.

Immunoprecipitation of beta-DG from C2C12 cell lysates solubilized under non-denaturing condition was performed with anti-beta-DG clone AS30 or AS19. The beta-DG/antibody complex was captured by protein A beads and run on SDS-PAGE, then reprobed with anti-alpha-DG and anti-beta-DG from R&D Systems. Both alpha-DG and beta-DG were immunoprecipitated, indicating they remain in complex after solubilization, and binding of anti-beta-DG antibody clones did not interfere the binding of alpha-DG to beta-DG (**FIG. 3L** for AS30; **FIG. 3P** for AS19).

After hybridoma clones were expanded and monoclonal antibodies purified, antibodies were rescreened by ELISA, FACS, and Biacore to confirm binding affinity. Results were extremely similar to those generated for antibodies from culture supernatant, confirming that the antibodies retained their kinetic characteristics after amplification.

To determine if antibodies could bind to muscle tissue, which contains abundant beta-DG and LG-4/5, immunofluorescence staining was done on mouse and human muscle tissue using purified antibodies. Unfixed tissue was used such that the native antigen conformation was preserved. Characteristic muscle sarcolemma staining was clearly demonstrated for human and mouse tissues, indicating specific LG-4/5 binding for C21 (**FIG. 4A**) and C3 (**FIG. 4B**), and specific beta-DG binding for AS30 (**FIG. 4C**) and AS19 (**FIG. 4D**). Sections stained with only secondary antibody did not reveal any fluorescent signal.

### Example 3: Generation of bispecific antibodies recognizing beta-DG and the LG-4/5 domain of the laminin-2 alpha subunit

### Methods

### Tetravalent bispecific tandem Ig (TBTI) antibody generation

VH and VL sequences obtained from generated hybridoma cells were codon-optimized for mammalian expression and synthesized (Genscript). To generate constructs expressing the light chains, one VL sequence specific to beta-DG, a (G4S)₂ linker, one VL sequence specific to LG-4/5, and human kappa chain (Genbank Q502W4) or murine kappa chain (Genbank BAB33404) were fused together and cloned into the transient episomal expression vector pXL, an analogue of the pTT vector described by Durocher et al. (Nucl. Acids Res. 2002, 30(2), E9). To generate constructs expressing the heavy chains, one VH sequence specific to beta-DG, a (G4S)₂ linker, one VH sequence specific to LG-4/5, and human IgG1 (Genbank Q569F4) or murine IgG1 (GenBank AAA75163.1) were fused together (Fig. 4A) and cloned into expression vector pXL. VH and VL sequences used were obtained from clones AN01, C3, and C21 for LG-4/5-specific binding, and from clone B6, AS19, and AS30 for beta-DG-specific binding.

These constructs were co-transfected into HEK293 FreeStyle 293-F or Expi293 cells (Thermo Fisher). After 7 days expression, antibodies were purified from the supernatant with a HiTrap MabSelect^{™} SuRe^{™} Protein A column (GE Healthcare).

### Crossover dual variable domain Ig (CODVIg) antibody generation

VH and VL sequences obtained from generated hybridoma cells were codon-optimized for mammalian expression and synthesized (Genscript). To generate constructs expressing the light chains, one VL sequence specific to LG-4/5, an L₁ linker, one VL sequence specific to beta-DG, a L₂ linker, and human kappa chain (Genbank Q502W4) or murine kappa chain (Genbank BAB33404) were fused together and cloned into expression vector pXL. To generate constructs expressing the heavy chains, one VH sequence specific to beta-DG, a L₃ linker, one VH sequence specific to LG-4/5, a L₄ linker, and human IgG1 (Genbank Q569F4) or murine IgG1 (GenBank AAA75163.1) were fused together and cloned into expression vector pXL. Specific combinations of linker sequences used are provided below.

These constructs were co-transfected into HEK293 FreeStyle 293-F or Expi293 cells (Thermo Fisher). After 7 days expression, antibodies were purified from the supernatant with a HiTrap MabSelect^{™} SuRe^{™} Protein A column (GE Healthcare).

### Sequential Biacore binding analysis

Parental monoclonal antibodies (AS19, C3 and C21) and three bispecific antibodies (AS19 x C3 and AS30 x C3 in TBTI, and AS30 x C3 in CODVIg) were each immobilized onto individual CM5 Series S Biacore chips (GE Healthcare). Human or murine LG-4/5, followed in sequence by human or murine beta-DG, was flown over each chip and binding was assessed.

### Double deck sandwich ELISA

96-well plates were coated with 50 ng human LG-4/5 and blocked with 5% fetal bovine serum in PBS. Each well was incubated with 1 µg of the generated bispecific antibodies (murine IgG backbone). After 2 hours, wells were washed with PBS and re-incubated with 16 ng to 1 µg per well of human beta-DG fused to the human hIgG1 Fc antibody region (hbeta-DG-hFc). After 2 hours, wells were washed with PBS, incubated with a HRP conjugated anti-hFc secondary antibody for 45 minutes, washed again with PBS, and developed for colorimetric measuring.

### Results

Antibodies were engineered into multiple bispecific formats including tetravalent bispecific tandem IgG format (TBTI; **FIG. 5A**) as well as crossover dual variable domain IgG format (CODVIg; **FIG. 5B**) as described above. To create these formats a light chain and a heavy chain plasmid for each construct was synthesized. One with a variable light chain region of an anti-βDG from the listed monoclonal sequences followed by a linker and then a variable

light chain region from an anti-LG4/5 monoclonal followed by an additional linker and then the constant light chain domain. The same principle was used to develop the heavy chain plasmids and then for expression these were co-transfected in mammalian cells. Multiple linker combinations were attempted and both variable region orientations were tested (i.e. having the anti-LG4/5 farther from the constant region rather than anti-βDG variable region and vice versa).

Bispecific antibodies (biAbs) recognizing beta-DG (using clones B06, AS19, and AS30) and LG-4/5 (using clones AN01, C3, and C21) were generated in TBTI or CODVIg format.

Multiple linker combinations were attempted and both variable region orientations were tested (i.e. having the anti-LG4/5 farther from the constant region rather than anti-βDG variable region and vice versa). For TBTI, (T1T2 and T5T6), the linker between the light chain variable regions consisted of 10 residues that were glycine or serine (*e.g*., GGGGSGGGGS; SEQ ID NO:294) and no linker was used between the second variable region and the constant region. The same linker (10 residues that were glycine or serine) was used between the heavy chain variable regions and no linker was used between the second heavy chain variable and the constant. For CODVIg format, two sets of linker lengths were used: 10-10-0-0 and 7-5-1-2 (# of residues for L₁-L₂-L₃-L₄). CODVIg C5C6 linkers consisted of 10 residues that were glycine or serine between variable light chains and 10 residues that were glycine or serine between the second variable region and the light constant region. No linkers were used on the heavy chain. Linker sequences for these combinations are as follows (depicted as L₁, L₂, L₃, L₄): GQPKAAP (SEQ ID NO:297), TKGPS (SEQ ID NO:298), S, RT; GGSGSSGSGG (SEQ ID NO:299), GGSGSSGSGG (SEQ ID NO:299), 0,0; and EPKSDKTHTSPPSP (SEQ ID NO:296), GG, EPKSDKTHTSPPSP (SEQ ID NO:296), GG. A list of bispecific antibodies created is provided in Table L below.

**Table L. CODY and TBTI bispecific antibody configurations tested.**

| **CODV with 10-10-0-0 linker** | **CODV with 7-5-1-2 linker** | **TBTI with (GGGGS)2 Linker** |
|---|---|---|
| | | |
| | anti-laminin-2 (AN01) x anti-beta-DG(clone B04) | anti-laminin-2(AN01) x anti-beta-DG(clone B04) |
| | anti-beta-DG(clone B04) x anti-laminin-2(AN01) | anti-beta-DG(clone B04) x anti-laminin-2(AN01) |
| | anti-laminin-2(AN01) x anti-beta-DG(clone B06) | anti-laminin-2(AN01) x anti-beta-DG(clone B06) |
| | ***anti-beta-DG(clone B06) x anti-laminin-2(AN01) =1331 and 1460** | anti-beta-DG(clone B06) x anti-laminin-2(AN01) |
| | | |
| anti-beta-DG(AS19) x anti-laminin-2(C3) | anti-beta-OG(AS19) x anti-laminin-2(C3) | ***anti-beta-DG(AS19) x anti-laminin-2(C3) =T1T2** |
| anti-laminin-2(C03) x anti-beta-DG(clone AS19) | anti-laminin-2(C03) x anti-beta-DG(clone AS19) | anti-laminin-2(C03) x anti-beta-DG(clone AS19) |
| anti-beta-DG(clone AS19) x anti-laminin-2(C3) | anti-beta-DG(clone AS19) x anti-laminin-2(C3) | anti-beta-DG(clone AS19) x anti-laminin-2(C3) |
| anti-laminin-2(C3) x anti-beta-DG(clone AS19) | anti-laminin-2(C3) x anti-beta-DG(clone AS19) | anti-laminin-2(C3) x anti-beta-DG(clone AS19) |
| | | |
| **anti-beta-DG(AS30) x anti-laminin-2(C3) = C5C6** | anti-beta-DG(AS30) x anti-laminin-2(C3) | **anti-beta-DG(AS30) x anti-laminin-2(C3) = T5T6** |
| anti-laminin-2(C03) x anti-beta-DG(clone AS30) | anti-laminin-2(C03) x anti-beta-DG(clone AS30) | anti-laminin-2(C03) x anti-beta-DG(clone AS30) |
| anti-beta-DG(clone AS30) x anti-laminin-2(C21) | anti-beta-DG(clone AS30) x anti-laminin-2(C21) | anti-beta-DG(clone AS30) x anti-laminin-2(C21) |
| anti-laminin-2(C21) x anti-beta-DG(clone AS30) | anti-laminin-2(C21) x anti-beta-DG(clone AS30) | anti-laminin-2(C21) x anti-beta-DG(clone AS30) |

To confirm that biAbs have the capacity to bind LG-4/5 and beta-DG at the same time, sequential Biacore analysis (GE Healthcare) and double deck Sandwich ELISA assays were performed.

Parental mAbs to LG-4/5 or beta-DG and biAbs of anti-LG4/5 and beta-DG were captured onto biacore chips, and then flowed with human LG4/5 and human beta-DG sequentially to determine their concurrent bindings to both antigens. Sequential Biacore analysis revealed that bispecific antibodies can bind either human (1^{st} peak, **FIG. 6A**) or murine LG-4/5 (1^{st} peak, **FIG. 6B**) first, and then further associate with human (2^{nd} peak, **FIG. 6A**) or murine beta-DG (2^{nd} peak, **FIG. 6B**)**.** In contrast, parental monoclonal antibodies were only able to bind to one target, either LG-4/5 (C3 and C21 in **FIGS. 6A** **&** **6B**) or beta-DG (AS19 in **FIGS. 6A** **&** 6B).

Double deck Sandwich ELISA revealed that bispecific antibodies can simultaneously bind hLG-4/5 and hbeta-DG. Colorimetric signals could be detected only when hLG-4/5 and hbeta-DG-hFc were both added in the assay (**FIG. 7**)**.** No signal was detected when parental monoclonal antibodies were used or when hbeta-DG-hFc was omitted. Three bispecific antibodies (biAbs), T1T2, T5T6, and C5C6, had the anticipated signal indicating simultaneous binding to both targets. The parental anti-LG4/5 or anti-beta-DG mAbs, or biAbs without beta-DG-hFc in the 2^{nd} step were all negative in this assay.

### Example 4: Intra-muscular injection of bi-specific antibodies into LARGE^{myd-3J/GrsrJ} mice.

### Methods

### LARGE^{myd-3J/}^{GrsrJ} mouse model

LARGE^{myd-3J/GrsrJ} (stock #008581) mice from Jackson lab is a mouse model of alpha-dystroglycanopathy caused by a mutation in the LARGE gene. The mutation of the LARGE gene maps between D8Mit65 and DMit249, with markers at 44.4 Mb and 83.8 Mb, respectively; the LARGE gene is located at 75.7 Mb. Mice homozygous for the LARGE generally begin to display evidence of muscle degeneration at two to three months of age, although some animals may exhibit symptoms as early as wean age. Inability to splay the hind legs outward when held up by the tail is an initial phenotype and this progresses with age to include swaying gait, then dragging of the hind legs.

### Bispecific antibody injection

A group of 10 LARGE^{myd-3J/GrsrJ} mice were given intramuscular injections into the left and right tibialis anterior (TA) muscles. The left TA received two injections of biAb (T1T2; murine Fc backbone) at 0.7 µg/µl in 50µl saline per injection. The right TA received two control injections of a 1:1 weight by weight mixture of parental AS19 and C3 antibodies at 0.7 µg/µl in 50µl saline per injection. The two injections were spaced 3 days apart.

### Exercise-induced tissue damage

One day after the last intramuscular injection, all mice received intraperitoneal injections (IP) of Evans blue dye (EBD) at 10 mg/ml with 50µl given per 10g body weight. One day after IP of EBD, all mice were exercised via a forced treadmill until exhaustion. The animals were euthanized with CO₂ according to standard IACUC protocol.

### Tissue preparation and immunofluorescence staining

After euthanasia, the TA muscles were removed, cut, and placed in optimum cutting temperature compound. The tissue was then rapidly frozen via a 2-methyl butane dry ice bath. The tissue was cryo-sectioned in a cryostat, at a thickness of 10 microns. Four different levels were cut (in triplicate) from the TA, 100 microns apart.

Slide sections were quickly dipped into cold PBS and fixed in ice-cold acetone for 15 minutes. Slides were washed and blocked (2% BSA and 1% normal goat serum in PBS) overnight at 4°C. The next day, slides were incubated with anti-mIgG Alexa Fluor 488 (Invitrogen) at 1:100 dilution for 2 hours (room temperature). Slides were washed and mounted using Vectashield mounting media with DAPI (Vector Labs). Slides were visualized with an inverted microscope (Olympus IX71) utilizing appropriate filter sets.

### Evans blue dye (EBD) myofiber damage evaluation

Tissue sections were processed as above except without immunofluorescence staining. All EBD positive fibers on each section were counted manually for both the left TA (biAb IM) and right TA (monoclonal parent antibody IM).

### Results

In order to determine whether biAbs are able to bind native antigens in mouse muscle tissues, unfixed frozen sections of wildtype or LARGE^{myd-3J/GrsrJ} mice, which are a murine model for alpha-dystroglycanopathy, were stained with biAbs (T1T2, T5T6, C5C6) or parental mAbs. The results indicated that biAbs were able to bind as well as the monospecific parental mAbs in both wild-type (**FIG. 8A**) and LARGE^{myd-3J/GrsrJ} (**FIG. 8B**) mouse muscle tissue sections.

Bispecific antibodies were then intramuscularly administered to wildtype or LARGE^{myd-3J/GrsrJ} mice (study outline is shown in **FIG. 8C**). To assess the effect of bispecific antibodies on exercise-induced tissue damage, immunofluorescence and Evans blue dye myofiber staining was performed on tissue from exercised mice. Immunofluorescence revealed that bispecific antibodies bound well to mouse muscle tissue (left or right tibialis anterior (TA) muscle) and was detectable 2 days after the last antibody injection (**FIG. 8E**). biAb treated left TAs had significantly fewer EBD positive fibers compared to the right contralateral control TAs (**FIG. 8D**). Evans blue dye penetrated many muscle fibers of LARGE^{myd-3J/GrsrJ} mouse tissue treated with a mixture of parental antibodies, indicating exercise-induced damage since the dye only penetrates and stains muscle fibers with membrane damage (**FIG. 8E**). In contrast, Evans blue dye penetrated significantly fewer muscle fibers of LARGE^{myd-3J/GrsrJ} mouse tissue treated with bispecific antibodies (**FIG. 8E**). This indicates that local injection of bispecific antibodies, but not parental monoclonal antibodies, protected muscle from exercise-induced damage in an *in vivo* mammalian model for alpha-dystroglycanopathy.

### Example 5: Systematic Delivery of bi-specific antibodies into LARGE^{myd-3J/GrsrJ} mice.

### Methods

### Antibody delivery

For exercise-induced tissue damage testing, four different groups of LARGE^{myd-3J/GrsrJ} mice were intravenously injected with a single dose of parental or bispecific antibody (with murine Fc region) via the lateral tail vein (IV) or intraperitoneally (IP). Each group received one of the following: parental anti-LG-4/5 (clone C3, IV), parental anti-beta-DG (clone AS19, IV), biAb (AS19 x C3, IV), and biAb (AS19 x C3, IP). One day after the injection, all mice received intraperitoneal injections (IP) of Evans blue dye (EBD) at 10 mg/ml with 50µl given per 10g body weight.

For behavioral testing, creatine kinase measurements, and biodistribution immunofluorescence experiments, LARGE^{myd-3J/GrsrJ} mice (aged 11-19 weeks) were randomized into two groups (n=16) before treatment. One group of mice was dosed at 30 mg biAb (T1T2) per kg mouse twice a week for 7 weeks. The second group of mice was dosed with a mixture of parental monoclonal antibodies (AS19 and C3, 15 mg antibody per kg mouse each) twice a week for 7 weeks. To prevent anaphylactic reaction, 5 mg per kg of diphenhydramine was pre-dosed intraperitoneally 10 minutes before administration of antibodies. Wildtype mice were treated with saline as a control.

### Exercise-induced tissue damage

1 day after intraperitoneal EBD injection, all mice were exercised via a forced treadmill until exhaustion. The animals were euthanized with CO₂ according to the standard IACUC protocol.

### Behavioral testing and creatine kinase measurements

For grip strength test, mice were allowed to acclimate to the testing room for 10 min before the test. The grip strength meter (Columbia Instruments, Columbus, OH) was mounted horizontally on a stable surface. The test mouse was gently placed on the top of the grid such that both of its front paws and hind paws were allowed to clasp onto the grid. The animal was then gently pulled backwards by its tail until the grip was released. The amount of force generated at the point of release was recorded on the strain gauge (grams). This procedure was performed 3 times for each animal and the grip force value was then calculated as the average of three tests.

For wire hang test, each animal was put on a wire screen, which was gently shifted side to side until the animal grabbed the wire. The wire-screen was then lifted to about 2 feet above a cushion pad and turned upside down. The time (latency) of animal from falling off the wire screen to the cushion pad was recorded, with a maximum cut-off time of 60 seconds. Each animal was tested twice with resting time of at least 5 min between tests.

Creatine kinase (CK) levels were measured at the beginning of the study (prior to bispecific antibody treatment) and at the end of the study (1 hr post-treadmill exercise after 7 weeks of bispecific antibody treatment) via standard colorimetric assay.

### Tissue preparation and immunofluorescence staining

For detection of bispecific antibodies in target organs, animals were euthanized 4 days after the last bispecific antibody intramuscular injection. TA muscles were removed, cut and placed in optimum cutting temperature compound. The tissue was then rapidly frozen via a 2-methyl butane dry ice bath. The tissue was cryo-sectioned in a cryostat, at a thickness of 10 microns.

For exercise-induced tissue damage samples, TA muscles were removed, cut and placed in optimum cutting temperature compound after exercise. The tissue was then rapidly frozen via a 2-methyl butane dry ice bath. The tissue was cryo-sectioned in a cryostat, at a thickness of 10 microns. Four different levels were cut (in triplicate) from the TA, 100 microns apart.

For both sets of tissue samples, slides were washed and blocked (2% BSA and 1% normal goat serum in PBS) overnight at 4°C. The next day, slides were incubated with anti-mIgG Alexa Fluor 488 (Invitrogen) at 1:100 dilution for 2 hours (room temperature). Slides were washed and mounted using Vectashield mounting media with DAPI (Vector Labs). Slides were visualized with an inverted microscope (Olympus IX71) utilizing appropriate filter sets.

### Evans blue dye (EBD) myofiber damage evaluation

Tissue sections were processed as above except without immunofluorescence staining. All EBD positive fibers on each section were counted manually for both the left TA (biAb IM) and right TA (monoclonal parent antibody IM).

### Results

LARGE^{myd-3J/GrsrJ} mice were dosed with 30 mg/kg of biAb (T1T2) and the parental antibodies as control, either by tail vein injection (IV) or intraperitoneally (IP) for comparison. Blood samples were collected by eye bleeding at 24, 48, 72, and 96 hrs after dosing, and the antibody levels were measured by ELISA coated with beta-DG (FIG. 8F) or LG4/5 (not shown). biAb had similar clearance rate of the parental mAbs. IP dosing resulted in high concentration but the overall pharmacokinetics of the biAb was similar to that dosed by IV. The anti-LG4/5 parental mAb had no signal when beta-DG was used for coating as expected.

Bispecific antibodies were next administered IV to wildtype or LARGE^{myd-3J/GrsrJ} mice. Behavioral testing revealed that LARGE^{myd-3J/GrsrJ} mice that were administered bispecific antibodies performed better on the grip strength test and wire hang test (**FIGS. 9A & 9B**), which are measures of muscle function, than mice treated with monoclonal parental antibodies (**FIGS. 9A & 9B**). Control wild-type mice treated with saline are also shown in **FIGS. 9A & 9B****.** These data demonstrate that bispecific antibodies improved muscle function. LARGE^{myd-3J/GrsrJ} mice that were administered bispecific antibodies also maintained performance on the treadmill test, whereas LARGE^{myd-3J/GrsrJ} mice treated with control antibody showed performance deterioration (**FIG. 9C**).

Despite poor performance in the treadmill test, LARGE^{myd-3J/GrsrJ} mice treated with control antibody showed increased CK levels. Significant elevation of serum CK levels indicates acute muscle damage as the result of lacking sarcolemma protection. By the end of the study, creatine kinase levels were significantly lower for LARGE^{myd-3J/GrsrJ} mice treated with bispecific antibodies compared to mice treated with monoclonal parental antibodies (**FIG. 9D**). Treatment with bispecific antibodies lowered the creatine kinase levels in LARGE^{myd-3J/GrsrJ} mice, indicating that bispecific antibodies helped to protect muscles from damage.

To assess the effect of bispecific antibodies on exercise-induced tissue damage, Evans blue dye myofiber staining was performed on tissue from exercised mice. Evans blue dye penetrated many muscle fibers of LARGE^{myd-3J/GrsrJ} mouse tissue treated with a mixture of parental antibodies, indicating exercise-induced damage since the dye only penetrates and stains muscle fibers with membrane damage. In contrast, Evans blue dye penetrated significantly fewer muscle fibers of LARGE^{myd-3J/GrsrJ} mouse tissue treated with bispecific antibodies than that of mice treated with parental antibodies (**FIG. 10**). This indicates that systematic delivery of bispecific antibodies, but not of parental monoclonal antibodies, protected muscle from exercise-induced damage.

For detection of bispecific antibodies in target organs, animals were euthanized 4 days after the last bispecific antibody intramuscular injection and immunofluorescence staining was performed. Staining revealed that even after 4 days, bispecific antibody T1T2 (AS19xC3) administered either by IV or intraperitoneally still specifically bound muscle tissue in the quadriceps, TA, diaphragm, and heart, but did not stain brain tissue, which was used as a negative control (**FIG. 11****,** first and second row). Parental monoclonal antibody AS19 did not stain muscle tissue well (**FIG. 11****,** third row), potentially due to a fast off-rate of the antibody (see Table K). However, parental monoclonal antibody C3 stained muscle tissue well (**FIG. 11****,** fourth row), as is consistent with **FIG. 4B****.**

The overall structure of the AS30 Fab bound to antigen beta-DG was determined, with the antigen shown between the heavy chain and light chain (**FIG. 12A**). **FIG. 12B** shows a close-up view of the CDR regions and the antigen. AS30 Fab and human βDG were mixed at 1:1 molar ratio and incubated on ice for 30 minutes before subjected to SEC Superdex 200 10/300 GL column (GE Healthcare) at 4°C. AS30:βDG complex was crystallized and its structure was determined with molecular replacement and refined to 2.55 Å. AS30 Fab and βDG sequence D⁷³⁸RDPEKSSEDD⁷⁴⁸ (SEQ ID NO:302) were visible in the electron density map. The crystal structure shows that AS30 antibody recognizes the linear peptide D⁷³⁸RDPEKSSEDD⁷⁴⁸ (SEQ ID NO:302) in βDG.

In addition, the overall structure of the C21 Fab bound to antigen human laminin-2 LG-5 domain was determined, with the antigen shown between the heavy chain and light chain (**FIG. 12AC**). **FIG. 12D** shows a close-up view of the CDR regions and the antigen. C21:LG5 complex structure was obtained in a similar fashion as AS30:βDG and was refined to 2.70 Å. C21Fab and human LG5 were both visible in the electrondensity, and C21 recognizes a conformational epitope on LG5.

### Example 6: Generation of trivalent, multispecific antibodies recognizing beta-DG and laminin-2

### Methods

### Antibody humanization

Humanization of the lead hybridoma antibodies was performed using both CDR grafting and 3D modeling techniques. Methods for antibody humanization are described in Jones et al., Nature 321: 522 (1986); Verhoeyen et al., Science 239: 1534 (1988); Sims et al., J Immunol 151: 2296 (1993); Chothia and Lesk, J Mol Biol 196: 901 (1987); Carter et al., Proc Natl Acad Sci USA 89: 4285 (1992); Presta et al., J Immunol 151: 2623 (1993); U.S. Pat. Nos. 5,589,205; 5,565,332; 6,180,370; 6,632,927; 7,241,877; 7,244,615; 7,244,832; 7,262,050; and U.S. Patent Publication No. 2004/0236078 (filed Apr. 30, 2004).

### Antibody expression and purification

The aDG trivalent antibodies were constructed by creating mammalian expression vectors with heavy chain constant regions that contain the knob-in-hole, NNAS, YTE, and RF variants and light chain constant regions. DNA variable domains with the desired linkers were synthesized and inserted in the desired heavy or light chain vectors. The configuration of each triAb is shown in Table M (numbering of antigen binding domains according to diagram in **FIG. 13****,** *i.e.,* VH1/VL1 and VH2/VL2 form CODV arm, and VH3/VL3 forms Fab arm). Amino acid sequences of the polypeptide chains of the triabs are provided in Table I2.

**Table M. triAb configurations.**

| ***triAb Name*** | ***VH1*/*VL1 binding domain*** | ***VH2*/*VL2 binding domain*** | ***VH3*/*VL3 binding domain*** |
|---|---|---|---|
| 3407 | C3_Hu10 | C3_Hu10 | AS30_Hu6 |
| 3423 | C3_Hu10 | C21_Hu11 | AS30_Hu6 |
| 3429 | C3_Hu11 | C21_Hu21 | AS30_Hu6 |
| 3437 | C21_Hu11 | C3_Hu11 | AS30_Hu6 |
| 3439 | C21_Hu21 | C3_Hu10 | AS30_Hu6 |

Trivalent antibodies were produced by transient cotransfection of four plasmids in Expi293F cells with Expifectamine (Thermo Fisher Scientific, A14635). Antibodies were purified with MabSelect SuRe columns (GE Healthcare, 11003494) followed by cation exchange with a HiTrap SP HP columns (GE Healthcare, 17115201). All proteins were then assessed for concentration, purity, and aggregation.

### Dual binding of antibodies to human antigens

A dual binding sandwich ELISA was performed by coating Thermo Nunc Immobilized SA 96 well plates with either 2 ug/mL of biotinylated N'Avi-HPC4-human LG4/5 or biotinylated human-beta DG-HPC4-Avi-C'. After overnight incubation at 4 °C, the plates were blocked with PBS+1% BSA+0.1% Tween for 1 hour at room temperature. After washing (BioTek ELx405 Select CW) with PBS, the trivalent or parental antibodies were added to the plate started at 8 ug/mL and a 2-fold dilution was performed across the plate, antibody was incubated for 1 hour at room temperature. After washing, the second antigen of beta-DG-mFe (Fig 1A) or LG4/5-mFc was added at 5 ug/mL. Following the second antigen, a secondary antibody of donkey anti-mouse (Jackson ImmunoResearch) at a 1:2,000 dilution was added for 30 minutes. ABTS was resuspended in ABTS buffer (Roche) and added to the wells for detection. Resulting signal was read with the Perkin Elmer EnVision Multimode Plate Reader at 405 nm.

For sequential dual binding of antigens to trivalent antibodies, a Series S Sensor Protein G chip (GE Healthcare, 29179315) was used with a T100 Biacore. This chip was used to immobilize trivalent or parental antibody to the surface (60 seconds with 5 ug/mL of antibody). After capture, 200 nM of LG4/5 was flowed over the chip for 60 seconds followed by 200 nM of beta-DG for 60 seconds. Binding to the trivalent antibody was observed by the change of mass detected on the chip in relative units (RU).

### Binding kinetics assay

Surface plasmon resonance ("SPR;" T100 Biacore; GE Healthcare) kinetics assay data with the trivalent antibodies was performed by immobilizing the antibodies (10 ug/mL) onto a Sensor S Protein G chip and then flowing serial dilutions of antigen over the chip (LG4/5: 80 nM-1.25 nM, BDG: 5 nM -0.31 nM and 4 nM-0.25 nM). Data was evaluated with a 1:1 binding model using the BIAevaluation software.

### Results

Trivalent antibodies (triAbs) were generated according to the format shown in **FIG. 13****.** These triAbs had a CODV arm with two distinct anti-laminin-2 antigen binding domains (VH-1/VL-1 and VH-2/VL-2 in **FIG. 13**) and a Fab arm (VH-3/VL-3 in **FIG. 13**) with an anti-beta-DG antigen binding domain.

To show binding of the triAbs to both antigens, a dual binding sandwich ELISA was performed as described above using 2 ug/mL of biotinylated N'Avi-HPC4-human LG4/5 (**FIG. 14A**) or biotinylated human-beta DG-HPC4-Avi-C'(**FIG. 14B**). In both orientations, all triAbs tested showed simultaneous dual binding.

Additionally, surface plasmon resonance was performed to show sequential binding of the human laminin-2 and beta-DG antigens (**FIG. 1C**). The triAbs showed binding to both antigens, while the monoclonal antibodies (humanized C3 and C21 variants for binding laminin-2 and humanized AS30 variant for binding beta-DG) only bound their respective antigen.

SPR was used as described above to analyze the kinetics of triAb binding to laminin-2 (Table N) or beta-DG (Table O).

**Table N. triAb binding to laminin-2 (SPR).**

| | **ka (1/Ms)** | **kd (1/s)** | **KD (M)** |
|---|---|---|---|
| 3407 | 1.57E+06 | 3.26E-03 | 2.08E-09 |
| 3423 | 2.70E+06 | 4.33E-03 | 1.60E-09 |
| 3429 | 2.13E+06 | 4.04E-03 | 1.90E-09 |
| 3437 | 2.41E+06 | 4.50E-03 | 1.87E-09 |
| 3439 | 3.07E+06 | 4.00E-03 | 1.30E-09 |
| AS30 Hu6 | 2.54E+06 | 2.02E-03 | 7.96E-10 |

**Table O. triAb binding to beta-DG (SPR).**

| | **ka (1/Ms)** | **kd (1/s)** | **KD (M)** |
|---|---|---|---|
| 3407 | 1.70E+05 | 2.53E-03 | 1.49E-08 |
| 3423 | 1.85E+05 | 2.28E-03 | 1.23E-08 |
| 3429 | 1.97E+05 | 2.19E-03 | 1.11E-08 |
| 3437 | 1.70E+05 | 2.31E-03 | 1.36E-08 |
| 3439 | 2.83E+05 | 2.89E-03 | 1.02E-08 |
| C3 Hu11 | 1.19E+05 | 1.17E-03 | 9.83E-09 |
| C21 Hu21 | 1.60E+05 | 1.44E-03 | 8.99E-09 |

As shown in Table N, five triAbs were able to bind human beta-DG with nanomolar affinity (K_{D} between 1.3-2.1 nM), comparable to that of the humanized AS30 antigen binding domain used in monovalent antibody format (0.8 nM). Similarly, Table O shows that the same triAbs were also able to bind human laminin-2 with nanomolar affinity (K_{D} between 10.2-14.9 nM), comparable to those of the humanized C3 and C21 antigen binding domains used in monovalent antibody format (9.8 nM and 9.0 nM, respectively).

These data demonstrate that bispecific anti-laminin-2/beta-DG antibodies in the trivalent format illustrated in **FIG. 13** were capable of simultaneous dual binding to their targets with similar binding affinity as compared with a traditional monovalent antibody format.

### Example 7: Improvement of muscle functions of LARGE^{myd-3J/GrsrJ} mice on behavioral tests after treatment with trivalent, multispecific antibodies recognizing beta-DG and laminin-2

### Methods

### Antibody administration

The LARGE/myd-3j mice described in Example 4 (ages at 8-16 weeks) were randomized into five groups based on their hind-leg splay score, wire-hang score, grip-strength and treadmill to ensure these scores were similar among groups (n=11) before treatment. Mice were then dosed at 30 mg/kg twice a week via tail vein injection (Monday and Thursday) with TriAbs (3407, 3437, and 3439) and a control TriAb that recognizes unrelated protein targets as well as a saline group as controls for up to 3.5 weeks with 7 doses. In addition, a wildtype mice group (n=6) was included as benchmarks for behavioral tests. To prevent anaphylactic reaction, 5 mg/kg diphenhydramine was pre-dosed IP 10 min before administration of antibodies. Grip-strength and wire-hang were tested weekly starting at week-2. Wildtype were treated with saline as benchmarks for various behavioral tests.

### Behavioral testing

For the hind-leg splay test, mice were lifted by their tails, and the positions of the hind legs relative to the body were recorded and graded.

The wire-hang test was conducted by placing mice on a wire grid and acclimating for 1 min; then, the wire grid with the mouse grasping on was slowly turned upside down at a defined speed of 2 sec, and the time the mouse held onto the grid was recorded, with a cutoff time of 60 sec. The test was repeated 3 times for each mouse and the results averaged.

Grip strength was evaluated by placing the mice on a Grip Strength Meter (Columbus Instruments), allowing the mouse to grasp the metal grid firmly, and pulling the tail horizontally until the mouse let go; then, the force was recorded. The test was repeated 5 times with a 1 min rest in between. Highest and lowest readings were removed for each mouse and the results were the average of the three remaining readings.

For treadmill run time, mice were placed onto individual lanes of a treadmill equipped with an electric shocking grid (Model 1055SRM Exer-3/6, Columbus Instruments). The animals were acclimated to the treadmill for 5 min, and then the mice were tested with a defined protocol with increasing speed. When a mouse spent more than 3 sec on the shocking grid without being able to run, the shocking grid was turned off and the total run time was recorded.

All behavioral tests were performed while blinded to mouse identity and treatment, with the results unblinded after testing.

### Results

The triAbs generated in Example 6 were tested for their effects on muscle function in LARGE/myd-3j mice.

Large/myd-3j mice showed significantly improved performance on grip-strength (**FIG. 15**) and wire-hang (**FIG. 16**) after two weeks of treatment with TriAb 3407, 3437, or 3439, as was observed with biAb treatment described previously (*see* Example 5). Treadmill performance was maintained or slightly improved with TriAbs treatment (**FIG. 17**), however without statistical significance as compared to controls, which instead demonstrated slight deterioration. Typically, statistically significant improvement on treadmill run time requires longer time of treatment.

Taken together, the results of multiple functional assays demonstrated that treatment with trivalent, bispecific anti-laminin-2/beta-DG antibodies led to improved muscle function in a murine model for alpha-dystroglycanopathy.

## Claims

1. A multispecific binding molecule comprising at least a first binding domain that binds an extracellular portion of dystroglycan and at least a second binding domain that binds laminin-2,
wherein the multispecific binding molecule is a multispecific binding protein comprising one or more polypeptide chains,
wherein the binding protein comprises four polypeptide chains, wherein a first polypeptide chain comprises a structure represented by the formula:
V_{L2}-L₁-V_{L1}-L₂-C_{L} [I]
and a second polypeptide chain comprises a structure represented by the formula:
V_{H1}-L₃-V_{H2}-L₋₄-C_{H1}-hinge-C_{H2}-C_{H3} [II]
and a third polypeptide chain comprises a structure represented by the formula:
V_{H3}-C_{H1}-hinge-C_{H2}-C_{H3} [III]
and a fourth polypeptide chain comprises a structure represented by the formula:
V_{L3}-C_{L} [IV]
wherein:
VL1 is a first immunoglobulin light chain variable domain;
VL2 is a second immunoglobulin light chain variable domain;
VL3 is a third immunoglobulin light chain variable domain;
VH1 is a first immunoglobulin heavy chain variable domain;
VH2 is a second immunoglobulin heavy chain variable domain;
VH3 is a third immunoglobulin heavy chain variable domain;
CL is an immunoglobulin light chain constant domain;
CH1 is an immunoglobulin CH1 heavy chain constant domain;
CH2 is an immunoglobulin CH2 heavy chain constant domain;
CH3 is an immunoglobulin CH3 heavy chain constant domain;
hinge is an immunoglobulin hinge region connecting the CH1 and CH2 domains; and
L1, L2, L3 and L4 are amino acid linkers;
wherein the polypeptide of formula I and the polypeptide of formula II form a cross-over light chain-heavy chain pair; and
wherein V_{H1} and V_{L1} form an antigen binding site, wherein V_{H2} and V_{L2} form an antigen binding site, and wherein V_{H3} and V_{L3} form an antigen binding site for a total of three antigen binding sites, and wherein the three antigen binding sites comprise at least one antigen binding site that binds the extracellular portion of dystroglycan and at least one antigen binding site that binds laminin-2.

2. The multispecific binding molecule of claim 1, comprising one antigen binding site that binds the extracellular portion of dystroglycan and two antigen binding sites that bind laminin-2, preferably wherein V_{H1} and V_{L1} form a first antigen binding site that binds laminin-2, V_{H2} and V_{L2} form a second antigen binding site that binds laminin-2, and V_{H3} and V_{L3} form a third antigen binding site that binds the extracellular portion of dystroglycan.

3. The multispecific binding molecule of claim 1, comprising two antigen binding sites that bind the extracellular portion of dystroglycan and one antigen binding site that binds laminin-2, preferably wherein V_{H1} and V_{L1} form a first antigen binding site that binds the extracellular portion of dystroglycan, V_{H2} and V_{L2} form a second antigen binding site that binds the extracellular portion of dystroglycan, and V_{H3} and V_{L3} form a third antigen binding site that binds laminin-2.

4. The multispecific binding molecule of any one of claims 1-3, wherein:
(a) the at least one antigen binding site that binds the extracellular portion of dystroglycan binds the extracellular portion of dystroglycan with an equilibrium dissociation constant (K_{D}) lower than about 1µM when assayed as part of a multispecific binding protein;
(b) the at least one antigen binding site that binds the extracellular portion of dystroglycan binds the extracellular portions of human and mouse dystroglycan;
(c) the at least one antigen binding site that binds the extracellular portion of dystroglycan binds beta-dystroglycann or alpha-dystroglycan;
(d) the at least one antigen binding site that binds laminin-2 binds human laminin-2;
(e) the at least one antigen binding site that binds laminin-2 binds mouse and human laminin-2; and/or
(f) the at least one antigen binding site that binds laminin-2 binds a polypeptide comprising a laminin G-like (LG) domain 4 of laminin-2, a laminin G-like (LG) domain 5 of laminin-2, or both.

5. The multispecific binding molecule of any one of claims 1-4,
wherein the at least one antigen binding site that binds the extracellular portion of dystroglycan comprises:
(a) a heavy chain variable domain (VH) comprising a CDR-H1 comprising an amino acid sequence selected from the group consisting of SEQ ID NOs:1-8, a CDR-H2 comprising an amino acid sequence selected from the group consisting of SEQ ID NOs:9-17, and a CDR-H3 comprising an amino acid sequence selected from the group consisting of SEQ ID NOs:18-27; and
(b) a light chain variable domain (VL) comprising a CDR-L1 comprising an amino acid sequence selected from the group consisting of SEQ ID NOs:28-37, a CDR-L2 comprising an amino acid sequence selected from the group consisting of SEQ ID NOs:38-42, and a CDR-L3 comprising an amino acid sequence selected from the group consisting of SEQ ID NOs:43-50; and/or
wherein the at least one antigen binding site that binds laminin-2 comprises:
(a) a heavy chain variable domain (VH) comprising a CDR-H1 comprising an amino acid sequence selected from the group consisting of SEQ ID NOs:51-55 and 81-95, a CDR-H2 comprising an amino acid sequence selected from the group consisting of SEQ ID NOs:56-60 and 96-110, and a CDR-H3 comprising an amino acid sequence selected from the group consisting of SEQ ID NOs:61-65 and 111-125; and
(b) a light chain variable domain (VL) comprising a CDR-L1 comprising an amino acid sequence selected from the group consisting of SEQ ID NOs:66-70 and 126-140, a CDR-L2 comprising an amino acid sequence selected from the group consisting of SEQ ID NOs:38, 71-75, and 141-154, and a CDR-L3 comprising an amino acid sequence selected from the group consisting of SEQ ID NOs:76-80 and 155-169.

6. The multispecific binding molecule of claim 5, wherein:
(a) the VH domain of the at least one antigen binding site that binds the extracellular portion of dystroglycan comprises a sequence selected from the group consisting of SEQ ID NOs:170, 172, 174, 176, 178, 180, 182, 184, 186, and 188; and
(b) the VL domain of the at least one antigen binding site that binds the extracellular portion of dystroglycan comprises a sequence selected from the group consisting of SEQ ID NOs:171, 173, 175, 177, 179, 181, 183, 185, 187, and 189; and/or
(c) the VH domain of the at least one antigen binding site that binds laminin-2 comprises a sequence selected from the group consisting of SEQ ID NOs:190, 192, 194, 196, 198, 200, 202, 204, 206, 208, 210, 212, 214, 216, 218, 220, 222, 224, 226, and 228; and
(d) the VL domain of the at least one antigen binding site that binds laminin-2 comprises a sequence selected from the group consisting of SEQ ID NOs: 191, 193, 195, 197, 199, 201, 203, 205, 207, 209, 211, 213, 215, 217, 219, 221, 223, 225, 227, and 229.

7. The multispecific binding molecule of any one of claims 1-3, wherein
the at least one antigen binding site that binds the extracellular portion of dystroglycan comprises:
(a) a heavy chain variable domain (VH) comprising a CDR-H1 comprising the sequence of SEQ ID NO:316, a CDR-H2 comprising the sequence of SEQ ID NO:318, and a CDR-H3 comprising the sequence of SEQ ID NO:320; and
(b) a light chain variable domain (VL) comprising a CDR-L1 comprising the sequence of SEQ ID NO:332, a CDR-L2 comprising the sequence of SEQ ID NO:334, and a CDR-L3 comprising the sequence of SEQ ID NO:336; and/or
the at least one antigen binding site that binds laminin-2 comprises:
(a) a heavy chain variable domain (VH) comprising a CDR-H1 comprising the sequence of SEQ ID NO:380, a CDR-H2 comprising the sequence of SEQ ID NO:382, and a CDR-H3 comprising the sequence of SEQ ID NO:384; and
(b) a light chain variable domain (VL) comprising a CDR-L1 comprising the sequence of SEQ ID NO:396, a CDR-L2 comprising the sequence of SEQ ID NO:398, and a CDR-L3 comprising the sequence of SEQ ID NO:400; or
the at least one antigen binding site that binds laminin-2 comprises:
(a) a heavy chain variable domain (VH) comprising a CDR-H1 comprising the sequence of SEQ ID NO:444, a CDR-H2 comprising the sequence of SEQ ID NO:446, and a CDR-H3 comprising the sequence of SEQ ID NO:448; and
(b) a light chain variable domain (VL) comprising a CDR-L1 comprising the sequence of SEQ ID NO:460, a CDR-L2 comprising the sequence of SEQ ID NO:462, and a CDR-L3 comprising the sequence of SEQ ID NO:464.

8. The multispecific binding molecule of claim 7, wherein:
(a) the VH domain of the at least one antigen binding site that binds the extracellular portion of dystroglycan comprises the sequence of SEQ ID NO:314; and
(b) the VL domain of the at least one antigen binding site that binds the extracellular portion of dystroglycan comprises the sequence of SEQ ID NO:330; or
(c) the VH domain of the at least one antigen binding site that binds the extracellular portion of dystroglycan comprises the sequence of SEQ ID NO:346; and
(d) the VL domain of the at least one antigen binding site that binds the extracellular portion of dystroglycan comprises the sequence of SEQ ID NO:362.

9. The multispecific binding molecule of any one of claims 1-3 and 5-8, wherein the at least one antigen binding site that binds laminin-2 comprises:
(a) a heavy chain variable domain (VH) comprising a CDR-H1 comprising the sequence of SEQ ID NO:380, a CDR-H2 comprising the sequence of SEQ ID NO:382, and a CDR-H3 comprising the sequence of SEQ ID NO:384; and
(b) a light chain variable domain (VL) comprising a CDR-L1 comprising the sequence of SEQ ID NO:396, a CDR-L2 comprising the sequence of SEQ ID NO:398, and a CDR-L3 comprising the sequence of SEQ ID NO:400, or
(a) a heavy chain variable domain (VH) comprising a CDR-H1 comprising the sequence of SEQ ID NO:380, a CDR-H2 comprising the sequence of SEQ ID NO:382, and a CDR-H3 comprising the sequence of SEQ ID NO:384; and
(b) a light chain variable domain (VL) comprising a CDR-L1 comprising the sequence of SEQ ID NO:428, a CDR-L2 comprising the sequence of SEQ ID NO:398, and a CDR-L3 comprising the sequence of SEQ ID NO:400.

10. The multispecific binding molecule of claim 9, wherein:
(a) the VH domain of the at least one antigen binding site that binds laminin-2 comprises the sequence of SEQ ID NO:378; and
(b) the VL domain of the at least one antigen binding site that binds laminin-2 comprises the sequence of SEQ ID NO:394; or
(a) the VH domain of the at least one antigen binding site that binds laminin-2 comprises the sequence of SEQ ID NO:410; and
(b) the VL domain of the at least one antigen binding site that binds laminin-2 comprises the sequence of SEQ ID NO:426.

11. The multispecific binding molecule of any one of claims 1-3 and 5-8, wherein the at least one antigen binding site that binds laminin-2 comprises:
(a) a heavy chain variable domain (VH) comprising a CDR-H1 comprising the sequence of SEQ ID NO:444, a CDR-H2 comprising the sequence of SEQ ID NO:446, and a CDR-H3 comprising the sequence of SEQ ID NO:448; and
(b) a light chain variable domain (VL) comprising a CDR-L1 comprising the sequence of SEQ ID NO:460, a CDR-L2 comprising the sequence of SEQ ID NO:462, and a CDR-L3 comprising the sequence of SEQ ID NO:464; or
(a) a heavy chain variable domain (VH) comprising a CDR-H1 comprising the sequence of SEQ ID NO:444, a CDR-H2 comprising the sequence of SEQ ID NO:478, and a CDR-H3 comprising the sequence of SEQ ID NO:448; and
(b) a light chain variable domain (VL) comprising a CDR-L1 comprising the sequence of SEQ ID NO:460, a CDR-L2 comprising the sequence of SEQ ID NO:462, and a CDR-L3 comprising the sequence of SEQ ID NO:464.

12. The multispecific binding molecule of 11, wherein:
(a) the VH domain of the at least one antigen binding site that binds laminin-2 comprises the sequence of SEQ ID NO:442; and
(b) the VL domain of the at least one antigen binding site that binds laminin-2 comprises the sequence of SEQ ID NO:458; or
(a) the VH domain of the at least one antigen binding site that binds laminin-2 comprises the sequence of SEQ ID NO:474; and
(b) the VL domain of the at least one antigen binding site that binds laminin-2 comprises the sequence of SEQ ID NO:490.

13. The multispecific binding molecule of any one of claims 1-12, wherein L₁ and L₂ comprise the sequence DKTHT (SEQ ID NO: 534), or wherein L₃ and L₄ comprise the sequence DKTHT (SEQ ID NO: 534), or wherein L₁, L₂, L₃, and L₄ comprise the sequence DKTHT (SEQ ID NO: 534).

14. A bispecific binding molecule comprising a first binding domain that binds an extracellular portion of dystroglycan and a second binding domain that binds laminin-2,
wherein the bispecific binding molecule is a bispecific binding protein comprising one or more polypeptide chains, and
wherein the bispecific binding molecule comprises four polypeptide chains that form four antigen binding sites, wherein two polypeptide chains comprise a structure represented by the formula:
V_{L1}-L₁-V_{L2}-L₂-C_{L} [I]
and two polypeptide chains comprise a structure represented by the formula:
V_{H2}-L₃-V_{H1}-L₄-C_{H1}-hinge-C_{H2}-C_{H3} [II]
wherein:
V_{L1} is a first immunoglobulin light chain variable domain;
V_{L2} is a second immunoglobulin light chain variable domain;
V_{H1} is a first immunoglobulin heavy chain variable domain;
V_{H2} is a second immunoglobulin heavy chain variable domain;
C_{L} is an immunoglobulin light chain constant domain;
C_{H1} is an immunoglobulin C_{H1} heavy chain constant domain;
C_{H2} is an immunoglobulin C_{H2} heavy chain constant domain;
C_{H3} is an immunoglobulin C_{H3} heavy chain constant domain;
hinge is an immunoglobulin hinge region connecting the C_{H1} and C_{H2} domains; and
L₁, L₂, L₃, and L₄ are amino acid linkers;
wherein the V_{H1} and V_{L1} domains form a V_{H1}/V_{L1} binding pair, and wherein the V_{H2} and V_{L2} domains form a V_{H2}/V_{L2} binding pair.

15. The bispecific binding molecule of claim 14, wherein L₁, L₂, L₃, and L₄ are each 0 to 50 amino acid residues in length, preferably wherein L₁, L₂, L₃, and L₄ are each 0 to 25 amino acid residues in length, or are each 0 to 14 amino acid residues in length.

16. A bispecific binding molecule comprising a first binding domain that binds an extracellular portion of dystroglycan and a second binding domain that binds laminin-2,
wherein the bispecific binding molecule is a bispecific binding protein comprising one or more polypeptide chains, and
wherein the bispecific binding molecule comprises two light chains comprising a structure represented by the formula:
V_{L1}-L₅-V_{L2}-L₆-C_{L} [III]
and two heavy chains comprising a structure represented by the formula:
V_{H1}-L₇-V_{H2}-L₈-C_{H1}-hinge-C_{H2}-C_{H3} [IV]
wherein:
V_{L1} is a first immunoglobulin light chain variable domain;
V_{L2} is a second immunoglobulin light chain variable domain;
V_{H1} is a first immunoglobulin heavy chain variable domain;
V_{H2} is a second immunoglobulin heavy chain variable domain;
C_{L} is an immunoglobulin light chain constant domain;
C_{H1} is an immunoglobulin C_{H1} heavy chain constant domain;
C_{H2} is an immunoglobulin C_{H2} heavy chain constant domain;
C_{H3} is an immunoglobulin C_{H3} heavy chain constant domain;
hinge is an immunoglobulin hinge region connecting the C_{H1} and C_{H2} domains; and
L₅, L₆, L₇, and L₈ are amino acid linkers;
wherein the V_{H1} and V_{L1} domains form a V_{H1}/V_{L1} binding pair, and wherein the V_{H2} and V_{L2} domains form a V_{H2}/V_{L2} binding pair.

17. An isolated nucleic acid molecule comprising a nucleotide sequence encoding the multispecific binding molecule of any one of claims 1-13 or the bispecific binding molecule of any one of claims 14-16.

18. A vector system comprising:
(a) one or more vectors encoding a first, second, third, and fourth polypeptide chain of a multispecific binding molecule of claim 1; or
(b) one or more vectors encoding a first, second, third, and fourth polypeptide chain of a bispecific binding molecule of claim 14, or
(c) one or more vectors encoding two light chains and two heavy chains of a bispecific binding molecule of claim 16.

19. An isolated host cell comprising the nucleic acid molecule of claim 17, or comprising the vector system of claim 18.

20. A method of producing a multispecific binding molecule or a bispecific binding molecule, the method comprising:
a) culturing a host cell of claim 19 under conditions such that the host cell expresses the multispecific binding molecule or bispecific binding molecule; and
b) isolating the multispecific binding molecule or bispecific binding molecule from the host cell.

21. The multispecific binding molecule of any one of claims 1-13 or the the bispecific binding molecule of any one of claims 14-16 for use in a method for treating or preventing an alpha-dystroglycanopathy in an individual.

22. A pharmaceutical composition comprising the multispecific binding molecule of any one of claims 1-13 or the bispecific binding molecule of any one of claims 14-16 and a pharmaceutically acceptable carrier.

## Patentansprüche

1. Multispezifisches Bindemolekül, umfassend wenigstens eine erste Bindedomäne, die einen extrazellulären Teil von Dystroglycan bindet, und wenigstens eine zweite Bindedomäne, die Laminin-2 bindet,
wobei es sich bei dem multispezifischen Bindemolekül um ein multispezifisches Bindeprotein handelt, das eine oder mehrere Polypeptidketten umfasst,
wobei das Bindeprotein vier Polypeptidketten umfasst, wobei eine erste Polypeptidkette eine durch die Formel
V_{L2}-L₁₋V_{L1-}L₂-C_{L} [I]
dargestellte Struktur umfasst und eine zweite Polypeptidkette eine durch die Formel
V_{H1}-L₃-V_{H2}-L₄-C_{H1}-hinge-C_{H2}-C_{H3} [II]
dargestellte Struktur umfasst und eine dritte Polypeptidkette eine durch die Formel
V_{H3}-_{H1}-hinge-C_{H2}-C_{H3} [III]
dargestellte Struktur umfasst und eine vierte Polypeptidkette eine durch die Formel
V_{L3}-C_{L} [IV]
dargestellte Struktur umfasst, wobei:
VL1 für eine erste variable Immunglobulin-Leichtkettendomäne steht;
VL2 für eine zweite variable Immunglobulin-Leichtkettendomäne steht;
VL3 für eine dritte variable Immunglobulin-Leichtkettendomäne steht;
VH1 für eine erste variable Immunglobulin-Schwerkettendomäne steht;
VH2 für eine zweite variable Immunglobulin-Schwerkettendomäne steht;
VH3 für eine dritte variable Immunglobulin-Schwerkettendomäne steht;
CL für eine konstante Immunglobulin-Leichtkettendomäne steht;
CH1 für eine konstante Immunglobulin-Schwerkettendomäne CH1 steht;
CH2 für eine konstante Immunglobulin-Schwerkettendomäne CH2 steht;
CH3 für eine konstante Immunglobulin-Schwerkettendomäne CH3 steht;
hinge für eine Immunglobulin-Scharnierregion steht, die die Domänen CH1 und CH2 verbindet; und
L1, L2, L3 und L4 für Aminosäure-Linker stehen;
wobei das Polypeptid der Formel I und das Polypeptid der Formel II ein Leichtkette-Schwerkette-Cross-over-Paar bilden;
wobei V_{H1} und V_{L1} eine Antigenbindestelle bilden, wobei V_{H2} und V_{L2} eine Antigenbindestelle bilden und wobei V_{H3} und V_{L3} eine Antigenbindestelle bilden, für insgesamt drei Antigenbindestellen, und wobei die drei Antigenbindestellen mindestens eine Antigenbindestelle, die den extrazellulären Teil von Dystroglycan bindet, und mindestens eine Antigenbindestelle, die Laminin-2 bindet, umfassen.

2. Multispezifisches Bindemolekül nach Anspruch 1, umfassend eine Antigenbindestelle, die den extrazellulären Teil von Dystroglycan bindet, und zwei Antigenbindestellen, die Laminin-2 binden, vorzugsweise wobei V_{H1} und V_{L1} eine erste Antigenbindestelle bilden, die Laminin-2 bindet, V_{H2} und V_{L2} eine zweite Antigenbindestelle bilden, die Laminin-2 bindet, und V_{H3} und V_{L3} eine dritte Antigenbindestelle bilden, die den extrazellulären Teil von Dystroglycan bindet.

3. Multispezifisches Bindemolekül nach Anspruch 1, umfassend zwei Antigenbindestellen, die den extrazellulären Teil von Dystroglycan binden, und eine Antigenbindestelle, die Laminin-2 bindet, vorzugsweise wobei V_{H1} und V_{L1} eine erste Antigenbindestelle bilden, die den extrazellulären Teil von Dystroglycan bindet, V_{H2} und V_{L2} eine zweite Antigenbindestelle bilden, die den extrazellulären Teil von Dystroglycan bindet, und V_{H3} und V_{L3} eine dritte Antigenbindestelle bilden, die Laminin-2 bindet.

4. Multispezifisches Bindemolekül nach einem der Ansprüche 1-3, wobei:
(a) die mindestens eine Antigenbindestelle, die den extrazellulären Teil von Dystroglycan bindet, den extrazellulären Teil von Dystroglycan mit einer Gleichgewichtsdissoziationskonstante (K_{D}) kleiner etwa 1 µM bindet, wenn sie als Teil eines multispezifischen Bindeproteins getestet wird;
(b) die mindestens eine Antigenbindestelle, die den extrazellulären Teil von Dystroglycan bindet, die extrazellulären Teile von Mensch- und Maus-Dystroglycan bindet;
(c) die mindestens eine Antigenbindestelle, die den extrazellulären Teil von Dystroglycan bindet, Beta-Dystroglycan oder Alpha-Dystroglycan bindet;
(d) die mindestens eine Antigenbindestelle, die Laminin-2 bindet, Mensch-Laminin-2 bindet;
(e) die mindestens eine Antigenbindestelle, die Laminin-2 bindet, Maus- und Mensch-Laminin-2 bindet; und/oder
(f) die mindestens eine Antigenbindestelle, die Laminin-2 bindet, ein Polypeptid bindet, das eine Laminin G ähnliche (LG) Domäne 4 von Laminin-2, eine Laminin G ähnliche (LG) Domäne 5 von Laminin-2 oder beide umfasst.

5. Multispezifisches Bindemolekül nach einem der Ansprüche 1-4,
wobei die mindestens eine Antigenbindestelle, die den extrazellulären Teil von Dystroglycan bindet, Folgendes umfasst:
(a) eine variable Schwerkettendomäne (VH), die eine CDR-H1, die eine aus der Gruppe bestehend aus SEQ ID NO:1-8 ausgewählte Aminosäuresequenz umfasst, eine CDR-H2, die eine aus der Gruppe bestehend aus SEQ ID NO:9-17 ausgewählte Aminosäuresequenz umfasst, und eine CDR-H3, die eine aus der Gruppe bestehend aus SEQ ID NO:18-27 ausgewählte Aminosäuresequenz umfasst, umfasst; und
(b) eine variable Leichtkettendomäne (VL), die eine CDR-L1, die eine aus der Gruppe bestehend aus SEQ ID NO:28-37 ausgewählte Aminosäuresequenz umfasst, eine CDR-L2, die eine aus der Gruppe bestehend aus SEQ ID NO:38-42 ausgewählte Aminosäuresequenz umfasst, und eine CDR-L3, die eine aus der Gruppe bestehend aus SEQ ID NO:43-50 ausgewählte Aminosäuresequenz umfasst, umfasst; und/oder wobei die mindestens eine Antigenbindestelle, die Laminin-2 bindet, Folgendes umfasst:
(a) eine variable Schwerkettendomäne (VH), die eine CDR-H1, die eine aus der Gruppe bestehend aus SEQ ID NO:51-55 und 81-95 ausgewählte Aminosäuresequenz umfasst, eine CDR-H2, die eine aus der Gruppe bestehend aus SEQ ID NO:56-60 und 96-110 ausgewählte Aminosäuresequenz umfasst, und eine CDR-H3, die eine aus der Gruppe bestehend aus SEQ ID NO:61-65 und 111-125 ausgewählte Aminosäuresequenz umfasst, umfasst; und
(b) eine variable Leichtkettendomäne (VL), die eine CDR-L1, die eine aus der Gruppe bestehend aus SEQ ID NO:66-70 und 126-140 ausgewählte Aminosäuresequenz umfasst, eine CDR-L2, die eine aus der Gruppe bestehend aus SEQ ID NO:38, 71-75 und 141-154 ausgewählte Aminosäuresequenz umfasst, und eine CDR-L3, die eine aus der Gruppe bestehend aus SEQ ID NO:76-80 und 155-169 ausgewählte Aminosäuresequenz umfasst, umfasst.

6. Multispezifisches Bindemolekül nach Anspruch 5, wobei:
(a) die VH-Domäne der mindestens einen Antigenbindestelle, die den extrazellulären Teil von Dystroglycan bindet, eine Sequenz umfasst, die aus der Gruppe bestehend aus SEQ ID NO:170, 172, 174, 176, 178, 180, 182, 184, 186 und 188 ausgewählt ist; und
(b) die VL-Domäne der mindestens einen Antigenbindestelle, die den extrazellulären Teil von Dystroglycan bindet, eine Sequenz umfasst, die aus der Gruppe bestehend aus SEQ ID NO:171, 173, 175, 177, 179, 181, 183, 185, 187 und 189 ausgewählt ist; und/oder
(c) die VH-Domäne der mindestens einen Antigenbindestelle, die Laminin-2 bindet, eine Sequenz umfasst, die aus der Gruppe bestehend aus SEQ ID NO:190, 192, 194, 196, 198, 200, 202, 204, 206, 208, 210, 212, 214, 216, 218, 220, 222, 224, 226 und 228 ausgewählt ist; und
(d) die VL-Domäne der mindestens einen Antigenbindestelle, die Laminin-2 bindet, eine Sequenz umfasst, die aus der Gruppe bestehend aus SEQ ID NO:191, 193, 195, 197, 199, 201, 203, 205, 207, 209, 211, 213, 215, 217, 219, 221, 223, 225, 227 und 229 ausgewählt ist.

7. Multispezifisches Bindemolekül nach einem der Ansprüche 1-3, wobei
die mindestens eine Antigenbindestelle, die den extrazellulären Teil von Dystroglycan bindet, Folgendes umfasst:
(a) eine variable Schwerkettendomäne (VH), die eine CDR-H1, die die Sequenz unter SEQ ID NO:316 umfasst, eine CDR-H2, die die Sequenz unter SEQ ID NO:318 umfasst, und eine CDR-H3, die die Sequenz unter SEQ ID NO:320 umfasst, umfasst; und
(b) eine variable Leichtkettendomäne (VL), die eine CDR-L1, die die Sequenz unter SEQ ID NO:332 umfasst, eine CDR-L2, die die Sequenz unter SEQ ID NO:334 umfasst, und eine CDR-L3, die die Sequenz unter SEQ ID NO:336 umfasst, umfasst; und/oder
die mindestens eine Antigenbindestelle, die Laminin-2 bindet, Folgendes umfasst:
(a) eine variable Schwerkettendomäne (VH), die eine CDR-H1, die die Sequenz unter SEQ ID NO:380 umfasst, eine CDR-H2, die die Sequenz unter SEQ ID NO:382 umfasst, und eine CDR-H3, die die Sequenz unter SEQ ID NO:384 umfasst, umfasst; und
(b) eine variable Leichtkettendomäne (VL), die eine CDR-L1, die die Sequenz unter SEQ ID NO:396 umfasst, eine CDR-L2, die die Sequenz unter SEQ ID NO:398 umfasst, und eine CDR-L3, die die Sequenz unter SEQ ID NO:400 umfasst, umfasst; oder
die mindestens eine Antigenbindestelle, die Laminin-2 bindet, Folgendes umfasst:
(a) eine variable Schwerkettendomäne (VH), die eine CDR-H1, die die Sequenz unter SEQ ID NO:444 umfasst, eine CDR-H2, die die Sequenz unter SEQ ID NO:446 umfasst, und eine CDR-H3, die die Sequenz unter SEQ ID NO:448 umfasst, umfasst; und
(b) eine variable Leichtkettendomäne (VL), die eine CDR-L1, die die Sequenz unter SEQ ID NO:460 umfasst, eine CDR-L2, die die Sequenz unter SEQ ID NO:462 umfasst, und eine CDR-L3, die die Sequenz unter SEQ ID NO:464 umfasst, umfasst.

8. Multispezifisches Bindemolekül nach Anspruch 7, wobei:
(a) die VH-Domäne der mindestens einen Antigenbindestelle, die den extrazellulären Teil von Dystroglycan bindet, die Sequenz unter SEQ ID NO:314 umfasst; und
(b) die VL-Domäne der mindestens einen Antigenbindestelle, die den extrazellulären Teil von Dystroglycan bindet, die Sequenz unter SEQ ID NO:330 umfasst; oder
(c) die VH-Domäne der mindestens einen Antigenbindestelle, die den extrazellulären Teil von Dystroglycan bindet, die Sequenz unter SEQ ID NO:346 umfasst; und
(d) die VL-Domäne der mindestens einen Antigenbindestelle, die den extrazellulären Teil von Dystroglycan bindet, die Sequenz unter SEQ ID NO:362 umfasst.

9. Multispezifisches Bindemolekül nach einem der Ansprüche 1-3 und 5-8, wobei die mindestens eine Antigenbindestelle, die Laminin-2 bindet, Folgendes umfasst:
(a) eine variable Schwerkettendomäne (VH), die eine CDR-H1, die die Sequenz unter SEQ ID NO:380 umfasst, eine CDR-H2, die die Sequenz unter SEQ ID NO:382 umfasst, und eine CDR-H3, die die Sequenz unter SEQ ID NO:384 umfasst, umfasst; und
(b) eine variable Leichtkettendomäne (VL), die eine CDR-L1, die die Sequenz unter SEQ ID NO:396 umfasst, eine CDR-L2, die die Sequenz unter SEQ ID NO:398 umfasst, und eine CDR-L3, die die Sequenz unter SEQ ID NO:400 umfasst, umfasst, oder
(a) eine variable Schwerkettendomäne (VH), die eine CDR-H1, die die Sequenz unter SEQ ID NO:380 umfasst, eine CDR-H2, die die Sequenz unter SEQ ID NO:382 umfasst, und eine CDR-H3, die die Sequenz unter SEQ ID NO:384 umfasst, umfasst; und
(b) eine variable Leichtkettendomäne (VL), die eine CDR-L1, die die Sequenz unter SEQ ID NO:428 umfasst, eine CDR-L2, die die Sequenz unter SEQ ID NO:398 umfasst, und eine CDR-L3, die die Sequenz unter SEQ ID NO:400 umfasst, umfasst.

10. Multispezifisches Bindemolekül nach Anspruch 9, wobei:
(a) die VH-Domäne der mindestens einen Antigenbindestelle, die Laminin-2 bindet, die Sequenz unter SEQ ID NO:378 umfasst; und
(b) die VL-Domäne der mindestens einen Antigenbindestelle, die Laminin-2 bindet, die Sequenz unter SEQ ID NO:394 umfasst; oder
(a) die VH-Domäne der mindestens einen Antigenbindestelle, die Laminin-2 bindet, die Sequenz unter SEQ ID NO:410 umfasst; und
(b) die VL-Domäne der mindestens einen Antigenbindestelle, die Laminin-2 bindet, die Sequenz unter SEQ ID NO:426 umfasst.

11. Multispezifisches Bindemolekül nach einem der Ansprüche 1-3 und 5-8, wobei die mindestens eine Antigenbindestelle, die Laminin-2 bindet, Folgendes umfasst:
(a) eine variable Schwerkettendomäne (VH), die eine CDR-H1, die die Sequenz unter SEQ ID NO:444 umfasst, eine CDR-H2, die die Sequenz unter SEQ ID NO:446 umfasst, und eine CDR-H3, die die Sequenz unter SEQ ID NO:448 umfasst, umfasst; und
(b) eine variable Leichtkettendomäne (VL), die eine CDR-L1, die die Sequenz unter SEQ ID NO:460 umfasst, eine CDR-L2, die die Sequenz unter SEQ ID NO:462 umfasst, und eine CDR-L3, die die Sequenz unter SEQ ID NO:464 umfasst, umfasst; oder
(a) eine variable Schwerkettendomäne (VH), die eine CDR-H1, die die Sequenz unter SEQ ID NO:444 umfasst, eine CDR-H2, die die Sequenz unter SEQ ID NO:478 umfasst, und eine CDR-H3, die die Sequenz unter SEQ ID NO:448 umfasst, umfasst; und
(b) eine variable Leichtkettendomäne (VL), die eine CDR-L1, die die Sequenz unter SEQ ID NO:460 umfasst, eine CDR-L2, die die Sequenz unter SEQ ID NO:462 umfasst, und eine CDR-L3, die die Sequenz unter SEQ ID NO:464 umfasst, umfasst.

12. Multispezifisches Bindemolekül unter 11, wobei:
(a) die VH-Domäne der mindestens einen Antigenbindestelle, die Laminin-2 bindet, die Sequenz unter SEQ ID NO:442 umfasst; und
(b) die VL-Domäne der mindestens einen Antigenbindestelle, die Laminin-2 bindet, die Sequenz unter SEQ ID NO:458 umfasst; oder
(a) die VH-Domäne der mindestens einen Antigenbindestelle, die Laminin-2 bindet, die Sequenz unter SEQ ID NO:474 umfasst; und
(b) die VL-Domäne der mindestens einen Antigenbindestelle, die Laminin-2 bindet, die Sequenz unter SEQ ID NO:490 umfasst.

13. Multispezifisches Bindemolekül nach einem der Ansprüche 1-12, wobei L₁ und L₂ die Sequenz DKTHT (SEQ ID NO:534) umfassen oder wobei L₃ und L₄ die Sequenz DKTHT (SEQ ID NO:534) umfassen oder wobei L₁, L₂, L₃ und L₄ die Sequenz DKTHT (SEQ ID NO:534) umfassen.

14. Bispezifisches Bindemolekül, umfassend eine erste Bindedomäne, die einen extrazellulären Teil von Dystroglycan bindet, und eine zweite Bindedomäne, die Laminin-2 bindet,
wobei es sich bei dem bispezifischen Bindemolekül um ein bispezifisches Bindeprotein handelt, das eine oder mehrere Polypeptidketten umfasst, und
wobei das bispezifische Bindemolekül vier Polypeptidketten umfasst, die vier Antigenbindestellen bilden, wobei zwei Polypeptidketten eine Struktur umfassen, die durch die Formel
V_{L1}-L₁-V_{L2}-L₂-C_{L} [I]
dargestellt ist, und zwei Polypeptidketten eine Struktur umfassen, die durch die Formel
V_{H2}-L₃-V_{H1}-L₄-C_{H1}-hinge-C_{H2}-C_{H3} [II]
dargestellt ist, wobei:
V_{L1} für eine erste variable Immunglobulin-Leichtkettendomäne steht;
V_{L2} für eine zweite variable Immunglobulin-Leichtkettendomäne steht;
V_{H1} für eine erste variable Immunglobulin-Schwerkettendomäne steht;
V_{H2} für eine zweite variable Immunglobulin-Schwerkettendomäne steht;
C_{L} für eine konstante Immunglobulin-Leichtkettendomäne steht;
C_{H1} für eine konstante Immunglobulin-Schwerkettendomäne C_{H1} steht;
C_{H2} für eine konstante Immunglobulin-Schwerkettendomäne C_{H2} steht;
C_{H3} für eine konstante Immunglobulin-Schwerkettendomäne C_{H3} steht;
hinge für eine Immunglobulin-Scharnierregion steht, die die Domänen C_{H1} und C_{H2} verbindet; und
L₁, L₂, L₃ und L₄ für Aminosäure-Linker stehen;
wobei die Domänen V_{H1} und V_{L1} ein V_{H1}/V_{L1}-Bindepaar bilden und wobei die Domänen V_{H2} und V_{L2} ein V_{H2}/V_{L2}-Bindepaar bilden.

15. Bispezifisches Bindemolekül nach Anspruch 14, wobei L₁, L₂, L₃ und L₄ jeweils eine Länge von 0 bis 50 Aminosäureresten aufweisen, vorzugsweise wobei L₁, L₂, L₃ und L₄ jeweils eine Länge von 0 bis 25 Aminosäureresten oder jeweils eine Länge von 0 bis 14 Aminosäureresten aufweisen.

16. Bispezifisches Bindemolekül, umfassend eine erste Bindedomäne, die einen extrazellulären Teil von Dystroglycan bindet, und eine zweite Bindedomäne, die Laminin-2 bindet,
wobei es sich bei dem bispezifischen Bindemolekül um ein bispezifisches Bindeprotein handelt, das eine oder mehrere Polypeptidketten umfasst, und
wobei das bispezifische Bindemolekül zwei leichte Ketten, die eine durch die Formel
V_{L1}-L₅-V_{L2}-L₆-C_{L} [III]
dargestellte Struktur umfassen, und zwei schwere Ketten, die eine durch die Formel
V_{H1}-L₇-V_{H2}-L₈-C_{H1}-hinge-C_{H2}-C_{H3} [IV]
dargestellte Struktur umfassen, umfasst, wobei:
V_{L1} für eine erste variable Immunglobulin-Leichtkettendomäne steht;
V_{L2} für eine zweite variable Immunglobulin-Leichtkettendomäne steht;
V_{H1} für eine erste variable Immunglobulin-Schwerkettendomäne steht;
V_{H2} für eine zweite variable Immunglobulin-Schwerkettendomäne steht;
C_{L} für eine konstante Immunglobulin-Leichtkettendomäne steht;
C_{H1} für eine konstante Immunglobulin-Schwerkettendomäne C_{H1} steht;
C_{H2} für eine konstante Immunglobulin-Schwerkettendomäne C_{H2} steht;
C_{H3} für eine konstante Immunglobulin-Schwerkettendomäne C_{H3} steht;
hinge für eine Immunglobulin-Scharnierregion steht, die die Domänen C_{H1} und C_{H2} verbindet; und
L₅, L₆, L₇ und L₈ für Aminosäure-Linker stehen;
wobei die Domänen V_{H1} und V_{L1} ein V_{H1}/V_{L1}-Bindepaar bilden und wobei die Domänen V_{H2} und V_{L2} ein V_{H2}/V_{L2}-Bindepaar bilden.

17. Isoliertes Nukleinsäuremolekül, umfassend eine Nukleotidsequenz, die das multispezifische Bindemolekül nach einem der Ansprüche 1-13 oder das bispezifische Bindemolekül nach einem der Ansprüche 14-16 codiert.

18. Vektorsystem, umfassend:
(a) einen oder mehrere Vektoren, die eine erste, zweite, dritte und vierte Polypeptidkette eines multispezifischen Bindemoleküls nach Anspruch 1 codieren; oder
(b) einen oder mehrere Vektoren, die eine erste, zweite, dritte und vierte Polypeptidkette eines bispezifischen Bindemoleküls nach Anspruch 14 codieren, oder
(c) einen oder mehrere Vektoren, die zwei leichte Ketten und zwei schwere Ketten eines bispezifischen Bindemoleküls nach Anspruch 16 codieren.

19. Isolierte Wirtszelle, umfassend das Nukleinsäuremolekül nach Anspruch 17 oder umfassend das Vektorsystem nach Anspruch 18.

20. Verfahren zur Herstellung eines multispezifischen Bindemoleküls oder eines bispezifischen Bindemoleküls, wobei das Verfahren Folgendes umfasst:
a) Kultivieren einer Wirtszelle nach Anspruch 19 unter solchen Bedingungen, dass die Wirtszelle das multispezifische Bindemolekül oder das bispezifische Bindemolekül exprimiert; und
b) Isolieren des multispezifischen Bindemoleküls oder bispezifischen Bindemoleküls aus der Wirtszelle.

21. Multispezifisches Bindemolekül nach einem der Ansprüche 1-13 oder bispezifisches Bindemolekül nach einem der Ansprüche 14-16 zur Verwendung bei einem Verfahren zur Behandlung oder Vorbeugung einer alpha-Dystroglycanopathie bei einem Individuum.

22. Pharmazeutische Zusammensetzung, umfassend das multispezifische Bindemolekül nach einem der Ansprüche 1-13 oder das bispezifische Bindemolekül nach einem der Ansprüche 14-16 und einen pharmazeutisch akzeptablen Träger.

## Revendications

1. Molécule de liaison multispécifique comprenant au moins un premier domaine de liaison qui se lie à une partie extracellulaire du dystroglycane et au moins un deuxième domaine de liaison qui se lie à la laminine 2, la molécule de liaison multispécifique étant une protéine de liaison multispécifique comprenant une ou plusieurs chaînes polypeptidiques,
la protéine de liaison comprenant quatre chaînes polypeptidiques, une première chaîne polypeptidique comprenant une structure représentée par la formule :
V_{L2}-L₁-V_{L1}-L₂-C_{L} [I]
et une deuxième chaîne polypeptidique comprenant une structure représentée par la formule :
V_{H1}-L₃-V_{H2}-L₄-C_{H1}-hinge-C_{H2}-C_{H3} [II]
et une troisième chaîne polypeptidique comprenant une structure représentée par la formule :
V_{H3}-C_{H1}-hinge-C_{H2}-C_{H3} [III]
et une quatrième chaîne polypeptidique comprenant une structure représentée par la formule :
V_{L3}-C_{L} [IV]
dans laquelle :
VL1 est un premier domaine variable de chaîne légère d'immunoglobuline ;
VL2 est un deuxième domaine variable de chaîne légère d'immunoglobuline ;
VL3 est un troisième domaine variable de chaîne légère d'immunoglobuline ;
VH1 est un premier domaine variable de chaîne lourde d'immunoglobuline ;
VH2 est un deuxième domaine variable de chaîne lourde d'immunoglobuline ;
VH3 est un troisième domaine variable de chaîne lourde d'immunoglobuline ;
CL est un domaine constant de chaîne légère d'immunoglobuline ;
CH1 est un domaine constant de chaîne lourde CH1 d'immunoglobuline ;
CH2 est un domaine constant de chaîne lourde CH2 d'immunoglobuline ;
CH3 est un domaine constant de chaîne lourde CH3 d'immunoglobuline ;
hinge est une région charnière d'immunoglobuline reliant les domaines CH1 et CH2 ; et
L1, L2, L3 et L4 sont des lieurs constitués d'acides aminés ;
dans laquelle le polypeptide de formule I et le polypeptide de formule II forment une paire chaîne légère-chaîne lourde recombinée ; et
dans laquelle V_{H1} et V_{L1} forment un site de liaison à un antigène, dans laquelle V_{H2} et V_{L2} forment un site de liaison à un antigène, et dans laquelle V_{H3} et V_{L3} forment un site de liaison à un antigène pour un total de trois sites de liaison à un antigène, et dans laquelle les trois sites de liaison à un antigène comprennent au moins un site de liaison à un antigène qui se lie à la partie extracellulaire du dystroglycane et au moins un site de liaison à un antigène qui se lie à la laminine 2.

2. Molécule de liaison multispécifique selon la revendication 1, comprenant un site de liaison à un antigène qui se lie à la partie extracellulaire du dystroglycane et deux sites de liaison à un antigène qui se lient à la laminine 2, de préférence dans laquelle V_{H1} et V_{L1} forment un premier site de liaison à un antigène qui se lie à la laminine 2, V_{H2} et V_{L2} forment un deuxième site de liaison à un antigène qui se lie à la laminine 2, et V_{H3} et V_{L3} forment un troisième site de liaison à un antigène qui se lie à la partie extracellulaire du dystroglycane.

3. Molécule de liaison multispécifique selon la revendication 1, comprenant deux sites de liaison à un antigène qui se lient à la partie extracellulaire du dystroglycane et un site de liaison à un antigène qui se lie à la laminine 2, de préférence dans laquelle V_{H1} et V_{L1} forment un premier site de liaison à un antigène qui se lie à la partie extracellulaire du dystroglycane, V_{H2} et V_{L2} forment un deuxième site de liaison à un antigène qui se lie à la partie extracellulaire du dystroglycane, et V_{H3} et V_{L3} forment un troisième site de liaison à un antigène qui se lie à la laminine 2.

4. Molécule de liaison multispécifique selon l'une quelconque des revendications 1 à 3, dans laquelle :
(a) l'au moins un site de liaison à un antigène qui se lie à la partie extracellulaire du dystroglycane se lie à la partie extracellulaire du dystroglycane avec une constante de dissociation à l'équilibre (K_{D}) inférieure à environ 1 µM, analysé comme faisant partie d'une protéine de liaison multispécifique ;
(b) l'au moins un site de liaison à un antigène qui se lie à la partie extracellulaire du dystroglycane se lie aux parties extracellulaires du dystroglycane humain et murin ;
(c) l'au moins un site de liaison à un antigène qui se lie à la partie extracellulaire du dystroglycane se lie au bêta-dystroglycane ou à l'alpha-dystroglycane ;
(d) l'au moins un site de liaison à un antigène qui se lie à la laminine 2 se lie à la laminine 2 humaine ;
(e) l'au moins un site de liaison à un antigène qui se lie à la laminine 2 se lie à la laminine 2 murine et humaine ; et/ou
(f) l'au moins un site de liaison à un antigène qui se lie à la laminine 2 se lie à un polypeptide comprenant un domaine 4 de type laminine G (LG) de la laminine 2, un domaine 5 de type laminine G (LG) de la laminine 2, ou les deux.

5. Molécule de liaison multispécifique selon l'une quelconque des revendications 1 à 4,
dans laquelle l'au moins un site de liaison à un antigène qui se lie à la partie extracellulaire du dystroglycane comprend :
(a) un domaine variable de chaîne lourde (VH) comprenant une CDR-H1 comprenant une séquence d'acides aminés choisie dans le groupe constitué par les SEQ ID NO : 1 à 8, une CDR-H2 comprenant une séquence d'acides aminés choisie dans le groupe constitué par les SEQ ID NO : 9 à 17, et une CDR-H3 comprenant une séquence d'acides aminés choisie dans le groupe constitué par les SEQ ID NO : 18 à 27 ; et
(b) un domaine variable de chaîne légère (VL) comprenant une CDR-L1 comprenant une séquence d'acides aminés choisie dans le groupe constitué par les SEQ ID NO : 28 à 37, une CDR-L2 comprenant une séquence d'acides aminés choisie dans le groupe constitué par les SEQ ID NO : 38 à 42, et une CDR-L3 comprenant une séquence d'acides aminés choisie dans le groupe constitué par les SEQ ID NO : 43 à 50 ; et/ou
dans laquelle l'au moins un site de liaison à un antigène qui se lie à la laminine 2 comprend :
(a) un domaine variable de chaîne lourde (VH) comprenant une CDR-H1 comprenant une séquence d'acides aminés choisie dans le groupe constitué par les SEQ ID NO : 51 à 55 et 81 à 95, une CDR-H2 comprenant une séquence d'acides aminés choisie dans le groupe constitué par les SEQ ID NO : 56 à 60 et 96 à 110, et une CDR-H3 comprenant une séquence d'acides aminés choisie dans le groupe constitué par les SEQ ID NO : 61 à 65 et 111 à 125 ; et
(b) un domaine variable de chaîne légère (VL) comprenant une CDR-L1 comprenant une séquence d'acides aminés choisie dans le groupe constitué par les SEQ ID NO : 66 à 70 et 126 à 140, une CDR-L2 comprenant une séquence d'acides aminés choisie dans le groupe constitué par les SEQ ID NO : 38, 71 à 75 et 141 à 154, et une CDR-L3 comprenant une séquence d'acides aminés choisie dans le groupe constitué par les SEQ ID NO : 76 à 80 et 155 à 169.

6. Molécule de liaison multispécifique selon la revendication 5, dans laquelle :
(a) le domaine VH de l'au moins un site de liaison à un antigène qui se lie à la partie extracellulaire du dystroglycane comprend une séquence choisie dans le groupe constitué par les SEQ ID NO : 170, 172, 174, 176, 178, 180, 182, 184, 186 et 188 ; et
(b) le domaine VL de l'au moins un site de liaison à un antigène qui se lie à la partie extracellulaire du dystroglycane comprend une séquence choisie dans le groupe constitué par les SEQ ID NO : 171, 173, 175, 177, 179, 181, 183, 185, 187 et 189 ; et/ou
(c) le domaine VH de l'au moins un site de liaison à un antigène qui se lie à la laminine 2 comprend une séquence choisie dans le groupe constitué par les SEQ ID NO : 190, 192, 194, 196, 198, 200, 202, 204, 206, 208, 210, 212, 214, 216, 218, 220, 222, 224, 226 et 228 ; et
(d) le domaine VL de l'au moins un site de liaison à un antigène qui se lie à la laminine 2 comprend une séquence choisie dans le groupe constitué par les SEQ ID NO : 191, 193, 195, 197, 199, 201, 203, 205, 207, 209, 211, 213, 215, 217, 219, 221, 223, 225, 227 et 229.

7. Molécule de liaison multispécifique selon l'une quelconque des revendications 1 à 3, dans laquelle l'au moins un site de liaison à un antigène qui se lie à la partie extracellulaire du dystroglycane comprend :
(a) un domaine variable de chaîne lourde (VH) comprenant une CDR-H1 comprenant la séquence de SEQ ID NO : 316, une CDR-H2 comprenant la séquence de SEQ ID NO : 318 et une CDR-H3 comprenant la séquence de SEQ ID NO : 320 ; et
(b) un domaine variable de chaîne légère (VL) comprenant une CDR-L1 comprenant la séquence de SEQ ID NO : 332, une CDR-L2 comprenant la séquence de SEQ ID NO : 334 et une CDR-L3 comprenant la séquence de SEQ ID NO : 336 ; et/ou l'au moins un site de liaison à un antigène qui se lie à la laminine 2 comprend :
(a) un domaine variable de chaîne lourde (VH) comprenant une CDR-H1 comprenant la séquence de SEQ ID NO : 380, une CDR-H2 comprenant la séquence de SEQ ID NO : 382 et une CDR-H3 comprenant la séquence de SEQ ID NO : 384 ; et
(b) un domaine variable de chaîne légère (VL) comprenant une CDR-L1 comprenant la séquence de SEQ ID NO : 396, une CDR-L2 comprenant la séquence de SEQ ID NO : 398 et une CDR-L3 comprenant la séquence de SEQ ID NO : 400 ; ou l'au moins un site de liaison à un antigène qui se lie à la laminine 2 comprend :
(a) un domaine variable de chaîne lourde (VH) comprenant une CDR-H1 comprenant la séquence de SEQ ID NO : 444, une CDR-H2 comprenant la séquence de SEQ ID NO : 446 et une CDR-H3 comprenant la séquence de SEQ ID NO : 448 ; et
(b) un domaine variable de chaîne légère (VL) comprenant une CDR-L1 comprenant la séquence de SEQ ID NO : 460, une CDR-L2 comprenant la séquence de SEQ ID NO : 462 et une CDR-L3 comprenant la séquence de SEQ ID NO : 464.

8. Molécule de liaison multispécifique selon la revendication 7, dans laquelle :
(a) le domaine VH de l'au moins un site de liaison à un antigène qui se lie à la partie extracellulaire du dystroglycane comprend la séquence de SEQ ID NO : 314 ; et
(b) le domaine VL de l'au moins un site de liaison à un antigène qui se lie à la partie extracellulaire du dystroglycane comprend la séquence de SEQ ID NO : 330 ; ou
(c) le domaine VH de l'au moins un site de liaison à un antigène qui se lie à la partie extracellulaire du dystroglycane comprend la séquence de SEQ ID NO : 346 ; et
(d) le domaine VL de l'au moins un site de liaison à un antigène qui se lie à la partie extracellulaire du dystroglycane comprend la séquence de SEQ ID NO : 362.

9. Molécule de liaison multispécifique selon l'une quelconque des revendications 1 à 3 et 5 à 8, dans laquelle l'au moins un site de liaison à un antigène qui se lie la laminine 2 comprend :
(a) un domaine variable de chaîne lourde (VH) comprenant une CDR-H1 comprenant la séquence de SEQ ID NO : 380, une CDR-H2 comprenant la séquence de SEQ ID NO : 382 et une CDR-H3 comprenant la séquence de SEQ ID NO : 384 ; et
(b) un domaine variable de chaîne légère (VL) comprenant une CDR-L1 comprenant la séquence de SEQ ID NO : 396, une CDR-L2 comprenant la séquence de SEQ ID NO : 398 et une CDR-L3 comprenant la séquence de SEQ ID NO : 400, ou
(a) un domaine variable de chaîne lourde (VH) comprenant une CDR-H1 comprenant la séquence de SEQ ID NO : 380, une CDR-H2 comprenant la séquence de SEQ ID NO : 382 et une CDR-H3 comprenant la séquence de SEQ ID NO : 384 ; et
(b) un domaine variable de chaîne légère (VL) comprenant une CDR-L1 comprenant la séquence de SEQ ID NO : 428, une CDR-L2 comprenant la séquence de SEQ ID NO : 398 et une CDR-L3 comprenant la séquence de SEQ ID NO : 400.

10. Molécule de liaison multispécifique selon la revendication 9, dans laquelle :
(a) le domaine VH de l'au moins un site de liaison à un antigène qui se lie à la laminine 2 comprend la séquence de SEQ ID NO : 378 ; et
(b) le domaine VL de l'au moins un site de liaison à un antigène qui se lie à la laminine 2 comprend la séquence de SEQ ID NO : 394 ; ou
(a) le domaine VH de l'au moins un site de liaison à un antigène qui se lie à la laminine 2 comprend la séquence de SEQ ID NO : 410 ; et
(b) le domaine VL de l'au moins un site de liaison à un antigène qui se lie à la laminine 2 comprend la séquence de SEQ ID NO : 426.

11. Molécule de liaison multispécifique selon l'une quelconque des revendications 1 à 3 et 5 à 8, dans laquelle l'au moins un site de liaison à un antigène qui se lie la laminine 2 comprend :
(a) un domaine variable de chaîne lourde (VH) comprenant une CDR-H1 comprenant la séquence de SEQ ID NO : 444, une CDR-H2 comprenant la séquence de SEQ ID NO : 446 et une CDR-H3 comprenant la séquence de SEQ ID NO : 448 ; et
(b) un domaine variable de chaîne légère (VL) comprenant une CDR-L1 comprenant la séquence de SEQ ID NO : 460, une CDR-L2 comprenant la séquence de SEQ ID NO : 462 et une CDR-L3 comprenant la séquence de SEQ ID NO : 464 ; ou
(a) un domaine variable de chaîne lourde (VH) comprenant une CDR-H1 comprenant la séquence de SEQ ID NO : 444, une CDR-H2 comprenant la séquence de SEQ ID NO : 478 et une CDR-H3 comprenant la séquence de SEQ ID NO : 448 ; et
(b) un domaine variable de chaîne légère (VL) comprenant une CDR-L1 comprenant la séquence de SEQ ID NO : 460, une CDR-L2 comprenant la séquence de SEQ ID NO : 462 et une CDR-L3 comprenant la séquence de SEQ ID NO : 464.

12. Molécule de liaison multispécifique selon la revendication 11, dans laquelle :
(a) le domaine VH de l'au moins un site de liaison à un antigène qui se lie à la laminine 2 comprend la séquence de SEQ ID NO : 442 ; et
(b) le domaine VL de l'au moins un site de liaison à un antigène qui se lie à la laminine 2 comprend la séquence de SEQ ID NO : 458 ; ou
(a) le domaine VH de l'au moins un site de liaison à un antigène qui se lie à la laminine 2 comprend la séquence de SEQ ID NO : 474 ; et
(b) le domaine VL de l'au moins un site de liaison à un antigène qui se lie à la laminine 2 comprend la séquence de SEQ ID NO : 490.

13. Molécule de liaison multispécifique selon l'une quelconque des revendications 1 à 12, dans laquelle L₁ et L₂ comprennent la séquence DKTHT (SEQ ID NO : 534), ou dans laquelle L₃ et L₄ comprennent la séquence DKTHT (SEQ ID NO : 534), ou dans laquelle L₁, L₂, L₃ et L₄ comprennent la séquence DKTHT (SEQ ID NO : 534).

14. Molécule de liaison bispécifique comprenant un premier domaine de liaison qui se lie à une partie extracellulaire du dystroglycane et un deuxième domaine de liaison qui se lie à la laminine 2,
la molécule de liaison bispécifique étant une protéine de liaison bispécifique comprenant une ou plusieurs chaînes polypeptidiques, et
la molécule de liaison bispécifique comprenant quatre chaînes polypeptidiques qui forment quatre sites de liaison à un antigène, deux chaînes polypeptidiques comprenant une structure représentée par la formule :
V_{L1}-L₁-V_{L2}-L₂-C_{L} [I]
et deux chaînes polypeptidiques comprenant une structure représentée par la formule :
V_{H2}-L₃-V_{H1}-L₄-C_{H1}-hinge-C_{H2}-C_{H3} [II]
dans laquelle :
V_{L1} est un premier domaine variable de chaîne légère d'immunoglobuline ;
V_{L2} est un deuxième domaine variable de chaîne légère d'immunoglobuline ;
V_{H1} est un premier domaine variable de chaîne lourde d'immunoglobuline ;
V_{H2} est un deuxième domaine variable de chaîne lourde d'immunoglobuline ;
C_{L} est un domaine constant de chaîne légère d'immunoglobuline ;
C_{H1} est un domaine constant de chaîne lourde C_{H1} d'immunoglobuline ;
C_{H2} est un domaine constant de chaîne lourde C_{H2} d'immunoglobuline ;
C_{H3} est un domaine constant de chaîne lourde C_{H3} d'immunoglobuline ;
hinge est une région charnière d'immunoglobuline reliant les domaines C_{H1} et C_{H2} ; et
L₁, L₂, L₃ et L₄ sont des lieurs constitués d'acides aminés ;
dans laquelle les domaines V_{H1} et V_{L1} forment une paire de liaison V_{H1}/V_{L1}, et les domaines V_{H2} et V_{L2} forment une paire de liaison V_{H2}/V_{L2}.

15. Molécule de liaison bispécifique selon la revendication 14, dans laquelle L₁, L₂, L₃ et L₄ ont chacun une longueur de 0 à 50 résidus d'acide aminé, de préférence dans laquelle L₁, L₂, L₃ et L₄ ont chacun une longueur de 0 à 25 résidus d'acide aminé, ou ont chacun une longueur de 0 à 14 résidus d'acide aminé.

16. Molécule de liaison bispécifique comprenant un premier domaine de liaison qui se lie à une partie extracellulaire du dystroglycane et un deuxième domaine de liaison qui se lie à la laminine 2,
la molécule de liaison bispécifique étant une protéine de liaison bispécifique comprenant une ou plusieurs chaînes polypeptidiques, et
la molécule de liaison bispécifique comprenant deux chaînes légères comprenant une structure représentée par la formule :
V_{L1}-L₅-V_{L2}-L₆-C_{L} [III]
et deux chaînes lourdes comprenant une structure représentée par la formule :
V_{H1}-L₇-V_{H2}-L₈-C_{H1}-hinge-C_{H2}-C_{H3} [IV]
dans laquelle :
V_{L1} est un premier domaine variable de chaîne légère d'immunoglobuline ;
V_{L2} est un deuxième domaine variable de chaîne légère d'immunoglobuline ;
V_{H1} est un premier domaine variable de chaîne lourde d'immunoglobuline ;
V_{H2} est un deuxième domaine variable de chaîne lourde d'immunoglobuline ;
C_{L} est un domaine constant de chaîne légère d'immunoglobuline ;
C_{H1} est un domaine constant de chaîne lourde C_{H1} d'immunoglobuline ;
C_{H2} est un domaine constant de chaîne lourde C_{H2} d'immunoglobuline ;
C_{H3} est un domaine constant de chaîne lourde C_{H3} d'immunoglobuline ;
hinge est une région charnière d'immunoglobuline reliant les domaines C_{H1} et C_{H2} ; et
L₅, L₆, L₇ et L₈ sont des lieurs constitués d'acides aminés ;
dans laquelle les domaines V_{H1} et V_{L1} forment une paire de liaison V_{H1}/V_{L1}, et les domaines V_{H2} et V_{L2} forment une paire de liaison V_{H2}/V_{L2}.

17. Molécule d'acide nucléique isolée comprenant une séquence nucléotidique codant pour la molécule de liaison multispécifique selon l'une quelconque des revendications 1 à 13 ou pour la molécule de liaison bispécifique selon l'une quelconque des revendications 14 à 16.

18. Système de vecteur comprenant :
(a) un ou plusieurs vecteurs codant pour une première, une deuxième, une troisième et une quatrième chaîne polypeptidique d'une molécule de liaison multispécifique selon la revendication 1 ; ou
(b) un ou plusieurs vecteurs codant pour une première, une deuxième, une troisième et une quatrième chaîne polypeptidique d'une molécule de liaison bispécifique selon la revendication 14, ou
(c) un ou plusieurs vecteurs codant pour deux chaînes légères et deux chaînes lourdes d'une molécule de liaison bispécifique selon la revendication 16.

19. Cellule hôte isolée comprenant la molécule d'acide nucléique selon la revendication 17, ou comprenant le système de vecteur selon la revendication 18.

20. Procédé de production d'une molécule de liaison multispécifique ou d'une molécule de liaison bispécifique, le procédé comprenant :
a) la culture d'une cellule hôte selon la revendication 19 dans des conditions telles que la cellule hôte exprime la molécule de liaison multispécifique ou la molécule de liaison bispécifique ; et
b) l'isolement de la molécule de liaison multispécifique ou de la molécule de liaison bispécifique à partir de la cellule hôte.

21. Molécule de liaison multispécifique selon l'une quelconque des revendications 1 à 13 ou molécule de liaison bispécifique selon l'une quelconque des revendications 14 à 16, destinée à être utilisée dans un procédé de traitement ou de prévention d'une alpha-dystroglycanopathie chez un individu.

22. Composition pharmaceutique comprenant la molécule de liaison multispécifique selon l'une quelconque des revendications 1 à 13 ou la molécule de liaison bispécifique selon l'une quelconque des revendications 14 à 16 et un excipient pharmaceutiquement acceptable.
